# EUROPEAN PATENT SPECIFICATION

(11) **EP 2 748 201 B1**
(45) Date of publication and mention of the grant of the patent: **13.12.2017**
(21) Application number: 12750364.7
(22) Date of filing: 21.08.2012
(51) Int. Cl.: C07K 16/28, C07K 16/30, C07K 16/40, C07K 16/46

(54) **BISPECIFIC T CELL ACTIVATING ANTIGEN BINDING MOLECULES**
BISPEZIFISCHE ANTIGENBINDENDE MOLEKÜLE ZUR T-ZELLEN-AKTIVIERUNG
MOLÉCULES BISPÉCIFIQUES DE LIAISON À L'ANTIGÈNE ACTIVANT LES LYMPHOCYTES T

(30) Priority: 23.08.2011 EP 11178370; 16.05.2012 EP 12168192
(43) Date of publication of application: 02.07.2014
(73) Proprietor: Roche Glycart AG, 8952 Schlieren (CH)
(72) Inventor: AST, Oliver, CH-8303 Bassersdorf (CH); BRUENKER, Peter, CH-8335 Hittnau (CH); FAUTI, Tanja, CH-8049 Zuerich (CH); FREIMOSER-GRUNDSCHOBER, Anne, CH-8050 Zuerich (CH); NEUMANN, Christiane, CH-8166 Niederweningen (CH); KLEIN, Christian, CH-8906 Bonstetten (CH); MOESSNER, Ekkehard, CH-8280 Kreuzlingen (CH); UMANA, Pablo, CH-8832 Wollerau (CH)
(74) Representative: Küng, Peter
(86) International application number: PCT/EP2012/066215
(87) International publication number: WO 2013/026833

(56) References cited:
- WO-A1-2009/018386
- WO-A1-2009/080251
- WO-A1-2009/080253
- WO-A1-2009/080254
- WO-A1-2010/115551
- WO-A1-2010/115552
- WO-A1-2010/115589
- WO-A1-2010/136172
- WO-A1-2010/145792
- WO-A1-2010/145793
- WO-A2-2007/073499
- WO-A2-2007/075270
- WO-A2-2008/119567
- W. SCHAEFER ET AL: "Immunoglobulin domain crossover as a generic approach for the production of bispecific IgG antibodies", PROCEEDINGS OF THE NATIONAL ACADEMY OF SCIENCES, vol. 108, no. 27, 5 July 2011 (2011-07-05) , pages 11187-11192, XP055003817, ISSN: 0027-8424, DOI: 10.1073/pnas.1019002108
- CHAN L A ET AL: "Variable region domain exchange in human IgGs promotes antibody complex formation with accompanying structural changes and altered effector functions", MOLECULAR IMMUNOLOGY, PERGAMON, GB, vol. 41, no. 5, 1 July 2004 (2004-07-01), pages 527-538, XP002519620, ISSN: 0161-5890, DOI: 10.1016/J.MOLIMM.2004.03.034
- WOLF ET AL: "BiTEs: bispecific antibody constructs with unique anti-tumor activity", DRUG DISCOVERY TODAY, ELSEVIER, RAHWAY, NJ, US, vol. 10, no. 18, 15 September 2005 (2005-09-15), pages 1237-1244, XP005103829, ISSN: 1359-6446, DOI: 10.1016/S1359-6446(05)03554-3
- DIRK NAGORSEN ET AL: "Immunomodulatory therapy of cancer with T cell-engaging BiTE antibody blinatumomab", EXPERIMENTAL CELL RESEARCH, ACADEMIC PRESS, US, vol. 317, no. 9, 10 March 2011 (2011-03-10), pages 1255-1260, XP028205663, ISSN: 0014-4827, DOI: 10.1016/J.YEXCR.2011.03.010 [retrieved on 2011-03-16] cited in the application
- BOSCH J J ET AL: "MCSP/CD3-bispecific single-chain antibody construct engages CD4+ and CD8+ T cells for lysis of MCSP-expressing human uveal melanoma cells", INTERNET CITATION, 21 April 2010 (2010-04-21), XP002685799, Retrieved from the Internet: URL:http://www.abstractsonline.com/Plan/Vi ewAbstract.aspx?mID=2521&sKey=ef81a5fa-b15 c-4c4e-aa57-870b6479e274&cKey=a695d122-de2 0-451f-aeba-daf2b2a898f1&mKey={0591FA3B-AF EF-49D2-8E65-55F41EE8117E} [retrieved on 2012-10-23]
- OSHIMI ET AL: "Increased lysis of patient CD10-positive leukemic cells by T cells coated with anti-CD3 Fab' antibody cross-linked to anti-CD10 Fab' antibody.", BLOOD, vol. 77, no. 5, 1 March 1991 (1991-03-01), pages 1044-1049, XP055041891, ISSN: 0006-4971
- TUTT A ET AL: "TRISPECIFIC F(AB')3 DERIVATIVES THAT USE COOPERATIVE SIGNALING VIA THE TCR/CD3 COMPLEX AND CD2 TO ACTIVATE AND REDIRECT RESTING CYTOTOXIC T CELLS", THE JOURNAL OF IMMUNOLOGY, THE AMERICAN ASSOCIATION OF IMMUNOLOGISTS, US, vol. 147, no. 1, 1 July 1991 (1991-07-01), pages 60-69, XP000863699, ISSN: 0022-1767
- MILLER KATHY ET AL: "Design, construction, and in vitro analyses of multivalent antibodies", THE JOURNAL OF IMMUNOLOGY, THE AMERICAN ASSOCIATION OF IMMUNOLOGISTS, US, vol. 170, no. 9, 1 May 2003 (2003-05-01), pages 4854-4861, XP002508787, ISSN: 0022-1767
- EVAN P BOOY ET AL: "Monoclonal and bispecific antibodies as novel therapeutics", ARCHIVUM IMMUNOLOGIAE ET THERAPIAE EXPERIMENTALIS, BIRKHAEUSER-VERLAG, BASEL, CH, vol. 54, no. 2, 1 April 2006 (2006-04-01), pages 85-101, XP019200147, ISSN: 1661-4917, DOI: 10.1007/S00005-006-0011-5

### SEQUENCE LISTING

<110> Roche Glycart AG
<120> Bispecific T cell activating antigen binding molecules
<130> 30598
<150> EP 11178370.0
   <151> 2011-08-23
<150> EP 12168192.8
   <151> 2012-05-16
<160> 266
<170> PatentIn version 3.5
<210> 1
   <211> 700
   <212> PRT
   <213> Artificial Sequence
<220>
   <223> V9 (scFab)-Fc(hole) P329G LALA
<400> 1
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Ser Gly Gly Gly Ser Glu
   210 215 220
   Gly Gly Gly Ser Glu Gly Gly Gly Ser Glu Gly Gly Gly Ser Glu Gly
   225 230 235 240
   Gly Gly Ser Gly Gly Gly Ser Gly Glu Val Gln Leu Val Glu Ser Gly
   245 250 255
   Gly Gly Leu Val Gln Pro Gly Gly Ser Leu Arg Leu Ser Cys Ala Ala
   260 265 270
   Ser Gly Tyr Ser Phe Thr Gly Tyr Thr Met Asn Trp Val Arg Gln Ala
   275 280 285
   Pro Gly Lys Gly Leu Glu Trp Val Ala Leu Ile Asn Pro Tyr Lys Gly
   290 295 300
   Val Ser Thr Tyr Asn Gln Lys Phe Lys Asp Arg Phe Thr Ile Ser Val
   305 310 315 320
   Asp Lys Ser Lys Asn Thr Ala Tyr Leu Gln Met Asn Ser Leu Arg Ala
   325 330 335
   Glu Asp Thr Ala Val Tyr Tyr Cys Ala Arg Ser Gly Tyr Tyr Gly Asp
   340 345 350
   Ser Asp Trp Tyr Phe Asp Val Trp Gly Gln Gly Thr Leu Val Thr Val
   355 360 365
   Ser Ser Ala Ser Thr Lys Gly Pro Ser Val Phe Pro Leu Ala Pro Ser
   370 375 380
   Ser Lys Ser Thr Ser Gly Gly Thr Ala Ala Leu Gly Cys Leu Val Lys
   385 390 395 400
   Asp Tyr Phe Pro Glu Pro Val Thr Val Ser Trp Asn Ser Gly Ala Leu
   405 410 415
   Thr Ser Gly Val His Thr Phe Pro Ala Val Leu Gln Ser Ser Gly Leu
               420                 425                 430
   Tyr Ser Leu Ser Ser Val Val Thr Val Pro Ser Ser Ser Leu Gly Thr
           435                 440                 445
   Gln Thr Tyr Ile Cys Asn Val Asn His Lys Pro Ser Asn Thr Lys Val
       450                 455                 460
   Asp Lys Lys Val Glu Pro Lys Ser Cys Asp Lys Thr His Thr Cys Pro
   465                 470                 475                 480
   Pro Cys Pro Ala Pro Glu Ala Ala Gly Gly Pro Ser Val Phe Leu Phe
                   485                 490                 495
   Pro Pro Lys Pro Lys Asp Thr Leu Met Ile Ser Arg Thr Pro Glu Val
               500                 505                 510
   Thr Cys Val Val Val Asp Val Ser His Glu Asp Pro Glu Val Lys Phe
           515                 520                 525
   Asn Trp Tyr Val Asp Gly Val Glu Val His Asn Ala Lys Thr Lys Pro
       530                 535                 540
   Arg Glu Glu Gln Tyr Asn Ser Thr Tyr Arg Val Val Ser Val Leu Thr
   545                 550                 555                 560
   Val Leu His Gln Asp Trp Leu Asn Gly Lys Glu Tyr Lys Cys Lys Val
                   565                 570                 575
   Ser Asn Lys Ala Leu Gly Ala Pro Ile Glu Lys Thr Ile Ser Lys Ala
               580                 585                 590
   Lys Gly Gln Pro Arg Glu Pro Gln Val Cys Thr Leu Pro Pro Ser Arg
           595                 600                 605
   Asp Glu Leu Thr Lys Asn Gln Val Ser Leu Ser Cys Ala Val Lys Gly
       610                 615                 620
   Phe Tyr Pro Ser Asp Ile Ala Val Glu Trp Glu Ser Asn Gly Gln Pro
   625                 630                 635                 640
   Glu Asn Asn Tyr Lys Thr Thr Pro Pro Val Leu Asp Ser Asp Gly Ser
                   645                 650                 655
   Phe Phe Leu Val Ser Lys Leu Thr Val Asp Lys Ser Arg Trp Gln Gln
               660                 665                 670
   Gly Asn Val Phe Ser Cys Ser Val Met His Glu Ala Leu His Asn His
           675                 680                 685
   Tyr Thr Gln Lys Ser Leu Ser Leu Ser Pro Gly Lys
       690                 695                 700
<210> 2
   <211> 2103
   <212> DNA
   <213> Artificial Sequence
<220>
   <223> V9 (scFab)-Fc(hole) P329G LALA
<400> 2
   gacatccaga tgacccagag cccctctagc ctgagcgcca gcgtgggcga cagagtgacc      60
   atcacctgtc gggccagcca ggacatcaga aactacctga actggtatca gcagaagccc     120
   ggcaaggccc ccaagctgct gatctactac acctctagac tggaaagcgg cgtgcccagc     180
   cggtttagcg gcagcggctc cggcaccgac tacaccctga ccatcagcag cctgcagccc     240
   gaggacttcg ccacctacta ctgccagcag ggcaacacac tcccctggac cttcggccag     300
   ggcaccaagg tggagatcaa gcgtacggtg gccgctccca gcgtgttcat cttccccccc     360
   agcgacgagc agctgaagtc cggcaccgcc agcgtcgtgt gcctgctgaa caacttctac     420
   ccccgggagg ccaaggtgca gtggaaggtg gacaacgccc tgcagagcgg caacagccag     480
   gaaagcgtca ccgagcagga cagcaaggac tccacctaca gcctgagcag caccctgacc     540
   ctgagcaagg ccgactacga gaagcacaag gtgtacgcct gcgaagtgac ccaccagggc     600
   ctgtccagcc ccgtgaccaa gagcttcaac cggggcgagt gcagcggcgg aggctctgga     660
   ggcggctctg aaggcggagg aagtgagggc ggaggctcag aaggcggcgg aagcgaaggt     720
   ggcggctctg gcggcggatc cggcgaggtg cagctggtcg agtccggcgg aggcctggtg     780
   cagcctggcg gcagcctgag actgagctgc gccgccagcg gctacagctt caccggctac     840
   accatgaact gggtccggca ggctcctggc aagggcctcg aatgggtggc cctgatcaac     900
   ccctacaagg gcgtgagcac ctacaaccag aagttcaagg accggttcac catcagcgtg     960
   gacaagagca agaacaccgc ctatctgcag atgaacagcc tgcgggccga ggacaccgcc    1020
   gtgtactact gcgccagaag cggctactac ggcgacagcg actggtactt cgacgtgtgg    1080
   ggccagggca cactggtcac cgtgtccagc gctagcacca agggcccatc ggtcttcccc    1140
   ctggcaccct cctccaagag cacctctggg ggcacagcgg ccctgggctg cctggtcaag    1200
   gactacttcc ccgaaccggt gacggtgtcg tggaactcag gcgccctgac cagcggcgtg    1260
   cacaccttcc cggctgtcct acagtcctca ggactctact ccctcagcag cgtggtgacc    1320
   gtgccctcca gcagcttggg cacccagacc tacatctgca acgtgaatca caagcccagc    1380
   aacaccaagg tggacaagaa agttgagccc aaatcttgtg acaaaactca cacatgccca    1440
   ccgtgcccag cacctgaagc tgcaggggga ccgtcagtct tcctcttccc cccaaaaccc      1500
   aaggacaccc tcatgatctc ccggacccct gaggtcacat gcgtggtggt ggacgtgagc      1560
   cacgaagacc ctgaggtcaa gttcaactgg tacgtggacg gcgtggaggt gcataatgcc      1620
   aagacaaagc cgcgggagga gcagtacaac agcacgtacc gtgtggtcag cgtcctcacc      1680
   gtcctgcacc aggactggct gaatggcaag gagtacaagt gcaaggtctc caacaaagcc      1740
   ctcggcgccc ccatcgagaa aaccatctcc aaagccaaag ggcagccccg agaaccacag      1800
   gtgtgcaccc tgcccccatc ccgggatgag ctgaccaaga accaggtcag cctctcgtgc      1860
   gcagtcaaag gcttctatcc cagcgacatc gccgtggagt gggagagcaa tgggcagccg      1920
   gagaacaact acaagaccac gcctcccgtg ctggactccg acggctcctt cttcctcgtg      1980
   agcaagctca ccgtggacaa gagcaggtgg cagcagggga acgtcttctc atgctccgtg      2040
   atgcatgagg ctctgcacaa ccactacacg cagaagagcc tctccctgtc tccgggtaaa      2100
   tga                                                                     2103
<210> 3
   <211> 442
   <212> PRT
   <213> Artificial Sequence
<220>
   <223> LC007 (VH-CH1)-Fc(knob) P329G LALA
<400> 3
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Gly Ala Pro Ile Glu Lys Thr Ile Ser Lys Ala Lys Gly
                   325                 330                 335
   Gln Pro Arg Glu Pro Gln Val Tyr Thr Leu Pro Pro Cys Arg Asp Glu
               340                 345                 350
   Leu Thr Lys Asn Gln Val Ser Leu Trp Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 4
   <211> 1329
   <212> DNA
   <213> Artificial Sequence
<220>
   <223> LC007 (VH-CH1)-Fc(knob) P329G LALA
<400> 4
   gaggtccagc tgcaggagtc aggacctggc ctcgtgaaac cttctcagtc tctgtctctc        60
   acctgctctg tcactggcta ctccatcacc agtggttatt actggaactg gatccggcag       120
   tttccaggaa acaagctgga atggatgggc tacataacct acgacggtag caataactac       180
   aacccatctc tcaaaaatcg aatctccatc actcgtgaca catctaagaa ccagtttttc       240
   ctgaagttga attctgtgac tactgaggac acagctacat attactgtgc ggactttgac       300
   tactggggcc aaggcaccac tctcacagtc tcctcagcta gcaccaaggg cccatcggtc       360
   ttccccctgg caccctcctc caagagcacc tctgggggca cagcggccct gggctgcctg       420
   gtcaaggact acttccccga accggtgacg gtgtcgtgga actcaggcgc cctgaccagc       480
   ggcgtgcaca ccttcccggc tgtcctacag tcctcaggac tctactccct cagcagcgtg       540
   gtgaccgtgc cctccagcag cttgggcacc cagacctaca tctgcaacgt gaatcacaag       600
   cccagcaaca ccaaggtgga caagaaagtt gagcccaaat cttgtgacaa gacccacacc       660
   tgtccccctt gccctgcccc tgaagctgct ggtggccctt ccgtgttcct gttcccccca       720
   aagcccaagg acaccctgat gatcagccgg acccccgaag tgacctgcgt ggtggtcgat       780
   gtgtcccacg aggaccctga agtgaagttc aattggtacg tggacggcgt ggaagtgcac       840
   aatgccaaga ccaagccgcg ggaggagcag tacaacagca cgtaccgtgt ggtcagcgtc       900
   ctcaccgtcc tgcaccagga ctggctgaat ggcaaggagt acaagtgcaa ggtctccaac       960
   aaagccctcg gcgcccccat cgagaaaacc atctccaaag ccaaagggca gccccgagaa      1020
   ccacaggtgt acaccctgcc cccatgccgg gatgagctga ccaagaacca ggtcagcctg      1080
   tggtgcctgg tcaaaggctt ctatcccagc gacatcgccg tggagtggga gagcaatggg      1140
   cagccggaga acaactacaa gaccacgcct cccgtgctgg actccgacgg ctccttcttc      1200
   ctctacagca agctcaccgt ggacaagagc aggtggcagc aggggaacgt cttctcatgc      1260
   tccgtgatgc atgaggctct gcacaaccac tacacgcaga agagcctctc cctgtctccg      1320
   ggtaaataa                                                              1329
<210> 5
   <211> 214
   <212> PRT
   <213> Artificial Sequence
<220>
   <223> LC007 (VL-CL)
<400> 5
   Asp Ile Val Leu Thr Gln Ser Pro Ser Ser Leu Ser Ala Ser Leu Gly
   1               5                   10                  15
   Asp Arg Val Thr Ile Ser Cys Ser Ala Ser Gln Gly Ile Arg Asn Tyr
               20                  25                  30
   Leu Asn Trp Tyr Gln Gln Arg Pro Asp Gly Thr Val Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Lys Leu Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 6
   <211> 645
   <212> DNA
   <213> Artificial Sequence
<220>
   <223> LC007 (VL-CL)
<400> 6
   gatattgtgc tcacacagtc tccatcctcc ctgtctgcct ctctgggaga cagagtcacc      60
   atcagttgca gtgcaagtca gggcattaga aattatttaa actggtatca gcagagacca     120
   gatggaactg ttaaactcct gatctattac acatcaagtt tacactcagg agtcccatca     180
   aggttcagtg gcagtgggtc tgggacagat tattctctca ccatcagcaa cctggaacct     240
   gaagatattg ccacttacta ttgtcagcag tatagtaagc ttccttggac gttcggtgga     300
   ggcaccaagc tggaaatcaa acgtacggtg gctgcaccat ctgtcttcat cttcccgcca     360
   tctgatgagc agttgaaatc tggaactgcc tctgttgtgt gcctgctgaa taacttctat     420
   cccagagagg ccaaagtaca gtggaaggtg gataacgccc tccaatcggg taactcccag     480
   gagagtgtca cagagcagga cagcaaggac agcacctaca gcctcagcag caccctgacg     540
   ctgagcaaag cagactacga gaaacacaaa gtctacgcct gcgaagtcac ccatcagggc     600
   ctgagctcgc ccgtcacaaa gagcttcaac aggggagagt gttag                     645
<210> 7
   <211> 704
   <212> PRT
   <213> Artificial Sequence
<220>
   <223> LC007 (scFab)-Fc(hole) P329G LALA
<400> 7
   Asp Ile Val Leu Thr Gln Ser Pro Ser Ser Leu Ser Ala Ser Leu Gly
   1               5                   10                  15
   Asp Arg Val Thr Ile Ser Cys Ser Ala Ser Gln Gly Ile Arg Asn Tyr
               20                  25                  30
   Leu Asn Trp Tyr Gln Gln Arg Pro Asp Gly Thr Val Lys Leu Leu Ile
           35                  40                  45
   Tyr Tyr Thr Ser Ser Leu His Ser Gly Val Pro Ser Arg Phe Ser Gly
   50 55 60
   Ser Gly Ser Gly Thr Asp Tyr Ser Leu Thr Ile Ser Asn Leu Glu Pro
   65 70 75 80
   Glu Asp Ile Ala Thr Tyr Tyr Cys Gln Gln Tyr Ser Lys Leu Pro Trp
   85 90 95
   Thr Phe Gly Gly Gly Thr Lys Leu Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Ser Gly Gly Gly Ser Glu
   210 215 220
   Gly Gly Gly Ser Glu Gly Gly Gly Ser Glu Gly Gly Gly Ser Glu Gly
   225 230 235 240
   Gly Gly Ser Gly Gly Gly Ser Gly Glu Val Gln Leu Gln Glu Ser Gly
   245 250 255
   Pro Gly Leu Val Lys Pro Ser Gln Ser Leu Ser Leu Thr Cys Ser Val
   260 265 270
   Thr Gly Tyr Ser Ile Thr Ser Gly Tyr Tyr Trp Asn Trp Ile Arg Gln
   275 280 285
   Phe Pro Gly Asn Lys Leu Glu Trp Met Gly Tyr Ile Thr Tyr Asp Gly
   290 295 300
   Ser Asn Asn Tyr Asn Pro Ser Leu Lys Asn Arg Ile Ser Ile Thr Arg
   305 310 315 320
   Asp Thr Ser Lys Asn Gln Phe Phe Leu Lys Leu Asn Ser Val Thr Thr
   325 330 335
   Glu Asp Thr Ala Thr Tyr Tyr Cys Ala Asp Phe Asp Tyr Trp Gly Gln
   340 345 350
   Gly Thr Thr Leu Thr Val Ser Ser Ala Ser Thr Lys Gly Pro Ser Val
   355 360 365
   Phe Pro Leu Ala Pro Ser Ser Lys Ser Thr Ser Gly Gly Thr Ala Ala
   370 375 380
   Leu Gly Cys Leu Val Lys Asp Tyr Phe Pro Glu Pro Val Thr Val Ser
   385 390 395 400
   Trp Asn Ser Gly Ala Leu Thr Ser Gly Val His Thr Phe Pro Ala Val
   405 410 415
   Leu Gln Ser Ser Gly Leu Tyr Ser Leu Ser Ser Val Val Thr Val Pro
   420 425 430
   Ser Ser Ser Leu Gly Thr Gln Thr Tyr Ile Cys Asn Val Asn His Lys
   435 440 445
   Pro Ser Asn Thr Lys Val Asp Lys Lys Val Glu Pro Lys Ser Cys Asp
   450 455 460
   Gly Gly Gly Gly Ser Gly Gly Gly Gly Ser Gly Ala Gln Asp Lys Thr
   465 470 475 480
   His Thr Cys Pro Pro Cys Pro Ala Pro Glu Ala Ala Gly Gly Pro Ser
   485 490 495
   Val Phe Leu Phe Pro Pro Lys Pro Lys Asp Thr Leu Met Ile Ser Arg
   500 505 510
   Thr Pro Glu Val Thr Cys Val Val Val Asp Val Ser His Glu Asp Pro
   515 520 525
   Glu Val Lys Phe Asn Trp Tyr Val Asp Gly Val Glu Val His Asn Ala
   530 535 540
   Lys Thr Lys Pro Arg Glu Glu Gln Tyr Asn Ser Thr Tyr Arg Val Val
   545 550 555 560
   Ser Val Leu Thr Val Leu His Gln Asp Trp Leu Asn Gly Lys Glu Tyr
   565 570 575
   Lys Cys Lys Val Ser Asn Lys Ala Leu Pro Ala Pro Ile Glu Lys Thr
               580                 585                 590
   Ile Ser Lys Ala Lys Gly Gln Pro Arg Glu Pro Gln Val Cys Thr Leu
           595                 600                 605
   Pro Pro Ser Arg Asp Glu Leu Thr Lys Asn Gln Val Ser Leu Ser Cys
       610                 615                 620
   Ala Val Lys Gly Phe Tyr Pro Ser Asp Ile Ala Val Glu Trp Glu Ser
   625                 630                 635                 640
   Asn Gly Gln Pro Glu Asn Asn Tyr Lys Thr Thr Pro Pro Val Leu Asp
                   645                 650                 655
   Ser Asp Gly Ser Phe Phe Leu Val Ser Lys Leu Thr Val Asp Lys Ser
               660                 665                 670
   Arg Trp Gln Gln Gly Asn Val Phe Ser Cys Ser Val Met His Glu Ala
           675                 680                 685
   Leu His Asn His Tyr Thr Gln Lys Ser Leu Ser Leu Ser Pro Gly Lys
       690                 695                 700
<210> 8
   <211> 2115
   <212> DNA
   <213> Artificial Sequence
<220>
   <223> LC007 (scFab)-Fc(hole) P329G LALA
<400> 8
   gacatcgtgc tgacccagag ccctagcagc ctgagcgcca gcctgggcga cagagtgacc 60
   atcagctgta gcgcctccca gggcatcaga aactacctga actggtatca gcagagaccc 120
   gacggcacag tgaagctgct gatctactac accagcagcc tgcacagcgg cgtgccaagc 180
   agattcagcg gcagcggctc cggcacagac tacagcctga ccatctccaa cctggaaccc 240
   gaggatatcg ccacctacta ctgccagcag tacagcaagc tgccctggac cttcggcgga 300
   ggcaccaagc tggaaatcaa gcggaccgtg gccgctccca gcgtgttcat cttcccaccc 360
   agcgacgagc agctgaagtc cggcacagcc agcgtcgtgt gcctgctgaa caacttctac 420
   ccccgggagg ccaaggtgca gtggaaggtg gacaacgccc tgcagagcgg caacagccag 480
   gaaagcgtca ccgagcagga cagcaaggac tccacctaca gcctgtccag caccctgacc 540
   ctgagcaagg ccgactacga gaagcacaag gtgtacgcct gcgaagtgac ccaccagggc 600
   ctgagcagcc ccgtgaccaa gagcttcaac cggggcgagt gtagtggcgg aggctctggc 660
   ggaggaagcg agggcggagg atctgaaggc ggcggatctg aggggggagg cagtgaaggg 720
   ggaggctcag ggggaggatc cggcgaggtg cagctgcagg aatctggccc tggcctggtc 780
   aagccaagcc agagtctgag cctgacctgc agcgtgaccg gctacagcat taccagcggc 840
   tactactgga actggattcg gcagttcccc ggcaataagc tggaatggat gggctacatc 900
   acctacgacg gcagcaacaa ctacaacccc agcctgaaga accggatcag catcacccgg 960
   gacaccagca agaaccagtt cttcctgaag ctgaacagcg tgaccaccga ggacaccgcc 1020
   acatactatt gcgccgactt cgactactgg ggccagggca ccaccctgac cgtgtccagc 1080
   gccagcacaa agggccctag cgtgttccct ctggccccca gcagcaagag cacaagcggc 1140
   ggaacagccg ccctgggctg cctcgtgaag gactacttcc ccgagcccgt gacagtgtct 1200
   tggaacagcg gagccctgac aagcggcgtg cacaccttcc ctgccgtgct gcagagcagc 1260
   ggcctgtact ccctgagcag cgtggtcacc gtgcctagca gcagcctggg cacccagacc 1320
   tacatctgca acgtgaacca caagcccagc aacaccaaag tggacaagaa ggtggagccc 1380
   aagagctgtg atggcggagg agggtccgga ggcggtggat ccggagctca ggacaaaact 1440
   cacacatgcc caccgtgccc agcacctgaa gctgcagggg gaccgtcagt cttcctcttc 1500
   cccccaaaac ccaaggacac cctcatgatc tcccggaccc ctgaggtcac atgcgtggtg 1560
   gtggacgtga gccacgaaga ccctgaggtc aagttcaact ggtacgtgga cggcgtggag 1620
   gtgcataatg ccaagacaaa gccgcgggag gagcagtaca acagcacgta ccgtgtggtc 1680
   agcgtcctca ccgtcctgca ccaggactgg ctgaatggca aggagtacaa gtgcaaggtc 1740
   tccaacaaag ccctcccagc ccccatcgag aaaaccatct ccaaagccaa agggcagccc 1800
   cgagaaccac aggtgtgcac cctgccccca tcccgggatg agctgaccaa gaaccaggtc 1860
   agcctctcgt gcgcagtcaa aggcttctat cccagcgaca tcgccgtgga gtgggagagc 1920
   aatgggcagc cggagaacaa ctacaagacc acgcctcccg tgctggactc cgacggctcc 1980
   ttcttcctcg tgagcaagct caccgtggac aagagcaggt ggcagcaggg gaacgtcttc 2040
   tcatgctccg tgatgcatga ggctctgcac aaccactaca cgcagaagag cctctccctg 2100
   tctccgggta aatga 2115
<210> 9
   <211> 466
   <212> PRT
   <213> Artificial Sequence
<220>
   <223> V9 (VH-CH1) -Fc(knob) LALA
<400> 9
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Gln Gly Thr Leu Val Thr Val Ser Ser Ala Ser Thr Lys Gly Pro
           115                 120                 125
   Ser Val Phe Pro Leu Ala Pro Ser Ser Lys Ser Thr Ser Gly Gly Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Gln Thr Tyr Ile Cys Asn Val Asn
           195                 200                 205
   His Lys Pro Ser Asn Thr Lys Val Asp Lys Lys Val Glu Pro Lys Ser
       210                 215                 220
   Cys Asp Gly Gly Gly Gly Ser Gly Gly Gly Gly Ser Gly Ala Gln Asp
   225                 230                 235                 240
   Lys Thr His Thr Cys Pro Pro Cys Pro Ala Pro Glu Ala Ala Gly Gly
                   245                 250                 255
   Pro Ser Val Phe Leu Phe Pro Pro Lys Pro Lys Asp Thr Leu Met Ile
               260                 265                 270
   Ser Arg Thr Pro Glu Val Thr Cys Val Val Val Asp Val Ser His Glu
           275                 280                 285
   Asp Pro Glu Val Lys Phe Asn Trp Tyr Val Asp Gly Val Glu Val His
   290 295 300
   Asn Ala Lys Thr Lys Pro Arg Glu Glu Gln Tyr Asn Ser Thr Tyr Arg
   305 310 315 320
   Val Val Ser Val Leu Thr Val Leu His Gln Asp Trp Leu Asn Gly Lys
   325 330 335
   Glu Tyr Lys Cys Lys Val Ser Asn Lys Ala Leu Pro Ala Pro Ile Glu
   340 345 350
   Lys Thr Ile Ser Lys Ala Lys Gly Gln Pro Arg Glu Pro Gln Val Tyr
   355 360 365
   Thr Leu Pro Pro Cys Arg Asp Glu Leu Thr Lys Asn Gln Val Ser Leu
   370 375 380
   Trp Cys Leu Val Lys Gly Phe Tyr Pro Ser Asp Ile Ala Val Glu Trp
   385 390 395 400
   Glu Ser Asn Gly Gln Pro Glu Asn Asn Tyr Lys Thr Thr Pro Pro Val
   405 410 415
   Leu Asp Ser Asp Gly Ser Phe Phe Leu Tyr Ser Lys Leu Thr Val Asp
   420 425 430
   Lys Ser Arg Trp Gln Gln Gly Asn Val Phe Ser Cys Ser Val Met His
   435 440 445
   Glu Ala Leu His Asn His Tyr Thr Gln Lys Ser Leu Ser Leu Ser Pro
   450 455 460
   Gly Lys
   465
<210> 10
   <211> 1401
   <212> DNA
   <213> Artificial Sequence
<220>
   <223> V9 (VH-CH1) -Fc(knob) LALA
<400> 10
   gaggtgcagc tggtcgagag cggaggcggc ctggtgcagc ctggcggcag cctgagactg      60
   agctgcgccg ccagcggcta cagcttcacc ggctacacca tgaactgggt ccggcaggca     120
   cctggcaagg gactggaatg ggtggccctg atcaacccct acaagggcgt gagcacctac     180
   aaccagaagt tcaaggaccg gttcaccatc agcgtggaca agagcaagaa caccgcctat     240
   ctgcagatga acagcctgcg ggccgaggac accgccgtgt actactgcgc cagaagcggc     300
   tactacggcg acagcgactg gtacttcgac gtgtggggcc agggcaccct cgtgaccgtg     360
   tctagcgcta gcaccaaggg cccctccgtg ttccccctgg cccccagcag caagagcacc     420
   agcggcggca cagccgctct gggctgcctg gtcaaggact acttccccga gcccgtgacc     480
   gtgtcctgga acagcggagc cctgacctcc ggcgtgcaca ccttccccgc cgtgctgcag     540
   agttctggcc tgtatagcct gagcagcgtg gtcaccgtgc cttctagcag cctgggcacc     600
   cagacctaca tctgcaacgt gaaccacaag cccagcaaca ccaaggtgga caagaaggtg     660
   gagcccaaga gctgcgacgg cggtggtggc tccggaggcg gtggatccgg agctcaggac     720
   aaaactcaca catgcccacc gtgcccagca cctgaagctg cagggggacc gtcagtcttc    780
   ctcttccccc caaaacccaa ggacaccctc atgatctccc ggacccctga ggtcacatgc     840
   gtggtggtgg acgtgagcca cgaagaccct gaggtcaagt tcaactggta cgtggacggc     900
   gtggaggtgc ataatgccaa gacaaagccg cgggaggagc agtacaacag cacgtaccgt     960
   gtggtcagcg tcctcaccgt cctgcaccag gactggctga atggcaagga gtacaagtgc    1020
   aaggtctcca acaaagccct cccagccccc atcgagaaaa ccatctccaa agccaaaggg    1080
   cagccccgag aaccacaggt gtacaccctg cccccatgcc gggatgagct gaccaagaac    1140
   caggtcagcc tgtggtgcct ggtcaaaggc ttctatccca gcgacatcgc cgtggagtgg    1200
   gagagcaatg ggcagccgga gaacaactac aagaccacgc ctcccgtgct ggactccgac    1260
   ggctccttct tcctctacag caagctcacc gtggacaaga gcaggtggca gcaggggaac    1320
   gtcttctcat gctccgtgat gcatgaggct ctgcacaacc actacacgca gaagagcctc    1380
   tccctgtctc cgggtaaatg a                                              1401
<210> 11
   <211> 214
   <212> PRT
   <213> Artificial Sequence
<220>
   <223> V9 (VL-CL)
<400> 11
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 12
   <211> 645
   <212> DNA
   <213> Artificial Sequence
<220>
   <223> V9 (VL-CL)
<400> 12
   gacatccaga tgacccagag ccccagcagc ctgagcgcca gcgtgggcga cagagtgacc      60
   atcacctgtc gggccagcca ggacatcaga aactacctga actggtatca gcagaagccc     120
   ggcaaggccc ccaagctgct gatctactac acctctagac tggaaagcgg cgtgcccagc     180
   cggtttagcg gcagcggctc cggcaccgac tacaccctga ccatcagcag cctgcagccc     240
   gaggacttcg ccacctacta ctgccagcag ggcaacacac tgccctggac cttcggccag     300
   ggcacaaagg tggagatcaa gcgtacggtg gctgcaccat ctgtcttcat cttcccgcca     360
   tctgatgagc agttgaaatc tggaactgcc tctgttgtgt gcctgctgaa taacttctat     420
   cccagagagg ccaaagtaca gtggaaggtg gataacgccc tccaatcggg taactcccag     480
   gagagtgtca cagagcagga cagcaaggac agcacctaca gcctcagcag caccctgacg     540
   ctgagcaaag cagactacga gaaacacaaa gtctacgcct gcgaagtcac ccatcagggc     600
   ctgagctcgc ccgtcacaaa gagcttcaac aggggagagt gttag                     645
<210> 13
   <211> 926
   <212> PRT
   <213> Artificial Sequence
<220>
   <223> V9 (scFab)-LC007 (VH-CH1)-Fc(knob) wt
<400> 13
<210> 14
   <211> 2781
   <212> DNA
   <213> Artificial Sequence
<220>
   <223> V9 (scFab)-LC007 (VH-CH1)-Fc(knob) wt
<400> 14
   gacatccaga tgacccagag cccctctagc ctgagcgcca gcgtgggcga cagagtgacc 60
   atcacctgtc gggccagcca ggacatcaga aactacctga actggtatca gcagaagccc 120
   ggcaaggccc ccaagctgct gatctactac acctctagac tggaaagcgg cgtgcccagc 180
   cggtttagcg gcagcggctc cggcaccgac tacaccctga ccatcagcag cctgcagccc 240
   gaggacttcg ccacctacta ctgccagcag ggcaacacac tcccctggac cttcggccag 300
   ggcaccaagg tggagatcaa gcgtacggtg gccgctccca gcgtgttcat cttccccccc 360
   agcgacgagc agctgaagtc cggcaccgcc agcgtcgtgt gcctgctgaa caacttctac 420
   ccccgggagg ccaaggtgca gtggaaggtg gacaacgccc tgcagagcgg caacagccag 480
   gaaagcgtca ccgagcagga cagcaaggac tccacctaca gcctgagcag caccctgacc 540
   ctgagcaagg ccgactacga gaagcacaag gtgtacgcct gcgaagtgac ccaccagggc 600
   ctgtccagcc ccgtgaccaa gagcttcaac cggggcgagt gcagcggcgg aggctctgga 660
   ggcggctctg aaggcggagg aagtgagggc ggaggctcag aaggcggcgg aagcgaaggt 720
   ggcggctctg gcggcggatc cggcgaggtg cagctggtcg agtccggcgg aggcctggtg 780
   cagcctggcg gcagcctgag actgagctgc gccgccagcg gctacagctt caccggctac 840
   accatgaact gggtccggca ggctcctggc aagggcctcg aatgggtggc cctgatcaac 900
   ccctacaagg gcgtgagcac ctacaaccag aagttcaagg accggttcac catcagcgtg 960
   gacaagagca agaacaccgc ctatctgcag atgaacagcc tgcgggccga ggacaccgcc 1020
   gtgtactact gcgccagaag cggctactac ggcgacagcg actggtactt cgacgtgtgg 1080
   ggccagggca cactggtcac cgtgtccagc gctagcacca agggcccctc cgtgttcccc 1140
   ctggccccca gcagcaagag caccagcggc ggcacagccg ccctcggctg cctggtcaag 1200
   gactacttcc ccgagcccgt gaccgtgtcc tggaacagcg gagccctgac ctccggcgtg 1260
   cacaccttcc ccgccgtgct gcagagcagc ggcctgtaca gcctgtccag cgtggtcacc 1320
   gtgccctcca gcagcctggg cacccagacc tacatctgca acgtgaacca caagcccagc 1380
   aataccaagg tggacaagaa ggtggagccc aagagctgcg acggcggtgg tggctccgga 1440
   ggcggtggat ctgaagtgca gctgcaggaa agcggccctg gcctggtcaa gcccagccag 1500
   agcctgagcc tgacctgtag cgtgaccggc tactccatca cctccggcta ctactggaat 1560
   tggattcggc agttccccgg caacaagctg gaatggatgg gctacatcac ctacgacggc 1620
   agcaacaact acaaccccag cctgaagaac cggatcagca tcacccggga caccagcaag 1680
   aaccagttct tcctgaagtt gaattctgtg actactgagg acacagctac atattactgt 1740
   gcggactttg actactgggg ccaaggcacc actctcacag tctcctcagc tagcaccaag 1800
   ggcccatcgg tcttccccct ggcaccctcc tccaagagca cctctggggg cacagcggcc 1860
   ctgggctgcc tggtcaagga ctacttcccc gaaccggtga cggtgtcgtg gaactcaggc 1920
   gccctgacca gcggcgtgca caccttcccg gctgtcctac agtcctcagg actctactcc 1980
   ctcagcagcg tggtgaccgt gccctccagc agcttgggca cccagaccta catctgcaac 2040
   gtgaatcaca agcccagcaa caccaaggtg gacaagaaag ttgagcccaa atcttgtgac 2100
   aaaactcaca catgcccacc gtgcccagca cctgaactcc tggggggacc gtcagtcttc 2160
   ctcttccccc caaaacccaa ggacaccctc atgatctccc ggacccctga ggtcacatgc 2220
   gtggtggtgg acgtgagcca cgaagaccct gaggtcaagt tcaactggta cgtggacggc 2280
   gtggaggtgc ataatgccaa gacaaagccg cgggaggagc agtacaacag cacgtaccgt 2340
   gtggtcagcg tcctcaccgt cctgcaccag gactggctga atggcaagga gtacaagtgc 2400
   aaggtctcca acaaagccct cccagccccc atcgagaaaa ccatctccaa agccaaaggg 2460
   cagccccgag aaccacaggt gtacaccctg cccccatgcc gggatgagct gaccaagaac 2520
   caggtcagcc tgtggtgcct ggtcaaaggc ttctatccca gcgacatcgc cgtggagtgg 2580
   gagagcaatg ggcagccgga gaacaactac aagaccacgc ctcccgtgct ggactccgac 2640
   ggctccttct tcctctacag caagctcacc gtggacaaga gcaggtggca gcaggggaac 2700
   gtcttctcat gctccgtgat gcatgaggct ctgcacaacc actacacgca gaagagcctc 2760
   tccctgtctc cgggtaaatg a 2781
<210> 15
   <211> 442
   <212> PRT
   <213> Artificial Sequence
<220>
   <223> LC007 (VH-CH1)-Fc(hole) wt
<400> 15
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Leu Leu Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Cys Thr Leu Pro Pro Ser Arg Asp Glu
               340                 345                 350
   Leu Thr Lys Asn Gln Val Ser Leu Ser Cys Ala Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Val Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440
<210> 16
   <211> 1329
   <212> DNA
   <213> Artificial Sequence
<220>
   <223> LC007 (VH-CH1)-Fc(hole) wt
<400> 16
   gaggtccagc tgcaggagtc aggacctggc ctcgtgaaac cttctcagtc tctgtctctc      60
   acctgctctg tcactggcta ctccatcacc agtggttatt actggaactg gatccggcag     120
   tttccaggaa acaagctgga atggatgggc tacataacct acgacggtag caataactac     180
   aacccatctc tcaaaaatcg aatctccatc actcgtgaca catctaagaa ccagtttttc     240
   ctgaagttga attctgtgac tactgaggac acagctacat attactgtgc ggactttgac     300
   tactggggcc aaggcaccac tctcacagtc tcctcagcta gcaccaaggg cccaagcgtg     360
   ttccctctgg cccccagcag caagagcaca agcggcggaa cagccgccct gggctgcctg     420
   gtcaaggact acttccccga gcccgtgaca gtgtcctgga acagcggagc cctgaccagc     480
   ggcgtgcaca cctttccagc cgtgctgcag agcagcggcc tgtacagcct gagcagcgtg     540
   gtcacagtgc ctagcagcag cctgggcacc cagacctaca tctgcaacgt gaaccacaag     600
   cccagcaaca ccaaggtgga caagaaggtg gagcccaaga gctgcgacaa gacccacacc     660
   tgtccccctt gtcctgcccc tgagctgctg ggcggaccca gcgtgttcct gttcccccca     720
   aagcccaagg acaccctgat gatcagccgg acccccgaag tgacctgcgt ggtggtggac     780
   gtgtcccacg aggaccctga agtgaagttc aattggtacg tggacggcgt ggaggtgcac     840
   aatgccaaga ccaagccccg ggaggaacag tacaacagca cctaccgggt ggtgtccgtg     900
   ctgaccgtgc tgcaccagga ctggctgaac ggcaaagagt acaagtgcaa ggtctccaac     960
   aaggccctgc ctgcccccat cgagaaaacc atcagcaagg ccaagggcca gcccagagaa    1020
   ccccaggtgt gcaccctgcc ccccagcaga gatgagctga ccaagaacca ggtgtccctg    1080
   agctgtgccg tcaagggctt ctaccccagc gatatcgccg tggagtggga gagcaacggc    1140
   cagcctgaga acaactacaa gaccaccccc cctgtgctgg acagcgacgg cagcttcttc    1200
   ctggtgtcca aactgaccgt ggacaagagc cggtggcagc agggcaacgt gttcagctgc    1260
   agcgtgatgc acgaggccct gcacaaccac tacacccaga agtccctgag cctgagcccc    1320
   ggcaagtga                                                            1329
<210> 17
   <211> 926
   <212> PRT
   <213> Artificial Sequence
<220>
   <223> V9 (scFab)-LC007 (VH-CH1)-Fc(knob) LALA
<400> 17
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Ser Gly Gly Gly Ser Glu
   210 215 220
   Gly Gly Gly Ser Glu Gly Gly Gly Ser Glu Gly Gly Gly Ser Glu Gly
   225 230 235 240
   Gly Gly Ser Gly Gly Gly Ser Gly Glu Val Gln Leu Val Glu Ser Gly
   245 250 255
   Gly Gly Leu Val Gln Pro Gly Gly Ser Leu Arg Leu Ser Cys Ala Ala
   260 265 270
   Ser Gly Tyr Ser Phe Thr Gly Tyr Thr Met Asn Trp Val Arg Gln Ala
   275 280 285
   Pro Gly Lys Gly Leu Glu Trp Val Ala Leu Ile Asn Pro Tyr Lys Gly
   290 295 300
   Val Ser Thr Tyr Asn Gln Lys Phe Lys Asp Arg Phe Thr Ile Ser Val
   305 310 315 320
   Asp Lys Ser Lys Asn Thr Ala Tyr Leu Gln Met Asn Ser Leu Arg Ala
   325 330 335
   Glu Asp Thr Ala Val Tyr Tyr Cys Ala Arg Ser Gly Tyr Tyr Gly Asp
   340 345 350
   Ser Asp Trp Tyr Phe Asp Val Trp Gly Gln Gly Thr Leu Val Thr Val
   355 360 365
   Ser Ser Ala Ser Thr Lys Gly Pro Ser Val Phe Pro Leu Ala Pro Ser
   370 375 380
   Ser Lys Ser Thr Ser Gly Gly Thr Ala Ala Leu Gly Cys Leu Val Lys
   385 390 395 400
   Asp Tyr Phe Pro Glu Pro Val Thr Val Ser Trp Asn Ser Gly Ala Leu
   405 410 415
   Thr Ser Gly Val His Thr Phe Pro Ala Val Leu Gln Ser Ser Gly Leu
   420 425 430
   Tyr Ser Leu Ser Ser Val Val Thr Val Pro Ser Ser Ser Leu Gly Thr
   435 440 445
   Gln Thr Tyr Ile Cys Asn Val Asn His Lys Pro Ser Asn Thr Lys Val
   450 455 460
   Asp Lys Lys Val Glu Pro Lys Ser Cys Asp Gly Gly Gly Gly Ser Gly
   465 470 475 480
   Gly Gly Gly Ser Glu Val Gln Leu Gln Glu Ser Gly Pro Gly Leu Val
   485 490 495
   Lys Pro Ser Gln Ser Leu Ser Leu Thr Cys Ser Val Thr Gly Tyr Ser
   500 505 510
   Ile Thr Ser Gly Tyr Tyr Trp Asn Trp Ile Arg Gln Phe Pro Gly Asn
   515 520 525
   Lys Leu Glu Trp Met Gly Tyr Ile Thr Tyr Asp Gly Ser Asn Asn Tyr
   530 535 540
   Asn Pro Ser Leu Lys Asn Arg Ile Ser Ile Thr Arg Asp Thr Ser Lys
   545 550 555 560
   Asn Gln Phe Phe Leu Lys Leu Asn Ser Val Thr Thr Glu Asp Thr Ala
   565 570 575
   Thr Tyr Tyr Cys Ala Asp Phe Asp Tyr Trp Gly Gln Gly Thr Thr Leu
   580 585 590
   Thr Val Ser Ser Ala Ser Thr Lys Gly Pro Ser Val Phe Pro Leu Ala
   595 600 605
   Pro Ser Ser Lys Ser Thr Ser Gly Gly Thr Ala Ala Leu Gly Cys Leu
   610 615 620
   Val Lys Asp Tyr Phe Pro Glu Pro Val Thr Val Ser Trp Asn Ser Gly
   625 630 635 640
   Ala Leu Thr Ser Gly Val His Thr Phe Pro Ala Val Leu Gln Ser Ser
   645 650 655
   Gly Leu Tyr Ser Leu Ser Ser Val Val Thr Val Pro Ser Ser Ser Leu
   660 665 670
   Gly Thr Gln Thr Tyr Ile Cys Asn Val Asn His Lys Pro Ser Asn Thr
   675 680 685
   Lys Val Asp Lys Lys Val Glu Pro Lys Ser Cys Asp Lys Thr His Thr
   690 695 700
   Cys Pro Pro Cys Pro Ala Pro Glu Ala Ala Gly Gly Pro Ser Val Phe
   705 710 715 720
   Leu Phe Pro Pro Lys Pro Lys Asp Thr Leu Met Ile Ser Arg Thr Pro
   725 730 735
   Glu Val Thr Cys Val Val Val Asp Val Ser His Glu Asp Pro Glu Val
   740 745 750
   Lys Phe Asn Trp Tyr Val Asp Gly Val Glu Val His Asn Ala Lys Thr
   755 760 765
   Lys Pro Arg Glu Glu Gln Tyr Asn Ser Thr Tyr Arg Val Val Ser Val
   770 775 780
   Leu Thr Val Leu His Gln Asp Trp Leu Asn Gly Lys Glu Tyr Lys Cys
   785 790 795 800
   Lys Val Ser Asn Lys Ala Leu Pro Ala Pro Ile Glu Lys Thr Ile Ser
   805 810 815
   Lys Ala Lys Gly Gln Pro Arg Glu Pro Gln Val Tyr Thr Leu Pro Pro
   820 825 830
   Cys Arg Asp Glu Leu Thr Lys Asn Gln Val Ser Leu Trp Cys Leu Val
   835 840 845
   Lys Gly Phe Tyr Pro Ser Asp Ile Ala Val Glu Trp Glu Ser Asn Gly
   850 855 860
   Gln Pro Glu Asn Asn Tyr Lys Thr Thr Pro Pro Val Leu Asp Ser Asp
   865 870 875 880
   Gly Ser Phe Phe Leu Tyr Ser Lys Leu Thr Val Asp Lys Ser Arg Trp
   885 890 895
   Gln Gln Gly Asn Val Phe Ser Cys Ser Val Met His Glu Ala Leu His
   900 905 910
   Asn His Tyr Thr Gln Lys Ser Leu Ser Leu Ser Pro Gly Lys
   915 920 925
<210> 18
   <211> 2781
   <212> DNA
   <213> Artificial Sequence
<220>
   <223> V9 (scFab)-LC007 (VH-CH1)-Fc(knob) LALA
<400> 18
   gacatccaga tgacccagag cccctctagc ctgagcgcca gcgtgggcga cagagtgacc 60
   atcacctgtc gggccagcca ggacatcaga aactacctga actggtatca gcagaagccc 120
   ggcaaggccc ccaagctgct gatctactac acctctagac tggaaagcgg cgtgcccagc 180
   cggtttagcg gcagcggctc cggcaccgac tacaccctga ccatcagcag cctgcagccc 240
   gaggacttcg ccacctacta ctgccagcag ggcaacacac tcccctggac cttcggccag 300
   ggcaccaagg tggagatcaa gcgtacggtg gccgctccca gcgtgttcat cttccccccc 360
   agcgacgagc agctgaagtc cggcaccgcc agcgtcgtgt gcctgctgaa caacttctac 420
   ccccgggagg ccaaggtgca gtggaaggtg gacaacgccc tgcagagcgg caacagccag 480
   gaaagcgtca ccgagcagga cagcaaggac tccacctaca gcctgagcag caccctgacc 540
   ctgagcaagg ccgactacga gaagcacaag gtgtacgcct gcgaagtgac ccaccagggc 600
   ctgtccagcc ccgtgaccaa gagcttcaac cggggcgagt gcagcggcgg aggctctgga 660
   ggcggctctg aaggcggagg aagtgagggc ggaggctcag aaggcggcgg aagcgaaggt 720
   ggcggctctg gcggcggatc cggcgaggtg cagctggtcg agtccggcgg aggcctggtg 780
   cagcctggcg gcagcctgag actgagctgc gccgccagcg gctacagctt caccggctac 840
   accatgaact gggtccggca ggctcctggc aagggcctcg aatgggtggc cctgatcaac 900
   ccctacaagg gcgtgagcac ctacaaccag aagttcaagg accggttcac catcagcgtg 960
   gacaagagca agaacaccgc ctatctgcag atgaacagcc tgcgggccga ggacaccgcc 1020
   gtgtactact gcgccagaag cggctactac ggcgacagcg actggtactt cgacgtgtgg 1080
   ggccagggca cactggtcac cgtgtccagc gctagcacca agggcccctc cgtgttcccc 1140
   ctggccccca gcagcaagag caccagcggc ggcacagccg ccctcggctg cctggtcaag 1200
   gactacttcc ccgagcccgt gaccgtgtcc tggaacagcg gagccctgac ctccggcgtg 1260
   cacaccttcc ccgccgtgct gcagagcagc ggcctgtaca gcctgtccag cgtggtcacc 1320
   gtgccctcca gcagcctggg cacccagacc tacatctgca acgtgaacca caagcccagc 1380
   aataccaagg tggacaagaa ggtggagccc aagagctgcg acggcggtgg tggctccgga 1440
   ggcggtggat ctgaagtgca gctgcaggaa agcggccctg gcctggtcaa gcccagccag 1500
   agcctgagcc tgacctgtag cgtgaccggc tactccatca cctccggcta ctactggaat 1560
   tggattcggc agttccccgg caacaagctg gaatggatgg gctacatcac ctacgacggc 1620
   agcaacaact acaaccccag cctgaagaac cggatcagca tcacccggga caccagcaag 1680
   aaccagttct tcctgaagtt gaattctgtg actactgagg acacagctac atattactgt 1740
   gcggactttg actactgggg ccaaggcacc actctcacag tctcctcagc tagcaccaag 1800
   ggcccatcgg tcttccccct ggcaccctcc tccaagagca cctctggggg cacagcggcc 1860
   ctgggctgcc tggtcaagga ctacttcccc gaaccggtga cggtgtcgtg gaactcaggc 1920
   gccctgacca gcggcgtgca caccttcccg gctgtcctac agtcctcagg actctactcc 1980
   ctcagcagcg tggtgaccgt gccctccagc agcttgggca cccagaccta catctgcaac 2040
   gtgaatcaca agcccagcaa caccaaggtg gacaagaaag ttgagcccaa atcttgtgac 2100
   aaaactcaca catgcccacc gtgcccagca cctgaagctg cagggggacc gtcagtcttc 2160
   ctcttccccc caaaacccaa ggacaccctc atgatctccc ggacccctga ggtcacatgc 2220
   gtggtggtgg acgtgagcca cgaagaccct gaggtcaagt tcaactggta cgtggacggc 2280
   gtggaggtgc ataatgccaa gacaaagccg cgggaggagc agtacaacag cacgtaccgt 2340
   gtggtcagcg tcctcaccgt cctgcaccag gactggctga atggcaagga gtacaagtgc 2400
   aaggtctcca acaaagccct cccagccccc atcgagaaaa ccatctccaa agccaaaggg 2460
   cagccccgag aaccacaggt gtacaccctg cccccatgcc gggatgagct gaccaagaac 2520
   caggtcagcc tgtggtgcct ggtcaaaggc ttctatccca gcgacatcgc cgtggagtgg 2580
   gagagcaatg ggcagccgga gaacaactac aagaccacgc ctcccgtgct ggactccgac 2640
   ggctccttct tcctctacag caagctcacc gtggacaaga gcaggtggca gcaggggaac 2700
   gtcttctcat gctccgtgat gcatgaggct ctgcacaacc actacacgca gaagagcctc 2760
   tccctgtctc cgggtaaatg a 2781
<210> 19
   <211> 442
   <212> PRT
   <213> Artificial Sequence
<220>
   <223> LC007 (VH-CH1)-Fc(hole) LALA
<400> 19
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Pro Ala Pro Ile Glu Lys Thr Ile Ser Lys Ala Lys Gly
                   325                 330                 335
   Gln Pro Arg Glu Pro Gln Val Cys Thr Leu Pro Pro Ser Arg Asp Glu
   340 345 350
   Leu Thr Lys Asn Gln Val Ser Leu Ser Cys Ala Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Val Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 20
   <211> 1329
   <212> DNA
   <213> Artificial Sequence
<220>
   <223> LC007 (VH-CH1)-Fc(hole) LALA
<400> 20
   gaggtccagc tgcaggagtc aggacctggc ctcgtgaaac cttctcagtc tctgtctctc      60
   acctgctctg tcactggcta ctccatcacc agtggttatt actggaactg gatccggcag     120
   tttccaggaa acaagctgga atggatgggc tacataacct acgacggtag caataactac     180
   aacccatctc tcaaaaatcg aatctccatc actcgtgaca catctaagaa ccagtttttc     240
   ctgaagttga attctgtgac tactgaggac acagctacat attactgtgc ggactttgac     300
   tactggggcc aaggcaccac tctcacagtc tcctcagcta gcaccaaggg cccatcggtc     360
   ttccccctgg caccctcctc caagagcacc tctgggggca cagcggccct gggctgcctg     420
   gtcaaggact acttccccga accggtgacg gtgtcgtgga actcaggcgc cctgaccagc     480
   ggcgtgcaca ccttcccggc tgtcctacag tcctcaggac tctactccct cagcagcgtg     540
   gtgaccgtgc cctccagcag cttgggcacc cagacctaca tctgcaacgt gaatcacaag     600
   cccagcaaca ccaaggtgga caagaaagtt gagcccaaat cttgtgacaa aactcacaca     660
   tgcccaccgt gcccagcacc tgaagctgca gggggaccgt cagtcttcct cttcccccca     720
   aaacccaagg acaccctcat gatctcccgg acccctgagg tcacatgcgt ggtggtggac     780
   gtgagccacg aagaccctga ggtcaagttc aactggtacg tggacggcgt ggaggtgcat     840
   aatgccaaga caaagccgcg ggaggagcag tacaacagca cgtaccgtgt ggtcagcgtc     900
   ctcaccgtcc tgcaccagga ctggctgaat ggcaaggagt acaagtgcaa ggtctccaac     960
   aaagccctcc cagcccccat cgagaaaacc atctccaaag ccaaagggca gccccgagaa    1020
   ccacaggtgt gcaccctgcc cccatcccgg gatgagctga ccaagaacca ggtcagcctc    1080
   tcgtgcgcag tcaaaggctt ctatcccagc gacatcgccg tggagtggga gagcaatggg    1140
   cagccggaga acaactacaa gaccacgcct cccgtgctgg actccgacgg ctccttcttc    1200
   ctcgtgagca agctcaccgt ggacaagagc aggtggcagc aggggaacgt cttctcatgc    1260
   tccgtgatgc atgaggctct gcacaaccac tacacgcaga agagcctctc cctgtctccg    1320
   ggtaaatga                                                            1329
<210> 21
   <211> 926
   <212> PRT
   <213> Artificial Sequence
<220>
   <223> V9 (scFab)-LC007 (VH-CH1)-Fc(knob) P329G LALA
<400> 21
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Ser Gly Gly Gly Ser Glu
   210 215 220
   Gly Gly Gly Ser Glu Gly Gly Gly Ser Glu Gly Gly Gly Ser Glu Gly
   225 230 235 240
   Gly Gly Ser Gly Gly Gly Ser Gly Glu Val Gln Leu Val Glu Ser Gly
   245 250 255
   Gly Gly Leu Val Gln Pro Gly Gly Ser Leu Arg Leu Ser Cys Ala Ala
   260 265 270
   Ser Gly Tyr Ser Phe Thr Gly Tyr Thr Met Asn Trp Val Arg Gln Ala
   275 280 285
   Pro Gly Lys Gly Leu Glu Trp Val Ala Leu Ile Asn Pro Tyr Lys Gly
   290 295 300
   Val Ser Thr Tyr Asn Gln Lys Phe Lys Asp Arg Phe Thr Ile Ser Val
   305 310 315 320
   Asp Lys Ser Lys Asn Thr Ala Tyr Leu Gln Met Asn Ser Leu Arg Ala
   325 330 335
   Glu Asp Thr Ala Val Tyr Tyr Cys Ala Arg Ser Gly Tyr Tyr Gly Asp
   340 345 350
   Ser Asp Trp Tyr Phe Asp Val Trp Gly Gln Gly Thr Leu Val Thr Val
   355 360 365
   Ser Ser Ala Ser Thr Lys Gly Pro Ser Val Phe Pro Leu Ala Pro Ser
   370 375 380
   Ser Lys Ser Thr Ser Gly Gly Thr Ala Ala Leu Gly Cys Leu Val Lys
   385 390 395 400
   Asp Tyr Phe Pro Glu Pro Val Thr Val Ser Trp Asn Ser Gly Ala Leu
   405 410 415
   Thr Ser Gly Val His Thr Phe Pro Ala Val Leu Gln Ser Ser Gly Leu
   420 425 430
   Tyr Ser Leu Ser Ser Val Val Thr Val Pro Ser Ser Ser Leu Gly Thr
   435 440 445
   Gln Thr Tyr Ile Cys Asn Val Asn His Lys Pro Ser Asn Thr Lys Val
   450 455 460
   Asp Lys Lys Val Glu Pro Lys Ser Cys Asp Gly Gly Gly Gly Ser Gly
   465 470 475 480
   Gly Gly Gly Ser Glu Val Gln Leu Gln Glu Ser Gly Pro Gly Leu Val
   485 490 495
   Lys Pro Ser Gln Ser Leu Ser Leu Thr Cys Ser Val Thr Gly Tyr Ser
   500 505 510
   Ile Thr Ser Gly Tyr Tyr Trp Asn Trp Ile Arg Gln Phe Pro Gly Asn
   515 520 525
   Lys Leu Glu Trp Met Gly Tyr Ile Thr Tyr Asp Gly Ser Asn Asn Tyr
   530 535 540
   Asn Pro Ser Leu Lys Asn Arg Ile Ser Ile Thr Arg Asp Thr Ser Lys
   545 550 555 560
   Asn Gln Phe Phe Leu Lys Leu Asn Ser Val Thr Thr Glu Asp Thr Ala
   565 570 575
   Thr Tyr Tyr Cys Ala Asp Phe Asp Tyr Trp Gly Gln Gly Thr Thr Leu
   580 585 590
   Thr Val Ser Ser Ala Ser Thr Lys Gly Pro Ser Val Phe Pro Leu Ala
   595 600 605
   Pro Ser Ser Lys Ser Thr Ser Gly Gly Thr Ala Ala Leu Gly Cys Leu
   610 615 620
   Val Lys Asp Tyr Phe Pro Glu Pro Val Thr Val Ser Trp Asn Ser Gly
   625 630 635 640
   Ala Leu Thr Ser Gly Val His Thr Phe Pro Ala Val Leu Gln Ser Ser
   645 650 655
   Gly Leu Tyr Ser Leu Ser Ser Val Val Thr Val Pro Ser Ser Ser Leu
   660 665 670
   Gly Thr Gln Thr Tyr Ile Cys Asn Val Asn His Lys Pro Ser Asn Thr
   675 680 685
   Lys Val Asp Lys Lys Val Glu Pro Lys Ser Cys Asp Lys Thr His Thr
   690 695 700
   Cys Pro Pro Cys Pro Ala Pro Glu Ala Ala Gly Gly Pro Ser Val Phe
   705 710 715 720
   Leu Phe Pro Pro Lys Pro Lys Asp Thr Leu Met Ile Ser Arg Thr Pro
   725 730 735
   Glu Val Thr Cys Val Val Val Asp Val Ser His Glu Asp Pro Glu Val
   740 745 750
   Lys Phe Asn Trp Tyr Val Asp Gly Val Glu Val His Asn Ala Lys Thr
   755 760 765
   Lys Pro Arg Glu Glu Gln Tyr Asn Ser Thr Tyr Arg Val Val Ser Val
   770 775 780
   Leu Thr Val Leu His Gln Asp Trp Leu Asn Gly Lys Glu Tyr Lys Cys
   785 790 795 800
   Lys Val Ser Asn Lys Ala Leu Gly Ala Pro Ile Glu Lys Thr Ile Ser
   805 810 815
   Lys Ala Lys Gly Gln Pro Arg Glu Pro Gln Val Tyr Thr Leu Pro Pro
   820 825 830
   Cys Arg Asp Glu Leu Thr Lys Asn Gln Val Ser Leu Trp Cys Leu Val
   835 840 845
   Lys Gly Phe Tyr Pro Ser Asp Ile Ala Val Glu Trp Glu Ser Asn Gly
   850 855 860
   Gln Pro Glu Asn Asn Tyr Lys Thr Thr Pro Pro Val Leu Asp Ser Asp
   865 870 875 880
   Gly Ser Phe Phe Leu Tyr Ser Lys Leu Thr Val Asp Lys Ser Arg Trp
   885 890 895
   Gln Gln Gly Asn Val Phe Ser Cys Ser Val Met His Glu Ala Leu His
   900 905 910
   Asn His Tyr Thr Gln Lys Ser Leu Ser Leu Ser Pro Gly Lys
   915 920 925
<210> 22
   <211> 2781
   <212> DNA
   <213> Artificial Sequence
<220>
   <223> V9 (scFab)-LC007 (VH-CH1)-Fc(knob) P329G LALA
<400> 22
   gacatccaga tgacccagag cccctctagc ctgagcgcca gcgtgggcga cagagtgacc 60
   atcacctgtc gggccagcca ggacatcaga aactacctga actggtatca gcagaagccc 120
   ggcaaggccc ccaagctgct gatctactac acctctagac tggaaagcgg cgtgcccagc 180
   cggtttagcg gcagcggctc cggcaccgac tacaccctga ccatcagcag cctgcagccc 240
   gaggacttcg ccacctacta ctgccagcag ggcaacacac tcccctggac cttcggccag 300
   ggcaccaagg tggagatcaa gcgtacggtg gccgctccca gcgtgttcat cttccccccc 360
   agcgacgagc agctgaagtc cggcaccgcc agcgtcgtgt gcctgctgaa caacttctac 420
   ccccgggagg ccaaggtgca gtggaaggtg gacaacgccc tgcagagcgg caacagccag 480
   gaaagcgtca ccgagcagga cagcaaggac tccacctaca gcctgagcag caccctgacc 540
   ctgagcaagg ccgactacga gaagcacaag gtgtacgcct gcgaagtgac ccaccagggc 600
   ctgtccagcc ccgtgaccaa gagcttcaac cggggcgagt gcagcggcgg aggctctgga 660
   ggcggctctg aaggcggagg aagtgagggc ggaggctcag aaggcggcgg aagcgaaggt 720
   ggcggctctg gcggcggatc cggcgaggtg cagctggtcg agtccggcgg aggcctggtg 780
   cagcctggcg gcagcctgag actgagctgc gccgccagcg gctacagctt caccggctac 840
   accatgaact gggtccggca ggctcctggc aagggcctcg aatgggtggc cctgatcaac 900
   ccctacaagg gcgtgagcac ctacaaccag aagttcaagg accggttcac catcagcgtg 960
   gacaagagca agaacaccgc ctatctgcag atgaacagcc tgcgggccga ggacaccgcc 1020
   gtgtactact gcgccagaag cggctactac ggcgacagcg actggtactt cgacgtgtgg 1080
   ggccagggca cactggtcac cgtgtccagc gctagcacca agggcccctc cgtgttcccc 1140
   ctggccccca gcagcaagag caccagcggc ggcacagccg ccctcggctg cctggtcaag 1200
   gactacttcc ccgagcccgt gaccgtgtcc tggaacagcg gagccctgac ctccggcgtg 1260
   cacaccttcc ccgccgtgct gcagagcagc ggcctgtaca gcctgtccag cgtggtcacc 1320
   gtgccctcca gcagcctggg cacccagacc tacatctgca acgtgaacca caagcccagc 1380
   aataccaagg tggacaagaa ggtggagccc aagagctgcg acggcggtgg tggctccgga 1440
   ggcggtggat ctgaagtgca gctgcaggaa agcggccctg gcctggtcaa gcccagccag 1500
   agcctgagcc tgacctgtag cgtgaccggc tactccatca cctccggcta ctactggaat 1560
   tggattcggc agttccccgg caacaagctg gaatggatgg gctacatcac ctacgacggc 1620
   agcaacaact acaaccccag cctgaagaac cggatcagca tcacccggga caccagcaag 1680
   aaccagttct tcctgaagtt gaattctgtg actactgagg acacagctac atattactgt 1740
   gcggactttg actactgggg ccaaggcacc actctcacag tctcctcagc tagcaccaag 1800
   ggcccatcgg tcttccccct ggcaccctcc tccaagagca cctctggggg cacagcggcc 1860
   ctgggctgcc tggtcaagga ctacttcccc gaaccggtga cggtgtcgtg gaactcaggc 1920
   gccctgacca gcggcgtgca caccttcccg gctgtcctac agtcctcagg actctactcc 1980
   ctcagcagcg tggtgaccgt gccctccagc agcttgggca cccagaccta catctgcaac 2040
   gtgaatcaca agcccagcaa caccaaggtg gacaagaaag ttgagcccaa atcttgtgac 2100
   aaaactcaca catgcccacc gtgcccagca cctgaagctg cagggggacc gtcagtcttc 2160
   ctcttccccc caaaacccaa ggacaccctc atgatctccc ggacccctga ggtcacatgc 2220
   gtggtggtgg acgtgagcca cgaagaccct gaggtcaagt tcaactggta cgtggacggc 2280
   gtggaggtgc ataatgccaa gacaaagccg cgggaggagc agtacaacag cacgtaccgt 2340
   gtggtcagcg tcctcaccgt cctgcaccag gactggctga atggcaagga gtacaagtgc 2400
   aaggtctcca acaaagccct cggcgccccc atcgagaaaa ccatctccaa agccaaaggg 2460
   cagccccgag aaccacaggt gtacaccctg cccccatgcc gggatgagct gaccaagaac 2520
   caggtcagcc tgtggtgcct ggtcaaaggc ttctatccca gcgacatcgc cgtggagtgg 2580
   gagagcaatg ggcagccgga gaacaactac aagaccacgc ctcccgtgct ggactccgac 2640
   ggctccttct tcctctacag caagctcacc gtggacaaga gcaggtggca gcaggggaac 2700
   gtcttctcat gctccgtgat gcatgaggct ctgcacaacc actacacgca gaagagcctc 2760
   tccctgtctc cgggtaaatg a 2781
<210> 23
   <211> 442
   <212> PRT
   <213> Artificial Sequence
<220>
   <223> LC007 (VH-CH1)-Fc(hole) P329G LALA
<400> 23
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65 70 75 80
   Leu Lys Leu Asn Ser Val Thr Thr Glu Asp Thr Ala Thr Tyr Tyr Cys
   85 90 95
   Ala Asp Phe Asp Tyr Trp Gly Gln Gly Thr Thr Leu Thr Val Ser Ser
   100 105 110
   Ala Ser Thr Lys Gly Pro Ser Val Phe Pro Leu Ala Pro Ser Ser Lys
   115 120 125
   Ser Thr Ser Gly Gly Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
   180 185 190
   Tyr Ile Cys Asn Val Asn His Lys Pro Ser Asn Thr Lys Val Asp Lys
   195 200 205
   Lys Val Glu Pro Lys Ser Cys Asp Lys Thr His Thr Cys Pro Pro Cys
   210 215 220
   Pro Ala Pro Glu Ala Ala Gly Gly Pro Ser Val Phe Leu Phe Pro Pro
   225 230 235 240
   Lys Pro Lys Asp Thr Leu Met Ile Ser Arg Thr Pro Glu Val Thr Cys
   245 250 255
   Val Val Val Asp Val Ser His Glu Asp Pro Glu Val Lys Phe Asn Trp
   260 265 270
   Tyr Val Asp Gly Val Glu Val His Asn Ala Lys Thr Lys Pro Arg Glu
   275 280 285
   Glu Gln Tyr Asn Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Ala Leu Gly Ala Pro Ile Glu Lys Thr Ile Ser Lys Ala Lys Gly
                   325                 330                 335
   Gln Pro Arg Glu Pro Gln Val Cys Thr Leu Pro Pro Ser Arg Asp Glu
               340                 345                 350
   Leu Thr Lys Asn Gln Val Ser Leu Ser Cys Ala Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Val Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440
<210> 24
   <211> 1329
   <212> DNA
   <213> Artificial Sequence
<220>
   <223> LC007 (VH-CH1)-Fc(hole) P329G LALA
<400> 24
   gaggtccagc tgcaggagtc aggacctggc ctcgtgaaac cttctcagtc tctgtctctc 60
   acctgctctg tcactggcta ctccatcacc agtggttatt actggaactg gatccggcag 120
   tttccaggaa acaagctgga atggatgggc tacataacct acgacggtag caataactac 180
   aacccatctc tcaaaaatcg aatctccatc actcgtgaca catctaagaa ccagtttttc 240
   ctgaagttga attctgtgac tactgaggac acagctacat attactgtgc ggactttgac 300
   tactggggcc aaggcaccac tctcacagtc tcctcagcta gcaccaaggg cccatcggtc 360
   ttccccctgg caccctcctc caagagcacc tctgggggca cagcggccct gggctgcctg 420
   gtcaaggact acttccccga accggtgacg gtgtcgtgga actcaggcgc cctgaccagc 480
   ggcgtgcaca ccttcccggc tgtcctacag tcctcaggac tctactccct cagcagcgtg 540
   gtgaccgtgc cctccagcag cttgggcacc cagacctaca tctgcaacgt gaatcacaag 600
   cccagcaaca ccaaggtgga caagaaagtt gagcccaaat cttgtgacaa aactcacaca 660
   tgcccaccgt gcccagcacc tgaagctgca gggggaccgt cagtcttcct cttcccccca 720
   aaacccaagg acaccctcat gatctcccgg acccctgagg tcacatgcgt ggtggtggac 780
   gtgagccacg aagaccctga ggtcaagttc aactggtacg tggacggcgt ggaggtgcat 840
   aatgccaaga caaagccgcg ggaggagcag tacaacagca cgtaccgtgt ggtcagcgtc 900
   ctcaccgtcc tgcaccagga ctggctgaat ggcaaggagt acaagtgcaa ggtctccaac 960
   aaagccctcg gcgcccccat cgagaaaacc atctccaaag ccaaagggca gccccgagaa 1020
   ccacaggtgt gcaccctgcc cccatcccgg gatgagctga ccaagaacca ggtcagcctc 1080
   tcgtgcgcag tcaaaggctt ctatcccagc gacatcgccg tggagtggga gagcaatggg 1140
   cagccggaga acaactacaa gaccacgcct cccgtgctgg actccgacgg ctccttcttc 1200
   ctcgtgagca agctcaccgt ggacaagagc aggtggcagc aggggaacgt cttctcatgc 1260
   tccgtgatgc atgaggctct gcacaaccac tacacgcaga agagcctctc cctgtctccg 1320
   ggtaaatga 1329
<210> 25
   <211> 926
   <212> PRT
   <213> Artificial Sequence
<220>
   <223> V9 (scFab)-LC007 (VH-CH1)-Fc(knob) P329G LALA N297D
<400> 25
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Ser Gly Gly Gly Ser Glu
   210 215 220
   Gly Gly Gly Ser Glu Gly Gly Gly Ser Glu Gly Gly Gly Ser Glu Gly
   225 230 235 240
   Gly Gly Ser Gly Gly Gly Ser Gly Glu Val Gln Leu Val Glu Ser Gly
   245 250 255
   Gly Gly Leu Val Gln Pro Gly Gly Ser Leu Arg Leu Ser Cys Ala Ala
   260 265 270
   Ser Gly Tyr Ser Phe Thr Gly Tyr Thr Met Asn Trp Val Arg Gln Ala
   275 280 285
   Pro Gly Lys Gly Leu Glu Trp Val Ala Leu Ile Asn Pro Tyr Lys Gly
   290 295 300
   Val Ser Thr Tyr Asn Gln Lys Phe Lys Asp Arg Phe Thr Ile Ser Val
   305 310 315 320
   Asp Lys Ser Lys Asn Thr Ala Tyr Leu Gln Met Asn Ser Leu Arg Ala
   325 330 335
   Glu Asp Thr Ala Val Tyr Tyr Cys Ala Arg Ser Gly Tyr Tyr Gly Asp
   340 345 350
   Ser Asp Trp Tyr Phe Asp Val Trp Gly Gln Gly Thr Leu Val Thr Val
   355 360 365
   Ser Ser Ala Ser Thr Lys Gly Pro Ser Val Phe Pro Leu Ala Pro Ser
   370 375 380
   Ser Lys Ser Thr Ser Gly Gly Thr Ala Ala Leu Gly Cys Leu Val Lys
   385 390 395 400
   Asp Tyr Phe Pro Glu Pro Val Thr Val Ser Trp Asn Ser Gly Ala Leu
   405 410 415
   Thr Ser Gly Val His Thr Phe Pro Ala Val Leu Gln Ser Ser Gly Leu
   420 425 430
   Tyr Ser Leu Ser Ser Val Val Thr Val Pro Ser Ser Ser Leu Gly Thr
   435 440 445
   Gln Thr Tyr Ile Cys Asn Val Asn His Lys Pro Ser Asn Thr Lys Val
   450 455 460
   Asp Lys Lys Val Glu Pro Lys Ser Cys Asp Gly Gly Gly Gly Ser Gly
   465 470 475 480
   Gly Gly Gly Ser Glu Val Gln Leu Gln Glu Ser Gly Pro Gly Leu Val
   485 490 495
   Lys Pro Ser Gln Ser Leu Ser Leu Thr Cys Ser Val Thr Gly Tyr Ser
   500 505 510
   Ile Thr Ser Gly Tyr Tyr Trp Asn Trp Ile Arg Gln Phe Pro Gly Asn
   515 520 525
   Lys Leu Glu Trp Met Gly Tyr Ile Thr Tyr Asp Gly Ser Asn Asn Tyr
   530 535 540
   Asn Pro Ser Leu Lys Asn Arg Ile Ser Ile Thr Arg Asp Thr Ser Lys
   545 550 555 560
   Asn Gln Phe Phe Leu Lys Leu Asn Ser Val Thr Thr Glu Asp Thr Ala
   565 570 575
   Thr Tyr Tyr Cys Ala Asp Phe Asp Tyr Trp Gly Gln Gly Thr Thr Leu
   580 585 590
   Thr Val Ser Ser Ala Ser Thr Lys Gly Pro Ser Val Phe Pro Leu Ala
   595 600 605
   Pro Ser Ser Lys Ser Thr Ser Gly Gly Thr Ala Ala Leu Gly Cys Leu
   610 615 620
   Val Lys Asp Tyr Phe Pro Glu Pro Val Thr Val Ser Trp Asn Ser Gly
   625 630 635 640
   Ala Leu Thr Ser Gly Val His Thr Phe Pro Ala Val Leu Gln Ser Ser
   645 650 655
   Gly Leu Tyr Ser Leu Ser Ser Val Val Thr Val Pro Ser Ser Ser Leu
               660                 665                 670
   Gly Thr Gln Thr Tyr Ile Cys Asn Val Asn His Lys Pro Ser Asn Thr
           675                 680                 685
   Lys Val Asp Lys Lys Val Glu Pro Lys Ser Cys Asp Lys Thr His Thr
       690                 695                 700
   Cys Pro Pro Cys Pro Ala Pro Glu Ala Ala Gly Gly Pro Ser Val Phe
   705                 710                 715                 720
   Leu Phe Pro Pro Lys Pro Lys Asp Thr Leu Met Ile Ser Arg Thr Pro
                   725                 730                 735
   Glu Val Thr Cys Val Val Val Asp Val Ser His Glu Asp Pro Glu Val
               740                 745                 750
   Lys Phe Asn Trp Tyr Val Asp Gly Val Glu Val His Asn Ala Lys Thr
           755                 760                 765
   Lys Pro Arg Glu Glu Gln Tyr Asp Ser Thr Tyr Arg Val Val Ser Val
       770                 775                 780
   Leu Thr Val Leu His Gln Asp Trp Leu Asn Gly Lys Glu Tyr Lys Cys
   785                 790                 795                 800
   Lys Val Ser Asn Lys Ala Leu Gly Ala Pro Ile Glu Lys Thr Ile Ser
                   805                 810                 815
   Lys Ala Lys Gly Gln Pro Arg Glu Pro Gln Val Tyr Thr Leu Pro Pro
               820                 825                 830
   Cys Arg Asp Glu Leu Thr Lys Asn Gln Val Ser Leu Trp Cys Leu Val
           835                 840                 845
   Lys Gly Phe Tyr Pro Ser Asp Ile Ala Val Glu Trp Glu Ser Asn Gly
       850                 855                 860
   Gln Pro Glu Asn Asn Tyr Lys Thr Thr Pro Pro Val Leu Asp Ser Asp
   865                 870                 875                 880
   Gly Ser Phe Phe Leu Tyr Ser Lys Leu Thr Val Asp Lys Ser Arg Trp
                   885                 890                 895
   Gln Gln Gly Asn Val Phe Ser Cys Ser Val Met His Glu Ala Leu His
               900                 905                 910
   Asn His Tyr Thr Gln Lys Ser Leu Ser Leu Ser Pro Gly Lys
   915 920 925
<210> 26
   <211> 2781
   <212> DNA
   <213> Artificial Sequence
<220>
   <223> V9 (scFab)-LC007 (VH-CH1)-Fc(knob) P329G LALA N297D
<400> 26
   gacatccaga tgacccagag cccctctagc ctgagcgcca gcgtgggcga cagagtgacc 60
   atcacctgtc gggccagcca ggacatcaga aactacctga actggtatca gcagaagccc 120
   ggcaaggccc ccaagctgct gatctactac acctctagac tggaaagcgg cgtgcccagc 180
   cggtttagcg gcagcggctc cggcaccgac tacaccctga ccatcagcag cctgcagccc 240
   gaggacttcg ccacctacta ctgccagcag ggcaacacac tcccctggac cttcggccag 300
   ggcaccaagg tggagatcaa gcgtacggtg gccgctccca gcgtgttcat cttccccccc 360
   agcgacgagc agctgaagtc cggcaccgcc agcgtcgtgt gcctgctgaa caacttctac 420
   ccccgggagg ccaaggtgca gtggaaggtg gacaacgccc tgcagagcgg caacagccag 480
   gaaagcgtca ccgagcagga cagcaaggac tccacctaca gcctgagcag caccctgacc 540
   ctgagcaagg ccgactacga gaagcacaag gtgtacgcct gcgaagtgac ccaccagggc 600
   ctgtccagcc ccgtgaccaa gagcttcaac cggggcgagt gcagcggcgg aggctctgga 660
   ggcggctctg aaggcggagg aagtgagggc ggaggctcag aaggcggcgg aagcgaaggt 720
   ggcggctctg gcggcggatc cggcgaggtg cagctggtcg agtccggcgg aggcctggtg 780
   cagcctggcg gcagcctgag actgagctgc gccgccagcg gctacagctt caccggctac 840
   accatgaact gggtccggca ggctcctggc aagggcctcg aatgggtggc cctgatcaac 900
   ccctacaagg gcgtgagcac ctacaaccag aagttcaagg accggttcac catcagcgtg 960
   gacaagagca agaacaccgc ctatctgcag atgaacagcc tgcgggccga ggacaccgcc 1020
   gtgtactact gcgccagaag cggctactac ggcgacagcg actggtactt cgacgtgtgg 1080
   ggccagggca cactggtcac cgtgtccagc gctagcacca agggcccctc cgtgttcccc 1140
   ctggccccca gcagcaagag caccagcggc ggcacagccg ccctcggctg cctggtcaag 1200
   gactacttcc ccgagcccgt gaccgtgtcc tggaacagcg gagccctgac ctccggcgtg 1260
   cacaccttcc ccgccgtgct gcagagcagc ggcctgtaca gcctgtccag cgtggtcacc 1320
   gtgccctcca gcagcctggg cacccagacc tacatctgca acgtgaacca caagcccagc 1380
   aataccaagg tggacaagaa ggtggagccc aagagctgcg acggcggtgg tggctccgga 1440
   ggcggtggat ctgaagtgca gctgcaggaa agcggccctg gcctggtcaa gcccagccag 1500
   agcctgagcc tgacctgtag cgtgaccggc tactccatca cctccggcta ctactggaat 1560
   tggattcggc agttccccgg caacaagctg gaatggatgg gctacatcac ctacgacggc      1620
   agcaacaact acaaccccag cctgaagaac cggatcagca tcacccggga caccagcaag      1680
   aaccagttct tcctgaagtt gaattctgtg actactgagg acacagctac atattactgt      1740
   gcggactttg actactgggg ccaaggcacc actctcacag tctcctcagc tagcaccaag      1800
   ggcccatcgg tcttccccct ggcaccctcc tccaagagca cctctggggg cacagcggcc      1860
   ctgggctgcc tggtcaagga ctacttcccc gaaccggtga cggtgtcgtg gaactcaggc      1920
   gccctgacca gcggcgtgca caccttcccg gctgtcctac agtcctcagg actctactcc      1980
   ctcagcagcg tggtgaccgt gccctccagc agcttgggca cccagaccta catctgcaac      2040
   gtgaatcaca agcccagcaa caccaaggtg gacaagaaag ttgagcccaa atcttgtgac      2100
   aaaactcaca catgcccacc gtgcccagca cctgaagctg cagggggacc gtcagtcttc      2160
   ctcttccccc caaaacccaa ggacaccctc atgatctccc ggacccctga ggtcacatgc      2220
   gtggtggtgg acgtgagcca cgaagaccct gaggtcaagt tcaactggta cgtggacggc      2280
   gtggaggtgc ataatgccaa gacaaagccg cgggaggagc agtacgacag cacgtaccgt      2340
   gtggtcagcg tcctcaccgt cctgcaccag gactggctga atggcaagga gtacaagtgc      2400
   aaggtctcca acaaagccct cggcgccccc atcgagaaaa ccatctccaa agccaaaggg      2460
   cagccccgag aaccacaggt gtacaccctg cccccatgcc gggatgagct gaccaagaac      2520
   caggtcagcc tgtggtgcct ggtcaaaggc ttctatccca gcgacatcgc cgtggagtgg      2580
   gagagcaatg ggcagccgga gaacaactac aagaccacgc ctcccgtgct ggactccgac      2640
   ggctccttct tcctctacag caagctcacc gtggacaaga gcaggtggca gcaggggaac      2700
   gtcttctcat gctccgtgat gcatgaggct ctgcacaacc actacacgca gaagagcctc      2760
   tccctgtctc cgggtaaatg a                                                2781
<210> 27
   <211> 442
   <212> PRT
   <213> Artificial Sequence
<220>
   <223> LC007 (VH-CH1)-Fc(hole) P329G LALA N297D
<400> 27
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asp Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Gly Ala Pro Ile Glu Lys Thr Ile Ser Lys Ala Lys Gly
                   325                 330                 335
   Gln Pro Arg Glu Pro Gln Val Cys Thr Leu Pro Pro Ser Arg Asp Glu
               340                 345                 350
   Leu Thr Lys Asn Gln Val Ser Leu Ser Cys Ala Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Val Ser Lys Leu Thr Val Asp Lys Ser Arg Trp Gln Gln Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Pro Gly Lys
           435                 440
<210> 28
   <211> 1329
   <212> DNA
   <213> Artificial Sequence
<220>
   <223> LC007 (VH-CH1)-Fc(hole) P329G LALA N297D
<400> 28
   gaggtccagc tgcaggagtc aggacctggc ctcgtgaaac cttctcagtc tctgtctctc 60
   acctgctctg tcactggcta ctccatcacc agtggttatt actggaactg gatccggcag 120
   tttccaggaa acaagctgga atggatgggc tacataacct acgacggtag caataactac 180
   aacccatctc tcaaaaatcg aatctccatc actcgtgaca catctaagaa ccagtttttc 240
   ctgaagttga attctgtgac tactgaggac acagctacat attactgtgc ggactttgac 300
   tactggggcc aaggcaccac tctcacagtc tcctcagcta gcaccaaggg cccatcggtc 360
   ttccccctgg caccctcctc caagagcacc tctgggggca cagcggccct gggctgcctg 420
   gtcaaggact acttccccga accggtgacg gtgtcgtgga actcaggcgc cctgaccagc 480
   ggcgtgcaca ccttcccggc tgtcctacag tcctcaggac tctactccct cagcagcgtg 540
   gtgaccgtgc cctccagcag cttgggcacc cagacctaca tctgcaacgt gaatcacaag 600
   cccagcaaca ccaaggtgga caagaaagtt gagcccaaat cttgtgacaa aactcacaca 660
   tgcccaccgt gcccagcacc tgaagctgca gggggaccgt cagtcttcct cttcccccca 720
   aaacccaagg acaccctcat gatctcccgg acccctgagg tcacatgcgt ggtggtggac 780
   gtgagccacg aagaccctga ggtcaagttc aactggtacg tggacggcgt ggaggtgcat 840
   aatgccaaga caaagccgcg ggaggagcag tacgacagca cgtaccgtgt ggtcagcgtc 900
   ctcaccgtcc tgcaccagga ctggctgaat ggcaaggagt acaagtgcaa ggtctccaac 960
   aaagccctcg gcgcccccat cgagaaaacc atctccaaag ccaaagggca gccccgagaa 1020
   ccacaggtgt gcaccctgcc cccatcccgg gatgagctga ccaagaacca ggtcagcctc 1080
   tcgtgcgcag tcaaaggctt ctatcccagc gacatcgccg tggagtggga gagcaatggg 1140
   cagccggaga acaactacaa gaccacgcct cccgtgctgg actccgacgg ctccttcttc 1200
   ctcgtgagca agctcaccgt ggacaagagc aggtggcagc aggggaacgt cttctcatgc 1260
   tccgtgatgc atgaggctct gcacaaccac tacacgcaga agagcctctc cctgtctccg 1320
   ggtaaatga 1329
<210> 29
   <211> 664
   <212> PRT
   <213> Artificial Sequence
<220>
   <223> V9 (VL-CH1)-LC007 (VH-CH1)-Fc(hole) P329G LALA
<400> 29
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Lys Asp Tyr Phe Pro Glu
   130 135 140
   Pro Val Thr Val Ser Trp Asn Ser Gly Ala Leu Thr Ser Gly Val His
   145 150 155 160
   Thr Phe Pro Ala Val Leu Gln Ser Ser Gly Leu Tyr Ser Leu Ser Ser
   165 170 175
   Val Val Thr Val Pro Ser Ser Ser Leu Gly Thr Gln Thr Tyr Ile Cys
   180 185 190
   Asn Val Asn His Lys Pro Ser Asn Thr Lys Val Asp Lys Lys Val Glu
   195 200 205
   Pro Lys Ser Cys Gly Gly Gly Gly Ser Gly Gly Gly Gly Ser Glu Val
   210 215 220
   Gln Leu Gln Glu Ser Gly Pro Gly Leu Val Lys Pro Ser Gln Ser Leu
   225 230 235 240
   Ser Leu Thr Cys Ser Val Thr Gly Tyr Ser Ile Thr Ser Gly Tyr Tyr
   245 250 255
   Trp Asn Trp Ile Arg Gln Phe Pro Gly Asn Lys Leu Glu Trp Met Gly
   260 265 270
   Tyr Ile Thr Tyr Asp Gly Ser Asn Asn Tyr Asn Pro Ser Leu Lys Asn
   275 280 285
   Arg Ile Ser Ile Thr Arg Asp Thr Ser Lys Asn Gln Phe Phe Leu Lys
   290 295 300
   Leu Asn Ser Val Thr Thr Glu Asp Thr Ala Thr Tyr Tyr Cys Ala Asp
   305 310 315 320
   Phe Asp Tyr Trp Gly Gln Gly Thr Thr Leu Thr Val Ser Ser Ala Ser
   325 330 335
   Thr Lys Gly Pro Ser Val Phe Pro Leu Ala Pro Ser Ser Lys Ser Thr
   340 345 350
   Ser Gly Gly Thr Ala Ala Leu Gly Cys Leu Val Lys Asp Tyr Phe Pro
   355 360 365
   Glu Pro Val Thr Val Ser Trp Asn Ser Gly Ala Leu Thr Ser Gly Val
       370                 375                 380
   His Thr Phe Pro Ala Val Leu Gln Ser Ser Gly Leu Tyr Ser Leu Ser
   385                 390                 395                 400
   Ser Val Val Thr Val Pro Ser Ser Ser Leu Gly Thr Gln Thr Tyr Ile
                   405                 410                 415
   Cys Asn Val Asn His Lys Pro Ser Asn Thr Lys Val Asp Lys Lys Val
               420                 425                 430
   Glu Pro Lys Ser Cys Asp Lys Thr His Thr Cys Pro Pro Cys Pro Ala
           435                 440                 445
   Pro Glu Ala Ala Gly Gly Pro Ser Val Phe Leu Phe Pro Pro Lys Pro
       450                 455                 460
   Lys Asp Thr Leu Met Ile Ser Arg Thr Pro Glu Val Thr Cys Val Val
   465                 470                 475                 480
   Val Asp Val Ser His Glu Asp Pro Glu Val Lys Phe Asn Trp Tyr Val
                   485                 490                 495
   Asp Gly Val Glu Val His Asn Ala Lys Thr Lys Pro Arg Glu Glu Gln
               500                 505                 510
   Tyr Asn Ser Thr Tyr Arg Val Val Ser Val Leu Thr Val Leu His Gln
           515                 520                 525
   Asp Trp Leu Asn Gly Lys Glu Tyr Lys Cys Lys Val Ser Asn Lys Ala
       530                 535                 540
   Leu Gly Ala Pro Ile Glu Lys Thr Ile Ser Lys Ala Lys Gly Gln Pro
   545                 550                 555                 560
   Arg Glu Pro Gln Val Cys Thr Leu Pro Pro Ser Arg Asp Glu Leu Thr
                   565                 570                 575
   Lys Asn Gln Val Ser Leu Ser Cys Ala Val Lys Gly Phe Tyr Pro Ser
               580                 585                 590
   Asp Ile Ala Val Glu Trp Glu Ser Asn Gly Gln Pro Glu Asn Asn Tyr
           595                 600                 605
   Lys Thr Thr Pro Pro Val Leu Asp Ser Asp Gly Ser Phe Phe Leu Val
       610                 615                 620
   Ser Lys Leu Thr Val Asp Lys Ser Arg Trp Gln Gln Gly Asn Val Phe
   625                 630                 635                 640
   Ser Cys Ser Val Met His Glu Ala Leu His Asn His Tyr Thr Gln Lys
   645 650 655
   Ser Leu Ser Leu Ser Pro Gly Lys
   660
<210> 30
   <211> 1995
   <212> DNA
   <213> Artificial Sequence
<220>
   <223> V9 (VL-CH1)-LC007 (VH-CH1)-Fc(hole) P329G LALA
<400> 30
   gatatccaga tgacccagag ccccagctct ctgagcgcca gcgtgggcga cagagtgacc      60
   atcacctgtc gggccagcca ggacatcaga aactacctga actggtatca gcagaagccc     120
   ggcaaggccc ccaagctgct gatctactac accagcagac tggaaagcgg cgtgccctcc     180
   agattttccg gcagcggctc cggcaccgac tacaccctga ccatcagcag cctgcagccc     240
   gaggatttcg ccacatatta ctgccagcag ggcaataccc tgccctggac cttcggacag     300
   ggcacaaaag tggaaatcaa gagcagcgct tccaccaaag gcccttccgt gtttcctctg     360
   gctcctagct ccaagtccac ctctggaggc accgctgctc tcggatgcct cgtgaaggat     420
   tattttcctg agcctgtgac agtgtcctgg aatagcggag cactgacctc tggagtgcat     480
   actttccccg ctgtgctgca gtcctctgga ctgtacagcc tgagcagcgt ggtgacagtg     540
   cccagcagca gcctgggcac ccagacctac atctgcaacg tgaaccacaa gcccagcaac     600
   accaaggtgg acaagaaggt ggaacccaag tcttgtggcg gaggcggatc cggcggaggg     660
   ggatctgagg tgcagctgca ggaaagcggc cctggcctgg tgaaacccag ccagagcctg     720
   agcctgacct gcagcgtgac cggctacagc atcaccagcg gctactactg gaactggatc     780
   agacagttcc ccggcaacaa gctggaatgg atgggctaca tcacctacga cggcagcaac     840
   aactacaacc ccagcctgaa gaacagaatc agcatcaccc gggacaccag caagaaccag     900
   ttcttcctga agctgaacag cgtgaccacc gaggacaccg ccacctacta ctgcgccgac     960
   ttcgactact ggggccaggg caccaccctg accgtgtcct ccgcctctac caagggcccc    1020
   agcgtgttcc ccctggcacc cagcagcaag agcacatctg gcggaacagc cgctctgggc    1080
   tgtctggtga aagactactt ccccgagccc gtgaccgtgt cttggaactc tggcgccctg    1140
   accagcggcg tgcacacctt tccagccgtg ctgcagagca gcggcctgta ctccctgtcc    1200
   tccgtggtca ccgtgccctc tagctccctg ggaacacaga catatatctg taatgtcaat    1260
   cacaagcctt ccaacaccaa agtcgataag aaagtcgagc ccaagagctg cgacaaaact    1320
   cacacatgcc caccgtgccc agcacctgaa gctgcagggg gaccgtcagt cttcctcttc    1380
   cccccaaaac ccaaggacac cctcatgatc tcccggaccc ctgaggtcac atgcgtggtg 1440
   gtggacgtga gccacgaaga ccctgaggtc aagttcaact ggtacgtgga cggcgtggag 1500
   gtgcataatg ccaagacaaa gccgcgggag gagcagtaca acagcacgta ccgtgtggtc 1560
   agcgtcctca ccgtcctgca ccaggactgg ctgaatggca aggagtacaa gtgcaaggtc 1620
   tccaacaaag ccctcggcgc ccccatcgag aaaaccatct ccaaagccaa agggcagccc 1680
   cgagaaccac aggtgtgcac cctgccccca tcccgggatg agctgaccaa gaaccaggtc 1740
   agcctctcgt gcgcagtcaa aggcttctat cccagcgaca tcgccgtgga gtgggagagc 1800
   aatgggcagc cggagaacaa ctacaagacc acgcctcccg tgctggactc cgacggctcc 1860
   ttcttcctcg tgagcaagct caccgtggac aagagcaggt ggcagcaggg gaacgtcttc 1920
   tcatgctccg tgatgcatga ggctctgcac aaccactaca cgcagaagag cctctccctg 1980
   tctccgggta aatga 1995
<210> 31
   <211> 229
   <212> PRT
   <213> Artificial Sequence
<220>
   <223> Fc(knob) wt
<400> 31
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Cys Arg Asp Glu Leu Thr Lys Asn Gln Val Ser
       130                 135                 140
   Leu Trp Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys Ser Gly
   225
<210> 32
   <211> 690
   <212> DNA
   <213> Artificial Sequence
<220>
   <223> Fc(knob) wt
<400> 32
   gacaaaactc acacatgccc accgtgccca gcacctgaac tcctgggggg accgtcagtc      60
   ttcctcttcc ccccaaaacc caaggacacc ctcatgatct cccggacccc tgaggtcaca     120
   tgcgtggtgg tggacgtgag ccacgaagac cctgaggtca agttcaactg gtacgtggac     180
   ggcgtggagg tgcataatgc caagacaaag ccgcgggagg agcagtacaa cagcacgtac     240
   cgtgtggtca gcgtcctcac cgtcctgcac caggactggc tgaatggcaa ggagtacaag     300
   tgcaaggtct ccaacaaagc cctcccagcc cccatcgaga aaaccatctc caaagccaaa     360
   gggcagcccc gagaaccaca ggtgtacacc ctgcccccat gccgggatga gctgaccaag     420
   aaccaggtca gcctgtggtg cctggtcaaa ggcttctatc ccagcgacat cgccgtggag     480
   tgggagagca atgggcagcc ggagaacaac tacaagacca cgcctcccgt gctggactcc     540
   gacggctcct tcttcctcta cagcaagctc accgtggaca agagcaggtg gcagcagggg     600
   aacgtcttct catgctccgt gatgcatgag gctctgcaca accactacac gcagaagagc     660
   ctctccctgt ctccgggtaa atccggatga                                      690
<210> 33
   <211> 229
   <212> PRT
   <213> Artificial Sequence
<220>
   <223> V9 (VH-CL)
<400> 33
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Ser Phe Thr Gly Tyr
   20 25 30
   Thr Met Asn Trp Val Arg Gln Ala Pro Gly Lys Gly Leu Glu Trp Val
   35 40 45
   Ala Leu Ile Asn Pro Tyr Lys Gly Val Ser Thr Tyr Asn Gln Lys Phe
   50 55 60
   Lys Asp Arg Phe Thr Ile Ser Val Asp Lys Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Ser Gly Tyr Tyr Gly Asp Ser Asp Trp Tyr Phe Asp Val Trp
   100 105 110
   Gly Gln Gly Thr Leu Val Thr Val Ser Ser Ala Ser Val Ala Ala Pro
   115 120 125
   Ser Val Phe Ile Phe Pro Pro Ser Asp Glu Gln Leu Lys Ser Gly Thr
   130 135 140
   Ala Ser Val Val Cys Leu Leu Asn Asn Phe Tyr Pro Arg Glu Ala Lys
   145 150 155 160
   Val Gln Trp Lys Val Asp Asn Ala Leu Gln Ser Gly Asn Ser Gln Glu
   165 170 175
   Ser Val Thr Glu Gln Asp Ser Lys Asp Ser Thr Tyr Ser Leu Ser Ser
   180 185 190
   Thr Leu Thr Leu Ser Lys Ala Asp Tyr Glu Lys His Lys Val Tyr Ala
   195 200 205
   Cys Glu Val Thr His Gln Gly Leu Ser Ser Pro Val Thr Lys Ser Phe
   210 215 220
   Asn Arg Gly Glu Cys
   225
<210> 34
   <211> 690
   <212> DNA
   <213> Artificial Sequence
<220>
   <223> V9 (VH-CL)
<400> 34
   gaggtgcagc tggtcgagag cggaggcggc ctggtgcagc ctggcggcag cctgagactg      60
   agctgcgccg ccagcggcta cagcttcacc ggctacacca tgaactgggt ccggcaggca     120
   cctggcaagg gactggaatg ggtggccctg atcaacccct acaagggcgt gagcacctac     180
   aaccagaagt tcaaggaccg gttcaccatc agcgtggaca agagcaagaa caccgcctat     240
   ctgcagatga acagcctgcg ggccgaggac accgccgtgt actactgcgc cagaagcggc     300
   tactacggcg acagcgactg gtacttcgac gtgtggggcc agggcaccct cgtgaccgtg     360
   tctagcgcta gcgtggctgc accatctgtc ttcatcttcc cgccatctga tgagcagttg     420
   aaatctggaa ctgcctctgt tgtgtgcctg ctgaataact tctatcccag agaggccaaa     480
   gtacagtgga aggtggataa cgccctccaa tcgggtaact cccaggagag tgtcacagag     540
   caggacagca aggacagcac ctacagcctc agcagcaccc tgacgctgag caaagcagac     600
   tacgagaaac acaaagtcta cgcctgcgaa gtcacccatc agggcctgag ctcgcccgtc     660
   acaaagagct tcaacagggg agagtgttga                                       690
<210> 35
   <211> 670
   <212> PRT
   <213> Artificial Sequence
<220>
   <223> FN18 (VL-CH1)-LC007 (VH-CH1)-Fc(hole) P329G LALA
<400> 35
   Asp Ile Val Met Ser Gln Ser Pro Ser Ser Leu Ala Val Ser Val Gly
   1               5                   10                  15
   Glu Lys Val Thr Met Ser Cys Lys Ser Ser Gln Ser Leu Leu Tyr Ser
               20                  25                  30
   Ser Asn Gln Lys Asn Tyr Leu Ala Trp Tyr Gln Gln Lys Pro Gly Gln
           35                  40                  45
   Ser Pro Lys Leu Leu Ile Asn Trp Ala Ser Thr Arg Glu Ser Gly Val
       50                  55                  60
   Pro Asp Arg Phe Thr Gly Ser Gly Ser Arg Thr Asp Phe Thr Leu Thr
   65                  70                  75                  80
   Ile Ser Ser Val Lys Ala Glu Asp Leu Ala Val Tyr Phe Cys Gln Gln
                   85                  90                  95
   Phe Tyr Ser Tyr Pro Pro Thr Phe Gly Gly Gly Thr Lys Leu Glu Ile
               100                 105                 110
   Lys Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Lys Val Glu Pro Lys Ser Cys Gly Gly Gly Gly Ser Gly
       210                 215                 220
   Gly Gly Gly Ser Glu Val Gln Leu Gln Glu Ser Gly Pro Gly Leu Val
   225                 230                 235                 240
   Lys Pro Ser Gln Ser Leu Ser Leu Thr Cys Ser Val Thr Gly Tyr Ser
                   245                 250                 255
   Ile Thr Ser Gly Tyr Tyr Trp Asn Trp Ile Arg Gln Phe Pro Gly Asn
               260                 265                 270
   Lys Leu Glu Trp Met Gly Tyr Ile Thr Tyr Asp Gly Ser Asn Asn Tyr
           275                 280                 285
   Asn Pro Ser Leu Lys Asn Arg Ile Ser Ile Thr Arg Asp Thr Ser Lys
       290                 295                 300
   Asn Gln Phe Phe Leu Lys Leu Asn Ser Val Thr Thr Glu Asp Thr Ala
   305                 310                 315                 320
   Thr Tyr Tyr Cys Ala Asp Phe Asp Tyr Trp Gly Gln Gly Thr Thr Leu
                   325                 330                 335
   Thr Val Ser Ser Ala Ser Thr Lys Gly Pro Ser Val Phe Pro Leu Ala
   340 345 350
   Pro Ser Ser Lys Ser Thr Ser Gly Gly Thr Ala Ala Leu Gly Cys Leu
   355 360 365
   Val Lys Asp Tyr Phe Pro Glu Pro Val Thr Val Ser Trp Asn Ser Gly
   370 375 380
   Ala Leu Thr Ser Gly Val His Thr Phe Pro Ala Val Leu Gln Ser Ser
   385 390 395 400
   Gly Leu Tyr Ser Leu Ser Ser Val Val Thr Val Pro Ser Ser Ser Leu
   405 410 415
   Gly Thr Gln Thr Tyr Ile Cys Asn Val Asn His Lys Pro Ser Asn Thr
   420 425 430
   Lys Val Asp Lys Lys Val Glu Pro Lys Ser Cys Asp Lys Thr His Thr
   435 440 445
   Cys Pro Pro Cys Pro Ala Pro Glu Ala Ala Gly Gly Pro Ser Val Phe
   450 455 460
   Leu Phe Pro Pro Lys Pro Lys Asp Thr Leu Met Ile Ser Arg Thr Pro
   465 470 475 480
   Glu Val Thr Cys Val Val Val Asp Val Ser His Glu Asp Pro Glu Val
   485 490 495
   Lys Phe Asn Trp Tyr Val Asp Gly Val Glu Val His Asn Ala Lys Thr
   500 505 510
   Lys Pro Arg Glu Glu Gln Tyr Asn Ser Thr Tyr Arg Val Val Ser Val
   515 520 525
   Leu Thr Val Leu His Gln Asp Trp Leu Asn Gly Lys Glu Tyr Lys Cys
   530 535 540
   Lys Val Ser Asn Lys Ala Leu Gly Ala Pro Ile Glu Lys Thr Ile Ser
   545 550 555 560
   Lys Ala Lys Gly Gln Pro Arg Glu Pro Gln Val Cys Thr Leu Pro Pro
   565 570 575
   Ser Arg Asp Glu Leu Thr Lys Asn Gln Val Ser Leu Ser Cys Ala Val
   580 585 590
   Lys Gly Phe Tyr Pro Ser Asp Ile Ala Val Glu Trp Glu Ser Asn Gly
   595 600 605
   Gln Pro Glu Asn Asn Tyr Lys Thr Thr Pro Pro Val Leu Asp Ser Asp
   610 615 620
   Gly Ser Phe Phe Leu Val Ser Lys Leu Thr Val Asp Lys Ser Arg Trp
   625 630 635 640
   Gln Gln Gly Asn Val Phe Ser Cys Ser Val Met His Glu Ala Leu His
   645 650 655
   Asn His Tyr Thr Gln Lys Ser Leu Ser Leu Ser Pro Gly Lys
   660 665 670
<210> 36
   <211> 2013
   <212> DNA
   <213> Artificial Sequence
<220>
   <223> FN18 (VL-CH1)-LC007 (VH-CH1)-Fc(hole) P329G LALA
<400> 36
   gacatcgtga tgagccagag ccccagcagc ctggccgtgt ccgtgggcga gaaagtgacc      60
   atgagctgca agagcagcca gagcctgctg tactcctcta accagaagaa ctacctggcc     120
   tggtatcagc agaagcccgg ccagtccccc aagctgctga tcaactgggc cagcacccgc     180
   gagagcggcg tgcccgatag attcacaggc agcggcagcc ggaccgactt caccctgacc     240
   atcagcagcg tgaaggccga ggatctggcc gtgtacttct gccagcagtt ctacagctac     300
   ccccccacct tcggcggagg cacgaagctg gaaatcaaga gcagcgcttc caccaaaggc     360
   ccttccgtgt ttcctctggc tcctagctcc aagtccacct ctggaggcac cgctgctctc     420
   ggatgcctcg tgaaggatta ttttcctgag cctgtgacag tgtcctggaa tagcggagca     480
   ctgacctctg gagtgcatac tttccccgct gtgctgcagt cctctggact gtacagcctg     540
   agcagcgtgg tgacagtgcc cagcagcagc ctgggcaccc agacctacat ctgcaacgtg     600
   aaccacaagc ccagcaacac caaggtggac aagaaggtgg aacccaagtc ttgtggcgga     660
   ggcggatccg gcggaggggg atctgaggtg cagctgcagg aaagcggccc tggcctggtg     720
   aaacccagcc agagcctgag cctgacctgc agcgtgaccg gctacagcat caccagcggc     780
   tactactgga actggatcag acagttcccc ggcaacaagc tggaatggat gggctacatc     840
   acctacgacg gcagcaacaa ctacaacccc agcctgaaga acagaatcag catcacccgg     900
   gacaccagca agaaccagtt cttcctgaag ctgaacagcg tgaccaccga ggacaccgcc     960
   acctactact gcgccgactt cgactactgg ggccagggca ccaccctgac cgtgtcctcc    1020
   gcctctacca agggccccag cgtgttcccc ctggcaccca gcagcaagag cacatctggc    1080
   ggaacagccg ctctgggctg tctggtgaaa gactacttcc ccgagcccgt gaccgtgtct    1140
   tggaactctg gcgccctgac cagcggcgtg cacacctttc cagccgtgct gcagagcagc      1200
   ggcctgtact ccctgtcctc cgtggtcacc gtgccctcta gctccctggg aacacagaca      1260
   tatatctgta atgtcaatca caagccttcc aacaccaaag tcgataagaa agtcgagccc      1320
   aagagctgcg acaaaactca cacatgccca ccgtgcccag cacctgaagc tgcaggggga      1380
   ccgtcagtct tcctcttccc cccaaaaccc aaggacaccc tcatgatctc ccggacccct      1440
   gaggtcacat gcgtggtggt ggacgtgagc cacgaagacc ctgaggtcaa gttcaactgg      1500
   tacgtggacg gcgtggaggt gcataatgcc aagacaaagc cgcgggagga gcagtacaac      1560
   agcacgtacc gtgtggtcag cgtcctcacc gtcctgcacc aggactggct gaatggcaag      1620
   gagtacaagt gcaaggtctc caacaaagcc ctcggcgccc ccatcgagaa aaccatctcc      1680
   aaagccaaag ggcagccccg agaaccacag gtgtgcaccc tgcccccatc ccgggatgag      1740
   ctgaccaaga accaggtcag cctctcgtgc gcagtcaaag gcttctatcc cagcgacatc      1800
   gccgtggagt gggagagcaa tgggcagccg gagaacaact acaagaccac gcctcccgtg      1860
   ctggactccg acggctcctt cttcctcgtg agcaagctca ccgtggacaa gagcaggtgg      1920
   cagcagggga acgtcttctc atgctccgtg atgcatgagg ctctgcacaa ccactacacg      1980
   cagaagagcc tctccctgtc tccgggtaaa tga                                   2013
<210> 37
   <211> 231
   <212> PRT
   <213> Artificial Sequence
<220>
   <223> FN18 (VH-CL)
<400> 37
   Gln Val Gln Leu Gln Gln Ser Glu Ala Glu Leu Ala Arg Pro Gly Ala
   1               5                   10                  15
   Ser Val Lys Met Ser Cys Lys Ala Ser Gly Tyr Thr Phe Thr Asp Tyr
               20                  25                  30
   Thr Ile His Trp Leu Lys Gln Arg Pro Gly Gln Gly Leu Asp Trp Ile
           35                  40                  45
   Gly Tyr Phe Asn Pro Ser Ser Glu Ser Thr Glu Tyr Asn Arg Lys Phe
       50                  55                  60
   Lys Asp Arg Thr Ile Leu Thr Ala Asp Arg Ser Ser Thr Thr Ala Tyr
   65                  70                  75                  80
   Met Gln Leu Ser Ser Leu Thr Ser Glu Asp Ser Ala Val Tyr Tyr Cys
                   85                  90                  95
   Ser Arg Lys Gly Glu Lys Leu Leu Gly Asn Arg Tyr Trp Tyr Phe Asp
               100                 105                 110
   Val Trp Gly Ala Gly Thr Ser Val Thr Val Ser Ser Ala Ser Val Ala
           115                 120                 125
   Ala Pro Ser Val Phe Ile Phe Pro Pro Ser Asp Glu Gln Leu Lys Ser
       130                 135                 140
   Gly Thr Ala Ser Val Val Cys Leu Leu Asn Asn Phe Tyr Pro Arg Glu
   145                 150                 155                 160
   Ala Lys Val Gln Trp Lys Val Asp Asn Ala Leu Gln Ser Gly Asn Ser
                   165                 170                 175
   Gln Glu Ser Val Thr Glu Gln Asp Ser Lys Asp Ser Thr Tyr Ser Leu
               180                 185                 190
   Ser Ser Thr Leu Thr Leu Ser Lys Ala Asp Tyr Glu Lys His Lys Val
           195                 200                 205
   Tyr Ala Cys Glu Val Thr His Gln Gly Leu Ser Ser Pro Val Thr Lys
       210                 215                 220
   Ser Phe Asn Arg Gly Glu Cys
   225                 230
<210> 38
   <211> 696
   <212> DNA
   <213> Artificial Sequence
<220>
   <223> FN18 (VH-CL)
<400> 38
   caggtgcagc tgcagcagag cgaggccgag ctggctagac ctggagccag cgtgaagatg        60
   agctgcaagg ccagcggcta caccttcacc gactacacca tccactggct gaagcagcgg       120
   cctggacagg gcctggactg gatcggctac ttcaacccca gcagcgagag caccgagtac       180
   aaccggaagt tcaaggaccg gaccatcctg accgccgaca gaagcagcac caccgcctac       240
   atgcagctga gcagcctgac cagcgaggac agcgccgtgt actactgcag ccggaagggc       300
   gagaagctgc tgggcaacag atactggtac ttcgacgtgt ggggagccgg caccagcgtg       360
   accgtgtcta gcgctagcgt ggctgcacca tctgtcttca tcttcccgcc atctgatgag       420
   cagttgaaat ctggaactgc ctctgttgtg tgcctgctga ataacttcta tcccagagag       480
   gccaaagtac agtggaaggt ggataacgcc ctccaatcgg gtaactccca ggagagtgtc       540
   acagagcagg acagcaagga cagcacctac agcctcagca gcaccctgac gctgagcaaa       600
   gcagactacg agaaacacaa agtctacgcc tgcgaagtca cccatcaggg cctgagctcg       660
   cccgtcacaa agagcttcaa caggggagag tgttga                                 696
<210> 39
   <211> 664
   <212> PRT
   <213> Artificial Sequence
<220>
   <223> 2C11 (VL-CH1)-LC007 (VH-CH1)-Fc(hole) P329G LALA
<400> 39
   Asp Ile Gln Met Thr Gln Ser Pro Ser Ser Leu Pro Ala Ser Leu Gly
   1               5                   10                  15
   Asp Arg Val Thr Ile Asn Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Asn Lys Leu Ala Asp Gly Val Pro Ser Arg Phe Ser Gly
       50                  55                  60
   Ser Gly Ser Gly Arg Asp Ser Ser Phe Thr Ile Ser Ser Leu Glu Ser
   65                  70                  75                  80
   Glu Asp Ile Gly Ser Tyr Tyr Cys Gln Gln Tyr Tyr Asn Tyr Pro Trp
                   85                  90                  95
   Thr Phe Gly Pro Gly Thr Lys Leu Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Gly Gly Gly Gly Ser Gly Gly Gly Gly Ser Glu Val
       210                 215                 220
   Gln Leu Gln Glu Ser Gly Pro Gly Leu Val Lys Pro Ser Gln Ser Leu
   225                 230                 235                 240
   Ser Leu Thr Cys Ser Val Thr Gly Tyr Ser Ile Thr Ser Gly Tyr Tyr
                   245                 250                 255
   Trp Asn Trp Ile Arg Gln Phe Pro Gly Asn Lys Leu Glu Trp Met Gly
               260                 265                 270
   Tyr Ile Thr Tyr Asp Gly Ser Asn Asn Tyr Asn Pro Ser Leu Lys Asn
           275                 280                 285
   Arg Ile Ser Ile Thr Arg Asp Thr Ser Lys Asn Gln Phe Phe Leu Lys
       290                 295                 300
   Leu Asn Ser Val Thr Thr Glu Asp Thr Ala Thr Tyr Tyr Cys Ala Asp
   305                 310                 315                 320
   Phe Asp Tyr Trp Gly Gln Gly Thr Thr Leu Thr Val Ser Ser Ala Ser
                   325                 330                 335
   Thr Lys Gly Pro Ser Val Phe Pro Leu Ala Pro Ser Ser Lys Ser Thr
               340                 345                 350
   Ser Gly Gly Thr Ala Ala Leu Gly Cys Leu Val Lys Asp Tyr Phe Pro
           355                 360                 365
   Glu Pro Val Thr Val Ser Trp Asn Ser Gly Ala Leu Thr Ser Gly Val
       370                 375                 380
   His Thr Phe Pro Ala Val Leu Gln Ser Ser Gly Leu Tyr Ser Leu Ser
   385                 390                 395                 400
   Ser Val Val Thr Val Pro Ser Ser Ser Leu Gly Thr Gln Thr Tyr Ile
                   405                 410                 415
   Cys Asn Val Asn His Lys Pro Ser Asn Thr Lys Val Asp Lys Lys Val
               420                 425                 430
   Glu Pro Lys Ser Cys Asp Lys Thr His Thr Cys Pro Pro Cys Pro Ala
           435                 440                 445
   Pro Glu Ala Ala Gly Gly Pro Ser Val Phe Leu Phe Pro Pro Lys Pro
       450                 455                 460
   Lys Asp Thr Leu Met Ile Ser Arg Thr Pro Glu Val Thr Cys Val Val
   465 470 475 480
   Val Asp Val Ser His Glu Asp Pro Glu Val Lys Phe Asn Trp Tyr Val
   485 490 495
   Asp Gly Val Glu Val His Asn Ala Lys Thr Lys Pro Arg Glu Glu Gln
   500 505 510
   Tyr Asn Ser Thr Tyr Arg Val Val Ser Val Leu Thr Val Leu His Gln
   515 520 525
   Asp Trp Leu Asn Gly Lys Glu Tyr Lys Cys Lys Val Ser Asn Lys Ala
   530 535 540
   Leu Gly Ala Pro Ile Glu Lys Thr Ile Ser Lys Ala Lys Gly Gln Pro
   545 550 555 560
   Arg Glu Pro Gln Val Cys Thr Leu Pro Pro Ser Arg Asp Glu Leu Thr
   565 570 575
   Lys Asn Gln Val Ser Leu Ser Cys Ala Val Lys Gly Phe Tyr Pro Ser
   580 585 590
   Asp Ile Ala Val Glu Trp Glu Ser Asn Gly Gln Pro Glu Asn Asn Tyr
   595 600 605
   Lys Thr Thr Pro Pro Val Leu Asp Ser Asp Gly Ser Phe Phe Leu Val
   610 615 620
   Ser Lys Leu Thr Val Asp Lys Ser Arg Trp Gln Gln Gly Asn Val Phe
   625 630 635 640
   Ser Cys Ser Val Met His Glu Ala Leu His Asn His Tyr Thr Gln Lys
   645 650 655
   Ser Leu Ser Leu Ser Pro Gly Lys
   660
<210> 40
   <211> 1995
   <212> DNA
   <213> Artificial Sequence
<220>
   <223> 2C11 (VL-CH1)-LC007 (VH-CH1)-Fc(hole) P329G LALA
<400> 40
   gacatccaga tgacccagag ccccagcagc ctgcctgcca gcctgggcga cagagtgacc 60
   atcaactgcc aggccagcca ggacatcagc aactacctga actggtatca gcagaagcct     120
   ggcaaggccc ccaagctgct gatctactac accaacaagc tggccgacgg cgtgcccagc     180
   agattcagcg gcagcggctc cggcagagac agcagcttca ccatctccag cctggaaagc     240
   gaggacatcg gcagctacta ctgccagcag tactacaact acccctggac cttcggccct     300
   ggcaccaagc tggaaatcaa gagcagcgct tccaccaaag gcccttccgt gtttcctctg     360
   gctcctagct ccaagtccac ctctggaggc accgctgctc tcggatgcct cgtgaaggat     420
   tattttcctg agcctgtgac agtgtcctgg aatagcggag cactgacctc tggagtgcat     480
   actttccccg ctgtgctgca gtcctctgga ctgtacagcc tgagcagcgt ggtgacagtg     540
   cccagcagca gcctgggcac ccagacctac atctgcaacg tgaaccacaa gcccagcaac     600
   accaaggtgg acaagaaggt ggaacccaag tcttgtggcg gaggcggatc cggcggaggg     660
   ggatctgagg tgcagctgca ggaaagcggc cctggcctgg tgaaacccag ccagagcctg     720
   agcctgacct gcagcgtgac cggctacagc atcaccagcg gctactactg gaactggatc     780
   agacagttcc ccggcaacaa gctggaatgg atgggctaca tcacctacga cggcagcaac     840
   aactacaacc ccagcctgaa gaacagaatc agcatcaccc gggacaccag caagaaccag     900
   ttcttcctga agctgaacag cgtgaccacc gaggacaccg ccacctacta ctgcgccgac     960
   ttcgactact ggggccaggg caccaccctg accgtgtcct ccgcctctac caagggcccc    1020
   agcgtgttcc ccctggcacc cagcagcaag agcacatctg gcggaacagc cgctctgggc    1080
   tgtctggtga aagactactt ccccgagccc gtgaccgtgt cttggaactc tggcgccctg    1140
   accagcggcg tgcacacctt tccagccgtg ctgcagagca gcggcctgta ctccctgtcc    1200
   tccgtggtca ccgtgccctc tagctccctg ggaacacaga catatatctg taatgtcaat    1260
   cacaagcctt ccaacaccaa agtcgataag aaagtcgagc ccaagagctg cgacaaaact    1320
   cacacatgcc caccgtgccc agcacctgaa gctgcagggg gaccgtcagt cttcctcttc    1380
   cccccaaaac ccaaggacac cctcatgatc tcccggaccc ctgaggtcac atgcgtggtg    1440
   gtggacgtga gccacgaaga ccctgaggtc aagttcaact ggtacgtgga cggcgtggag    1500
   gtgcataatg ccaagacaaa gccgcgggag gagcagtaca acagcacgta ccgtgtggtc    1560
   agcgtcctca ccgtcctgca ccaggactgg ctgaatggca aggagtacaa gtgcaaggtc    1620
   tccaacaaag ccctcggcgc ccccatcgag aaaaccatct ccaaagccaa agggcagccc    1680
   cgagaaccac aggtgtgcac cctgccccca tcccgggatg agctgaccaa gaaccaggtc    1740
   agcctctcgt gcgcagtcaa aggcttctat cccagcgaca tcgccgtgga gtgggagagc    1800
   aatgggcagc cggagaacaa ctacaagacc acgcctcccg tgctggactc cgacggctcc    1860
   ttcttcctcg tgagcaagct caccgtggac aagagcaggt ggcagcaggg gaacgtcttc    1920
   tcatgctccg tgatgcatga ggctctgcac aaccactaca cgcagaagag cctctccctg    1980
   tctccgggta aatga                                                     1995
<210> 41
   <211> 223
   <212> PRT
   <213> Artificial Sequence
<220>
   <223> 2C11 (VH-CL)
<400> 41
   Glu Val Gln Leu Val Glu Ser Gly Gly Gly Leu Val Gln Pro Gly Lys
   1               5                   10                  15
   Ser Leu Lys Leu Ser Cys Glu Ala Ser Gly Phe Thr Phe Ser Gly Tyr
               20                  25                  30
   Gly Met His Trp Val Arg Gln Ala Pro Gly Arg Gly Leu Glu Ser Val
           35                  40                  45
   Ala Tyr Ile Thr Ser Ser Ser Ile Asn Ile Lys Tyr Ala Asp Ala Val
       50                  55                  60
   Lys Gly Arg Phe Thr Val Ser Arg Asp Asn Ala Lys Asn Leu Leu Phe
   65                  70                  75                  80
   Leu Gln Met Asn Ile Leu Lys Ser Glu Asp Thr Ala Met Tyr Tyr Cys
                   85                  90                  95
   Ala Arg Phe Asp Trp Asp Lys Asn Tyr Trp Gly Gln Gly Thr Met Val
               100                 105                 110
   Thr Val Ser Ser Ala Ser Val Ala Ala Pro Ser Val Phe Ile Phe Pro
           115                 120                 125
   Pro Ser Asp Glu Gln Leu Lys Ser Gly Thr Ala Ser Val Val Cys Leu
       130                 135                 140
   Leu Asn Asn Phe Tyr Pro Arg Glu Ala Lys Val Gln Trp Lys Val Asp
   145                 150                 155                 160
   Asn Ala Leu Gln Ser Gly Asn Ser Gln Glu Ser Val Thr Glu Gln Asp
                   165                 170                 175
   Ser Lys Asp Ser Thr Tyr Ser Leu Ser Ser Thr Leu Thr Leu Ser Lys
               180                 185                 190
   Ala Asp Tyr Glu Lys His Lys Val Tyr Ala Cys Glu Val Thr His Gln
           195                 200                 205
   Gly Leu Ser Ser Pro Val Thr Lys Ser Phe Asn Arg Gly Glu Cys
             210                 215                 220
<210> 42
   <211> 672
   <212> DNA
   <213> Artificial Sequence
<220>
   <223> 2C11 (VH-CL)
<400> 42
   gaggtgcagc tggtggaaag cggcggaggc ctggtgcagc ccggcaagag cctgaagctg       60
   agctgcgagg ccagcggctt caccttcagc ggctacggca tgcactgggt gagacaggcc      120
   cctggcagag gactggaaag cgtggcctac atcaccagca gcagcatcaa cattaagtac      180
   gccgacgccg tgaagggccg gttcaccgtg tccagggata acgccaagaa cctgctgttc      240
   ctgcagatga acatcctgaa gtccgaggac accgctatgt attactgcgc cagattcgac      300
   tgggacaaga actactgggg ccagggcacc atggtcacag tgtctagcgc tagcgtggct      360
   gcaccatctg tcttcatctt cccgccatct gatgagcagt tgaaatctgg aactgcctct      420
   gttgtgtgcc tgctgaataa cttctatccc agagaggcca aagtacagtg gaaggtggat      480
   aacgccctcc aatcgggtaa ctcccaggag agtgtcacag agcaggacag caaggacagc      540
   acctacagcc tcagcagcac cctgacgctg agcaaagcag actacgagaa acacaaagtc      600
   tacgcctgcg aagtcaccca tcagggcctg agctcgcccg tcacaaagag cttcaacagg      660
   ggagagtgtt ga                                                          672
<210> 43
   <211> 700
   <212> PRT
   <213> Artificial Sequence
<220>
   <223> V9 (scFab)-Fc(knob) P329G LALA
<400> 43
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Ser Gly Gly Gly Ser Glu
   210 215 220
   Gly Gly Gly Ser Glu Gly Gly Gly Ser Glu Gly Gly Gly Ser Glu Gly
   225 230 235 240
   Gly Gly Ser Gly Gly Gly Ser Gly Glu Val Gln Leu Val Glu Ser Gly
   245 250 255
   Gly Gly Leu Val Gln Pro Gly Gly Ser Leu Arg Leu Ser Cys Ala Ala
   260 265 270
   Ser Gly Tyr Ser Phe Thr Gly Tyr Thr Met Asn Trp Val Arg Gln Ala
   275 280 285
   Pro Gly Lys Gly Leu Glu Trp Val Ala Leu Ile Asn Pro Tyr Lys Gly
   290 295 300
   Val Ser Thr Tyr Asn Gln Lys Phe Lys Asp Arg Phe Thr Ile Ser Val
   305 310 315 320
   Asp Lys Ser Lys Asn Thr Ala Tyr Leu Gln Met Asn Ser Leu Arg Ala
   325 330 335
   Glu Asp Thr Ala Val Tyr Tyr Cys Ala Arg Ser Gly Tyr Tyr Gly Asp
               340                 345                 350
   Ser Asp Trp Tyr Phe Asp Val Trp Gly Gln Gly Thr Leu Val Thr Val
           355                 360                 365
   Ser Ser Ala Ser Thr Lys Gly Pro Ser Val Phe Pro Leu Ala Pro Ser
       370                 375                 380
   Ser Lys Ser Thr Ser Gly Gly Thr Ala Ala Leu Gly Cys Leu Val Lys
   385                 390                 395                 400
   Asp Tyr Phe Pro Glu Pro Val Thr Val Ser Trp Asn Ser Gly Ala Leu
                   405                 410                 415
   Thr Ser Gly Val His Thr Phe Pro Ala Val Leu Gln Ser Ser Gly Leu
               420                 425                 430
   Tyr Ser Leu Ser Ser Val Val Thr Val Pro Ser Ser Ser Leu Gly Thr
           435                 440                 445
   Gln Thr Tyr Ile Cys Asn Val Asn His Lys Pro Ser Asn Thr Lys Val
       450                 455                 460
   Asp Lys Lys Val Glu Pro Lys Ser Cys Asp Lys Thr His Thr Cys Pro
   465                 470                 475                 480
   Pro Cys Pro Ala Pro Glu Ala Ala Gly Gly Pro Ser Val Phe Leu Phe
                   485                 490                 495
   Pro Pro Lys Pro Lys Asp Thr Leu Met Ile Ser Arg Thr Pro Glu Val
               500                 505                 510
   Thr Cys Val Val Val Asp Val Ser His Glu Asp Pro Glu Val Lys Phe
           515                 520                 525
   Asn Trp Tyr Val Asp Gly Val Glu Val His Asn Ala Lys Thr Lys Pro
       530                 535                 540
   Arg Glu Glu Gln Tyr Asn Ser Thr Tyr Arg Val Val Ser Val Leu Thr
   545                 550                 555                 560
   Val Leu His Gln Asp Trp Leu Asn Gly Lys Glu Tyr Lys Cys Lys Val
                   565                 570                 575
   Ser Asn Lys Ala Leu Gly Ala Pro Ile Glu Lys Thr Ile Ser Lys Ala
               580                 585                 590
   Lys Gly Gln Pro Arg Glu Pro Gln Val Tyr Thr Leu Pro Pro Cys Arg
   595 600 605
   Asp Glu Leu Thr Lys Asn Gln Val Ser Leu Trp Cys Leu Val Lys Gly
   610 615 620
   Phe Tyr Pro Ser Asp Ile Ala Val Glu Trp Glu Ser Asn Gly Gln Pro
   625 630 635 640
   Glu Asn Asn Tyr Lys Thr Thr Pro Pro Val Leu Asp Ser Asp Gly Ser
   645 650 655
   Phe Phe Leu Tyr Ser Lys Leu Thr Val Asp Lys Ser Arg Trp Gln Gln
   660 665 670
   Gly Asn Val Phe Ser Cys Ser Val Met His Glu Ala Leu His Asn His
   675 680 685
   Tyr Thr Gln Lys Ser Leu Ser Leu Ser Pro Gly Lys
   690 695 700
<210> 44
   <211> 2103
   <212> DNA
   <213> Artificial Sequence
<220>
   <223> V9 (scFab)-Fc(knob) P329G LALA
<400> 44
   gacatccaga tgacccagag cccctctagc ctgagcgcca gcgtgggcga cagagtgacc      60
   atcacctgtc gggccagcca ggacatcaga aactacctga actggtatca gcagaagccc     120
   ggcaaggccc ccaagctgct gatctactac acctctagac tggaaagcgg cgtgcccagc     180
   cggtttagcg gcagcggctc cggcaccgac tacaccctga ccatcagcag cctgcagccc     240
   gaggacttcg ccacctacta ctgccagcag ggcaacacac tcccctggac cttcggccag     300
   ggcaccaagg tggagatcaa gcgtacggtg gccgctccca gcgtgttcat cttccccccc     360
   agcgacgagc agctgaagtc cggcaccgcc agcgtcgtgt gcctgctgaa caacttctac     420
   ccccgggagg ccaaggtgca gtggaaggtg gacaacgccc tgcagagcgg caacagccag     480
   gaaagcgtca ccgagcagga cagcaaggac tccacctaca gcctgagcag caccctgacc     540
   ctgagcaagg ccgactacga gaagcacaag gtgtacgcct gcgaagtgac ccaccagggc     600
   ctgtccagcc ccgtgaccaa gagcttcaac cggggcgagt gcagcggcgg aggctctgga     660
   ggcggctctg aaggcggagg aagtgagggc ggaggctcag aaggcggcgg aagcgaaggt     720
   ggcggctctg gcggcggatc cggcgaggtg cagctggtcg agtccggcgg aggcctggtg     780
   cagcctggcg gcagcctgag actgagctgc gccgccagcg gctacagctt caccggctac     840
   accatgaact gggtccggca ggctcctggc aagggcctcg aatgggtggc cctgatcaac 900
   ccctacaagg gcgtgagcac ctacaaccag aagttcaagg accggttcac catcagcgtg 960
   gacaagagca agaacaccgc ctatctgcag atgaacagcc tgcgggccga ggacaccgcc 1020
   gtgtactact gcgccagaag cggctactac ggcgacagcg actggtactt cgacgtgtgg 1080
   ggccagggca cactggtcac cgtgtccagc gctagcacca agggccctag cgtgttccct 1140
   ctggccccta gcagcaagag cacaagtgga ggaacagccg ccctgggctg cctggtcaag 1200
   gactacttcc ccgagcccgt gaccgtgtcc tggaattctg gcgccctgac aagcggcgtg 1260
   cacacatttc cagccgtgct gcagagcagc ggcctgtact ctctgagcag cgtcgtgacc 1320
   gtgccctcta gctctctggg cacccagacc tacatctgca acgtgaacca caagcccagc 1380
   aacaccaaag tggacaagaa ggtggaaccc aagagctgcg acaagaccca cacctgtccc 1440
   ccttgccctg cccctgaagc tgctggtggc ccttccgtgt tcctgttccc cccaaagccc 1500
   aaggacaccc tgatgatcag ccggaccccc gaagtgacct gcgtggtggt cgatgtgtcc 1560
   cacgaggacc ctgaagtgaa gttcaattgg tacgtggacg gcgtggaagt gcacaatgcc 1620
   aagaccaagc cgcgggagga gcagtacaac agcacgtacc gtgtggtcag cgtcctcacc 1680
   gtcctgcacc aggactggct gaatggcaag gagtacaagt gcaaggtctc caacaaagcc 1740
   ctcggcgccc ccatcgagaa aaccatctcc aaagccaaag ggcagccccg agaaccacag 1800
   gtgtacaccc tgcccccatg ccgggatgag ctgaccaaga accaggtcag cctgtggtgc 1860
   ctggtcaaag gcttctatcc cagcgacatc gccgtggagt gggagagcaa tgggcagccg 1920
   gagaacaact acaagaccac gcctcccgtg ctggactccg acggctcctt cttcctctac 1980
   agcaagctca ccgtggacaa gagcaggtgg cagcagggga acgtcttctc atgctccgtg 2040
   atgcatgagg ctctgcacaa ccactacacg cagaagagcc tctccctgtc tccgggtaaa 2100
   taa 2103
<210> 45
   <211> 450
   <212> PRT
   <213> Artificial Sequence
<220>
   <223> GA201 (VH-CH1)-Fc(hole) P329G LALA
<400> 45
   Gln Val Gln Leu Val Gln Ser Gly Ala Glu Val Lys Lys Pro Gly Ser
   1               5                   10                  15
   Ser Val Lys Val Ser Cys Lys Ala Ser Gly Phe Thr Phe Thr Asp Tyr
               20                  25                  30
   Lys Ile His Trp Val Arg Gln Ala Pro Gly Gln Gly Leu Glu Trp Met
           35                  40                  45
   Gly Tyr Phe Asn Pro Asn Ser Gly Tyr Ser Thr Tyr Ala Gln Lys Phe
       50                  55                  60
   Gln Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Leu Ser Pro Gly Gly Tyr Tyr Val Met Asp Ala Trp Gly Gln
               100                 105                 110
   Gly Thr Thr Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Gly Ala Pro Ile Glu
                   325                 330                 335
   Lys Thr Ile Ser Lys Ala Lys Gly Gln Pro Arg Glu Pro Gln Val Cys
               340                 345                 350
   Thr Leu Pro Pro Ser Arg Asp Glu Leu Thr Lys Asn Gln Val Ser Leu
           355                 360                 365
   Ser Cys Ala Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Val Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 46
   <211> 1353
   <212> DNA
   <213> Artificial Sequence
<220>
   <223> GA201 (VH-CH1)-Fc(hole) P329G LALA
<400> 46
   caggtgcagc tggtgcagtc tggggctgag gtgaagaagc ctgggtcctc ggtgaaggtc     60
   tcctgcaagg cctctggttt cacattcact gactacaaga tacactgggt gcgacaggcc    120
   cctggacaag ggctcgagtg gatgggatat ttcaacccta acagcggtta tagtacctac    180
   gcacagaagt tccagggcag ggtcaccatt accgcggaca aatccacgag cacagcctac    240
   atggagctga gcagcctgag atctgaggac acggccgtgt attactgtgc gagactatcc    300
   ccaggcggtt actatgttat ggatgcctgg ggccaaggga ccaccgtgac cgtctcctca    360
   gctagcacca agggcccatc ggtcttcccc ctggcaccct cctccaagag cacctctggg    420
   ggcacagcgg ccctgggctg cctggtcaag gactacttcc ccgaaccggt gacggtgtcg    480
   tggaactcag gcgccctgac cagcggcgtg cacaccttcc cggctgtcct acagtcctca    540
   ggactctact ccctcagcag cgtggtgacc gtgccctcca gcagcttggg cacccagacc    600
   tacatctgca acgtgaatca caagcccagc aacaccaagg tggacaagaa agttgagccc    660
   aaatcttgtg acaaaactca cacatgccca ccgtgcccag cacctgaagc tgcaggggga    720
   ccgtcagtct tcctcttccc cccaaaaccc aaggacaccc tcatgatctc ccggacccct    780
   gaggtcacat gcgtggtggt ggacgtgagc cacgaagacc ctgaggtcaa gttcaactgg    840
   tacgtggacg gcgtggaggt gcataatgcc aagacaaagc cgcgggagga gcagtacaac    900
   agcacgtacc gtgtggtcag cgtcctcacc gtcctgcacc aggactggct gaatggcaag    960
   gagtacaagt gcaaggtctc caacaaagcc ctcggcgccc ccatcgagaa aaccatctcc   1020
   aaagccaaag ggcagccccg agaaccacag gtgtgcaccc tgcccccatc ccgggatgag   1080
   ctgaccaaga accaggtcag cctctcgtgc gcagtcaaag gcttctatcc cagcgacatc   1140
   gccgtggagt gggagagcaa tgggcagccg gagaacaact acaagaccac gcctcccgtg   1200
   ctggactccg acggctcctt cttcctcgtg agcaagctca ccgtggacaa gagcaggtgg   1260
   cagcagggga acgtcttctc atgctccgtg atgcatgagg ctctgcacaa ccactacacg   1320
   cagaagagcc tctccctgtc tccgggtaaa tga                                1353
<210> 47
   <211> 213
   <212> PRT
   <213> Artificial Sequence
<220>
   <223> GA201 (VL-CL)
<400> 47
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Asn Asn Tyr
               20                  25                  30
   Leu Asn Trp Tyr Gln Gln Lys Pro Gly Lys Ala Pro Lys Arg Leu Ile
           35                  40                  45
   Tyr Asn Thr Asn Asn Leu Gln Thr Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Phe Pro Thr
                   85                  90                  95
   Phe Gly Gln Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 48
   <211> 642
   <212> DNA
   <213> Artificial Sequence
<220>
   <223> GA201 (VL-CL)
<400> 48
   gatatccaga tgacccagtc tccatcctcc ctgtctgcat ctgtcggaga ccgggtcacc      60
   atcacctgcc gggcaagtca gggcattaac aattacttaa attggtacca gcagaagcca     120
   gggaaagccc ctaagcgcct gatctataat accaacaact tgcagacagg cgtcccatca     180
   aggttcagcg gcagtggatc cgggacagaa ttcactctca ccatcagcag cctgcagcct     240
   gaagattttg ccacctatta ctgcttgcag cataatagtt ttcccacgtt tggccagggc     300
   accaagctcg agatcaagcg tacggtggct gcaccatctg tcttcatctt cccgccatct     360
   gatgagcagt tgaaatctgg aactgcctct gttgtgtgcc tgctgaataa cttctatccc     420
   agagaggcca aagtacagtg gaaggtggat aacgccctcc aatcgggtaa ctcccaggag     480
   agtgtcacag agcaggacag caaggacagc acctacagcc tcagcagcac cctgacgctg     540
   agcaaagcag actacgagaa acacaaagtc tacgcctgcg aagtcaccca tcagggcctg     600
   agctcgcccg tcacaaagag cttcaacagg ggagagtgtt ag                        642
<210> 49
   <211> 697
   <212> PRT
   <213> Artificial Sequence
<220>
   <223> GA201 (scFab)-Fc(knob) P329G LALA
<400> 49
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Asn Asn Tyr
               20                  25                  30
   Leu Asn Trp Tyr Gln Gln Lys Pro Gly Lys Ala Pro Lys Arg Leu Ile
           35                  40                  45
   Tyr Asn Thr Asn Asn Leu Gln Thr Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Leu Gln His Asn Ser Phe Pro Thr
                   85                  90                  95
   Phe Gly Gln Gly Thr Lys Leu Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys Ser Gly Gly Gly Ser Gly Gly Gly Ser Glu Gly
       210                 215                 220
   Gly Gly Ser Glu Gly Gly Gly Ser Glu Gly Gly Gly Ser Glu Gly Gly
   225 230 235 240
   Gly Ser Gly Gly Gly Ser Gly Gln Val Gln Leu Val Gln Ser Gly Ala
   245 250 255
   Glu Val Lys Lys Pro Gly Ser Ser Val Lys Val Ser Cys Lys Ala Ser
   260 265 270
   Gly Phe Thr Phe Thr Asp Tyr Lys Ile His Trp Val Arg Gln Ala Pro
   275 280 285
   Gly Gln Gly Leu Glu Trp Met Gly Tyr Phe Asn Pro Asn Ser Gly Tyr
   290 295 300
   Ser Thr Tyr Ala Gln Lys Phe Gln Gly Arg Val Thr Ile Thr Ala Asp
   305 310 315 320
   Lys Ser Thr Ser Thr Ala Tyr Met Glu Leu Ser Ser Leu Arg Ser Glu
   325 330 335
   Asp Thr Ala Val Tyr Tyr Cys Ala Arg Leu Ser Pro Gly Gly Tyr Tyr
   340 345 350
   Val Met Asp Ala Trp Gly Gln Gly Thr Thr Val Thr Val Ser Ser Ala
   355 360 365
   Ser Thr Lys Gly Pro Ser Val Phe Pro Leu Ala Pro Ser Ser Lys Ser
   370 375 380
   Thr Ser Gly Gly Thr Ala Ala Leu Gly Cys Leu Val Lys Asp Tyr Phe
   385 390 395 400
   Pro Glu Pro Val Thr Val Ser Trp Asn Ser Gly Ala Leu Thr Ser Gly
   405 410 415
   Val His Thr Phe Pro Ala Val Leu Gln Ser Ser Gly Leu Tyr Ser Leu
   420 425 430
   Ser Ser Val Val Thr Val Pro Ser Ser Ser Leu Gly Thr Gln Thr Tyr
   435 440 445
   Ile Cys Asn Val Asn His Lys Pro Ser Asn Thr Lys Val Asp Lys Lys
   450 455 460
   Val Glu Pro Lys Ser Cys Asp Lys Thr His Thr Cys Pro Pro Cys Pro
   465 470 475 480
   Ala Pro Glu Ala Ala Gly Gly Pro Ser Val Phe Leu Phe Pro Pro Lys
                   485                 490                 495
   Pro Lys Asp Thr Leu Met Ile Ser Arg Thr Pro Glu Val Thr Cys Val
               500                 505                 510
   Val Val Asp Val Ser His Glu Asp Pro Glu Val Lys Phe Asn Trp Tyr
           515                 520                 525
   Val Asp Gly Val Glu Val His Asn Ala Lys Thr Lys Pro Arg Glu Glu
       530                 535                 540
   Gln Tyr Asn Ser Thr Tyr Arg Val Val Ser Val Leu Thr Val Leu His
   545                 550                 555                 560
   Gln Asp Trp Leu Asn Gly Lys Glu Tyr Lys Cys Lys Val Ser Asn Lys
                   565                 570                 575
   Ala Leu Gly Ala Pro Ile Glu Lys Thr Ile Ser Lys Ala Lys Gly Gln
               580                 585                 590
   Pro Arg Glu Pro Gln Val Tyr Thr Leu Pro Pro Cys Arg Asp Glu Leu
           595                 600                 605
   Thr Lys Asn Gln Val Ser Leu Trp Cys Leu Val Lys Gly Phe Tyr Pro
       610                 615                 620
   Ser Asp Ile Ala Val Glu Trp Glu Ser Asn Gly Gln Pro Glu Asn Asn
   625                 630                 635                 640
   Tyr Lys Thr Thr Pro Pro Val Leu Asp Ser Asp Gly Ser Phe Phe Leu
                   645                 650                 655
   Tyr Ser Lys Leu Thr Val Asp Lys Ser Arg Trp Gln Gln Gly Asn Val
               660                 665                 670
   Phe Ser Cys Ser Val Met His Glu Ala Leu His Asn His Tyr Thr Gln
           675                 680                 685
   Lys Ser Leu Ser Leu Ser Pro Gly Lys
       690                 695
<210> 50
   <211> 2094
   <212> DNA
   <213> Artificial Sequence
<220>
   <223> GA201 (scFab)-Fc(knob) P329G LALA
<400> 50
   gatatccaga tgacccagtc tccatcctcc ctgtctgcat ctgtcggaga ccgggtcacc 60
   atcacctgcc gggcaagtca gggcattaac aattacttaa attggtacca gcagaagcca 120
   gggaaagccc ctaagcgcct gatctataat accaacaact tgcagacagg cgtcccatca 180
   aggttcagcg gcagtggatc cgggacagaa ttcactctca ccatcagcag cctgcagcct 240
   gaagattttg ccacctatta ctgcttgcag cataatagtt ttcccacgtt tggccagggc 300
   accaagctcg agatcaagcg tacggtggcc gctcccagcg tgttcatctt cccccccagc 360
   gacgagcagc tgaaatctgg caccgccagc gtcgtgtgcc tgctgaacaa cttctacccc 420
   cgggaggcca aggtgcagtg gaaggtggac aacgccctgc agagcggcaa cagccaggaa 480
   agcgtcaccg agcaggacag caaggactcc acctatagcc tgtccagcac cctgaccctg 540
   agcaaggccg actacgagaa gcacaaggtg tacgcctgcg aagtgaccca ccagggcctg 600
   agcagccccg tgaccaagag cttcaaccgg ggcgagtgca gcggcggagg tagcggaggc 660
   ggctctgagg gcggaggaag cgagggcgga ggctccgaag gcggcggaag cgaaggtggc 720
   ggctctggcg gcggatccgg ccaggtgcag ctggtgcagt ctggggctga ggtgaagaag 780
   cctgggtcct cggtgaaggt ctcctgcaag gcctctggtt tcacattcac tgactacaag 840
   atacactggg tgcgacaggc ccctggacaa gggctcgagt ggatgggata tttcaaccct 900
   aacagcggtt atagtaccta cgcacagaag ttccagggca gggtcaccat taccgcggac 960
   aaatccacga gcacagccta catggagctg agcagcctga gatctgagga cacggccgtg 1020
   tattactgtg cgagactatc cccaggcggt tactatgtta tggatgcctg gggccaaggg 1080
   accaccgtga ccgtctcctc agctagcacc aagggcccta gcgtgttccc tctggcccct 1140
   agcagcaaga gcacaagtgg aggaacagcc gccctgggct gcctggtcaa ggactacttc 1200
   cccgagcccg tgaccgtgtc ctggaattct ggcgccctga caagcggcgt gcacacattt 1260
   ccagccgtgc tgcagagcag cggcctgtac tctctgagca gcgtcgtgac cgtgccctct 1320
   agctctctgg gcacccagac ctacatctgc aacgtgaacc acaagcccag caacaccaaa 1380
   gtggacaaga aggtggaacc caagagctgc gacaagaccc acacctgtcc cccttgccct 1440
   gcccctgaag ctgctggtgg cccttccgtg ttcctgttcc ccccaaagcc caaggacacc 1500
   ctgatgatca gccggacccc cgaagtgacc tgcgtggtgg tcgatgtgtc ccacgaggac 1560
   cctgaagtga agttcaattg gtacgtggac ggcgtggaag tgcacaatgc caagaccaag 1620
   ccgcgggagg agcagtacaa cagcacgtac cgtgtggtca gcgtcctcac cgtcctgcac 1680
   caggactggc tgaatggcaa ggagtacaag tgcaaggtct ccaacaaagc cctcggcgcc 1740
   cccatcgaga aaaccatctc caaagccaaa gggcagcccc gagaaccaca ggtgtacacc 1800
   ctgcccccat gccgggatga gctgaccaag aaccaggtca gcctgtggtg cctggtcaaa 1860
   ggcttctatc ccagcgacat cgccgtggag tgggagagca atgggcagcc ggagaacaac 1920
   tacaagacca cgcctcccgt gctggactcc gacggctcct tcttcctcta cagcaagctc 1980
   accgtggaca agagcaggtg gcagcagggg aacgtcttct catgctccgt gatgcatgag      2040
   gctctgcaca accactacac gcagaagagc ctctccctgt ctccgggtaa ataa            2094
<210> 51
   <211> 452
   <212> PRT
   <213> Artificial Sequence
<220>
   <223> V9 (VH-CH1)-Fc(hole) P329G LALA
<400> 51
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Gln Gly Thr Leu Val Thr Val Ser Ser Ala Ser Thr Lys Gly Pro
           115                 120                 125
   Ser Val Phe Pro Leu Ala Pro Ser Ser Lys Ser Thr Ser Gly Gly Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Gln Thr Tyr Ile Cys Asn Val Asn
           195                 200                 205
   His Lys Pro Ser Asn Thr Lys Val Asp Lys Lys Val Glu Pro Lys Ser
       210                 215                 220
   Cys Asp Lys Thr His Thr Cys Pro Pro Cys Pro Ala Pro Glu Ala Ala
   225                 230                 235                 240
   Gly Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Lys Phe Asn Trp Tyr Val Asp Gly Val Glu
           275                 280                 285
   Val His Asn Ala Lys Thr Lys Pro Arg Glu Glu Gln Tyr Asn Ser Thr
       290                 295                 300
   Tyr Arg Val Val Ser Val Leu Thr Val Leu His Gln Asp Trp Leu Asn
   305                 310                 315                 320
   Gly Lys Glu Tyr Lys Cys Lys Val Ser Asn Lys Ala Leu Gly Ala Pro
                   325                 330                 335
   Ile Glu Lys Thr Ile Ser Lys Ala Lys Gly Gln Pro Arg Glu Pro Gln
               340                 345                 350
   Val Cys Thr Leu Pro Pro Ser Arg Asp Glu Leu Thr Lys Asn Gln Val
           355                 360                 365
   Ser Leu Ser Cys Ala Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Val Leu Asp Ser Asp Gly Ser Phe Phe Leu Val Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
       450
<210> 52
   <211> 1359
   <212> DNA
   <213> Artificial Sequence
<220>
   <223> V9 (VH-CH1)-Fc(hole) P329G LALA
<400> 52
   gaggtgcagc tggtcgagag cggaggcggc ctggtgcagc ctggcggcag cctgagactg      60
   agctgcgccg ccagcggcta cagcttcacc ggctacacca tgaactgggt ccggcaggca     120
   cctggcaagg gactggaatg ggtggccctg atcaacccct acaagggcgt gagcacctac     180
   aaccagaagt tcaaggaccg gttcaccatc agcgtggaca agagcaagaa caccgcctat     240
   ctgcagatga acagcctgcg ggccgaggac accgccgtgt actactgcgc cagaagcggc     300
   tactacggcg acagcgactg gtacttcgac gtgtggggcc agggcaccct cgtgaccgtg     360
   tctagcgcta gcaccaaggg cccctccgtg ttccccctgg cccccagcag caagagcacc     420
   agcggcggca cagccgctct gggctgcctg gtcaaggact acttccccga gcccgtgacc     480
   gtgtcctgga acagcggagc cctgacctcc ggcgtgcaca ccttccccgc cgtgctgcag     540
   agttctggcc tgtatagcct gagcagcgtg gtcaccgtgc cttctagcag cctgggcacc     600
   cagacctaca tctgcaacgt gaaccacaag cccagcaaca ccaaggtgga caagaaggtg     660
   gagcccaaga gctgcgacaa aactcacaca tgcccaccgt gcccagcacc tgaagctgca     720
   gggggaccgt cagtcttcct cttcccccca aaacccaagg acaccctcat gatctcccgg     780
   acccctgagg tcacatgcgt ggtggtggac gtgagccacg aagaccctga ggtcaagttc     840
   aactggtacg tggacggcgt ggaggtgcat aatgccaaga caaagccgcg ggaggagcag     900
   tacaacagca cgtaccgtgt ggtcagcgtc ctcaccgtcc tgcaccagga ctggctgaat     960
   ggcaaggagt acaagtgcaa ggtctccaac aaagccctcg gcgcccccat cgagaaaacc    1020
   atctccaaag ccaaagggca gccccgagaa ccacaggtgt gcaccctgcc cccatcccgg    1080
   gatgagctga ccaagaacca ggtcagcctc tcgtgcgcag tcaaaggctt ctatcccagc    1140
   gacatcgccg tggagtggga gagcaatggg cagccggaga acaactacaa gaccacgcct    1200
   cccgtgctgg actccgacgg ctccttcttc ctcgtgagca agctcaccgt ggacaagagc    1260
   aggtggcagc aggggaacgt cttctcatgc tccgtgatgc atgaggctct gcacaaccac    1320
   tacacgcaga agagcctctc cctgtctccg ggtaaatga                           1359
<210> 53
   <211> 934
   <212> PRT
   <213> Artificial Sequence
<220>
   <223> V9 (scFab)-GA201 (VH-CH1)-Fc(knob) P329G LALA
<400> 53
<210> 54
   <211> 2805
   <212> DNA
   <213> Artificial Sequence
<220>
   <223> V9 (scFab)-GA201 (VH-CH1)-Fc(knob) P329G LALA
<400> 54
   gacatccaga tgacccagag cccctctagc ctgagcgcca gcgtgggcga cagagtgacc     60
   atcacctgtc gggccagcca ggacatcaga aactacctga actggtatca gcagaagccc    120
   ggcaaggccc ccaagctgct gatctactac acctctagac tggaaagcgg cgtgcccagc    180
   cggtttagcg gcagcggctc cggcaccgac tacaccctga ccatcagcag cctgcagccc    240
   gaggacttcg ccacctacta ctgccagcag ggcaacacac tcccctggac cttcggccag    300
   ggcaccaagg tggagatcaa gcgtacggtg gccgctccca gcgtgttcat cttccccccc    360
   agcgacgagc agctgaagtc cggcaccgcc agcgtcgtgt gcctgctgaa caacttctac 420
   ccccgggagg ccaaggtgca gtggaaggtg gacaacgccc tgcagagcgg caacagccag 480
   gaaagcgtca ccgagcagga cagcaaggac tccacctaca gcctgagcag caccctgacc 540
   ctgagcaagg ccgactacga gaagcacaag gtgtacgcct gcgaagtgac ccaccagggc 600
   ctgtccagcc ccgtgaccaa gagcttcaac cggggcgagt gcagcggcgg aggctctgga 660
   ggcggctctg aaggcggagg aagtgagggc ggaggctcag aaggcggcgg aagcgaaggt 720
   ggcggctctg gcggcggatc cggcgaggtg cagctggtcg agtccggcgg aggcctggtg 780
   cagcctggcg gcagcctgag actgagctgc gccgccagcg gctacagctt caccggctac 840
   accatgaact gggtccggca ggctcctggc aagggcctcg aatgggtggc cctgatcaac 900
   ccctacaagg gcgtgagcac ctacaaccag aagttcaagg accggttcac catcagcgtg 960
   gacaagagca agaacaccgc ctatctgcag atgaacagcc tgcgggccga ggacaccgcc 1020
   gtgtactact gcgccagaag cggctactac ggcgacagcg actggtactt cgacgtgtgg 1080
   ggccagggca cactggtcac cgtgtccagc gctagcacca agggcccctc cgtgttcccc 1140
   ctggccccca gcagcaagag caccagcggc ggcacagccg ccctcggctg cctggtcaag 1200
   gactacttcc ccgagcccgt gaccgtgtcc tggaacagcg gagccctgac ctccggcgtg 1260
   cacaccttcc ccgccgtgct gcagagcagc ggcctgtaca gcctgtccag cgtggtcacc 1320
   gtgccctcca gcagcctggg cacccagacc tacatctgca acgtgaacca caagcccagc 1380
   aataccaagg tggacaagaa ggtggagccc aagagctgcg acggcggtgg tggctccgga 1440
   ggcggtggat ctcaggtgca gctggtgcag tctggggctg aggtgaagaa gcctgggtcc 1500
   tcggtgaagg tctcctgcaa ggcctctggt ttcacattca ctgactacaa gatacactgg 1560
   gtgcgacagg cccctggaca agggctcgag tggatgggat atttcaaccc taacagcggt 1620
   tatagtacct acgcacagaa gttccagggc agggtcacca ttaccgcgga caaatccacg 1680
   agcacagcct acatggagct gagcagcctg agatctgagg acacggccgt gtattactgt 1740
   gcgagactat ccccaggcgg ttactatgtt atggatgcct ggggccaagg gaccaccgtg 1800
   accgtctcct cagctagcac caagggcccc tccgtgttcc ccctggcccc cagcagcaag 1860
   agcaccagcg gcggcacagc cgctctgggc tgcctggtca aggactactt ccccgagccc 1920
   gtgaccgtgt cctggaacag cggagccctg acctccggcg tgcacacctt ccccgccgtg 1980
   ctgcagagtt ctggcctgta tagcctgagc agcgtggtca ccgtgccttc tagcagcctg 2040
   ggcacccaga cctacatctg caacgtgaac cacaagccca gcaacaccaa ggtggacaag 2100
   aaggtggagc ccaagagctg cgacaaaact cacacatgcc caccgtgccc agcacctgaa 2160
   gctgcagggg gaccgtcagt cttcctcttc cccccaaaac ccaaggacac cctcatgatc 2220
   tcccggaccc ctgaggtcac atgcgtggtg gtggacgtga gccacgaaga ccctgaggtc 2280
   aagttcaact ggtacgtgga cggcgtggag gtgcataatg ccaagacaaa gccgcgggag 2340
   gagcagtaca acagcacgta ccgtgtggtc agcgtcctca ccgtcctgca ccaggactgg     2400
   ctgaatggca aggagtacaa gtgcaaggtc tccaacaaag ccctcggcgc ccccatcgag     2460
   aaaaccatct ccaaagccaa agggcagccc cgagaaccac aggtgtacac cctgccccca     2520
   tgccgggatg agctgaccaa gaaccaggtc agcctgtggt gcctggtcaa aggcttctat     2580
   cccagcgaca tcgccgtgga gtgggagagc aatgggcagc cggagaacaa ctacaagacc     2640
   acgcctcccg tgctggactc cgacggctcc ttcttcctct acagcaagct caccgtggac     2700
   aagagcaggt ggcagcaggg gaacgtcttc tcatgctccg tgatgcatga ggctctgcac     2760
   aaccactaca cgcagaagag cctctccctg tctccgggta aatga                     2805
<210> 55
   <211> 694
   <212> PRT
   <213> Artificial Sequence
<220>
   <223> 3F2 (scFab)-Fc(knob) P329G LALA
<400> 55
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145 150 155 160
   Gln Glu Ser Val Thr Glu Gln Asp Ser Lys Asp Ser Thr Tyr Ser Leu
   165 170 175
   Ser Ser Thr Leu Thr Leu Ser Lys Ala Asp Tyr Glu Lys His Lys Val
   180 185 190
   Tyr Ala Cys Glu Val Thr His Gln Gly Leu Ser Ser Pro Val Thr Lys
   195 200 205
   Ser Phe Asn Arg Gly Glu Cys Gly Gly Gly Gly Ser Gly Gly Gly Gly
   210 215 220
   Ser Gly Gly Gly Gly Ser Gly Gly Gly Gly Ser Gly Gly Gly Gly Ser
   225 230 235 240
   Gly Gly Gly Gly Ser Gly Gly Glu Val Gln Leu Leu Glu Ser Gly Gly
   245 250 255
   Gly Leu Val Gln Pro Gly Gly Ser Leu Arg Leu Ser Cys Ala Ala Ser
   260 265 270
   Gly Phe Thr Phe Ser Ser Tyr Ala Met Ser Trp Val Arg Gln Ala Pro
   275 280 285
   Gly Lys Gly Leu Glu Trp Val Ser Ala Ile Ser Gly Ser Gly Gly Ser
   290 295 300
   Thr Tyr Tyr Ala Asp Ser Val Lys Gly Arg Phe Thr Ile Ser Arg Asp
   305 310 315 320
   Asn Ser Lys Asn Thr Leu Tyr Leu Gln Met Asn Ser Leu Arg Ala Glu
   325 330 335
   Asp Thr Ala Val Tyr Tyr Cys Ala Lys Gly Trp Phe Gly Gly Phe Asn
   340 345 350
   Tyr Trp Gly Gln Gly Thr Leu Val Thr Val Ser Ser Ala Ser Thr Lys
   355 360 365
   Gly Pro Ser Val Phe Pro Leu Ala Pro Ser Ser Lys Ser Thr Ser Gly
   370 375 380
   Gly Thr Ala Ala Leu Gly Cys Leu Val Lys Asp Tyr Phe Pro Glu Pro
   385 390 395 400
   Val Thr Val Ser Trp Asn Ser Gly Ala Leu Thr Ser Gly Val His Thr
   405 410 415
   Phe Pro Ala Val Leu Gln Ser Ser Gly Leu Tyr Ser Leu Ser Ser Val
   420 425 430
   Val Thr Val Pro Ser Ser Ser Leu Gly Thr Gln Thr Tyr Ile Cys Asn
   435 440 445
   Val Asn His Lys Pro Ser Asn Thr Lys Val Asp Lys Lys Val Glu Pro
   450 455 460
   Lys Ser Cys Asp Lys Thr His Thr Cys Pro Pro Cys Pro Ala Pro Glu
   465 470 475 480
   Ala Ala Gly Gly Pro Ser Val Phe Leu Phe Pro Pro Lys Pro Lys Asp
   485 490 495
   Thr Leu Met Ile Ser Arg Thr Pro Glu Val Thr Cys Val Val Val Asp
   500 505 510
   Val Ser His Glu Asp Pro Glu Val Lys Phe Asn Trp Tyr Val Asp Gly
   515 520 525
   Val Glu Val His Asn Ala Lys Thr Lys Pro Arg Glu Glu Gln Tyr Asn
   530 535 540
   Ser Thr Tyr Arg Val Val Ser Val Leu Thr Val Leu His Gln Asp Trp
   545 550 555 560
   Leu Asn Gly Lys Glu Tyr Lys Cys Lys Val Ser Asn Lys Ala Leu Gly
   565 570 575
   Ala Pro Ile Glu Lys Thr Ile Ser Lys Ala Lys Gly Gln Pro Arg Glu
   580 585 590
   Pro Gln Val Tyr Thr Leu Pro Pro Cys Arg Asp Glu Leu Thr Lys Asn
   595 600 605
   Gln Val Ser Leu Trp Cys Leu Val Lys Gly Phe Tyr Pro Ser Asp Ile
   610 615 620
   Ala Val Glu Trp Glu Ser Asn Gly Gln Pro Glu Asn Asn Tyr Lys Thr
   625 630 635 640
   Thr Pro Pro Val Leu Asp Ser Asp Gly Ser Phe Phe Leu Tyr Ser Lys
   645 650 655
   Leu Thr Val Asp Lys Ser Arg Trp Gln Gln Gly Asn Val Phe Ser Cys
   660 665 670
   Ser Val Met His Glu Ala Leu His Asn His Tyr Thr Gln Lys Ser Leu
           675                 680                 685
   Ser Leu Ser Pro Gly Lys
       690
<210> 56
   <211> 2085
   <212> DNA
   <213> Artificial Sequence
<220>
   <223> 3F2 (scFab)-Fc(knob) P329G LALA
<400> 56
   gagatcgtgc tgacacagag ccccggaacc ctgtctctga gccctggcga aagagccacc 60
   ctgagctgta gagccagcca gagcgtgacc agcagctacc tggcctggta tcagcagaag 120
   cctggacagg cccccagact gctgatcaat gtgggcagca gacgggccac cggcatccct 180
   gatagatttt ctggcagcgg cagcggcacc gacttcaccc tgaccatcag cagactggaa 240
   cccgaggact tcgccgtgta ctactgccag cagggcatca tgctgccccc tacatttggc 300
   cagggcacca aggtggaaat caagcgtacg gtggccgctc ccagcgtgtt catcttccca 360
   cctagcgacg agcagctgaa gtctggcaca gccagcgtcg tgtgcctgct gaacaacttc 420
   tacccccgcg aggccaaggt gcagtggaag gtggacaacg ccctgcagag cggcaacagc 480
   caggaaagcg tcaccgagca ggacagcaag gactccacct acagcctgag cagcaccctg 540
   accctgagca aggccgacta cgagaagcac aaggtgtacg cctgcgaagt gacccaccag 600
   ggcctgtcta gccccgtgac caagagcttc aaccggggag aatgtggcgg cggaggatct 660
   ggtggcggag gtagtggtgg tggtggatct ggcggaggcg gatccggcgg aggtggaagc 720
   ggaggtggtg gaagtggggg agaagtgcag ctgctggaaa gtggcggagg cctggtgcag 780
   cctggcggat ctctgagact gagctgtgcc gccagcggct tcacctttag cagctacgcc 840
   atgagctggg tccgacaggc ccctggaaag ggactggaat gggtgtccgc catctctggc 900
   tctggcggca gcacctacta cgccgatagc gtgaagggcc ggttcaccat cagccgggac 960
   aacagcaaga acaccctgta cctgcagatg aacagcctgc gggccgagga taccgccgtg 1020
   tattattgcg ccaagggatg gttcggcggc ttcaactatt ggggccaggg aaccctggtc 1080
   accgtgtcta gtgctagcac caagggccct agcgtgttcc ctctggcccc tagcagcaag 1140
   agcacaagtg gaggaacagc cgccctgggc tgcctggtca aggactactt ccccgagccc 1200
   gtgaccgtgt cctggaattc tggcgccctg acaagcggcg tgcacacatt tccagccgtg 1260
   ctgcagagca gcggcctgta ctctctgagc agcgtcgtga ccgtgccctc tagctctctg 1320
   ggcacccaga cctacatctg caacgtgaac cacaagccca gcaacaccaa agtggacaag 1380
   aaggtggaac ccaagagctg cgacaagacc cacacctgtc ccccttgccc tgcccctgaa 1440
   gctgctggtg gcccttccgt gttcctgttc cccccaaagc ccaaggacac cctgatgatc      1500
   agccggaccc ccgaagtgac ctgcgtggtg gtcgatgtgt cccacgagga ccctgaagtg      1560
   aagttcaatt ggtacgtgga cggcgtggaa gtgcacaatg ccaagaccaa gccgcgggag      1620
   gagcagtaca acagcacgta ccgtgtggtc agcgtcctca ccgtcctgca ccaggactgg      1680
   ctgaatggca aggagtacaa gtgcaaggtc tccaacaaag ccctcggcgc ccccatcgag      1740
   aaaaccatct ccaaagccaa agggcagccc cgagaaccac aggtgtacac cctgccccca      1800
   tgccgggatg agctgaccaa gaaccaggtc agcctgtggt gcctggtcaa aggcttctat      1860
   cccagcgaca tcgccgtgga gtgggagagc aatgggcagc cggagaacaa ctacaagacc      1920
   acgcctcccg tgctggactc cgacggctcc ttcttcctct acagcaagct caccgtggac      1980
   aagagcaggt ggcagcaggg gaacgtcttc tcatgctccg tgatgcatga ggctctgcac      2040
   aaccactaca cgcagaagag cctctccctg tctccgggta aataa                     2085
<210> 57
   <211> 931
   <212> PRT
   <213> Artificial Sequence
<220>
   <223> V9 (scFab)-3F2 (VH-CH1)-Fc(knob) P329G LALA
<400> 57
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys Ser Gly Gly Gly Ser Gly Gly Gly Ser Glu
       210                 215                 220
   Gly Gly Gly Ser Glu Gly Gly Gly Ser Glu Gly Gly Gly Ser Glu Gly
   225                 230                 235                 240
   Gly Gly Ser Gly Gly Gly Ser Gly Glu Val Gln Leu Val Glu Ser Gly
                   245                 250                 255
   Gly Gly Leu Val Gln Pro Gly Gly Ser Leu Arg Leu Ser Cys Ala Ala
               260                 265                 270
   Ser Gly Tyr Ser Phe Thr Gly Tyr Thr Met Asn Trp Val Arg Gln Ala
           275                 280                 285
   Pro Gly Lys Gly Leu Glu Trp Val Ala Leu Ile Asn Pro Tyr Lys Gly
       290                 295                 300
   Val Ser Thr Tyr Asn Gln Lys Phe Lys Asp Arg Phe Thr Ile Ser Val
   305                 310                 315                 320
   Asp Lys Ser Lys Asn Thr Ala Tyr Leu Gln Met Asn Ser Leu Arg Ala
                   325                 330                 335
   Glu Asp Thr Ala Val Tyr Tyr Cys Ala Arg Ser Gly Tyr Tyr Gly Asp
               340                 345                 350
   Ser Asp Trp Tyr Phe Asp Val Trp Gly Gln Gly Thr Leu Val Thr Val
           355                 360                 365
   Ser Ser Ala Ser Thr Lys Gly Pro Ser Val Phe Pro Leu Ala Pro Ser
       370                 375                 380
   Ser Lys Ser Thr Ser Gly Gly Thr Ala Ala Leu Gly Cys Leu Val Lys
   385 390 395 400
   Asp Tyr Phe Pro Glu Pro Val Thr Val Ser Trp Asn Ser Gly Ala Leu
   405 410 415
   Thr Ser Gly Val His Thr Phe Pro Ala Val Leu Gln Ser Ser Gly Leu
   420 425 430
   Tyr Ser Leu Ser Ser Val Val Thr Val Pro Ser Ser Ser Leu Gly Thr
   435 440 445
   Gln Thr Tyr Ile Cys Asn Val Asn His Lys Pro Ser Asn Thr Lys Val
   450 455 460
   Asp Lys Lys Val Glu Pro Lys Ser Cys Asp Gly Gly Gly Gly Ser Gly
   465 470 475 480
   Gly Gly Gly Ser Glu Val Gln Leu Leu Glu Ser Gly Gly Gly Leu Val
   485 490 495
   Gln Pro Gly Gly Ser Leu Arg Leu Ser Cys Ala Ala Ser Gly Phe Thr
   500 505 510
   Phe Ser Ser Tyr Ala Met Ser Trp Val Arg Gln Ala Pro Gly Lys Gly
   515 520 525
   Leu Glu Trp Val Ser Ala Ile Ser Gly Ser Gly Gly Ser Thr Tyr Tyr
   530 535 540
   Ala Asp Ser Val Lys Gly Arg Phe Thr Ile Ser Arg Asp Asn Ser Lys
   545 550 555 560
   Asn Thr Leu Tyr Leu Gln Met Asn Ser Leu Arg Ala Glu Asp Thr Ala
   565 570 575
   Val Tyr Tyr Cys Ala Lys Gly Trp Phe Gly Gly Phe Asn Tyr Trp Gly
   580 585 590
   Gln Gly Thr Leu Val Thr Val Ser Ser Ala Ser Thr Lys Gly Pro Ser
   595 600 605
   Val Phe Pro Leu Ala Pro Ser Ser Lys Ser Thr Ser Gly Gly Thr Ala
   610 615 620
   Ala Leu Gly Cys Leu Val Lys Asp Tyr Phe Pro Glu Pro Val Thr Val
   625 630 635 640
   Ser Trp Asn Ser Gly Ala Leu Thr Ser Gly Val His Thr Phe Pro Ala
                   645                 650                 655
   Val Leu Gln Ser Ser Gly Leu Tyr Ser Leu Ser Ser Val Val Thr Val
               660                 665                 670
   Pro Ser Ser Ser Leu Gly Thr Gln Thr Tyr Ile Cys Asn Val Asn His
           675                 680                 685
   Lys Pro Ser Asn Thr Lys Val Asp Lys Lys Val Glu Pro Lys Ser Cys
       690                 695                 700
   Asp Lys Thr His Thr Cys Pro Pro Cys Pro Ala Pro Glu Ala Ala Gly
   705                 710                 715                 720
   Gly Pro Ser Val Phe Leu Phe Pro Pro Lys Pro Lys Asp Thr Leu Met
                   725                 730                 735
   Ile Ser Arg Thr Pro Glu Val Thr Cys Val Val Val Asp Val Ser His
               740                 745                 750
   Glu Asp Pro Glu Val Lys Phe Asn Trp Tyr Val Asp Gly Val Glu Val
           755                 760                 765
   His Asn Ala Lys Thr Lys Pro Arg Glu Glu Gln Tyr Asn Ser Thr Tyr
       770                 775                 780
   Arg Val Val Ser Val Leu Thr Val Leu His Gln Asp Trp Leu Asn Gly
   785                 790                 795                 800
   Lys Glu Tyr Lys Cys Lys Val Ser Asn Lys Ala Leu Gly Ala Pro Ile
                   805                 810                 815
   Glu Lys Thr Ile Ser Lys Ala Lys Gly Gln Pro Arg Glu Pro Gln Val
               820                 825                 830
   Tyr Thr Leu Pro Pro Cys Arg Asp Glu Leu Thr Lys Asn Gln Val Ser
           835                 840                 845
   Leu Trp Cys Leu Val Lys Gly Phe Tyr Pro Ser Asp Ile Ala Val Glu
       850                 855                 860
   Trp Glu Ser Asn Gly Gln Pro Glu Asn Asn Tyr Lys Thr Thr Pro Pro
   865                 870                 875                 880
   Val Leu Asp Ser Asp Gly Ser Phe Phe Leu Tyr Ser Lys Leu Thr Val
                   885                 890                 895
   Asp Lys Ser Arg Trp Gln Gln Gly Asn Val Phe Ser Cys Ser Val Met
               900                 905                 910
   His Glu Ala Leu His Asn His Tyr Thr Gln Lys Ser Leu Ser Leu Ser
   915 920 925
   Pro Gly Lys
   930
<210> 58
   <211> 2796
   <212> DNA
   <213> Artificial Sequence
<220>
   <223> V9 (scFab)-3F2 (VH-CH1)-Fc(knob) P329G LALA
<400> 58
   gacatccaga tgacccagag cccctctagc ctgagcgcca gcgtgggcga cagagtgacc 60
   atcacctgtc gggccagcca ggacatcaga aactacctga actggtatca gcagaagccc 120
   ggcaaggccc ccaagctgct gatctactac acctctagac tggaaagcgg cgtgcccagc 180
   cggtttagcg gcagcggctc cggcaccgac tacaccctga ccatcagcag cctgcagccc 240
   gaggacttcg ccacctacta ctgccagcag ggcaacacac tcccctggac cttcggccag 300
   ggcaccaagg tggagatcaa gcgtacggtg gccgctccca gcgtgttcat cttccccccc 360
   agcgacgagc agctgaagtc cggcaccgcc agcgtcgtgt gcctgctgaa caacttctac 420
   ccccgggagg ccaaggtgca gtggaaggtg gacaacgccc tgcagagcgg caacagccag 480
   gaaagcgtca ccgagcagga cagcaaggac tccacctaca gcctgagcag caccctgacc 540
   ctgagcaagg ccgactacga gaagcacaag gtgtacgcct gcgaagtgac ccaccagggc 600
   ctgtccagcc ccgtgaccaa gagcttcaac cggggcgagt gcagcggcgg aggctctgga 660
   ggcggctctg aaggcggagg aagtgagggc ggaggctcag aaggcggcgg aagcgaaggt 720
   ggcggctctg gcggcggatc cggcgaggtg cagctggtcg agtccggcgg aggcctggtg 780
   cagcctggcg gcagcctgag actgagctgc gccgccagcg gctacagctt caccggctac 840
   accatgaact gggtccggca ggctcctggc aagggcctcg aatgggtggc cctgatcaac 900
   ccctacaagg gcgtgagcac ctacaaccag aagttcaagg accggttcac catcagcgtg 960
   gacaagagca agaacaccgc ctatctgcag atgaacagcc tgcgggccga ggacaccgcc 1020
   gtgtactact gcgccagaag cggctactac ggcgacagcg actggtactt cgacgtgtgg 1080
   ggccagggca cactggtcac cgtgtccagc gctagcacca agggcccctc cgtgttcccc 1140
   ctggccccca gcagcaagag caccagcggc ggcacagccg ccctcggctg cctggtcaag 1200
   gactacttcc ccgagcccgt gaccgtgtcc tggaacagcg gagccctgac ctccggcgtg 1260
   cacaccttcc ccgccgtgct gcagagcagc ggcctgtaca gcctgtccag cgtggtcacc 1320
   gtgccctcca gcagcctggg cacccagacc tacatctgca acgtgaacca caagcccagc 1380
   aataccaagg tggacaagaa ggtggagccc aagagctgcg acggcggtgg tggctccgga 1440
   ggaggaggca gcgaggtgca gctgctggaa tctggaggcg gcctggtgca gcctggcggc 1500
   agcctgagac tgtcttgcgc cgccagcggc ttcaccttca gcagctacgc catgagctgg 1560
   gtccgacagg ctcctggcaa gggactggaa tgggtgtccg ccatctccgg cagcggaggc 1620
   agcacctact acgccgacag cgtgaagggc cggttcacca tcagcagaga caacagcaag 1680
   aacaccctgt acctgcagat gaacagcctg cgggccgagg ataccgccgt gtattattgc 1740
   gccaagggat ggttcggcgg cttcaactac tggggccagg gaaccctggt gacagtgtcc 1800
   agcgccagca ccaagggccc ctccgtgttt cctctggccc ccagcagcaa gagcacctct 1860
   ggcggaacag ccgccctggg ctgcctggtg aaagactact tccccgagcc cgtgaccgtg 1920
   tcctggaact ctggcgccct gaccagcggc gtgcacacct ttccagccgt gctgcagagc 1980
   agcggcctgt actccctgag cagcgtggtg acagtgccct ccagcagcct gggcacccag 2040
   acctacatct gcaacgtgaa ccacaagccc agcaacacca aagtggacaa gaaggtggaa 2100
   cccaagagct gcgacaaaac tcacacatgc ccaccgtgcc cagcacctga agctgcaggg 2160
   ggaccgtcag tcttcctctt ccccccaaaa cccaaggaca ccctcatgat ctcccggacc 2220
   cctgaggtca catgcgtggt ggtggacgtg agccacgaag accctgaggt caagttcaac 2280
   tggtacgtgg acggcgtgga ggtgcataat gccaagacaa agccgcggga ggagcagtac 2340
   aacagcacgt accgtgtggt cagcgtcctc accgtcctgc accaggactg gctgaatggc 2400
   aaggagtaca agtgcaaggt ctccaacaaa gccctcggcg cccccatcga gaaaaccatc 2460
   tccaaagcca aagggcagcc ccgagaacca caggtgtaca ccctgccccc atgccgggat 2520
   gagctgacca agaaccaggt cagcctgtgg tgcctggtca aaggcttcta tcccagcgac 2580
   atcgccgtgg agtgggagag caatgggcag ccggagaaca actacaagac cacgcctccc 2640
   gtgctggact ccgacggctc cttcttcctc tacagcaagc tcaccgtgga caagagcagg 2700
   tggcagcagg ggaacgtctt ctcatgctcc gtgatgcatg aggctctgca caaccactac 2760
   acgcagaaga gcctctccct gtctccgggt aaatga 2796
<210> 59
   <211> 447
   <212> PRT
   <213> Artificial Sequence
<220>
   <223> 3F2 (VH-CH1)-Fc(hole) P329G LALA
<400> 59
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Ala Ala Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Gly Ala Pro Ile Glu Lys Thr Ile
                   325                 330                 335
   Ser Lys Ala Lys Gly Gln Pro Arg Glu Pro Gln Val Cys Thr Leu Pro
               340                 345                 350
   Pro Ser Arg Asp Glu Leu Thr Lys Asn Gln Val Ser Leu Ser Cys Ala
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Val Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 60
   <211> 1344
   <212> DNA
   <213> Artificial Sequence
<220>
   <223> 3F2 (VH-CH1)-Fc(hole) P329G LALA
<400> 60
   gaggtgcagc tgctggaatc tggaggcggc ctggtgcagc ctggcggcag cctgagactg 60
   tcttgcgccg ccagcggctt caccttcagc agctacgcca tgagctgggt ccgacaggct 120
   cctggcaagg gactggaatg ggtgtccgcc atctccggca gcggaggcag cacctactac 180
   gccgacagcg tgaagggccg gttcaccatc agcagagaca acagcaagaa caccctgtac 240
   ctgcagatga acagcctgcg ggccgaggat accgccgtgt attattgcgc caagggatgg 300
   ttcggcggct tcaactactg gggccaggga accctggtga cagtgtccag cgccagcacc 360
   aagggcccct ccgtgtttcc tctggccccc agcagcaaga gcacctctgg cggaacagcc 420
   gccctgggct gcctggtgaa agactacttc cccgagcccg tgaccgtgtc ctggaactct 480
   ggcgccctga ccagcggcgt gcacaccttt ccagccgtgc tgcagagcag cggcctgtac 540
   tccctgagca gcgtggtgac agtgccctcc agcagcctgg gcacccagac ctacatctgc 600
   aacgtgaacc acaagcccag caacaccaaa gtggacaaga aggtggaacc caagagctgc 660
   gacaaaactc acacatgccc accgtgccca gcacctgaag ctgcaggggg accgtcagtc 720
   ttcctcttcc ccccaaaacc caaggacacc ctcatgatct cccggacccc tgaggtcaca 780
   tgcgtggtgg tggacgtgag ccacgaagac cctgaggtca agttcaactg gtacgtggac 840
   ggcgtggagg tgcataatgc caagacaaag ccgcgggagg agcagtacaa cagcacgtac 900
   cgtgtggtca gcgtcctcac cgtcctgcac caggactggc tgaatggcaa ggagtacaag 960
   tgcaaggtct ccaacaaagc cctcggcgcc cccatcgaga aaaccatctc caaagccaaa 1020
   gggcagcccc gagaaccaca ggtgtgcacc ctgcccccat cccgggatga gctgaccaag 1080
   aaccaggtca gcctctcgtg cgcagtcaaa ggcttctatc ccagcgacat cgccgtggag 1140
   tgggagagca atgggcagcc ggagaacaac tacaagacca cgcctcccgt gctggactcc 1200
   gacggctcct tcttcctcgt gagcaagctc accgtggaca agagcaggtg gcagcagggg 1260
   aacgtcttct catgctccgt gatgcatgag gctctgcaca accactacac gcagaagagc 1320
   ctctccctgt ctccgggtaa atga 1344
<210> 61
   <211> 215
   <212> PRT
   <213> Artificial Sequence
<220>
   <223> 3F2 (VL-CL)
<400> 61
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 62
   <211> 648
   <212> DNA
   <213> Artificial Sequence
<220>
   <223> 3F2 (VL-CL)
<400> 62
   gagatcgtgc tgacccagtc tcccggcacc ctgagcctga gccctggcga gagagccacc      60
   ctgagctgca gagccagcca gagcgtgacc agcagctacc tggcctggta tcagcagaag     120
   cccggccagg cccccagact gctgatcaac gtgggcagca gacgggccac cggcatcccc     180
   gatagattca gcggcagcgg ctccggcacc gacttcaccc tgaccatcag ccggctggaa     240
   cccgaggact tcgccgtgta ctactgccag cagggcatca tgctgccccc caccttcggc     300
   cagggcacca aggtggaaat caagcggacc gtggccgctc ccagcgtgtt catcttccca     360
   cccagcgacg agcagctgaa gtccggcaca gccagcgtgg tgtgcctgct gaacaacttc     420
   tacccccgcg aggccaaggt gcagtggaag gtggacaacg ccctgcagag cggcaacagc      480
   caggaatccg tgaccgagca ggacagcaag gactccacct acagcctgag cagcaccctg     540
   accctgagca aggccgacta cgagaagcac aaggtgtacg cctgcgaagt gacccaccag     600
   ggcctgtcca gccccgtgac caagagcttc aaccggggcg agtgctga                  648
<210> 63
   <211> 673
   <212> PRT
   <213> Artificial Sequence
<220>
   <223> CH1A1A (VH-CH1)- V9 (VL-CH1)-Fc(knob) P329G LALA
<400> 63
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Gly Gly Gly Gly Ser Gly Gly Gly Gly Ser Asp Ile Gln Met Thr Gln
   225 230 235 240
   Ser Pro Ser Ser Leu Ser Ala Ser Val Gly Asp Arg Val Thr Ile Thr
   245 250 255
   Cys Arg Ala Ser Gln Asp Ile Arg Asn Tyr Leu Asn Trp Tyr Gln Gln
   260 265 270
   Lys Pro Gly Lys Ala Pro Lys Leu Leu Ile Tyr Tyr Thr Ser Arg Leu
   275 280 285
   Glu Ser Gly Val Pro Ser Arg Phe Ser Gly Ser Gly Ser Gly Thr Asp
   290 295 300
   Tyr Thr Leu Thr Ile Ser Ser Leu Gln Pro Glu Asp Phe Ala Thr Tyr
   305 310 315 320
   Tyr Cys Gln Gln Gly Asn Thr Leu Pro Trp Thr Phe Gly Gln Gly Thr
   325 330 335
   Lys Val Glu Ile Lys Ser Ser Ala Ser Thr Lys Gly Pro Ser Val Phe
   340 345 350
   Pro Leu Ala Pro Ser Ser Lys Ser Thr Ser Gly Gly Thr Ala Ala Leu
   355 360 365
   Gly Cys Leu Val Lys Asp Tyr Phe Pro Glu Pro Val Thr Val Ser Trp
   370 375 380
   Asn Ser Gly Ala Leu Thr Ser Gly Val His Thr Phe Pro Ala Val Leu
   385 390 395 400
   Gln Ser Ser Gly Leu Tyr Ser Leu Ser Ser Val Val Thr Val Pro Ser
   405 410 415
   Ser Ser Leu Gly Thr Gln Thr Tyr Ile Cys Asn Val Asn His Lys Pro
   420 425 430
   Ser Asn Thr Lys Val Asp Lys Lys Val Glu Pro Lys Ser Cys Asp Lys
   435 440 445
   Thr His Thr Cys Pro Pro Cys Pro Ala Pro Glu Ala Ala Gly Gly Pro
   450 455 460
   Ser Val Phe Leu Phe Pro Pro Lys Pro Lys Asp Thr Leu Met Ile Ser
   465 470 475 480
   Arg Thr Pro Glu Val Thr Cys Val Val Val Asp Val Ser His Glu Asp
   485 490 495
   Pro Glu Val Lys Phe Asn Trp Tyr Val Asp Gly Val Glu Val His Asn
   500 505 510
   Ala Lys Thr Lys Pro Arg Glu Glu Gln Tyr Asn Ser Thr Tyr Arg Val
   515 520 525
   Val Ser Val Leu Thr Val Leu His Gln Asp Trp Leu Asn Gly Lys Glu
   530 535 540
   Tyr Lys Cys Lys Val Ser Asn Lys Ala Leu Gly Ala Pro Ile Glu Lys
   545 550 555 560
   Thr Ile Ser Lys Ala Lys Gly Gln Pro Arg Glu Pro Gln Val Tyr Thr
   565 570 575
   Leu Pro Pro Cys Arg Asp Glu Leu Thr Lys Asn Gln Val Ser Leu Trp
   580 585 590
   Cys Leu Val Lys Gly Phe Tyr Pro Ser Asp Ile Ala Val Glu Trp Glu
   595 600 605
   Ser Asn Gly Gln Pro Glu Asn Asn Tyr Lys Thr Thr Pro Pro Val Leu
   610 615 620
   Asp Ser Asp Gly Ser Phe Phe Leu Tyr Ser Lys Leu Thr Val Asp Lys
   625 630 635 640
   Ser Arg Trp Gln Gln Gly Asn Val Phe Ser Cys Ser Val Met His Glu
   645 650 655
   Ala Leu His Asn His Tyr Thr Gln Lys Ser Leu Ser Leu Ser Pro Gly
   660 665 670
   Lys
<210> 64
   <211> 2022
   <212> DNA
   <213> Artificial Sequence
<220>
   <223> CH1A1A (VH-CH1)- V9 (VL-CH1)-Fc(knob) P329G LALA
<400> 64
   caggtgcagc tggtgcagtc tggcgccgaa gtgaagaaac ctggcgccag cgtgaaggtg 60
   tcctgcaagg ccagcggcta caccttcacc gagttcggca tgaactgggt ccgacaggcc 120
   cctggacagg gcctggaatg gatgggctgg atcaacacca agaccggcga ggccacctac 180
   gtggaagagt tcaagggcag agtgaccttc accaccgaca ccagcaccag caccgcctac 240
   atggaactgc ggagcctgag aagcgacgac accgccgtgt actactgcgc cagatgggac 300
   ttcgcctact atgtggaagc catggactac tggggccagg gcaccaccgt gaccgtgtct 360
   agtgctagca caaagggccc cagcgtgttc cctctggccc ctagcagcaa gagcacatct 420
   ggcggaacag ccgccctggg ctgcctggtc aaggactact ttcccgagcc cgtgacagtg 480
   tcctggaact ctggcgccct gacaagcggc gtgcacacct ttccagccgt gctgcagagc 540
   agcggcctgt actctctgag cagcgtggtc accgtgccta gctctagcct gggcacccag 600
   acctacatct gcaacgtgaa ccacaagccc agcaacacca aggtggacaa gaaggtggaa 660
   cccaagagct gcggcggagg cggatccgga ggcggaggat ctgatatcca gatgacccag 720
   agccccagca gcctgtctgc cagcgtgggc gacagagtga ccattacctg cagagccagc 780
   caggacatca gaaactacct gaactggtat cagcagaagc ccggcaaggc ccccaagctg 840
   ctgatctact acaccagcag actggaatcc ggcgtgccca gcagattttc cggcagcggc 900
   tctggcaccg actacaccct gacaatcagc agcctgcagc ccgaggactt cgccacctac 960
   tactgccagc agggcaacac cctgccctgg acatttggac agggcacaaa ggtggaaatc 1020
   aagagcagcg cctccaccaa gggcccttcc gtgtttccac tggcccccag ctctaagagc 1080
   accagcggag gaacagctgc tctgggatgt ctcgtgaagg attacttccc cgaacctgtg 1140
   accgtcagct ggaacagcgg cgctctgaca tctggggtgc acacattccc cgctgtcctg 1200
   cagtcctccg gcctgtacag tctgtccagc gtcgtgacag tgcctagcag ctccctggga 1260
   acacagacat atatctgtaa tgtcaatcac aagccctcta ataccaaggt cgacaaaaaa 1320
   gtcgagccca agtcctgcga caagacccac acctgtcccc cttgtcctgc ccctgaagct 1380
   gctggcggcc cttctgtgtt cctgttcccc ccaaagccca aggacaccct gatgatcagc 1440
   cggacccccg aagtgacctg cgtggtggtg gatgtgtccc acgaggaccc tgaagtgaag 1500
   ttcaattggt acgtggacgg cgtggaagtg cacaacgcca agacaaagcc gcgggaggag 1560
   cagtacaaca gcacgtaccg tgtggtcagc gtcctcaccg tcctgcacca ggactggctg 1620
   aatggcaagg agtacaagtg caaggtctcc aacaaagccc tcggcgcccc catcgagaaa 1680
   accatctcca aagccaaagg gcagccccga gaaccacagg tgtacaccct gcccccatgc 1740
   cgggatgagc tgaccaagaa ccaggtcagc ctgtggtgcc tggtcaaagg cttctatccc 1800
   agcgacatcg ccgtggagtg ggagagcaat gggcagccgg agaacaacta caagaccacg 1860
   cctcccgtgc tggactccga cggctccttc ttcctctaca gcaagctcac cgtggacaag 1920
   agcaggtggc agcaggggaa cgtcttctca tgctccgtga tgcatgaggc tctgcacaac 1980
   cactacacgc agaagagcct ctccctgtct ccgggtaaat ga 2022
<210> 65
   <211> 451
   <212> PRT
   <213> Artificial Sequence
<220>
   <223> CH1A1A (VH-CH1)-Fc(hole) P329G LALA
<400> 65
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Ala Ala Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Gly Ala Pro Ile
   325 330 335
   Glu Lys Thr Ile Ser Lys Ala Lys Gly Gln Pro Arg Glu Pro Gln Val
   340 345 350
   Cys Thr Leu Pro Pro Ser Arg Asp Glu Leu Thr Lys Asn Gln Val Ser
   355 360 365
   Leu Ser Cys Ala Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Val Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 66
   <211> 1356
   <212> DNA
   <213> Artificial Sequence
<220>
   <223> CH1A1A (VH-CH1)-Fc(hole) P329G LALA
<400> 66
   caggtgcagc tggtgcagtc tggcgccgaa gtgaagaaac ctggagctag tgtgaaggtg      60
   tcctgcaagg ccagcggcta caccttcacc gagttcggca tgaactgggt ccgacaggct     120
   ccaggccagg gcctcgaatg gatgggctgg atcaacacca agaccggcga ggccacctac     180
   gtggaagagt tcaagggcag agtgaccttc accacggaca ccagcaccag caccgcctac     240
   atggaactgc ggagcctgag aagcgacgac accgccgtgt actactgcgc cagatgggac     300
   ttcgcctatt acgtggaagc catggactac tggggccagg gcaccaccgt gaccgtgtct     360
   agcgctagca ccaagggccc ctccgtgttc cccctggccc ccagcagcaa gagcaccagc     420
   ggcggcacag ccgctctggg ctgcctggtc aaggactact tccccgagcc cgtgaccgtg     480
   tcctggaaca gcggagccct gacctccggc gtgcacacct tccccgccgt gctgcagagt     540
   tctggcctgt atagcctgag cagcgtggtc accgtgcctt ctagcagcct gggcacccag     600
   acctacatct gcaacgtgaa ccacaagccc agcaacacca aggtggacaa gaaggtggag     660
   cccaagagct gcgacaaaac tcacacatgc ccaccgtgcc cagcacctga agctgcaggg     720
   ggaccgtcag tcttcctctt ccccccaaaa cccaaggaca ccctcatgat ctcccggacc     780
   cctgaggtca catgcgtggt ggtggacgtg agccacgaag accctgaggt caagttcaac     840
   tggtacgtgg acggcgtgga ggtgcataat gccaagacaa agccgcggga ggagcagtac     900
   aacagcacgt accgtgtggt cagcgtcctc accgtcctgc accaggactg gctgaatggc     960
   aaggagtaca agtgcaaggt ctccaacaaa gccctcggcg cccccatcga gaaaaccatc    1020
   tccaaagcca aagggcagcc ccgagaacca caggtgtgca ccctgccccc atcccgggat    1080
   gagctgacca agaaccaggt cagcctctcg tgcgcagtca aaggcttcta tcccagcgac    1140
   atcgccgtgg agtgggagag caatgggcag ccggagaaca actacaagac cacgcctccc    1200
   gtgctggact ccgacggctc cttcttcctc gtgagcaagc tcaccgtgga caagagcagg    1260
   tggcagcagg ggaacgtctt ctcatgctcc gtgatgcatg aggctctgca caaccactac    1320
   acgcagaaga gcctctccct gtctccgggt aaatga                               1356
<210> 67
   <211> 215
   <212> PRT
   <213> Artificial Sequence
<220>
   <223> CH1A1A (VL-CL)
<400> 67
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 68
   <211> 648
   <212> DNA
   <213> Artificial Sequence
<220>
   <223> CH1A1A (VL-CL)
<400> 68
   gatatccaga tgacccagtc tccatcctcc ctgtctgcat ctgtgggaga cagagtcacc        60
   atcacttgca aggccagtgc ggctgtgggt acgtatgttg cgtggtatca gcagaaacca       120
   gggaaagcac ctaagctcct gatctattcg gcatcctacc gcaaaagggg agtcccatca       180
   aggttcagtg gcagtggatc tgggacagat ttcactctca ccatcagcag tctgcaacct       240
   gaagatttcg caacttacta ctgtcaccaa tattacacct atcctctatt cacgtttggc       300
   cagggcacca agctcgagat caagcgtacg gtggctgcac catctgtctt catcttcccg       360
   ccatctgatg agcagttgaa atctggaact gcctctgttg tgtgcctgct gaataacttc       420
   tatcccagag aggccaaagt acagtggaag gtggataacg ccctccaatc gggtaactcc       480
   caggagagtg tcacagagca ggacagcaag gacagcacct acagcctcag cagcaccctg       540
   acgctgagca aagcagacta cgagaaacac aaagtctacg cctgcgaagt cacccatcag       600
   ggcctgagct cgcccgtcac aaagagcttc aacaggggag agtgttag                    648
<210> 69
   <211> 11
   <212> PRT
   <213> Artificial Sequence
<220>
   <223> LC007 HCDR1
<400> 69
   Gly Tyr Ser Ile Thr Ser Gly Tyr Tyr Trp Asn
   1               5                   10
<210> 70
   <211> 33
   <212> DNA
   <213> Artificial Sequence
<220>
   <223> LC007 HCDR1
<400> 70
   ggctactcca tcaccagtgg ttattactgg aac 33
<210> 71
   <211> 16
   <212> PRT
   <213> Artificial Sequence
<220>
   <223> LC007 HCDR2
<400> 71
   Tyr Ile Thr Tyr Asp Gly Ser Asn Asn Tyr Asn Pro Ser Leu Lys Asn
   1               5                   10                  15
<210> 72
   <211> 48
   <212> DNA
   <213> Artificial Sequence
<220>
   <223> LC007 HCDR2
<400> 72
   tacataacct acgacggtag caataactac aacccatctc tcaaaaat 48
<210> 73
   <211> 3
   <212> PRT
   <213> Artificial Sequence
<220>
   <223> LC007 HCDR3
<400> 73
   Phe Asp Tyr
   1
<210> 74
   <211> 9
   <212> DNA
   <213> Artificial Sequence
<220>
   <223> LC007 HCDR3
<400> 74
   tttgactac 9
<210> 75
   <211> 112
   <212> PRT
   <213> Artificial Sequence
<220>
   <223> LC007 VH
<400> 75
   Glu Val Gln Leu Gln Glu Ser Gly Pro Gly Leu Val Lys Pro Ser Gln
   1 5 10 15
   Ser Leu Ser Leu Thr Cys Ser Val Thr Gly Tyr Ser Ile Thr Ser Gly
   20 25 30
   Tyr Tyr Trp Asn Trp Ile Arg Gln Phe Pro Gly Asn Lys Leu Glu Trp
   35 40 45
   Met Gly Tyr Ile Thr Tyr Asp Gly Ser Asn Asn Tyr Asn Pro Ser Leu
   50 55 60
   Lys Asn Arg Ile Ser Ile Thr Arg Asp Thr Ser Lys Asn Gln Phe Phe
   65 70 75 80
   Leu Lys Leu Asn Ser Val Thr Thr Glu Asp Thr Ala Thr Tyr Tyr Cys
   85 90 95
   Ala Asp Phe Asp Tyr Trp Gly Gln Gly Thr Thr Leu Thr Val Ser Ser
   100 105 110
<210> 76
   <211> 336
   <212> DNA
   <213> Artificial Sequence
<220>
   <223> LC007 VH
<400> 76
   gaggtccagc tgcaggagtc aggacctggc ctcgtgaaac cttctcagtc tctgtctctc 60
   acctgctctg tcactggcta ctccatcacc agtggttatt actggaactg gatccggcag 120
   tttccaggaa acaagctgga atggatgggc tacataacct acgacggtag caataactac 180
   aacccatctc tcaaaaatcg aatctccatc actcgtgaca catctaagaa ccagtttttc 240
   ctgaagttga attctgtgac tactgaggac acagctacat attactgtgc ggactttgac 300
   tactggggcc aaggcaccac tctcacagtc tcctca 336
<210> 77
   <211> 11
   <212> PRT
   <213> Artificial Sequence
<220>
   <223> LC007 LCDR1
<400> 77
   Ser Ala Ser Gln Gly Ile Arg Asn Tyr Leu Asn
   1 5 10
<210> 78
   <211> 33
   <212> DNA
   <213> Artificial Sequence
<220>
   <223> LC007 LCDR1
<400> 78
   agtgcaagtc agggcattag aaattattta aac 33
<210> 79
   <211> 7
   <212> PRT
   <213> Artificial Sequence
<220>
   <223> LC007 LCDR2
<400> 79
   Tyr Thr Ser Ser Leu His Ser
   1 5
<210> 80
   <211> 21
   <212> DNA
   <213> Artificial Sequence
<220>
   <223> LC007 LCDR2
<400> 80
   tacacatcaa gtttacactc a 21
<210> 81
   <211> 9
   <212> PRT
   <213> Artificial Sequence
<220>
   <223> LC007 LCDR3
<400> 81
   Gln Gln Tyr Ser Lys Leu Pro Trp Thr
   1 5
<210> 82
   <211> 27
   <212> DNA
   <213> Artificial Sequence
<220>
   <223> LC007 LCDR3
<400> 82
   cagcagtata gtaagcttcc ttggacg 27
<210> 83
   <211> 107
   <212> PRT
   <213> Artificial Sequence
<220>
   <223> LC007 VL
<400> 83
   Asp Ile Val Leu Thr Gln Ser Pro Ser Ser Leu Ser Ala Ser Leu Gly
   1               5                   10                  15
   Asp Arg Val Thr Ile Ser Cys Ser Ala Ser Gln Gly Ile Arg Asn Tyr
               20                  25                  30
   Leu Asn Trp Tyr Gln Gln Arg Pro Asp Gly Thr Val Lys Leu Leu Ile
           35                  40                  45
   Tyr Tyr Thr Ser Ser Leu His Ser Gly Val Pro Ser Arg Phe Ser Gly
       50                  55                  60
   Ser Gly Ser Gly Thr Asp Tyr Ser Leu Thr Ile Ser Asn Leu Glu Pro
   65                  70                  75                  80
   Glu Asp Ile Ala Thr Tyr Tyr Cys Gln Gln Tyr Ser Lys Leu Pro Trp
                   85                  90                  95
   Thr Phe Gly Gly Gly Thr Lys Leu Glu Ile Lys
               100                 105
<210> 84
   <211> 321
   <212> DNA
   <213> Artificial Sequence
<220>
   <223> LC007 VL
<400> 84
   gatattgtgc tcacacagtc tccatcctcc ctgtctgcct ctctgggaga cagagtcacc        60
   atcagttgca gtgcaagtca gggcattaga aattatttaa actggtatca gcagagacca       120
   gatggaactg ttaaactcct gatctattac acatcaagtt tacactcagg agtcccatca       180
   aggttcagtg gcagtgggtc tgggacagat tattctctca ccatcagcaa cctggaacct       240
   gaagatattg ccacttacta ttgtcagcag tatagtaagc ttccttggac gttcggtgga       300
   ggcaccaagc tggaaatcaa a                                                 321
<210> 85
   <211> 5
   <212> PRT
   <213> Artificial Sequence
<220>
   <223> GA201 HCDR1
<400> 85
   Asp Tyr Lys Ile His
   1               5
<210> 86
   <211> 15
   <212> DNA
   <213> Artificial Sequence
<220>
   <223> GA201 HCDR1
<400> 86
   gactacaaga tacac 15
<210> 87
   <211> 17
   <212> PRT
   <213> Artificial Sequence
<220>
   <223> GA201 HCDR2
<400> 87
   Tyr Phe Asn Pro Asn Ser Gly Tyr Ser Thr Tyr Ala Gln Lys Phe Gln
   1               5                   10                  15
   Gly
<210> 88
   <211> 51
   <212> DNA
   <213> Artificial Sequence
<220>
   <223> GA201 HCDR2
<400> 88
   tatttcaacc ctaacagcgg ttatagtacc tacgcacaga agttccaggg c 51
<210> 89
   <211> 11
   <212> PRT
   <213> Artificial Sequence
<220>
   <223> GA201 HCDR3
<400> 89
   Leu Ser Pro Gly Gly Tyr Tyr Val Met Asp Ala
   1 5 10
<210> 90
   <211> 33
   <212> DNA
   <213> Artificial Sequence
<220>
   <223> GA201 HCDR3
<400> 90
   ctatccccag gcggttacta tgttatggat gcc 33
<210> 91
   <211> 120
   <212> PRT
   <213> Artificial Sequence
<220>
   <223> GA201 VH
<400> 91
<210> 92
   <211> 360
   <212> DNA
   <213> Artificial Sequence
<220>
   <223> GA201 VH
<400> 92
   caggtgcagc tggtgcagtc tggggctgag gtgaagaagc ctgggtcctc ggtgaaggtc 60
   tcctgcaagg cctctggttt cacattcact gactacaaga tacactgggt gcgacaggcc 120
   cctggacaag ggctcgagtg gatgggatat ttcaacccta acagcggtta tagtacctac 180
   gcacagaagt tccagggcag ggtcaccatt accgcggaca aatccacgag cacagcctac 240
   atggagctga gcagcctgag atctgaggac acggccgtgt attactgtgc gagactatcc 300
   ccaggcggtt actatgttat ggatgcctgg ggccaaggga ccaccgtgac cgtctcctca 360
<210> 93
   <211> 11
   <212> PRT
   <213> Artificial Sequence
<220>
   <223> GA201 LCDR1
<400> 93
   Arg Ala Ser Gln Gly Ile Asn Asn Tyr Leu Asn
   1 5 10
<210> 94
   <211> 33
   <212> DNA
   <213> Artificial Sequence
<220>
   <223> GA201 LCDR1
<400> 94
   cgggcaagtc agggcattaa caattactta aat 33
<210> 95
   <211> 7
   <212> PRT
   <213> Artificial Sequence
<220>
   <223> GA201 LCDR2
<400> 95
   Asn Thr Asn Asn Leu Gln Thr
   1 5
<210> 96
   <211> 21
   <212> DNA
   <213> Artificial Sequence
<220>
   <223> GA201 LCDR2
<400> 96
   aataccaaca acttgcagac a 21
<210> 97
   <211> 8
   <212> PRT
   <213> Artificial Sequence
<220>
   <223> GA201 LCDR3
<400> 97
   Leu Gln His Asn Ser Phe Pro Thr
   1 5
<210> 98
   <211> 24
   <212> DNA
   <213> Artificial Sequence
<220>
   <223> GA201 LCDR3
<400> 98
   ttgcagcata atagttttcc cacg 24
<210> 99
   <211> 106
   <212> PRT
   <213> Artificial Sequence
<220>
   <223> GA201 VL
<400> 99
<210> 100
   <211> 318
   <212> DNA
   <213> Artificial Sequence
<220>
   <223> GA201 VL
<400> 100
   gatatccaga tgacccagtc tccatcctcc ctgtctgcat ctgtcggaga ccgggtcacc      60
   atcacctgcc gggcaagtca gggcattaac aattacttaa attggtacca gcagaagcca     120
   gggaaagccc ctaagcgcct gatctataat accaacaact tgcagacagg cgtcccatca     180
   aggttcagcg gcagtggatc cgggacagaa ttcactctca ccatcagcag cctgcagcct     240
   gaagattttg ccacctatta ctgcttgcag cataatagtt ttcccacgtt tggccagggc     300
   accaagctcg agatcaag                                                   318
<210> 101
   <211> 5
   <212> PRT
   <213> Artificial Sequence
<220>
   <223> 3F2 HCDR1
<400> 101
   Ser Tyr Ala Met Ser
   1               5
<210> 102
   <211> 15
   <212> DNA
   <213> Artificial Sequence
<220>
   <223> 3F2 HCDR1
<400> 102
   agctacgcca tgagc 15
<210> 103
   <211> 16
   <212> PRT
   <213> Artificial Sequence
<220>
   <223> 3F2 HCDR2
<400> 103
   Ala Ile Ser Gly Ser Gly Gly Ser Thr Tyr Tyr Ala Asp Ser Val Lys
   1               5                   10                  15
<210> 104
   <211> 48
   <212> DNA
   <213> Artificial Sequence
<220>
   <223> 3F2 HCDR2
<400> 104
   gccatctccg gcagcggagg cagcacctac tacgccgaca gcgtgaag 48
<210> 105
   <211> 8
   <212> PRT
   <213> Artificial Sequence
<220>
   <223> 3F2 HCDR3
<400> 105
   Tyr Cys Ala Lys Gly Trp Phe Gly
   1 5
<210> 106
   <211> 24
   <212> DNA
   <213> Artificial Sequence
<220>
   <223> 3F2 HCDR3
<400> 106
   tattgcgcca agggatggtt cggc 24
<210> 107
   <211> 117
   <212> PRT
   <213> Artificial Sequence
<220>
   <223> 3F2 VH
<400> 107
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 108
   <211> 351
   <212> DNA
   <213> Artificial Sequence
<220>
   <223> 3F2 VH
<400> 108
   gaggtgcagc tgctggaatc tggaggcggc ctggtgcagc ctggcggcag cctgagactg 60
   tcttgcgccg ccagcggctt caccttcagc agctacgcca tgagctgggt ccgacaggct 120
   cctggcaagg gactggaatg ggtgtccgcc atctccggca gcggaggcag cacctactac 180
   gccgacagcg tgaagggccg gttcaccatc agcagagaca acagcaagaa caccctgtac 240
   ctgcagatga acagcctgcg ggccgaggat accgccgtgt attattgcgc caagggatgg 300
   ttcggcggct tcaactactg gggccaggga accctggtga cagtgtccag c 351
<210> 109
   <211> 11
   <212> PRT
   <213> Artificial Sequence
<220>
   <223> 3F2 LCDR1
<400> 109
   Arg Ala Ser Gln Ser Val Thr Ser Ser Tyr Leu
   1               5                   10
<210> 110
   <211> 33
   <212> DNA
   <213> Artificial Sequence
<220>
   <223> 3F2 LCDR1
<400> 110
   agagccagcc agagcgtgac cagcagctac ctg 33
<210> 111
   <211> 7
   <212> PRT
   <213> Artificial Sequence
<220>
   <223> 3F2 LCDR2
<400> 111
   Asn Val Gly Ser Arg Arg Ala
   1               5
<210> 112
   <211> 21
   <212> DNA
   <213> Artificial Sequence
<220>
   <223> 3F2 LCDR2
<400> 112
   aacgtgggca gcagacgggc c 21
<210> 113
   <211> 9
   <212> PRT
   <213> Artificial Sequence
<220>
   <223> 3F2 LCDR3
<400> 113
   Cys Gln Gln Gly Ile Met Leu Pro Pro
   1               5
<210> 114
   <211> 27
   <212> DNA
   <213> Artificial Sequence
<220>
   <223> 3F2 LCDR3
<400> 114
   tgccagcagg gcatcatgct gcccccc 27
<210> 115
   <211> 108
   <212> PRT
   <213> Artificial Sequence
<220>
   <223> 3F2 VL
<400> 115
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 116
   <211> 324
   <212> DNA
   <213> Artificial Sequence
<220>
   <223> 3F2 VL
<400> 116
   gagatcgtgc tgacccagtc tcccggcacc ctgagcctga gccctggcga gagagccacc 60
   ctgagctgca gagccagcca gagcgtgacc agcagctacc tggcctggta tcagcagaag 120
   cccggccagg cccccagact gctgatcaac gtgggcagca gacgggccac cggcatcccc 180
   gatagattca gcggcagcgg ctccggcacc gacttcaccc tgaccatcag ccggctggaa 240
   cccgaggact tcgccgtgta ctactgccag cagggcatca tgctgccccc caccttcggc 300
   cagggcacca aggtggaaat caag 324
<210> 117
   <211> 5
   <212> PRT
   <213> Artificial Sequence
<220>
   <223> CH1A1A HCDR1
<400> 117
   Glu Phe Gly Met Asn
   1 5
<210> 118
   <211> 15
   <212> DNA
   <213> Artificial Sequence
<220>
   <223> CH1A1A HCDR1
<400> 118
   gagttcggca tgaac 15
<210> 119
   <211> 17
   <212> PRT
   <213> Artificial Sequence
<220>
   <223> CH1A1A HCDR2
<400> 119
   Trp Ile Asn Thr Lys Thr Gly Glu Ala Thr Tyr Val Glu Glu Phe Lys
   1 5 10 15
   Gly
<210> 120
   <211> 51
   <212> DNA
   <213> Artificial Sequence
<220>
   <223> CH1A1A HCDR2
<400> 120
   tggatcaaca ccaagaccgg cgaggccacc tacgtggaag agttcaaggg c 51
<210> 121
   <211> 12
   <212> PRT
   <213> Artificial Sequence
<220>
   <223> CH1A1A HCDR3
<400> 121
   Trp Asp Phe Ala Tyr Tyr Val Glu Ala Met Asp Tyr
   1 5 10
<210> 122
   <211> 36
   <212> DNA
   <213> Artificial Sequence
<220>
   <223> CH1A1A HCDR3
<400> 122
   tgggacttcg cctattacgt ggaagccatg gactac 36
<210> 123
   <211> 121
   <212> PRT
   <213> Artificial Sequence
<220>
   <223> CH1A1A VH
<400> 123
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser
           115                 120
<210> 124
   <211> 363
   <212> DNA
   <213> Artificial Sequence
<220>
   <223> CH1A1A VH
<400> 124
   caggtgcagc tggtgcagtc tggcgccgaa gtgaagaaac ctggagctag tgtgaaggtg      60
   tcctgcaagg ccagcggcta caccttcacc gagttcggca tgaactgggt ccgacaggct     120
   ccaggccagg gcctcgaatg gatgggctgg atcaacacca agaccggcga ggccacctac     180
   gtggaagagt tcaagggcag agtgaccttc accacggaca ccagcaccag caccgcctac     240
   atggaactgc ggagcctgag aagcgacgac accgccgtgt actactgcgc cagatgggac     300
   ttcgcctatt acgtggaagc catggactac tggggccagg gcaccaccgt gaccgtgtct     360
   agc                                                                   363
<210> 125
   <211> 11
   <212> PRT
   <213> Artificial Sequence
<220>
   <223> CH1A1A LCDR1
<400> 125
   Lys Ala Ser Ala Ala Val Gly Thr Tyr Val Ala
   1 5 10
<210> 126
   <211> 33
   <212> DNA
   <213> Artificial Sequence
<220>
   <223> CH1A1A LCDR1
<400> 126
   aaggccagtg cggctgtggg tacgtatgtt gcg 33
<210> 127
   <211> 7
   <212> PRT
   <213> Artificial Sequence
<220>
   <223> CH1A1A LCDR2
<400> 127
   Ser Ala Ser Tyr Arg Lys Arg
   1 5
<210> 128
   <211> 21
   <212> DNA
   <213> Artificial Sequence
<220>
   <223> CH1A1A LCDR2
<400> 128
   tcggcatcct accgcaaaag g 21
<210> 129
   <211> 10
   <212> PRT
   <213> Artificial Sequence
<220>
   <223> CH1A1A LCDR3
<400> 129
   His Gln Tyr Tyr Thr Tyr Pro Leu Phe Thr
   1 5 10
<210> 130
   <211> 30
   <212> DNA
   <213> Artificial Sequence
<220>
   <223> CH1A1A LCDR3
<400> 130
   caccaatatt acacctatcc tctattcacg 30
<210> 131
   <211> 108
   <212> PRT
   <213> Artificial Sequence
<220>
   <223> CH1A1A VL
<400> 131
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys
               100                 105
<210> 132
   <211> 324
   <212> DNA
   <213> Artificial Sequence
<220>
   <223> CH1A1A VL
<400> 132
   gatatccaga tgacccagtc tccatcctcc ctgtctgcat ctgtgggaga cagagtcacc 60
   atcacttgca aggccagtgc ggctgtgggt acgtatgttg cgtggtatca gcagaaacca 120
   gggaaagcac ctaagctcct gatctattcg gcatcctacc gcaaaagggg agtcccatca 180
   aggttcagtg gcagtggatc tgggacagat ttcactctca ccatcagcag tctgcaacct 240
   gaagatttcg caacttacta ctgtcaccaa tattacacct atcctctatt cacgtttggc 300
   cagggcacca agctcgagat caag 324
<210> 133
   <211> 10
   <212> PRT
   <213> Artificial Sequence
<220>
   <223> Anti-CD33 HCDR1
<400> 133
   Gly Tyr Thr Ile Thr Asp Ser Asn Ile His
   1 5 10
<210> 134
   <211> 30
   <212> DNA
   <213> Artificial Sequence
<220>
   <223> Anti-CD33 HCDR1
<400> 134
   ggctacacca tcaccgacag caacatccac 30
<210> 135
   <211> 13
   <212> PRT
   <213> Artificial Sequence
<220>
   <223> Anti-CD33 HCDR2
<400> 135
   Tyr Ile Tyr Pro Tyr Asn Gly Gly Thr Asp Tyr Asn Gln
   1 5 10
<210> 136
   <211> 39
   <212> DNA
   <213> Artificial Sequence
<220>
   <223> Anti-CD33 HCDR2
<400> 136
   tacatctacc cctacaacgg cggcaccgac tacaaccag 39
<210> 137
   <211> 7
   <212> PRT
   <213> Artificial Sequence
<220>
   <223> Anti-CD33 HCDR3
<400> 137
   Gly Asn Pro Trp Leu Ala Tyr
   1               5
<210> 138
   <211> 21
   <212> DNA
   <213> Artificial Sequence
<220>
   <223> Anti-CD33 HCDR3
<400> 138
   ggcaacccct ggctggccta t 21
<210> 139
   <211> 116
   <212> PRT
   <213> Artificial Sequence
<220>
   <223> Anti-CD33 VH
<400> 139
<210> 140
   <211> 348
   <212> DNA
   <213> Artificial Sequence
<220>
   <223> Anti-CD33 VH
<400> 140
   gaagtgcagc tggtgcagtc tggcgccgaa gtgaagaaac ccggcagcag cgtgaaggtg       60
   tcctgcaagg ccagcggcta caccatcacc gacagcaaca tccactgggt ccgacaggcc      120
   cctgggcaga gcctggaatg gatcggctac atctacccct acaacggcgg caccgactac      180
   aaccagaagt tcaagaaccg ggccaccctg accgtggaca accccaccaa caccgcctac      240
   atggaactga gcagcctgcg gagcgaggac accgccttct actactgcgt gaacggcaac      300
   ccctggctgg cctattgggg ccagggaacc ctggtcaccg tgtctagc                   348
<210> 141
   <211> 15
   <212> PRT
   <213> Artificial Sequence
<220>
   <223> Anti-CD33 LCDR1
<400> 141
   Arg Ala Ser Glu Ser Leu Asp Asn Tyr Gly Ile Arg Phe Leu Thr
   1               5                   10                  15
<210> 142
   <211> 45
   <212> DNA
   <213> Artificial Sequence
<220>
   <223> Anti-CD33 LCDR1
<400> 142
   cgggccagcg agagcctgga caactacggc atccggtttc tgacc 45
<210> 143
   <211> 7
   <212> PRT
   <213> Artificial Sequence
<220>
   <223> Anti-CD33 LCDR2
<400> 143
   Ala Ala Ser Asn Gln Gly Ser
   1               5
<210> 144
   <211> 21
   <212> DNA
   <213> Artificial Sequence
<220>
   <223> Anti-CD33 LCDR2
<400> 144
   gccgccagca accagggcag c 21
<210> 145
   <211> 9
   <212> PRT
   <213> Artificial Sequence
<220>
   <223> Anti-CD33 LCDR3
<400> 145
   Gln Gln Thr Lys Glu Val Pro Trp Ser
   1               5
<210> 146
   <211> 27
   <212> DNA
   <213> Artificial Sequence
<220>
   <223> Anti-CD33 LCDR3
<400> 146
   cagcagacca aagaggtgcc ctggtcc 27
<210> 147
   <211> 111
   <212> PRT
   <213> Artificial Sequence
<220>
   <223> Anti-CD33 VL
<400> 147
<210> 148
   <211> 333
   <212> DNA
   <213> Artificial Sequence
<220>
   <223> Anti-CD33 VL
<400> 148
   gacatccagc tgacccagag ccccagcacc ctgtctgcca gcgtgggcga cagagtgacc 60
   atcacctgtc gggccagcga gagcctggac aactacggca tccggtttct gacctggttc 120
   cagcagaagc ccggcaaggc ccccaagctg ctgatgtacg ccgccagcaa ccagggcagc 180
   ggcgtgccaa gcagattcag cggcagcggc tccggcaccg agttcaccct gaccatcagc 240
   agcctgcagc ccgacgactt cgccacctac tactgccagc agaccaaaga ggtgccctgg 300
   tccttcggcc agggcaccaa ggtggaagtg aag 333
<210> 149
   <211> 227
   <212> PRT
   <213> Homo sapiens
<400> 149
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 150
   <211> 15
   <212> PRT
   <213> Artificial Sequence
<220>
   <223> Linker
<400> 150
   Glu Pro Lys Ser Cys Gly Gly Gly Gly Ser Gly Gly Gly Gly Ser
   1               5                   10                  15
<210> 151
   <211> 16
   <212> PRT
   <213> Artificial Sequence
<220>
   <223> Linker
<400> 151
   Glu Pro Lys Ser Cys Asp Gly Gly Gly Gly Ser Gly Gly Gly Gly Ser
   1               5                   10                  15
<210> 152
   <211> 32
   <212> PRT
   <213> Artificial Sequence
<220>
   <223> Linker
<400> 152
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser Gly Gly Gly Gly Ser Gly Gly
               20                  25                  30
<210> 153
   <211> 34
   <212> PRT
   <213> Artificial Sequence
<220>
   <223> Linker
<400> 153
   Ser Gly Gly Gly Ser Gly Gly Gly Ser Glu Gly Gly Gly Ser Glu Gly
   1               5                   10                  15
   Gly Gly Ser Glu Gly Gly Gly Ser Glu Gly Gly Gly Ser Gly Gly Gly
               20                  25                  30
   Ser Gly
<210> 154
   <211> 19
   <212> PRT
   <213> Artificial Sequence
<220>
   <223> Leader 1
<400> 154
   Met Asp Trp Thr Trp Arg Ile Leu Phe Leu Val Ala Ala Ala Thr Gly
   1               5                   10                  15
   Ala His Ser
<210> 155
   <211> 57
   <212> DNA
   <213> Artificial Sequence
<220>
   <223> Leader 1
<400> 155
   atggactgga cctggagaat cctcttcttg gtggcagcag ccacaggagc ccactcc 57
<210> 156
   <211> 57
   <212> DNA
   <213> Artificial Sequence
<220>
   <223> Leader 1
<400> 156
   atggactgga cctggaggat cctcttcttg gtggcagcag ccacaggagc ccactcc 57
<210> 157
   <211> 22
   <212> PRT
   <213> Artificial Sequence
<220>
   <223> Leader 2
<400> 157
   Met Asp Met Arg Val Pro Ala Gln Leu Leu Gly Leu Leu Leu Leu Trp
   1 5 10 15
   Phe Pro Gly Ala Arg Cys
   20
<210> 158
   <211> 66
   <212> DNA
   <213> Artificial Sequence
<220>
   <223> Leader 2
<400> 158
   atggacatga gggtccccgc tcagctcctg ggcctcctgc tgctctggtt cccaggtgcc 60
   aggtgt 66
<210> 159
   <211> 19
   <212> PRT
   <213> Artificial Sequence
<220>
   <223> Leader 3
<400> 159
   Met Gly Trp Ser Cys Ile Ile Leu Phe Leu Val Ala Thr Ala Thr Gly
   1 5 10 15
   Val His Ser
<210> 160
   <211> 57
   <212> DNA
   <213> Artificial Sequence
<220>
   <223> Leader 3
<400> 160
   atgggatgga gctgtatcat cctcttcttg gtagcaacag ctaccggtgt gcattcc 57
<210> 161
   <211> 57
   <212> DNA
   <213> Artificial Sequence
<220>
   <223> Leader 3
<400> 161
   atgggctggt cctgcatcat cctgtttctg gtggctaccg ccactggagt gcattcc 57
<210> 162
   <211> 57
   <212> DNA
   <213> Artificial Sequence
<220>
   <223> Leader 3
<400> 162
   atgggctggt cctgcatcat cctgtttctg gtcgccacag ccaccggcgt gcactct 57
<210> 163
   <211> 5
   <212> PRT
   <213> Artificial Sequence
<220>
   <223> V9 HCDR1
<400> 163
   Gly Tyr Thr Met Asn
   1 5
<210> 164
   <211> 15
   <212> DNA
   <213> Artificial Sequence
<220>
   <223> V9 HCDR1
<400> 164
   ggctacacca tgaac 15
<210> 165
   <211> 17
   <212> PRT
   <213> Artificial Sequence
<220>
   <223> V9 HCDR2
<400> 165
   Leu Ile Asn Pro Tyr Lys Gly Val Ser Thr Tyr Asn Gln Lys Phe Lys
   1 5 10 15
   Asp
<210> 166
   <211> 51
   <212> DNA
   <213> Artificial Sequence
<220>
   <223> V9 HCDR2
<400> 166
   ctgatcaacc cctacaaggg cgtgagcacc tacaaccaga agttcaagga c 51
<210> 167
   <211> 13
   <212> PRT
   <213> Artificial Sequence
<220>
   <223> V9 HCDR3
<400> 167
   Ser Gly Tyr Tyr Gly Asp Ser Asp Trp Tyr Phe Asp Val
   1 5 10
<210> 168
   <211> 39
   <212> DNA
   <213> Artificial Sequence
<220>
   <223> V9 HCDR3
<400> 168
   agcggctact acggcgacag cgactggtac ttcgacgtg 39
<210> 169
   <211> 122
   <212> PRT
   <213> Artificial Sequence
<220>
   <223> V9 VH
<400> 169
<210> 170
   <211> 366
   <212> DNA
   <213> Artificial Sequence
<220>
   <223> V9 VH
<400> 170
   gaggtgcagc tggtcgagtc cggcggaggc ctggtgcagc ctggcggcag cctgagactg      60
   agctgcgccg ccagcggcta cagcttcacc ggctacacca tgaactgggt ccggcaggct     120
   cctggcaagg gcctcgaatg ggtggccctg atcaacccct acaagggcgt gagcacctac     180
   aaccagaagt tcaaggaccg gttcaccatc agcgtggaca agagcaagaa caccgcctat     240
   ctgcagatga acagcctgcg ggccgaggac accgccgtgt actactgcgc cagaagcggc     300
   tactacggcg acagcgactg gtacttcgac gtgtggggcc agggcacact ggtcaccgtg     360
   tccagc                                                                366
<210> 171
   <211> 11
   <212> PRT
   <213> Artificial Sequence
<220>
   <223> V9 LCDR1
<400> 171
   Arg Ala Ser Gln Asp Ile Arg Asn Tyr Leu Asn
   1 5 10
<210> 172
   <211> 33
   <212> DNA
   <213> Artificial Sequence
<220>
   <223> V9 LCDR1
<400> 172
   cgggccagcc aggacatcag aaactacctg aac 33
<210> 173
   <211> 7
   <212> PRT
   <213> Artificial Sequence
<220>
   <223> V9 LCDR2
<400> 173
   Tyr Thr Ser Arg Leu Glu Ser
   1 5
<210> 174
   <211> 21
   <212> DNA
   <213> Artificial Sequence
<220>
   <223> V9 LCDR2
<400> 174
   tacacctcta gactggaaag c 21
<210> 175
   <211> 9
   <212> PRT
   <213> Artificial Sequence
<220>
   <223> V9 LCDR3
<400> 175
   Gln Gln Gly Asn Thr Leu Pro Trp Thr
   1 5
<210> 176
   <211> 27
   <212> DNA
   <213> Artificial Sequence
<220>
   <223> V9 LCDR3
<400> 176
   cagcagggca acacactccc ctggacc 27
<210> 177
   <211> 107
   <212> PRT
   <213> Artificial Sequence
<220>
   <223> V9 VL
<400> 177
<210> 178
   <211> 321
   <212> DNA
   <213> Artificial Sequence
<220>
   <223> V9 VL
<400> 178
   gacatccaga tgacccagag cccctctagc ctgagcgcca gcgtgggcga cagagtgacc 60
   atcacctgtc gggccagcca ggacatcaga aactacctga actggtatca gcagaagccc 120
   ggcaaggccc ccaagctgct gatctactac acctctagac tggaaagcgg cgtgcccagc 180
   cggtttagcg gcagcggctc cggcaccgac tacaccctga ccatcagcag cctgcagccc 240
   gaggacttcg ccacctacta ctgccagcag ggcaacacac tcccctggac cttcggccag 300
   ggcaccaagg tggagatcaa g 321
<210> 179
   <211> 454
   <212> PRT
   <213> Artificial Sequence
<220>
   <223> V9(VH-CL)-LC007(VL-CL)
<400> 179
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Ser Phe Thr Gly Tyr
   20 25 30
   Thr Met Asn Trp Val Arg Gln Ala Pro Gly Lys Gly Leu Glu Trp Val
   35 40 45
   Ala Leu Ile Asn Pro Tyr Lys Gly Val Ser Thr Tyr Asn Gln Lys Phe
   50 55 60
   Lys Asp Arg Phe Thr Ile Ser Val Asp Lys Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Ser Gly Tyr Tyr Gly Asp Ser Asp Trp Tyr Phe Asp Val Trp
   100 105 110
   Gly Gln Gly Thr Leu Val Thr Val Ser Ser Ala Ser Val Ala Ala Pro
   115 120 125
   Ser Val Phe Ile Phe Pro Pro Ser Asp Glu Gln Leu Lys Ser Gly Thr
       130                 135                 140
   Ala Ser Val Val Cys Leu Leu Asn Asn Phe Tyr Pro Arg Glu Ala Lys
   145                 150                 155                 160
   Val Gln Trp Lys Val Asp Asn Ala Leu Gln Ser Gly Asn Ser Gln Glu
                   165                 170                 175
   Ser Val Thr Glu Gln Asp Ser Lys Asp Ser Thr Tyr Ser Leu Ser Ser
               180                 185                 190
   Thr Leu Thr Leu Ser Lys Ala Asp Tyr Glu Lys His Lys Val Tyr Ala
           195                 200                 205
   Cys Glu Val Thr His Gln Gly Leu Ser Ser Pro Val Thr Lys Ser Phe
       210                 215                 220
   Asn Arg Gly Glu Cys Asp Gly Gly Gly Gly Ser Gly Gly Gly Gly Ser
   225                 230                 235                 240
   Asp Ile Val Leu Thr Gln Ser Pro Ser Ser Leu Ser Ala Ser Leu Gly
                   245                 250                 255
   Asp Arg Val Thr Ile Ser Cys Ser Ala Ser Gln Gly Ile Arg Asn Tyr
               260                 265                 270
   Leu Asn Trp Tyr Gln Gln Arg Pro Asp Gly Thr Val Lys Leu Leu Ile
           275                 280                 285
   Tyr Tyr Thr Ser Ser Leu His Ser Gly Val Pro Ser Arg Phe Ser Gly
       290                 295                 300
   Ser Gly Ser Gly Thr Asp Tyr Ser Leu Thr Ile Ser Asn Leu Glu Pro
   305                 310                 315                 320
   Glu Asp Ile Ala Thr Tyr Tyr Cys Gln Gln Tyr Ser Lys Leu Pro Trp
                   325                 330                 335
   Thr Phe Gly Gly Gly Thr Lys Leu Glu Ile Lys Arg Thr Val Ala Ala
               340                 345                 350
   Pro Ser Val Phe Ile Phe Pro Pro Ser Asp Glu Gln Leu Lys Ser Gly
           355                 360                 365
   Thr Ala Ser Val Val Cys Leu Leu Asn Asn Phe Tyr Pro Arg Glu Ala
       370                 375                 380
   Lys Val Gln Trp Lys Val Asp Asn Ala Leu Gln Ser Gly Asn Ser Gln
   385                 390                 395                 400
   Glu Ser Val Thr Glu Gln Asp Ser Lys Asp Ser Thr Tyr Ser Leu Ser
                   405                 410                 415
   Ser Thr Leu Thr Leu Ser Lys Ala Asp Tyr Glu Lys His Lys Val Tyr
               420                 425                 430
   Ala Cys Glu Val Thr His Gln Gly Leu Ser Ser Pro Val Thr Lys Ser
           435                 440                 445
   Phe Asn Arg Gly Glu Cys
       450
<210> 180
   <211> 1362
   <212> DNA
   <213> Artificial Sequence
<220>
   <223> V9(VH-CL)-LC007(VL-CL)
<400> 180
   gaggtgcagc tggtggaatc tggcggcgga ctggtgcagc ctggcggatc tctgagactg 60
   agctgtgccg ccagcggcta cagcttcacc ggctacacca tgaactgggt gcgccaggcc 120
   cctggcaagg gactggaatg ggtggccctg atcaacccct acaagggcgt gtccacctac 180
   aaccagaagt tcaaggaccg gttcaccatc agcgtggaca agagcaagaa caccgcctac 240
   ctgcagatga acagcctgcg ggccgaggac accgccgtgt actattgtgc cagaagcggc 300
   tactacggcg acagcgactg gtacttcgac gtgtggggcc agggcacact cgtgaccgtg 360
   tcaagcgcta gcgtggccgc tcccagcgtg ttcatcttcc cacctagcga cgagcagctg 420
   aagtccggca cagcctctgt cgtgtgcctg ctgaacaact tctacccccg cgaggccaag 480
   gtgcagtgga aggtggacaa tgccctgcag agcggcaaca gccaggaaag cgtgaccgag 540
   caggacagca aggatagcac ctacagcctg agcagcaccc tgaccctgag caaggccgac 600
   tacgagaagc acaaggtgta cgcctgcgaa gtgacccacc agggcctgtc tagccccgtg 660
   accaagagct tcaaccgggg cgagtgtgat ggcggaggcg gatccggggg aggcggctct 720
   gatattgtgc tgacccagag ccccagcagc ctgtctgcct ctctgggcga cagagtgacc 780
   atcagctgta gcgcctctca gggcatccgg aactacctga actggtatca gcagcggccc 840
   gacggcaccg tgaagctgct gatctactac accagctccc tgcactccgg cgtgcccagc 900
   agattttctg gcagcggctc cggcaccgac tactccctga ccatctccaa cctggaaccc 960
   gaggatatcg ccacctacta ctgccagcag tactccaagc tgccctggac ctttggaggc 1020
   ggcaccaagc tggaaatcaa gcgtacggtg gctgccccct ccgtgtttat ctttccccca 1080
   tccgatgaac agctgaaaag cggcaccgcc agcgtcgtgt gtctgctgaa caatttttac 1140
   cctagggaag ctaaagtgca gtggaaagtg gataacgcac tgcagtccgg caactcccag 1200
   gaatctgtga cagaacagga ctctaaggac agcacatact ccctgtcctc caccctgaca 1260
   ctgtctaagg ctgattatga gaaacacaaa gtgtatgctt gtgaagtgac acatcaggga 1320
   ctgagcagcc ctgtgacaaa gtccttcaac agaggcgagt gc 1362
<210> 181
   <211> 227
   <212> PRT
   <213> Artificial Sequence
<220>
   <223> Fc(knob) P329G LALA
<400> 181
   Asp Lys Thr His Thr Cys Pro Pro Cys Pro Ala Pro Glu Ala Ala Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Gly Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Cys Arg Asp Glu Leu Thr Lys Asn Gln Val Ser
       130                 135                 140
   Leu Trp Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 182
   <211> 681
   <212> DNA
   <213> Artificial Sequence
<220>
   <223> Fc(knob) P329G LALA
<400> 182
   gacaaaactc acacatgccc accgtgccca gcacctgaag ctgcaggggg accgtcagtc      60
   ttcctcttcc ccccaaaacc caaggacacc ctcatgatct cccggacccc tgaggtcaca     120
   tgcgtggtgg tggacgtgag ccacgaagac cctgaggtca agttcaactg gtacgtggac     180
   ggcgtggagg tgcataatgc caagacaaag ccgcgggagg agcagtacaa cagcacgtac     240
   cgtgtggtca gcgtcctcac cgtcctgcac caggactggc tgaatggcaa ggagtacaag     300
   tgcaaggtct ccaacaaagc cctcggcgcc cccatcgaga aaaccatctc caaagccaaa     360
   gggcagcccc gagaaccaca ggtgtacacc ctgcccccat gccgggatga gctgaccaag     420
   aaccaggtca gcctgtggtg cctggtcaaa ggcttctatc ccagcgacat cgccgtggag     480
   tgggagagca atgggcagcc ggagaacaac tacaagacca cgcctcccgt gctggactcc     540
   gacggctcct tcttcctcta cagcaagctc accgtggaca agagcaggtg gcagcagggg     600
   aacgtcttct catgctccgt gatgcatgag gctctgcaca accactacac gcagaagagc     660
   ctctccctgt ctccgggtaa a                                               681
<210> 183
   <211> 212
   <212> PRT
   <213> Artificial Sequence
<220>
   <223> V9(VL-CH1)
<400> 183
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys
       210
<210> 184
   <211> 636
   <212> DNA
   <213> Artificial Sequence
<220>
   <223> V9(VL-CH1)
<400> 184
   gatattcaga tgacccagag ccccagctct ctgagcgcca gcgtgggcga cagagtgacc      60
   atcacctgtc gggccagcca ggacatcaga aactacctga actggtatca gcagaagccc     120
   ggcaaggccc ccaagctgct gatctactac accagcagac tggaaagcgg cgtgccctcc     180
   agattttccg gcagcggctc cggcaccgac tacaccctga ccatcagcag cctgcagccc     240
   gaggatttcg ccacatatta ctgccagcag ggcaataccc tgccctggac cttcggacag     300
   ggcacaaaag tggaaatcaa gagcagcgct tccaccaaag gcccttccgt gtttcctctg     360
   gctcctagct ccaagtccac ctctggaggc accgctgctc tcggatgcct cgtgaaggat     420
   tattttcctg agcctgtgac agtgtcctgg aatagcggag cactgacctc tggagtgcat     480
   actttccccg ctgtgctgca gtcctctgga ctgtacagcc tgagcagcgt ggtgacagtg     540
   cccagcagca gcctgggcac ccagacctac atctgcaacg tgaaccacaa gcccagcaac     600
   accaaggtgg acaagaaggt ggaacccaag tcttgt                               636
<210> 185
   <211> 456
   <212> PRT
   <213> Artificial Sequence
<220>
   <223> V9(VH-CL)-Fc(knob) P329G LALA
<400> 185
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Gln Gly Thr Leu Val Thr Val Ser Ser Ala Ser Val Ala Ala Pro
           115                 120                 125
   Ser Val Phe Ile Phe Pro Pro Ser Asp Glu Gln Leu Lys Ser Gly Thr
       130                 135                 140
   Ala Ser Val Val Cys Leu Leu Asn Asn Phe Tyr Pro Arg Glu Ala Lys
   145 150 155 160
   Val Gln Trp Lys Val Asp Asn Ala Leu Gln Ser Gly Asn Ser Gln Glu
   165 170 175
   Ser Val Thr Glu Gln Asp Ser Lys Asp Ser Thr Tyr Ser Leu Ser Ser
   180 185 190
   Thr Leu Thr Leu Ser Lys Ala Asp Tyr Glu Lys His Lys Val Tyr Ala
   195 200 205
   Cys Glu Val Thr His Gln Gly Leu Ser Ser Pro Val Thr Lys Ser Phe
   210 215 220
   Asn Arg Gly Glu Cys Asp Lys Thr His Thr Cys Pro Pro Cys Pro Ala
   225 230 235 240
   Pro Glu Ala Ala Gly Gly Pro Ser Val Phe Leu Phe Pro Pro Lys Pro
   245 250 255
   Lys Asp Thr Leu Met Ile Ser Arg Thr Pro Glu Val Thr Cys Val Val
   260 265 270
   Val Asp Val Ser His Glu Asp Pro Glu Val Lys Phe Asn Trp Tyr Val
   275 280 285
   Asp Gly Val Glu Val His Asn Ala Lys Thr Lys Pro Arg Glu Glu Gln
   290 295 300
   Tyr Asn Ser Thr Tyr Arg Val Val Ser Val Leu Thr Val Leu His Gln
   305 310 315 320
   Asp Trp Leu Asn Gly Lys Glu Tyr Lys Cys Lys Val Ser Asn Lys Ala
   325 330 335
   Leu Gly Ala Pro Ile Glu Lys Thr Ile Ser Lys Ala Lys Gly Gln Pro
   340 345 350
   Arg Glu Pro Gln Val Tyr Thr Leu Pro Pro Cys Arg Asp Glu Leu Thr
   355 360 365
   Lys Asn Gln Val Ser Leu Trp Cys Leu Val Lys Gly Phe Tyr Pro Ser
   370 375 380
   Asp Ile Ala Val Glu Trp Glu Ser Asn Gly Gln Pro Glu Asn Asn Tyr
   385 390 395 400
   Lys Thr Thr Pro Pro Val Leu Asp Ser Asp Gly Ser Phe Phe Leu Tyr
   405 410 415
   Ser Lys Leu Thr Val Asp Lys Ser Arg Trp Gln Gln Gly Asn Val Phe
   420 425 430
   Ser Cys Ser Val Met His Glu Ala Leu His Asn His Tyr Thr Gln Lys
   435 440 445
   Ser Leu Ser Leu Ser Pro Gly Lys
   450 455
<210> 186
   <211> 1368
   <212> DNA
   <213> Artificial Sequence
<220>
   <223> V9(VH-CL)-Fc(knob) P329G LALA
<400> 186
   gaggtgcagc tggtcgagag cggaggcggc ctggtgcagc ctggcggcag cctgagactg 60
   agctgcgccg ccagcggcta cagcttcacc ggctacacca tgaactgggt ccggcaggca 120
   cctggcaagg gactggaatg ggtggccctg atcaacccct acaagggcgt gagcacctac 180
   aaccagaagt tcaaggaccg gttcaccatc agcgtggaca agagcaagaa caccgcctat 240
   ctgcagatga acagcctgcg ggccgaggac accgccgtgt actactgcgc cagaagcggc 300
   tactacggcg acagcgactg gtacttcgac gtgtggggcc agggcaccct cgtgaccgtg 360
   tctagcgcta gcgtggccgc tccctccgtg tttatctttc ccccatccga tgaacagctg 420
   aaaagcggca ccgcctccgt cgtgtgtctg ctgaacaatt tttaccctag ggaagctaaa 480
   gtgcagtgga aagtggataa cgcactgcag tccggcaact cccaggaatc tgtgacagaa 540
   caggactcca aggacagcac ctactccctg tcctccaccc tgacactgtc taaggctgat 600
   tatgagaaac acaaagtcta cgcctgcgaa gtcacccatc agggcctgag ctcgcccgtc 660
   acaaagagct tcaacagggg agagtgtgac aagacccaca cctgtccccc ttgtcctgcc 720
   cctgaagctg ctggcggccc ttctgtgttc ctgttccccc caaagcccaa ggacaccctg 780
   atgatcagcc ggacccccga agtgacctgc gtggtggtgg atgtgtccca cgaggaccct 840
   gaagtgaagt tcaattggta cgtggacggc gtggaagtgc acaacgccaa gacaaagccg 900
   cgggaggagc agtacaacag cacgtaccgt gtggtcagcg tcctcaccgt cctgcaccag 960
   gactggctga atggcaagga gtacaagtgc aaggtctcca acaaagccct cggcgccccc 1020
   atcgagaaaa ccatctccaa agccaaaggg cagccccgag aaccacaggt gtacaccctg 1080
   cccccatgcc gggatgagct gaccaagaac caggtcagcc tgtggtgcct ggtcaaaggc 1140
   ttctatccca gcgacatcgc cgtggagtgg gagagcaatg ggcagccgga gaacaactac 1200
   aagaccacgc ctcccgtgct ggactccgac ggctccttct tcctctacag caagctcacc 1260
   gtggacaaga gcaggtggca gcaggggaac gtcttctcat gctccgtgat gcatgaggct 1320
   ctgcacaacc actacacgca gaagagcctc tccctgtctc cgggtaaa 1368
<210> 187
   <211> 682
   <212> PRT
   <213> Artificial Sequence
<220>
   <223> LC007(VH-CH1)-V9(VH-CL)-Fc(knob) P329G LALA
<400> 187
   Glu Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Tyr Trp Asn Trp Ile Arg Gln Phe Pro Gly Asn Lys Leu Glu Trp
           35                  40                  45
   Met Gly Tyr Ile Thr Tyr Asp Gly Ser Asn Asn Tyr Asn Pro Ser Leu
       50                  55                  60
   Lys Asn Arg Ile Ser Ile Thr Arg Asp Thr Ser Lys Asn Gln Phe Phe
   65                  70                  75                  80
   Leu Lys Leu Asn Ser Val Thr Thr Glu Asp Thr Ala Thr Tyr Tyr Cys
                   85                  90                  95
   Ala Asp Phe Asp Tyr Trp Gly Gln Gly Thr Thr Leu Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Gly Gly Gly Gly Ser Gly Gly Gly
       210                 215                 220
   Gly Ser Glu Val Gln Leu Val Glu Ser Gly Gly Gly Leu Val Gln Pro
   225                 230                 235                 240
   Gly Gly Ser Leu Arg Leu Ser Cys Ala Ala Ser Gly Tyr Ser Phe Thr
                   245                 250                 255
   Gly Tyr Thr Met Asn Trp Val Arg Gln Ala Pro Gly Lys Gly Leu Glu
               260                 265                 270
   Trp Val Ala Leu Ile Asn Pro Tyr Lys Gly Val Ser Thr Tyr Asn Gln
           275                 280                 285
   Lys Phe Lys Asp Arg Phe Thr Ile Ser Val Asp Lys Ser Lys Asn Thr
       290                 295                 300
   Ala Tyr Leu Gln Met Asn Ser Leu Arg Ala Glu Asp Thr Ala Val Tyr
   305                 310                 315                 320
   Tyr Cys Ala Arg Ser Gly Tyr Tyr Gly Asp Ser Asp Trp Tyr Phe Asp
                   325                 330                 335
   Val Trp Gly Gln Gly Thr Leu Val Thr Val Ser Ser Ala Ser Val Ala
               340                 345                 350
   Ala Pro Ser Val Phe Ile Phe Pro Pro Ser Asp Glu Gln Leu Lys Ser
           355                 360                 365
   Gly Thr Ala Ser Val Val Cys Leu Leu Asn Asn Phe Tyr Pro Arg Glu
       370                 375                 380
   Ala Lys Val Gln Trp Lys Val Asp Asn Ala Leu Gln Ser Gly Asn Ser
   385                 390                 395                 400
   Gln Glu Ser Val Thr Glu Gln Asp Ser Lys Asp Ser Thr Tyr Ser Leu
                   405                 410                 415
   Ser Ser Thr Leu Thr Leu Ser Lys Ala Asp Tyr Glu Lys His Lys Val
               420                 425                 430
   Tyr Ala Cys Glu Val Thr His Gln Gly Leu Ser Ser Pro Val Thr Lys
           435                 440                 445
   Ser Phe Asn Arg Gly Glu Cys Asp Lys Thr His Thr Cys Pro Pro Cys
       450                 455                 460
   Pro Ala Pro Glu Ala Ala Gly Gly Pro Ser Val Phe Leu Phe Pro Pro
   465                 470                 475                 480
   Lys Pro Lys Asp Thr Leu Met Ile Ser Arg Thr Pro Glu Val Thr Cys
                   485                 490                 495
   Val Val Val Asp Val Ser His Glu Asp Pro Glu Val Lys Phe Asn Trp
               500                 505                 510
   Tyr Val Asp Gly Val Glu Val His Asn Ala Lys Thr Lys Pro Arg Glu
           515                 520                 525
   Glu Gln Tyr Asn Ser Thr Tyr Arg Val Val Ser Val Leu Thr Val Leu
       530                 535                 540
   His Gln Asp Trp Leu Asn Gly Lys Glu Tyr Lys Cys Lys Val Ser Asn
   545                 550                 555                 560
   Lys Ala Leu Gly Ala Pro Ile Glu Lys Thr Ile Ser Lys Ala Lys Gly
                   565                 570                 575
   Gln Pro Arg Glu Pro Gln Val Tyr Thr Leu Pro Pro Cys Arg Asp Glu
               580                 585                 590
   Leu Thr Lys Asn Gln Val Ser Leu Trp Cys Leu Val Lys Gly Phe Tyr
           595                 600                 605
   Pro Ser Asp Ile Ala Val Glu Trp Glu Ser Asn Gly Gln Pro Glu Asn
       610                 615                 620
   Asn Tyr Lys Thr Thr Pro Pro Val Leu Asp Ser Asp Gly Ser Phe Phe
   625                 630                 635                 640
   Leu Tyr Ser Lys Leu Thr Val Asp Lys Ser Arg Trp Gln Gln Gly Asn
                   645                 650                 655
   Val Phe Ser Cys Ser Val Met His Glu Ala Leu His Asn His Tyr Thr
               660                 665                 670
   Gln Lys Ser Leu Ser Leu Ser Pro Gly Lys
           675                 680
<210> 188
   <211> 2046
   <212> DNA
   <213> Artificial Sequence
<220>
   <223> LC007(VH-CH1)- V9(VH-CL)-Fc(knob) P329G LALA
<400> 188
   gaggtgcagc tgcaggaatc tggccctggc ctggtcaagc caagccagag tctgagcctg 60
   acctgcagcg tgaccggcta cagcattacc agcggctact actggaactg gattcggcag 120
   ttccccggca ataagctgga atggatgggc tacatcacct acgacggcag caacaactac 180
   aaccccagcc tgaagaaccg gatcagcatc acccgggaca ccagcaagaa ccagttcttc 240
   ctgaagctga acagcgtgac caccgaggac accgccacat actattgcgc cgacttcgac 300
   tactggggcc agggcaccac cctgaccgtg tccagcgcca gcacaaaggg ccctagcgtg 360
   ttccctctgg cccccagcag caagagcaca agcggcggaa cagccgccct gggctgcctc 420
   gtgaaggact acttccccga gcccgtgaca gtgtcttgga acagcggagc cctgacaagc 480
   ggcgtgcaca ccttccctgc cgtgctgcag agcagcggcc tgtactccct gagcagcgtg 540
   gtcaccgtgc ctagcagcag cctgggcacc cagacctaca tctgcaacgt gaaccacaag 600
   cccagcaaca ccaaagtgga caagaaggtg gagcccaaga gctgtgatgg cggaggaggg 660
   tccggaggcg gaggatccga agtgcagctg gtggaatctg gcggaggcct ggtgcagcct 720
   ggcggatctc tgagactgag ctgtgccgcc agcggctaca gcttcaccgg ctacaccatg 780
   aactgggtgc gccaggcccc tggcaaggga ctggaatggg tggccctgat caacccctac 840
   aagggcgtgt ccacatacaa ccagaagttc aaggaccggt tcaccatcag cgtggacaag 900
   agcaagaaca ccgcctacct gcagatgaac agcctgcggg ccgaggacac cgccgtgtac 960
   tattgtgcca gaagcggcta ctacggcgac agcgactggt acttcgacgt gtggggccag 1020
   ggcacactcg tgaccgtgtc aagcgctagc gtggccgctc cctccgtgtt tatctttccc 1080
   ccatccgatg aacagctgaa aagcggcacc gcctccgtcg tgtgtctgct gaacaatttt 1140
   taccctaggg aagctaaagt gcagtggaaa gtggataacg cactgcagtc cggcaactcc 1200
   caggaatctg tgacagaaca ggactccaag gacagcacct actccctgtc ctccaccctg 1260
   acactgtcta aggctgatta tgagaaacac aaagtctacg cctgcgaagt cacccatcag 1320
   ggcctgagct cgcccgtcac aaagagcttc aacaggggag agtgtgacaa gacccacacc 1380
   tgtccccctt gtcctgcccc tgaagctgct ggcggccctt ctgtgttcct gttcccccca 1440
   aagcccaagg acaccctgat gatcagccgg acccccgaag tgacctgcgt ggtggtggat 1500
   gtgtcccacg aggaccctga agtgaagttc aattggtacg tggacggcgt ggaagtgcac 1560
   aacgccaaga caaagccgcg ggaggagcag tacaacagca cgtaccgtgt ggtcagcgtc 1620
   ctcaccgtcc tgcaccagga ctggctgaat ggcaaggagt acaagtgcaa ggtctccaac 1680
   aaagccctcg gcgcccccat cgagaaaacc atctccaaag ccaaagggca gccccgagaa 1740
   ccacaggtgt acaccctgcc cccatgccgg gatgagctga ccaagaacca ggtcagcctg 1800
   tggtgcctgg tcaaaggctt ctatcccagc gacatcgccg tggagtggga gagcaatggg 1860
   cagccggaga acaactacaa gaccacgcct cccgtgctgg actccgacgg ctccttcttc 1920
   ctctacagca agctcaccgt ggacaagagc aggtggcagc aggggaacgt cttctcatgc 1980
   tccgtgatgc atgaggctct gcacaaccac tacacgcaga agagcctctc cctgtctccg 2040
   ggtaaa 2046
<210> 189
   <211> 214
   <212> PRT
   <213> Artificial Sequence
<220>
   <223> M4-3 ML2(VL-CL)
<400> 189
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Tyr
   20 25 30
   Leu Asn Trp Tyr Gln Gln Lys Pro Gly Lys Ala Pro Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Lys Leu Pro Trp
   85 90 95
   Thr Phe Gly Gln Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 190
   <211> 642
   <212> DNA
   <213> Artificial Sequence
<220>
   <223> M4-3 ML2(VL-CL)
<400> 190
   gacatccaga tgacccagag ccccagcagc ctgagcgcca gcgtgggcga cagagtgacc      60
   atcacctgcc gggccagcca gggcatccgg aactacctga actggtatca gcagaagccc     120
   ggcaaggccc ccaagctgct gatctactac accagcagcc tgcacagcgg cgtgcctagc     180
   cggtttagcg gcagcggctc cggcaccgac ttcaccctga ccattagctc cctgcagccc     240
   gaggacttcg ccacctacta ctgccagcag tacagcaagc tgccctggac cttcggccag     300
   ggaacaaagg tggagatcaa gcgtacggtg gctgcaccat ctgtcttcat cttcccgcca     360
   tctgatgagc agttgaaatc tggaactgcc tctgttgtgt gcctgctgaa taacttctat     420
   cccagagagg ccaaagtaca gtggaaggtg gataacgccc tccaatcggg taactcccag     480
   gagagtgtca cagagcagga cagcaaggac agcacctaca gcctcagcag caccctgacg     540
   ctgagcaaag cagactacga gaaacacaaa gtctacgcct gcgaagtcac ccatcagggc     600
   ctgagctcgc ccgtcacaaa gagcttcaac aggggagagt gt                        642
<210> 191
   <211> 664
   <212> PRT
   <213> Artificial Sequence
<220>
   <223> V9(VL-CH1)-M4-3 ML2(VH-CH1)-Fc(knob) P329G LALA
<400> 191
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Gln Gln Gly Asn Thr Leu Pro Trp
   85 90 95
   Thr Phe Gly Gln Gly Thr Lys Val Glu Ile Lys Ser Ser Ala Ser Thr
   100 105 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Lys Asp Tyr Phe Pro Glu
   130 135 140
   Pro Val Thr Val Ser Trp Asn Ser Gly Ala Leu Thr Ser Gly Val His
   145 150 155 160
   Thr Phe Pro Ala Val Leu Gln Ser Ser Gly Leu Tyr Ser Leu Ser Ser
   165 170 175
   Val Val Thr Val Pro Ser Ser Ser Leu Gly Thr Gln Thr Tyr Ile Cys
   180 185 190
   Asn Val Asn His Lys Pro Ser Asn Thr Lys Val Asp Lys Lys Val Glu
   195 200 205
   Pro Lys Ser Cys Gly Gly Gly Gly Ser Gly Gly Gly Gly Ser Gln Val
   210 215 220
   Gln Leu Gln Glu Ser Gly Pro Gly Leu Val Lys Pro Ser Gln Thr Leu
   225 230 235 240
   Ser Leu Thr Cys Thr Val Ser Gly Gly Ser Ile Thr Ser Gly Tyr Tyr
   245 250 255
   Trp Asn Trp Ile Arg Gln His Pro Gly Lys Gly Leu Glu Trp Ile Gly
   260 265 270
   Tyr Ile Thr Tyr Asp Gly Ser Asn Asn Tyr Asn Pro Ser Leu Lys Ser
   275 280 285
   Arg Val Thr Ile Ser Arg Asp Thr Ser Lys Asn Gln Phe Ser Leu Lys
   290 295 300
   Leu Ser Ser Val Thr Ala Ala Asp Thr Ala Val Tyr Tyr Cys Ala Asp
   305 310 315 320
   Phe Asp Tyr Trp Gly Gln Gly Thr Leu Val Thr Val Ser Ser Ala Ser
   325 330 335
   Thr Lys Gly Pro Ser Val Phe Pro Leu Ala Pro Ser Ser Lys Ser Thr
   340 345 350
   Ser Gly Gly Thr Ala Ala Leu Gly Cys Leu Val Lys Asp Tyr Phe Pro
   355 360 365
   Glu Pro Val Thr Val Ser Trp Asn Ser Gly Ala Leu Thr Ser Gly Val
   370 375 380
   His Thr Phe Pro Ala Val Leu Gln Ser Ser Gly Leu Tyr Ser Leu Ser
   385 390 395 400
   Ser Val Val Thr Val Pro Ser Ser Ser Leu Gly Thr Gln Thr Tyr Ile
   405 410 415
   Cys Asn Val Asn His Lys Pro Ser Asn Thr Lys Val Asp Lys Lys Val
   420 425 430
   Glu Pro Lys Ser Cys Asp Lys Thr His Thr Cys Pro Pro Cys Pro Ala
   435 440 445
   Pro Glu Ala Ala Gly Gly Pro Ser Val Phe Leu Phe Pro Pro Lys Pro
   450 455 460
   Lys Asp Thr Leu Met Ile Ser Arg Thr Pro Glu Val Thr Cys Val Val
   465 470 475 480
   Val Asp Val Ser His Glu Asp Pro Glu Val Lys Phe Asn Trp Tyr Val
   485 490 495
   Asp Gly Val Glu Val His Asn Ala Lys Thr Lys Pro Arg Glu Glu Gln
   500 505 510
   Tyr Asn Ser Thr Tyr Arg Val Val Ser Val Leu Thr Val Leu His Gln
   515 520 525
   Asp Trp Leu Asn Gly Lys Glu Tyr Lys Cys Lys Val Ser Asn Lys Ala
   530 535 540
   Leu Gly Ala Pro Ile Glu Lys Thr Ile Ser Lys Ala Lys Gly Gln Pro
   545 550 555 560
   Arg Glu Pro Gln Val Tyr Thr Leu Pro Pro Cys Arg Asp Glu Leu Thr
   565 570 575
   Lys Asn Gln Val Ser Leu Trp Cys Leu Val Lys Gly Phe Tyr Pro Ser
               580                 585                 590
   Asp Ile Ala Val Glu Trp Glu Ser Asn Gly Gln Pro Glu Asn Asn Tyr
           595                 600                 605
   Lys Thr Thr Pro Pro Val Leu Asp Ser Asp Gly Ser Phe Phe Leu Tyr
       610                 615                 620
   Ser Lys Leu Thr Val Asp Lys Ser Arg Trp Gln Gln Gly Asn Val Phe
   625                 630                 635                 640
   Ser Cys Ser Val Met His Glu Ala Leu His Asn His Tyr Thr Gln Lys
                   645                 650                 655
   Ser Leu Ser Leu Ser Pro Gly Lys
               660
<210> 192
   <211> 1992
   <212> DNA
   <213> Artificial Sequence
<220>
   <223> V9(VL-CH1)-M4-3 ML2(VH-CH1)-Fc(knob) P329G LALA (DNA)
<400> 192
   gatatccaga tgacccagag ccccagctct ctgagcgcca gcgtgggcga cagagtgacc      60
   atcacctgtc gggccagcca ggacatcaga aactacctga actggtatca gcagaagccc     120
   ggcaaggccc ccaagctgct gatctactac accagcagac tggaaagcgg cgtgccctcc     180
   agattttccg gcagcggctc cggcaccgac tacaccctga ccatcagcag cctgcagccc     240
   gaggatttcg ccacatatta ctgccagcag ggcaataccc tgccctggac cttcggacag     300
   ggcacaaaag tggaaatcaa gagcagcgct tccaccaaag gcccttccgt gtttcctctg     360
   gctcctagct ccaagtccac ctctggaggc accgctgctc tcggatgcct cgtgaaggat     420
   tattttcctg agcctgtgac agtgtcctgg aatagcggag cactgacctc tggagtgcat     480
   actttccccg ctgtgctgca gtcctctgga ctgtacagcc tgagcagcgt ggtgacagtg     540
   cccagcagca gcctgggcac ccagacctac atctgcaacg tgaaccacaa gcccagcaac     600
   accaaggtgg acaagaaggt ggaacccaag tcttgtggcg gaggcggatc cggcggaggg     660
   ggatctcagg tgcagctgca ggaaagcggc cctggcctgg tcaagcccag ccagaccctg     720
   agcctgacct gcaccgtgtc cggcggcagc atcaccagcg gctactactg gaactggatt     780
   cggcagcacc ccggcaaggg cctggaatgg atcggctaca tcacctacga cggcagcaac     840
   aactacaacc ccagcctgaa gtccagagtg accatcagcc gggacaccag caagaaccag     900
   ttcagcctga agctgtccag cgtgacagcc gccgacaccg ccgtgtacta ctgcgccgac     960
   ttcgactact ggggccaggg caccctggtc accgtgtcca gcgctagcac caagggcccc 1020
   agcgtgttcc ccctggcacc cagcagcaag agcacatctg gcggaacagc cgctctgggc 1080
   tgtctggtga aagactactt ccccgagccc gtgaccgtgt cttggaactc tggcgccctg 1140
   accagcggcg tgcacacctt tccagccgtg ctgcagagca gcggcctgta ctccctgtcc 1200
   tccgtggtca ccgtgccctc tagctccctg ggaacacaga catatatctg taatgtcaat 1260
   cacaagcctt ccaacaccaa agtcgataag aaagtcgagc ccaagagctg cgacaaaact 1320
   cacacatgcc caccgtgccc agcacctgaa gctgcagggg gaccgtcagt cttcctcttc 1380
   cccccaaaac ccaaggacac cctcatgatc tcccggaccc ctgaggtcac atgcgtggtg 1440
   gtggacgtga gccacgaaga ccctgaggtc aagttcaact ggtacgtgga cggcgtggag 1500
   gtgcataatg ccaagacaaa gccgcgggag gagcagtaca acagcacgta ccgtgtggtc 1560
   agcgtcctca ccgtcctgca ccaggactgg ctgaatggca aggagtacaa gtgcaaggtc 1620
   tccaacaaag ccctcggcgc ccccatcgag aaaaccatct ccaaagccaa agggcagccc 1680
   cgagaaccac aggtgtacac cctgccccca tgccgggatg agctgaccaa gaaccaggtc 1740
   agcctgtggt gcctggtcaa aggcttctat cccagcgaca tcgccgtgga gtgggagagc 1800
   aatgggcagc cggagaacaa ctacaagacc acgcctcccg tgctggactc cgacggctcc 1860
   ttcttcctct acagcaagct caccgtggac aagagcaggt ggcagcaggg gaacgtcttc 1920
   tcatgctccg tgatgcatga ggctctgcac aaccactaca cgcagaagag cctctccctg 1980
   tctccgggta aa 1992
<210> 193
   <211> 442
   <212> PRT
   <213> Artificial Sequence
<220>
   <223> M4-3 ML2(VH-CH1)-Fc(hole) P329G LALA
<400> 193
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
   Ala Ser Thr Lys Gly Pro Ser Val Phe Pro Leu Ala Pro Ser Ser Lys
           115                 120                 125
   Ser Thr Ser Gly Gly Thr Ala Ala Leu Gly Cys Leu Val Lys Asp Tyr
       130                 135                 140
   Phe Pro Glu Pro Val Thr Val Ser Trp Asn Ser Gly Ala Leu Thr Ser
   145                 150                 155                 160
   Gly Val His Thr Phe Pro Ala Val Leu Gln Ser Ser Gly Leu Tyr Ser
                   165                 170                 175
   Leu Ser Ser Val Val Thr Val Pro Ser Ser Ser Leu Gly Thr Gln Thr
               180                 185                 190
   Tyr Ile Cys Asn Val Asn His Lys Pro Ser Asn Thr Lys Val Asp Lys
           195                 200                 205
   Lys Val Glu Pro Lys Ser Cys Asp Lys Thr His Thr Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser His Glu Asp Pro Glu Val Lys Phe Asn Trp
               260                 265                 270
   Tyr Val Asp Gly Val Glu Val His Asn Ala Lys Thr Lys Pro Arg Glu
           275                 280                 285
   Glu Gln Tyr Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Ala Leu Gly Ala Pro Ile Glu Lys Thr Ile Ser Lys Ala Lys Gly
                   325                 330                 335
   Gln Pro Arg Glu Pro Gln Val Cys Thr Leu Pro Pro Ser Arg Asp Glu
   340 345 350
   Leu Thr Lys Asn Gln Val Ser Leu Ser Cys Ala Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Val Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 194
   <211> 1326
   <212> DNA
   <213> Artificial Sequence
<220>
   <223> M4-3 ML2(VH-CH1)-Fc(hole) P329G LALA
<400> 194
   caggtgcagc tgcaggaaag cggccctggc ctggtcaagc ccagccagac cctgagcctg      60
   acctgcaccg tgtccggcgg cagcatcacc agcggctact actggaactg gatccggcag     120
   caccccggca agggcctgga atggatcggc tacatcacct acgacggcag caacaactac     180
   aaccccagcc tgaagtccag agtgaccatc agccgggaca ccagcaagaa ccagttcagc     240
   ctgaagctgt ccagcgtgac agccgccgac accgccgtgt actactgcgc cgacttcgac     300
   tactggggcc agggcaccct ggtcaccgtg tccagcgcta gcaccaaggg cccctccgtg     360
   ttccccctgg cccccagcag caagagcacc agcggcggca cagccgctct gggctgcctg     420
   gtcaaggact acttccccga gcccgtgacc gtgtcctgga acagcggagc cctgacctcc     480
   ggcgtgcaca ccttccccgc cgtgctgcag agttctggcc tgtatagcct gagcagcgtg     540
   gtcaccgtgc cttctagcag cctgggcacc cagacctaca tctgcaacgt gaaccacaag     600
   cccagcaaca ccaaggtgga caagaaggtg gagcccaaga gctgcgacaa aactcacaca     660
   tgcccaccgt gcccagcacc tgaagctgca gggggaccgt cagtcttcct cttcccccca     720
   aaacccaagg acaccctcat gatctcccgg acccctgagg tcacatgcgt ggtggtggac     780
   gtgagccacg aagaccctga ggtcaagttc aactggtacg tggacggcgt ggaggtgcat     840
   aatgccaaga caaagccgcg ggaggagcag tacaacagca cgtaccgtgt ggtcagcgtc     900
   ctcaccgtcc tgcaccagga ctggctgaat ggcaaggagt acaagtgcaa ggtctccaac     960
   aaagccctcg gcgcccccat cgagaaaacc atctccaaag ccaaagggca gccccgagaa    1020
   ccacaggtgt gcaccctgcc cccatcccgg gatgagctga ccaagaacca ggtcagcctc    1080
   tcgtgcgcag tcaaaggctt ctatcccagc gacatcgccg tggagtggga gagcaatggg    1140
   cagccggaga acaactacaa gaccacgcct cccgtgctgg actccgacgg ctccttcttc    1200
   ctcgtgagca agctcaccgt ggacaagagc aggtggcagc aggggaacgt cttctcatgc    1260
   tccgtgatgc atgaggctct gcacaaccac tacacgcaga agagcctctc cctgtctccg    1320
   ggtaaa                                                               1326
<210> 195
   <211> 681
   <212> PRT
   <213> Artificial Sequence
<220>
   <223> V9(VH-CL)-M4-3 ML2(VH-CH1)-Fc(knob) P329G LALA
<400> 195
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Ser Phe Thr Gly Tyr
               20                  25                  30
   Thr Met Asn Trp Val Arg Gln Ala Pro Gly Lys Gly Leu Glu Trp Val
           35                  40                  45
   Ala Leu Ile Asn Pro Tyr Lys Gly Val Ser Thr Tyr Asn Gln Lys Phe
       50                  55                  60
   Lys Asp Arg Phe Thr Ile Ser Val Asp Lys Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Ser Gly Tyr Tyr Gly Asp Ser Asp Trp Tyr Phe Asp Val Trp
               100                 105                 110
   Gly Gln Gly Thr Leu Val Thr Val Ser Ser Ala Ser Val Ala Ala Pro
           115                 120                 125
   Ser Val Phe Ile Phe Pro Pro Ser Asp Glu Gln Leu Lys Ser Gly Thr
       130                 135                 140
   Ala Ser Val Val Cys Leu Leu Asn Asn Phe Tyr Pro Arg Glu Ala Lys
   145                 150                 155                 160
   Val Gln Trp Lys Val Asp Asn Ala Leu Gln Ser Gly Asn Ser Gln Glu
                   165                 170                 175
   Ser Val Thr Glu Gln Asp Ser Lys Asp Ser Thr Tyr Ser Leu Ser Ser
               180                 185                 190
   Thr Leu Thr Leu Ser Lys Ala Asp Tyr Glu Lys His Lys Val Tyr Ala
           195                 200                 205
   Cys Glu Val Thr His Gln Gly Leu Ser Ser Pro Val Thr Lys Ser Phe
       210                 215                 220
   Asn Arg Gly Glu Cys Gly Gly Gly Gly Ser Gly Gly Gly Gly Ser Gln
   225                 230                 235                 240
   Val Gln Leu Gln Glu Ser Gly Pro Gly Leu Val Lys Pro Ser Gln Thr
                   245                 250                 255
   Leu Ser Leu Thr Cys Thr Val Ser Gly Gly Ser Ile Thr Ser Gly Tyr
               260                 265                 270
   Tyr Trp Asn Trp Ile Arg Gln His Pro Gly Lys Gly Leu Glu Trp Ile
           275                 280                 285
   Gly Tyr Ile Thr Tyr Asp Gly Ser Asn Asn Tyr Asn Pro Ser Leu Lys
       290                 295                 300
   Ser Arg Val Thr Ile Ser Arg Asp Thr Ser Lys Asn Gln Phe Ser Leu
   305                 310                 315                 320
   Lys Leu Ser Ser Val Thr Ala Ala Asp Thr Ala Val Tyr Tyr Cys Ala
                   325                 330                 335
   Asp Phe Asp Tyr Trp Gly Gln Gly Thr Leu Val Thr Val Ser Ser Ala
               340                 345                 350
   Ser Thr Lys Gly Pro Ser Val Phe Pro Leu Ala Pro Ser Ser Lys Ser
           355                 360                 365
   Thr Ser Gly Gly Thr Ala Ala Leu Gly Cys Leu Val Lys Asp Tyr Phe
       370                 375                 380
   Pro Glu Pro Val Thr Val Ser Trp Asn Ser Gly Ala Leu Thr Ser Gly
   385                 390                 395                 400
   Val His Thr Phe Pro Ala Val Leu Gln Ser Ser Gly Leu Tyr Ser Leu
   405 410 415
   Ser Ser Val Val Thr Val Pro Ser Ser Ser Leu Gly Thr Gln Thr Tyr
   420 425 430
   Ile Cys Asn Val Asn His Lys Pro Ser Asn Thr Lys Val Asp Lys Lys
   435 440 445
   Val Glu Pro Lys Ser Cys Asp Lys Thr His Thr Cys Pro Pro Cys Pro
   450 455 460
   Ala Pro Glu Ala Ala Gly Gly Pro Ser Val Phe Leu Phe Pro Pro Lys
   465 470 475 480
   Pro Lys Asp Thr Leu Met Ile Ser Arg Thr Pro Glu Val Thr Cys Val
   485 490 495
   Val Val Asp Val Ser His Glu Asp Pro Glu Val Lys Phe Asn Trp Tyr
   500 505 510
   Val Asp Gly Val Glu Val His Asn Ala Lys Thr Lys Pro Arg Glu Glu
   515 520 525
   Gln Tyr Asn Ser Thr Tyr Arg Val Val Ser Val Leu Thr Val Leu His
   530 535 540
   Gln Asp Trp Leu Asn Gly Lys Glu Tyr Lys Cys Lys Val Ser Asn Lys
   545 550 555 560
   Ala Leu Gly Ala Pro Ile Glu Lys Thr Ile Ser Lys Ala Lys Gly Gln
   565 570 575
   Pro Arg Glu Pro Gln Val Tyr Thr Leu Pro Pro Cys Arg Asp Glu Leu
   580 585 590
   Thr Lys Asn Gln Val Ser Leu Trp Cys Leu Val Lys Gly Phe Tyr Pro
   595 600 605
   Ser Asp Ile Ala Val Glu Trp Glu Ser Asn Gly Gln Pro Glu Asn Asn
   610 615 620
   Tyr Lys Thr Thr Pro Pro Val Leu Asp Ser Asp Gly Ser Phe Phe Leu
   625 630 635 640
   Tyr Ser Lys Leu Thr Val Asp Lys Ser Arg Trp Gln Gln Gly Asn Val
   645 650 655
   Phe Ser Cys Ser Val Met His Glu Ala Leu His Asn His Tyr Thr Gln
   660 665 670
   Lys Ser Leu Ser Leu Ser Pro Gly Lys
   675 680
<210> 196
   <211> 2043
   <212> DNA
   <213> Artificial Sequence
<220>
   <223> V9(VH-CL)-M4-3 ML2(VH-CH1)-Fc(knob) P329G LALA (DNA)
<400> 196
   gaggtgcagc tggtcgagag cggaggcggc ctggtgcagc ctggcggcag cctgagactg 60
   agctgcgccg ccagcggcta cagcttcacc ggctacacca tgaactgggt ccggcaggca 120
   cctggcaagg gactggaatg ggtggccctg atcaacccct acaagggcgt gagcacctac 180
   aaccagaagt tcaaggaccg gttcaccatc agcgtggaca agagcaagaa caccgcctat 240
   ctgcagatga acagcctgcg ggccgaggac accgccgtgt actactgcgc cagaagcggc 300
   tactacggcg acagcgactg gtacttcgac gtgtggggcc agggcaccct cgtgaccgtg 360
   tctagcgcta gcgtggctgc accatctgtc ttcatcttcc cgccatctga tgagcagttg 420
   aaatctggaa ctgcctctgt tgtgtgcctg ctgaataact tctatcccag agaggccaaa 480
   gtacagtgga aggtggataa cgccctccaa tcgggtaact cccaggagag tgtcacagag 540
   caggacagca aggacagcac ctacagcctc agcagcaccc tgacgctgag caaagcagac 600
   tacgagaaac acaaagtcta cgcctgcgaa gtcacccatc agggcctgag ctcgcccgtc 660
   acaaagagct tcaacagggg agagtgtggc ggaggcggat ccggcggagg gggatctcag 720
   gtgcagctgc aggaaagcgg ccctggcctg gtcaagccca gccagaccct gagcctgacc 780
   tgcaccgtgt ccggcggcag catcaccagc ggctactact ggaactggat tcggcagcac 840
   cccggcaagg gcctggaatg gatcggctac atcacctacg acggcagcaa caactacaac 900
   cccagcctga agtccagagt gaccatcagc cgggacacca gcaagaacca gttcagcctg 960
   aagctgtcca gcgtgacagc cgccgacacc gccgtgtact actgcgccga cttcgactac 1020
   tggggccagg gcaccctggt caccgtgtcc agcgctagca ccaagggccc cagcgtgttc 1080
   cccctggcac ccagcagcaa gagcacatct ggcggaacag ccgctctggg ctgtctggtg 1140
   aaagactact tccccgagcc cgtgaccgtg tcttggaact ctggcgccct gaccagcggc 1200
   gtgcacacct ttccagccgt gctgcagagc agcggcctgt actccctgtc ctccgtggtc 1260
   accgtgccct ctagctccct gggaacacag acatatatct gtaatgtcaa tcacaagcct 1320
   tccaacacca aagtcgataa gaaagtcgag cccaagagct gcgacaaaac tcacacatgc 1380
   ccaccgtgcc cagcacctga agctgcaggg ggaccgtcag tcttcctctt ccccccaaaa 1440
   cccaaggaca ccctcatgat ctcccggacc cctgaggtca catgcgtggt ggtggacgtg 1500
   agccacgaag accctgaggt caagttcaac tggtacgtgg acggcgtgga ggtgcataat    1560
   gccaagacaa agccgcggga ggagcagtac aacagcacgt accgtgtggt cagcgtcctc    1620
   accgtcctgc accaggactg gctgaatggc aaggagtaca agtgcaaggt ctccaacaaa    1680
   gccctcggcg cccccatcga gaaaaccatc tccaaagcca aagggcagcc ccgagaacca    1740
   caggtgtaca ccctgccccc atgccgggat gagctgacca agaaccaggt cagcctgtgg    1800
   tgcctggtca aaggcttcta tcccagcgac atcgccgtgg agtgggagag caatgggcag    1860
   ccggagaaca actacaagac cacgcctccc gtgctggact ccgacggctc cttcttcctc    1920
   tacagcaagc tcaccgtgga caagagcagg tggcagcagg ggaacgtctt ctcatgctcc    1980
   gtgatgcatg aggctctgca caaccactac acgcagaaga gcctctccct gtctccgggt    2040
   aaa                                                                  2043
<210> 197
   <211> 690
   <212> PRT
   <213> Artificial Sequence
<220>
   <223> CH1A1A(VH-CH1)- V9(VH-CL)-Fc(knob) P329G LALA
<400> 197
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Gly Gly Gly Gly Ser Gly Gly Gly Gly Ser Glu Val Gln Leu Val Glu
   225 230 235 240
   Ser Gly Gly Gly Leu Val Gln Pro Gly Gly Ser Leu Arg Leu Ser Cys
   245 250 255
   Ala Ala Ser Gly Tyr Ser Phe Thr Gly Tyr Thr Met Asn Trp Val Arg
   260 265 270
   Gln Ala Pro Gly Lys Gly Leu Glu Trp Val Ala Leu Ile Asn Pro Tyr
   275 280 285
   Lys Gly Val Ser Thr Tyr Asn Gln Lys Phe Lys Asp Arg Phe Thr Ile
   290 295 300
   Ser Val Asp Lys Ser Lys Asn Thr Ala Tyr Leu Gln Met Asn Ser Leu
   305 310 315 320
   Arg Ala Glu Asp Thr Ala Val Tyr Tyr Cys Ala Arg Ser Gly Tyr Tyr
   325 330 335
   Gly Asp Ser Asp Trp Tyr Phe Asp Val Trp Gly Gln Gly Thr Leu Val
   340 345 350
   Thr Val Ser Ser Ala Ser Val Ala Ala Pro Ser Val Phe Ile Phe Pro
   355 360 365
   Pro Ser Asp Glu Gln Leu Lys Ser Gly Thr Ala Ser Val Val Cys Leu
   370 375 380
   Leu Asn Asn Phe Tyr Pro Arg Glu Ala Lys Val Gln Trp Lys Val Asp
   385 390 395 400
   Asn Ala Leu Gln Ser Gly Asn Ser Gln Glu Ser Val Thr Glu Gln Asp
                   405                 410                 415
   Ser Lys Asp Ser Thr Tyr Ser Leu Ser Ser Thr Leu Thr Leu Ser Lys
               420                 425                 430
   Ala Asp Tyr Glu Lys His Lys Val Tyr Ala Cys Glu Val Thr His Gln
           435                 440                 445
   Gly Leu Ser Ser Pro Val Thr Lys Ser Phe Asn Arg Gly Glu Cys Asp
       450                 455                 460
   Lys Thr His Thr Cys Pro Pro Cys Pro Ala Pro Glu Ala Ala Gly Gly
   465                 470                 475                 480
   Pro Ser Val Phe Leu Phe Pro Pro Lys Pro Lys Asp Thr Leu Met Ile
                   485                 490                 495
   Ser Arg Thr Pro Glu Val Thr Cys Val Val Val Asp Val Ser His Glu
               500                 505                 510
   Asp Pro Glu Val Lys Phe Asn Trp Tyr Val Asp Gly Val Glu Val His
           515                 520                 525
   Asn Ala Lys Thr Lys Pro Arg Glu Glu Gln Tyr Asn Ser Thr Tyr Arg
       530                 535                 540
   Val Val Ser Val Leu Thr Val Leu His Gln Asp Trp Leu Asn Gly Lys
   545                 550                 555                 560
   Glu Tyr Lys Cys Lys Val Ser Asn Lys Ala Leu Gly Ala Pro Ile Glu
                   565                 570                 575
   Lys Thr Ile Ser Lys Ala Lys Gly Gln Pro Arg Glu Pro Gln Val Tyr
               580                 585                 590
   Thr Leu Pro Pro Cys Arg Asp Glu Leu Thr Lys Asn Gln Val Ser Leu
           595                 600                 605
   Trp Cys Leu Val Lys Gly Phe Tyr Pro Ser Asp Ile Ala Val Glu Trp
       610                 615                 620
   Glu Ser Asn Gly Gln Pro Glu Asn Asn Tyr Lys Thr Thr Pro Pro Val
   625                 630                 635                 640
   Leu Asp Ser Asp Gly Ser Phe Phe Leu Tyr Ser Lys Leu Thr Val Asp
                   645                 650                 655
   Lys Ser Arg Trp Gln Gln Gly Asn Val Phe Ser Cys Ser Val Met His
   660 665 670
   Glu Ala Leu His Asn His Tyr Thr Gln Lys Ser Leu Ser Leu Ser Pro
   675 680 685
   Gly Lys
   690
<210> 198
   <211> 2070
   <212> DNA
   <213> Artificial Sequence
<220>
   <223> CH1A1A(VH-CH1)-V9(VH-CL)-Fc(knob) P329G LALA
<400> 198
   caggtgcagc tggtgcagtc tggcgccgaa gtgaagaaac ctggcgccag cgtgaaggtg        60
   tcctgcaagg ccagcggcta caccttcacc gagttcggca tgaactgggt ccgacaggcc       120
   cctggacagg gcctggaatg gatgggctgg atcaacacca agaccggcga ggccacctac       180
   gtggaagagt tcaagggcag agtgaccttc accaccgaca ccagcaccag caccgcctac       240
   atggaactgc ggagcctgag aagcgacgac accgccgtgt actactgcgc cagatgggac       300
   ttcgcctact atgtggaagc catggactac tggggccagg gcaccaccgt gaccgtgtct       360
   agtgctagca caaagggccc cagcgtgttc cctctggccc ctagcagcaa gagcacatct       420
   ggcggaacag ccgccctggg ctgcctggtc aaggactact ttcccgagcc cgtgacagtg        480
   tcctggaact ctggcgccct gacaagcggc gtgcacacct ttccagccgt gctgcagagc       540
   agcggcctgt actctctgag cagcgtggtc accgtgccta gctctagcct gggcacccag       600
   acctacatct gcaacgtgaa ccacaagccc agcaacacca aggtggacaa gaaggtggaa       660
   cccaagagct gcggcggagg cggatccgga ggcggaggat ccgaagtgca gctggtggaa       720
   tctggcggag gcctggtgca gcctggcgga tctctgagac tgagctgtgc cgccagcggc       780
   tacagcttca ccggctacac catgaactgg gtgcgccagg cccctggcaa gggactggaa       840
   tgggtggccc tgatcaaccc ctacaagggc gtgtccacat acaaccagaa gttcaaggac       900
   cggttcacca tcagcgtgga caagagcaag aacaccgcct acctgcagat gaacagcctg       960
   cgggccgagg acaccgccgt gtactattgt gccagaagcg gctactacgg cgacagcgac      1020
   tggtacttcg acgtgtgggg ccagggcaca ctcgtgaccg tgtcaagcgc tagcgtggcc      1080
   gctccctccg tgtttatctt tcccccatcc gatgaacagc tgaaaagcgg caccgcctcc      1140
   gtcgtgtgtc tgctgaacaa tttttaccct agggaagcta aagtgcagtg gaaagtggat      1200
   aacgcactgc agtccggcaa ctcccaggaa tctgtgacag aacaggactc caaggacagc      1260
   acctactccc tgtcctccac cctgacactg tctaaggctg attatgagaa acacaaagtc      1320
   tacgcctgcg aagtcaccca tcagggcctg agctcgcccg tcacaaagag cttcaacagg 1380
   ggagagtgtg acaagaccca cacctgtccc ccttgtcctg cccctgaagc tgctggcggc 1440
   ccttctgtgt tcctgttccc cccaaagccc aaggacaccc tgatgatcag ccggaccccc 1500
   gaagtgacct gcgtggtggt ggatgtgtcc cacgaggacc ctgaagtgaa gttcaattgg 1560
   tacgtggacg gcgtggaagt gcacaacgcc aagacaaagc cgcgggagga gcagtacaac 1620
   agcacgtacc gtgtggtcag cgtcctcacc gtcctgcacc aggactggct gaatggcaag 1680
   gagtacaagt gcaaggtctc caacaaagcc ctcggcgccc ccatcgagaa aaccatctcc 1740
   aaagccaaag ggcagccccg agaaccacag gtgtacaccc tgcccccatg ccgggatgag 1800
   ctgaccaaga accaggtcag cctgtggtgc ctggtcaaag gcttctatcc cagcgacatc 1860
   gccgtggagt gggagagcaa tgggcagccg gagaacaact acaagaccac gcctcccgtg 1920
   ctggactccg acggctcctt cttcctctac agcaagctca ccgtggacaa gagcaggtgg 1980
   cagcagggga acgtcttctc atgctccgtg atgcatgagg ctctgcacaa ccactacacg 2040
   cagaagagcc tctccctgtc tccgggtaaa 2070
<210> 199
   <211> 214
   <212> PRT
   <213> Artificial Sequence
<220>
   <223> H2C(VL-CH1)
<400> 199
   Gln Thr Val Val Thr Gln Glu Pro Ser Leu Thr Val Ser Pro Gly Gly
   1               5                   10                  15
   Thr Val Thr Leu Thr Cys Gly Ser Ser Thr Gly Ala Val Thr Ser Gly
               20                  25                  30
   Tyr Tyr Pro Asn Trp Val Gln Gln Lys Pro Gly Gln Ala Pro Arg Gly
           35                  40                  45
   Leu Ile Gly Gly Thr Lys Phe Leu Ala Pro Gly Thr Pro Ala Arg Phe
       50                  55                  60
   Ser Gly Ser Leu Leu Gly Gly Lys Ala Ala Leu Thr Leu Ser Gly Val
   65                  70                  75                  80
   Gln Pro Glu Asp Glu Ala Glu Tyr Tyr Cys Ala Leu Trp Tyr Ser Asn
                   85                  90                  95
   Arg Trp Val Phe Gly Gly Gly Thr Lys Leu Thr Val Leu Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Lys Lys
           195                 200                 205
   Val Glu Pro Lys Ser Cys
       210
<210> 200
   <211> 642
   <212> DNA
   <213> Artificial Sequence
<220>
   <223> H2C(VL-CH1)
<400> 200
   cagaccgtgg tgacacagga acccagcctg accgtctccc ctggcggcac cgtgaccctg      60
   acctgtggaa gcagcacagg cgccgtgacc agcggctact accccaactg ggtgcagcag     120
   aagcccggcc aggcccctag aggactgatc ggcggcacca agtttctggc ccctggcacc     180
   cccgccagat tctctggctc tctgctgggc ggcaaggccg ccctgacact gtctggcgtg     240
   cagcctgagg acgaggccga gtactactgc gccctgtggt acagcaacag atgggtgttc     300
   ggcggaggca ccaagctgac cgtgctgagc agcgcttcca ccaaaggccc ttccgtgttt     360
   cctctggctc ctagctccaa gtccacctct ggaggcaccg ctgctctcgg atgcctcgtg     420
   aaggattatt ttcctgagcc tgtgacagtg tcctggaata gcggagcact gacctctgga     480
   gtgcatactt tccccgctgt gctgcagtcc tctggactgt acagcctgag cagcgtggtg     540
   acagtgccca gcagcagcct gggcacccag acctacatct gcaacgtgaa ccacaagccc     600
   agcaacacca aggtggacaa gaaggtggaa cccaagtctt gt                        642
<210> 201
   <211> 684
   <212> PRT
   <213> Artificial Sequence
<220>
   <223> H2C(VH-CL)-M4-3 ML2(VH-CH1)-Fc(knob) P329G LALA
<400> 201
   Glu Val Gln Leu Val Glu Ser Gly Gly Gly Leu Val Gln Pro Gly Gly
   1               5                   10                  15
   Ser Leu Lys Leu Ser Cys Ala Ala Ser Gly Phe Thr Phe Asn Lys Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Lys Asn Thr
   65                  70                  75                  80
   Ala Tyr Leu Gln Met Asn Asn Leu Lys Thr Glu Asp Thr Ala Val Tyr
                   85                  90                  95
   Tyr Cys Val Arg His Gly Asn Phe Gly Asn Ser Tyr Ile Ser Tyr Trp
               100                 105                 110
   Ala Tyr Trp Gly Gln Gly Thr Leu Val Thr Val Ser Ser Ala Ser Val
           115                 120                 125
   Ala Ala Pro Ser Val Phe Ile Phe Pro Pro Ser Asp Glu Gln Leu Lys
       130                 135                 140
   Ser Gly Thr Ala Ser Val Val Cys Leu Leu Asn Asn Phe Tyr Pro Arg
   145                 150                 155                 160
   Glu Ala Lys Val Gln Trp Lys Val Asp Asn Ala Leu Gln Ser Gly Asn
                   165                 170                 175
   Ser Gln Glu Ser Val Thr Glu Gln Asp Ser Lys Asp Ser Thr Tyr Ser
               180                 185                 190
   Leu Ser Ser Thr Leu Thr Leu Ser Lys Ala Asp Tyr Glu Lys His Lys
           195                 200                 205
   Val Tyr Ala Cys Glu Val Thr His Gln Gly Leu Ser Ser Pro Val Thr
       210                 215                 220
   Lys Ser Phe Asn Arg Gly Glu Cys Gly Gly Gly Gly Ser Gly Gly Gly
   225                 230                 235                 240
   Gly Ser Gln Val Gln Leu Gln Glu Ser Gly Pro Gly Leu Val Lys Pro
                   245                 250                 255
   Ser Gln Thr Leu Ser Leu Thr Cys Thr Val Ser Gly Gly Ser Ile Thr
               260                 265                 270
   Ser Gly Tyr Tyr Trp Asn Trp Ile Arg Gln His Pro Gly Lys Gly Leu
           275                 280                 285
   Glu Trp Ile Gly Tyr Ile Thr Tyr Asp Gly Ser Asn Asn Tyr Asn Pro
       290                 295                 300
   Ser Leu Lys Ser Arg Val Thr Ile Ser Arg Asp Thr Ser Lys Asn Gln
   305                 310                 315                 320
   Phe Ser Leu Lys Leu Ser Ser Val Thr Ala Ala Asp Thr Ala Val Tyr
                   325                 330                 335
   Tyr Cys Ala Asp Phe Asp Tyr Trp Gly Gln Gly Thr Leu Val Thr Val
               340                 345                 350
   Ser Ser Ala Ser Thr Lys Gly Pro Ser Val Phe Pro Leu Ala Pro Ser
           355                 360                 365
   Ser Lys Ser Thr Ser Gly Gly Thr Ala Ala Leu Gly Cys Leu Val Lys
       370                 375                 380
   Asp Tyr Phe Pro Glu Pro Val Thr Val Ser Trp Asn Ser Gly Ala Leu
   385                 390                 395                 400
   Thr Ser Gly Val His Thr Phe Pro Ala Val Leu Gln Ser Ser Gly Leu
                   405                 410                 415
   Tyr Ser Leu Ser Ser Val Val Thr Val Pro Ser Ser Ser Leu Gly Thr
               420                 425                 430
   Gln Thr Tyr Ile Cys Asn Val Asn His Lys Pro Ser Asn Thr Lys Val
           435                 440                 445
   Asp Lys Lys Val Glu Pro Lys Ser Cys Asp Lys Thr His Thr Cys Pro
       450                 455                 460
   Pro Cys Pro Ala Pro Glu Ala Ala Gly Gly Pro Ser Val Phe Leu Phe
   465                 470                 475                 480
   Pro Pro Lys Pro Lys Asp Thr Leu Met Ile Ser Arg Thr Pro Glu Val
                   485                 490                 495
   Thr Cys Val Val Val Asp Val Ser His Glu Asp Pro Glu Val Lys Phe
                   500                 505                 510
   Asn Trp Tyr Val Asp Gly Val Glu Val His Asn Ala Lys Thr Lys Pro
           515                 520                 525
   Arg Glu Glu Gln Tyr Asn Ser Thr Tyr Arg Val Val Ser Val Leu Thr
       530                 535                 540
   Val Leu His Gln Asp Trp Leu Asn Gly Lys Glu Tyr Lys Cys Lys Val
   545                 550                 555                 560
   Ser Asn Lys Ala Leu Gly Ala Pro Ile Glu Lys Thr Ile Ser Lys Ala
                   565                 570                 575
   Lys Gly Gln Pro Arg Glu Pro Gln Val Tyr Thr Leu Pro Pro Cys Arg
               580                 585                 590
   Asp Glu Leu Thr Lys Asn Gln Val Ser Leu Trp Cys Leu Val Lys Gly
           595                 600                 605
   Phe Tyr Pro Ser Asp Ile Ala Val Glu Trp Glu Ser Asn Gly Gln Pro
       610                 615                 620
   Glu Asn Asn Tyr Lys Thr Thr Pro Pro Val Leu Asp Ser Asp Gly Ser
   625                 630                 635                 640
   Phe Phe Leu Tyr Ser Lys Leu Thr Val Asp Lys Ser Arg Trp Gln Gln
                   645                 650                 655
   Gly Asn Val Phe Ser Cys Ser Val Met His Glu Ala Leu His Asn His
               660                 665                 670
   Tyr Thr Gln Lys Ser Leu Ser Leu Ser Pro Gly Lys
           675                 680
<210> 202
   <211> 2052
   <212> DNA
   <213> Artificial Sequence
<220>
   <223> H2C(VH-CL)-M4-3 ML2(VH-CH1)-Fc(knob) P329G LALA
<400> 202
   gaggtgcagc tggtggaaag cggcggagga ctggtgcagc ctggcggaag cctgaagctg      60
   tcttgcgccg ccagcggctt caccttcaac aaatacgcca tgaactgggt gcgccaggcc     120
   cctggcaagg gactggaatg ggtggcccgg atcagaagca agtacaacaa ctacgccacc     180
   tactacgccg acagcgtgaa ggaccggttc accatcagcc gggacgacag caagaacacc     240
   gcctacctgc agatgaacaa cctgaaaacc gaggacaccg ccgtgtacta ctgcgtgcgg      300
   cacggcaact tcggcaacag ctacatcagc tactgggcct actggggaca gggcaccctg      360
   gtgacagtgt ccagcgctag cgtggctgca ccatctgtct tcatcttccc gccatctgat      420
   gagcagttga aatctggaac tgcctctgtt gtgtgcctgc tgaataactt ctatcccaga      480
   gaggccaaag tacagtggaa ggtggataac gccctccaat cgggtaactc ccaggagagt      540
   gtcacagagc aggacagcaa ggacagcacc tacagcctca gcagcaccct gacgctgagc      600
   aaagcagact acgagaaaca caaagtctac gcctgcgaag tcacccatca gggcctgagc      660
   tcgcccgtca caaagagctt caacagggga gagtgtggcg gaggcggatc cggcggaggg      720
   ggatctcagg tgcagctgca ggaaagcggc cctggcctgg tcaagcccag ccagaccctg      780
   agcctgacct gcaccgtgtc cggcggcagc atcaccagcg gctactactg gaactggatt      840
   cggcagcacc ccggcaaggg cctggaatgg atcggctaca tcacctacga cggcagcaac      900
   aactacaacc ccagcctgaa gtccagagtg accatcagcc gggacaccag caagaaccag      960
   ttcagcctga agctgtccag cgtgacagcc gccgacaccg ccgtgtacta ctgcgccgac     1020
   ttcgactact ggggccaggg caccctggtc accgtgtcca gcgctagcac caagggcccc     1080
   agcgtgttcc ccctggcacc cagcagcaag agcacatctg gcggaacagc cgctctgggc     1140
   tgtctggtga aagactactt ccccgagccc gtgaccgtgt cttggaactc tggcgccctg     1200
   accagcggcg tgcacacctt tccagccgtg ctgcagagca gcggcctgta ctccctgtcc     1260
   tccgtggtca ccgtgccctc tagctccctg ggaacacaga catatatctg taatgtcaat     1320
   cacaagcctt ccaacaccaa agtcgataag aaagtcgagc ccaagagctg cgacaaaact     1380
   cacacatgcc caccgtgccc agcacctgaa gctgcagggg gaccgtcagt cttcctcttc     1440
   cccccaaaac ccaaggacac cctcatgatc tcccggaccc ctgaggtcac atgcgtggtg     1500
   gtggacgtga gccacgaaga ccctgaggtc aagttcaact ggtacgtgga cggcgtggag     1560
   gtgcataatg ccaagacaaa gccgcgggag gagcagtaca acagcacgta ccgtgtggtc     1620
   agcgtcctca ccgtcctgca ccaggactgg ctgaatggca aggagtacaa gtgcaaggtc     1680
   tccaacaaag ccctcggcgc ccccatcgag aaaaccatct ccaaagccaa agggcagccc     1740
   cgagaaccac aggtgtacac cctgccccca tgccgggatg agctgaccaa gaaccaggtc     1800
   agcctgtggt gcctggtcaa aggcttctat cccagcgaca tcgccgtgga gtgggagagc     1860
   aatgggcagc cggagaacaa ctacaagacc acgcctcccg tgctggactc cgacggctcc     1920
   ttcttcctct acagcaagct caccgtggac aagagcaggt ggcagcaggg gaacgtcttc     1980
   tcatgctccg tgatgcatga ggctctgcac aaccactaca cgcagaagag cctctccctg     2040
   tctccgggta aa                                                         2052
<210> 203
   <211> 212
   <212> PRT
   <213> Artificial Sequence
<220>
   <223> 431/26(VL-CL)
<400> 203
   Asp Ile Gln Met Thr Gln Ser Pro Ser Ser Leu Ser Ala Ser Val Gly
   1               5                   10                  15
   Asp Arg Val Thr Ile Thr Cys Ser Thr Ser Ser Ser Val Ser Tyr Met
               20                  25                  30
   His Trp Tyr Gln Gln Lys Pro Gly Lys Ala Pro Lys Leu Leu Ile Tyr
           35                  40                  45
   Ser Thr Ser Asn Leu Ala Ser Gly Val Pro Ser Arg Phe Ser Gly Ser
       50                  55                  60
   Gly Ser Gly Thr Asp Phe Thr Phe Thr Ile Ser Ser Leu Gln Pro Glu
   65                  70                  75                  80
   Asp Ile Ala Thr Tyr Tyr Cys His Gln Trp Ser Ser Tyr Pro Thr Phe
                   85                  90                  95
   Gly Gln Gly Thr Lys Val Glu Ile Lys Arg Thr Val Ala Ala Pro Ser
               100                 105                 110
   Val Phe Ile Phe Pro Pro Ser Asp Glu Gln Leu Lys Ser Gly Thr Ala
           115                 120                 125
   Ser Val Val Cys Leu Leu Asn Asn Phe Tyr Pro Arg Glu Ala Lys Val
       130                 135                 140
   Gln Trp Lys Val Asp Asn Ala Leu Gln Ser Gly Asn Ser Gln Glu Ser
   145                 150                 155                 160
   Val Thr Glu Gln Asp Ser Lys Asp Ser Thr Tyr Ser Leu Ser Ser Thr
                   165                 170                 175
   Leu Thr Leu Ser Lys Ala Asp Tyr Glu Lys His Lys Val Tyr Ala Cys
               180                 185                 190
   Glu Val Thr His Gln Gly Leu Ser Ser Pro Val Thr Lys Ser Phe Asn
           195                 200                 205
   Arg Gly Glu Cys
       210
<210> 204
   <211> 636
   <212> DNA
   <213> Artificial Sequence
<220>
   <223> 431/26(VL-CL)
<400> 204
   gacatccaga tgacccagag ccccagcagc ctgtctgcca gcgtgggcga cagagtgacc      60
   atcacctgta gcaccagcag cagcgtgtcc tacatgcact ggtatcagca gaagcccggc     120
   aaggccccca agctgctgat ctacagcacc tccaatctgg ccagcggcgt gcccagcaga     180
   ttttctggca gcggctccgg caccgacttc accttcacca tcagctccct gcagcccgag     240
   gatatcgcca cctactactg ccaccagtgg tccagctacc ccacctttgg ccagggcacc     300
   aaggtggaaa tcaagcgtac ggtggctgca ccatctgtct tcatcttccc gccatctgat     360
   gagcagttga aatctggaac tgcctctgtt gtgtgcctgc tgaataactt ctatcccaga     420
   gaggccaaag tacagtggaa ggtggataac gccctccaat cgggtaactc ccaggagagt     480
   gtcacagagc aggacagcaa ggacagcacc tacagcctca gcagcaccct gacgctgagc     540
   aaagcagact acgagaaaca caaagtctac gcctgcgaag tcacccatca gggcctgagc     600
   tcgcccgtca caaagagctt caacagggga gagtgt                               636
<210> 205
   <211> 689
   <212> PRT
   <213> Artificial Sequence
<220>
   <223> 431/26(VH-CH1)-V9(VH-CL)-Fc(knob) P329G LALA
<400> 205
   Gln Val Gln Leu Gln Glu Ser Gly Pro Gly Leu Val Arg Pro Ser Gln
   1               5                   10                  15
   Thr Leu Ser Leu Thr Cys Thr Val Ser Gly Phe Thr Ile Ser Ser Gly
               20                  25                  30
   Tyr Ser Trp His Trp Val Arg Gln Pro Pro Gly Arg Gly Leu Glu Trp
           35                  40                  45
   Ile Gly Tyr Ile Gln Tyr Ser Gly Ile Thr Asn Tyr Asn Pro Ser Leu
       50                  55                  60
   Lys Ser Arg Val Thr Met Leu Val Asp Thr Ser Lys Asn Gln Phe Ser
   65                  70                  75                  80
   Leu Arg Leu Ser Ser Val Thr Ala Ala Asp Thr Ala Val Tyr Tyr Cys
                   85                  90                  95
   Ala Arg Glu Asp Tyr Asp Tyr His Trp Tyr Phe Asp Val Trp Gly Gln
   100 105 110
   Gly Ser Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Gly
   210 215 220
   Gly Gly Gly Ser Gly Gly Gly Gly Ser Glu Val Gln Leu Val Glu Ser
   225 230 235 240
   Gly Gly Gly Leu Val Gln Pro Gly Gly Ser Leu Arg Leu Ser Cys Ala
   245 250 255
   Ala Ser Gly Tyr Ser Phe Thr Gly Tyr Thr Met Asn Trp Val Arg Gln
   260 265 270
   Ala Pro Gly Lys Gly Leu Glu Trp Val Ala Leu Ile Asn Pro Tyr Lys
   275 280 285
   Gly Val Ser Thr Tyr Asn Gln Lys Phe Lys Asp Arg Phe Thr Ile Ser
   290 295 300
   Val Asp Lys Ser Lys Asn Thr Ala Tyr Leu Gln Met Asn Ser Leu Arg
   305 310 315 320
   Ala Glu Asp Thr Ala Val Tyr Tyr Cys Ala Arg Ser Gly Tyr Tyr Gly
   325 330 335
   Asp Ser Asp Trp Tyr Phe Asp Val Trp Gly Gln Gly Thr Leu Val Thr
   340 345 350
   Val Ser Ser Ala Ser Val Ala Ala Pro Ser Val Phe Ile Phe Pro Pro
   355 360 365
   Ser Asp Glu Gln Leu Lys Ser Gly Thr Ala Ser Val Val Cys Leu Leu
   370 375 380
   Asn Asn Phe Tyr Pro Arg Glu Ala Lys Val Gln Trp Lys Val Asp Asn
   385 390 395 400
   Ala Leu Gln Ser Gly Asn Ser Gln Glu Ser Val Thr Glu Gln Asp Ser
   405 410 415
   Lys Asp Ser Thr Tyr Ser Leu Ser Ser Thr Leu Thr Leu Ser Lys Ala
   420 425 430
   Asp Tyr Glu Lys His Lys Val Tyr Ala Cys Glu Val Thr His Gln Gly
   435 440 445
   Leu Ser Ser Pro Val Thr Lys Ser Phe Asn Arg Gly Glu Cys Asp Lys
   450 455 460
   Thr His Thr Cys Pro Pro Cys Pro Ala Pro Glu Ala Ala Gly Gly Pro
   465 470 475 480
   Ser Val Phe Leu Phe Pro Pro Lys Pro Lys Asp Thr Leu Met Ile Ser
   485 490 495
   Arg Thr Pro Glu Val Thr Cys Val Val Val Asp Val Ser His Glu Asp
   500 505 510
   Pro Glu Val Lys Phe Asn Trp Tyr Val Asp Gly Val Glu Val His Asn
   515 520 525
   Ala Lys Thr Lys Pro Arg Glu Glu Gln Tyr Asn Ser Thr Tyr Arg Val
   530 535 540
   Val Ser Val Leu Thr Val Leu His Gln Asp Trp Leu Asn Gly Lys Glu
   545 550 555 560
   Tyr Lys Cys Lys Val Ser Asn Lys Ala Leu Gly Ala Pro Ile Glu Lys
   565 570 575
   Thr Ile Ser Lys Ala Lys Gly Gln Pro Arg Glu Pro Gln Val Tyr Thr
   580 585 590
   Leu Pro Pro Cys Arg Asp Glu Leu Thr Lys Asn Gln Val Ser Leu Trp
   595 600 605
   Cys Leu Val Lys Gly Phe Tyr Pro Ser Asp Ile Ala Val Glu Trp Glu
   610 615 620
   Ser Asn Gly Gln Pro Glu Asn Asn Tyr Lys Thr Thr Pro Pro Val Leu
   625 630 635 640
   Asp Ser Asp Gly Ser Phe Phe Leu Tyr Ser Lys Leu Thr Val Asp Lys
   645 650 655
   Ser Arg Trp Gln Gln Gly Asn Val Phe Ser Cys Ser Val Met His Glu
   660 665 670
   Ala Leu His Asn His Tyr Thr Gln Lys Ser Leu Ser Leu Ser Pro Gly
   675 680 685
   Lys
<210> 206
   <211> 2067
   <212> DNA
   <213> Artificial Sequence
<220>
   <223> 431/26(VH-CH1)-V9(VH-CL)-Fc(knob) P329G LALA
<400> 206
   caggtgcagc tgcaggaatc tggccctgga ctcgtgcggc ctagccagac actgagcctg      60
   acctgtaccg tgtccggctt caccatcagc agcggctaca gctggcattg ggtgcgccag     120
   ccacctggca gaggcctgga atggatcggc tacatccagt acagcggcat caccaactac     180
   aaccccagcc tgaagtccag agtgaccatg ctggtggaca cctccaagaa ccagttcagc     240
   ctgcggctga gcagcgtgac agccgccgat acagccgtgt actactgcgc cagagaggac     300
   tacgactacc actggtactt cgacgtgtgg ggccagggct ctctcgtgac cgtgtcaagc     360
   gctagcacaa agggccccag cgtgttccct ctggccccta gcagcaagag cacatctggc     420
   ggaacagccg ccctgggctg cctggtcaag gactactttc ccgagcccgt gacagtgtcc     480
   tggaactctg gcgccctgac aagcggcgtg cacacctttc cagccgtgct gcagagcagc     540
   ggcctgtact ctctgagcag cgtggtcacc gtgcctagct ctagcctggg cacccagacc     600
   tacatctgca acgtgaacca caagcccagc aacaccaagg tggacaagaa ggtggaaccc     660
   aagagctgcg gcggaggcgg atccggaggc ggaggatccg aagtgcagct ggtggaatct     720
   ggcggaggcc tggtgcagcc tggcggatct ctgagactga gctgtgccgc cagcggctac     780
   agcttcaccg gctacaccat gaactgggtg cgccaggccc ctggcaaggg actggaatgg     840
   gtggccctga tcaaccccta caagggcgtg tccacataca accagaagtt caaggaccgg     900
   ttcaccatca gcgtggacaa gagcaagaac accgcctacc tgcagatgaa cagcctgcgg     960
   gccgaggaca ccgccgtgta ctattgtgcc agaagcggct actacggcga cagcgactgg    1020
   tacttcgacg tgtggggcca gggcacactc gtgaccgtgt caagcgctag cgtggccgct 1080
   ccctccgtgt ttatctttcc cccatccgat gaacagctga aaagcggcac cgcctccgtc 1140
   gtgtgtctgc tgaacaattt ttaccctagg gaagctaaag tgcagtggaa agtggataac 1200
   gcactgcagt ccggcaactc ccaggaatct gtgacagaac aggactccaa ggacagcacc 1260
   tactccctgt cctccaccct gacactgtct aaggctgatt atgagaaaca caaagtctac 1320
   gcctgcgaag tcacccatca gggcctgagc tcgcccgtca caaagagctt caacagggga 1380
   gagtgtgaca agacccacac ctgtccccct tgtcctgccc ctgaagctgc tggcggccct 1440
   tctgtgttcc tgttcccccc aaagcccaag gacaccctga tgatcagccg gacccccgaa 1500
   gtgacctgcg tggtggtgga tgtgtcccac gaggaccctg aagtgaagtt caattggtac 1560
   gtggacggcg tggaagtgca caacgccaag acaaagccgc gggaggagca gtacaacagc 1620
   acgtaccgtg tggtcagcgt cctcaccgtc ctgcaccagg actggctgaa tggcaaggag 1680
   tacaagtgca aggtctccaa caaagccctc ggcgccccca tcgagaaaac catctccaaa 1740
   gccaaagggc agccccgaga accacaggtg tacaccctgc ccccatgccg ggatgagctg 1800
   accaagaacc aggtcagcct gtggtgcctg gtcaaaggct tctatcccag cgacatcgcc 1860
   gtggagtggg agagcaatgg gcagccggag aacaactaca agaccacgcc tcccgtgctg 1920
   gactccgacg gctccttctt cctctacagc aagctcaccg tggacaagag caggtggcag 1980
   caggggaacg tcttctcatg ctccgtgatg catgaggctc tgcacaacca ctacacgcag 2040
   aagagcctct ccctgtctcc gggtaaa 2067
<210> 207
   <211> 450
   <212> PRT
   <213> Artificial Sequence
<220>
   <223> 431/26(VH-CH1)-Fc(hole) P329G LALA
<400> 207
   Gln Val Gln Leu Gln Glu Ser Gly Pro Gly Leu Val Arg Pro Ser Gln
   1               5                   10                  15
   Thr Leu Ser Leu Thr Cys Thr Val Ser Gly Phe Thr Ile Ser Ser Gly
               20                  25                  30
   Tyr Ser Trp His Trp Val Arg Gln Pro Pro Gly Arg Gly Leu Glu Trp
           35                  40                  45
   Ile Gly Tyr Ile Gln Tyr Ser Gly Ile Thr Asn Tyr Asn Pro Ser Leu
       50                  55                  60
   Lys Ser Arg Val Thr Met Leu Val Asp Thr Ser Lys Asn Gln Phe Ser
   65                  70                  75                  80
   Leu Arg Leu Ser Ser Val Thr Ala Ala Asp Thr Ala Val Tyr Tyr Cys
                   85                  90                  95
   Ala Arg Glu Asp Tyr Asp Tyr His Trp Tyr Phe Asp Val Trp Gly Gln
               100                 105                 110
   Gly Ser Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Ala Ala Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Gly Ala Pro Ile Glu
                   325                 330                 335
   Lys Thr Ile Ser Lys Ala Lys Gly Gln Pro Arg Glu Pro Gln Val Cys
               340                 345                 350
   Thr Leu Pro Pro Ser Arg Asp Glu Leu Thr Lys Asn Gln Val Ser Leu
           355                 360                 365
   Ser Cys Ala Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Val Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 208
   <211> 1350
   <212> DNA
   <213> Artificial Sequence
<220>
   <223> 431/26(VH-CH1)-Fc(hole) P329G LALA
<400> 208
   caggtgcagc tgcaggaatc tggccctgga ctcgtgcggc ctagccagac actgagcctg 60
   acctgtaccg tgtccggctt caccatcagc agcggctaca gctggcattg ggtgcgccag 120
   ccacctggca gaggcctgga atggatcggc tacatccagt acagcggcat caccaactac 180
   aaccccagcc tgaagtccag agtgaccatg ctggtggaca cctccaagaa ccagttcagc 240
   ctgcggctga gcagcgtgac agccgccgat acagccgtgt actactgcgc cagagaggac 300
   tacgactacc actggtactt cgacgtgtgg ggccagggct ctctcgtgac cgtgtcaagc     360
   gctagcacca agggcccctc cgtgttcccc ctggccccca gcagcaagag caccagcggc     420
   ggcacagccg ctctgggctg cctggtcaag gactacttcc ccgagcccgt gaccgtgtcc 480
   tggaacagcg gagccctgac ctccggcgtg cacaccttcc ccgccgtgct gcagagttct 540
   ggcctgtata gcctgagcag cgtggtcacc gtgccttcta gcagcctggg cacccagacc 600
   tacatctgca acgtgaacca caagcccagc aacaccaagg tggacaagaa ggtggagccc 660
   aagagctgcg acaaaactca cacatgccca ccgtgcccag cacctgaagc tgcaggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacaac 900
   agcacgtacc gtgtggtcag cgtcctcacc gtcctgcacc aggactggct gaatggcaag 960
   gagtacaagt gcaaggtctc caacaaagcc ctcggcgccc ccatcgagaa aaccatctcc 1020
   aaagccaaag ggcagccccg agaaccacag gtgtgcaccc tgcccccatc ccgggatgag 1080
   ctgaccaaga accaggtcag cctctcgtgc gcagtcaaag gcttctatcc cagcgacatc 1140
   gccgtggagt gggagagcaa tgggcagccg gagaacaact acaagaccac gcctcccgtg 1200
   ctggactccg acggctcctt cttcctcgtg agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtaaa 1350
<210> 209
   <211> 464
   <212> PRT
   <213> Artificial Sequence
<220>
   <223> CH1A1A(VL-CL)-V9 (VH-CL)
<400> 209
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys Gly Gly Gly Gly Ser Gly Gly Gly Gly
       210                 215                 220
   Ser Gly Gly Gly Gly Ser Gly Gly Gly Gly Ser Glu Val Gln Leu Val
   225                 230                 235                 240
   Glu Ser Gly Gly Gly Leu Val Gln Pro Gly Gly Ser Leu Arg Leu Ser
                   245                 250                 255
   Cys Ala Ala Ser Gly Tyr Ser Phe Thr Gly Tyr Thr Met Asn Trp Val
               260                 265                 270
   Arg Gln Ala Pro Gly Lys Gly Leu Glu Trp Val Ala Leu Ile Asn Pro
           275                 280                 285
   Tyr Lys Gly Val Ser Thr Tyr Asn Gln Lys Phe Lys Asp Arg Phe Thr
       290                 295                 300
   Ile Ser Val Asp Lys Ser Lys Asn Thr Ala Tyr Leu Gln Met Asn Ser
   305                 310                 315                 320
   Leu Arg Ala Glu Asp Thr Ala Val Tyr Tyr Cys Ala Arg Ser Gly Tyr
                   325                 330                 335
   Tyr Gly Asp Ser Asp Trp Tyr Phe Asp Val Trp Gly Gln Gly Thr Leu
               340                 345                 350
   Val Thr Val Ser Ser Ala Ser Val Ala Ala Pro Ser Val Phe Ile Phe
           355                 360                 365
   Pro Pro Ser Asp Glu Gln Leu Lys Ser Gly Thr Ala Ser Val Val Cys
       370                 375                 380
   Leu Leu Asn Asn Phe Tyr Pro Arg Glu Ala Lys Val Gln Trp Lys Val
   385                 390                 395                 400
   Asp Asn Ala Leu Gln Ser Gly Asn Ser Gln Glu Ser Val Thr Glu Gln
                   405                 410                 415
   Asp Ser Lys Asp Ser Thr Tyr Ser Leu Ser Ser Thr Leu Thr Leu Ser
               420                 425                 430
   Lys Ala Asp Tyr Glu Lys His Lys Val Tyr Ala Cys Glu Val Thr His
           435                 440                 445
   Gln Gly Leu Ser Ser Pro Val Thr Lys Ser Phe Asn Arg Gly Glu Cys
       450                 455                 460
<210> 210
   <211> 1392
   <212> DNA
   <213> Artificial Sequence
<220>
   <223> CH1A1A(VL-CL)-V9 (VH-CL)
<400> 210
   gatatccaga tgacccagag ccccagcagc ctgtctgcca gcgtgggcga cagagtgacc 60
   atcacatgca aggcctctgc cgccgtgggc acatacgtgg cctggtatca gcagaagccc 120
   ggcaaggccc ccaagctgct gatctacagc gccagctacc ggaagagagg cgtgcccagc 180
   agattttccg gcagcggctc tggcaccgac ttcaccctga ccatcagctc cctgcagccc 240
   gaggacttcg ccacctacta ctgccaccag tactacacct accccctgtt caccttcggc 300
   cagggcacca agctcgagat caagcgtacg gtggccgctc ccagcgtgtt catcttccca 360
   cctagcgacg agcagctgaa gtccggcaca gcctctgtcg tgtgcctgct gaacaacttc 420
   tacccccgcg aggccaaggt gcagtggaag gtggacaatg ccctgcagag cggcaacagc 480
   caggaaagcg tgaccgagca ggacagcaag gactccacct acagcctgag cagcaccctg 540
   acactgagca aggccgacta cgagaagcac aaggtgtacg cctgcgaagt gacccaccag 600
   ggcctgtcta gccccgtgac caagagcttc aaccggggcg aatgtggcgg cggaggatcc 660
   ggcggaggcg gctccggagg cggaggaagt ggcggagggg gatctgaagt gcagctggtg 720
   gaatctggcg gaggcctggt gcagcctggc ggatctctga gactgagctg tgccgccagc 780
   ggctacagct tcaccggcta caccatgaac tgggtgcgcc aggcccctgg caagggactg 840
   gaatgggtgg ccctgatcaa cccctacaag ggcgtgtcca catacaacca gaagttcaag 900
   gaccggttca ccatcagcgt ggacaagagc aagaacaccg cctacctgca gatgaacagc 960
   ctgcgggccg aggacaccgc cgtgtactac tgtgccagaa gcggctacta cggcgacagc 1020
   gactggtact tcgacgtgtg gggccaggga accctcgtga ccgtgtcaag cgctagcgtg 1080
   gccgcaccct ctgtgtttat ctttccaccc tctgacgaac agctgaaaag cggcaccgcc 1140
   agcgtcgtgt gtctgctgaa caatttttac cctagggaag ctaaagtgca gtggaaagtg 1200
   gataacgcac tgcagtccgg caactcccag gaatctgtga cagaacagga ctccaaggac 1260
   agcacatact ccctgtccag cacactgacc ctgtctaagg ccgattatga gaaacacaaa 1320
   gtgtatgctt gtgaagtgac acatcaggga ctgagcagcc ctgtgacaaa gtccttcaac 1380
   agaggcgagt gt 1392
<210> 211
   <211> 451
   <212> PRT
   <213> Artificial Sequence
<220>
   <223> CH1A1A(VH-CH1)-Fc(knob) P329G LALA
<400> 211
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Ala Ala Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Gly Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Cys Arg Asp Glu Leu Thr Lys Asn Gln Val Ser
           355                 360                 365
   Leu Trp Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
   435 440 445
   Pro Gly Lys
   450
<210> 212
   <211> 1353
   <212> DNA
   <213> Artificial Sequence
<220>
   <223> CH1A1A(VH-CH1)-Fc(knob) P329G LALA
<400> 212
   caggtgcagc tggtgcagtc tggcgccgaa gtgaagaaac ctggagctag tgtgaaggtg 60
   tcctgcaagg ccagcggcta caccttcacc gagttcggca tgaactgggt ccgacaggct 120
   ccaggccagg gcctcgaatg gatgggctgg atcaacacca agaccggcga ggccacctac 180
   gtggaagagt tcaagggcag agtgaccttc accacggaca ccagcaccag caccgcctac 240
   atggaactgc ggagcctgag aagcgacgac accgccgtgt actactgcgc cagatgggac 300
   ttcgcctatt acgtggaagc catggactac tggggccagg gcaccaccgt gaccgtgtct 360
   agcgctagca ccaagggccc atcggtcttc cccctggcac cctcctccaa gagcacctct 420
   gggggcacag cggccctggg ctgcctggtc aaggactact tccccgaacc ggtgacggtg 480
   tcgtggaact caggcgccct gaccagcggc gtgcacacct tcccggctgt cctacagtcc 540
   tcaggactct actccctcag cagcgtggtg accgtgccct ccagcagctt gggcacccag 600
   acctacatct gcaacgtgaa tcacaagccc agcaacacca aggtggacaa gaaagttgag 660
   cccaaatctt gtgacaaaac tcacacatgc ccaccgtgcc cagcacctga agctgcaggg 720
   ggaccgtcag tcttcctctt ccccccaaaa cccaaggaca ccctcatgat ctcccggacc 780
   cctgaggtca catgcgtggt ggtggacgtg agccacgaag accctgaggt caagttcaac 840
   tggtacgtgg acggcgtgga ggtgcataat gccaagacaa agccgcggga ggagcagtac 900
   aacagcacgt accgtgtggt cagcgtcctc accgtcctgc accaggactg gctgaatggc 960
   aaggagtaca agtgcaaggt ctccaacaaa gccctcggcg cccccatcga gaaaaccatc 1020
   tccaaagcca aagggcagcc ccgagaacca caggtgtaca ccctgccccc atgccgggat 1080
   gagctgacca agaaccaggt cagcctgtgg tgcctggtca aaggcttcta tcccagcgac 1140
   atcgccgtgg agtgggagag caatgggcag ccggagaaca actacaagac cacgcctccc 1200
   gtgctggact ccgacggctc cttcttcctc tacagcaagc tcaccgtgga caagagcagg 1260
   tggcagcagg ggaacgtctt ctcatgctcc gtgatgcatg aggctctgca caaccactac 1320
   acgcagaaga gcctctccct gtctccgggt aaa 1353
<210> 213
   <211> 227
   <212> PRT
   <213> Artificial Sequence
<220>
   <223> Fc(hole) P329G LALA
<400> 213
   Asp Lys Thr His Thr Cys Pro Pro Cys Pro Ala Pro Glu Ala Ala Gly
   1 5 10 15
   Gly Pro Ser Val Phe Leu Phe Pro Pro Lys Pro Lys Asp Thr Leu Met
   20 25 30
   Ile Ser Arg Thr Pro Glu Val Thr Cys Val Val Val Asp Val Ser His
   35 40 45
   Glu Asp Pro Glu Val Lys Phe Asn Trp Tyr Val Asp Gly Val Glu Val
   50 55 60
   His Asn Ala Lys Thr Lys Pro Arg Glu Glu Gln Tyr Asn Ser Thr Tyr
   65 70 75 80
   Arg Val Val Ser Val Leu Thr Val Leu His Gln Asp Trp Leu Asn Gly
   85 90 95
   Lys Glu Tyr Lys Cys Lys Val Ser Asn Lys Ala Leu Gly Ala Pro Ile
   100 105 110
   Glu Lys Thr Ile Ser Lys Ala Lys Gly Gln Pro Arg Glu Pro Gln Val
   115 120 125
   Cys Thr Leu Pro Pro Ser Arg Asp Glu Leu Thr Lys Asn Gln Val Ser
   130 135 140
   Leu Ser Cys Ala Val Lys Gly Phe Tyr Pro Ser Asp Ile Ala Val Glu
   145 150 155 160
   Trp Glu Ser Asn Gly Gln Pro Glu Asn Asn Tyr Lys Thr Thr Pro Pro
   165 170 175
   Val Leu Asp Ser Asp Gly Ser Phe Phe Leu Val Ser Lys Leu Thr Val
   180 185 190
   Asp Lys Ser Arg Trp Gln Gln Gly Asn Val Phe Ser Cys Ser Val Met
   195 200 205
   His Glu Ala Leu His Asn His Tyr Thr Gln Lys Ser Leu Ser Leu Ser
   210 215 220
   Pro Gly Lys
   225
<210> 214
   <211> 681
   <212> DNA
   <213> Artificial Sequence
<220>
   <223> Fc(hole) P329G LALA
<400> 214
   gacaaaactc acacatgccc accgtgccca gcacctgaag ctgcaggggg accgtcagtc      60
   ttcctcttcc ccccaaaacc caaggacacc ctcatgatct cccggacccc tgaggtcaca     120
   tgcgtggtgg tggacgtgag ccacgaagac cctgaggtca agttcaactg gtacgtggac     180
   ggcgtggagg tgcataatgc caagacaaag ccgcgggagg agcagtacaa cagcacgtac     240
   cgtgtggtca gcgtcctcac cgtcctgcac caggactggc tgaatggcaa ggagtacaag     300
   tgcaaggtct ccaacaaagc cctcggcgcc cccatcgaga aaaccatctc caaagccaaa     360
   gggcagcccc gagaaccaca ggtgtgcacc ctgcccccat cccgggatga gctgaccaag     420
   aaccaggtca gcctctcgtg cgcagtcaaa ggcttctatc ccagcgacat cgccgtggag     480
   tgggagagca atgggcagcc ggagaacaac tacaagacca cgcctcccgt gctggactcc     540
   gacggctcct tcttcctcgt gagcaagctc accgtggaca agagcaggtg gcagcagggg     600
   aacgtcttct catgctccgt gatgcatgag gctctgcaca accactacac gcagaagagc     660
   ctctccctgt ctccgggtaa a                                               681
<210> 215
   <211> 214
   <212> PRT
   <213> Artificial Sequence
<220>
   <223> CH2527(VL-CH1)
<400> 215
   Gln Ala Val Val Thr Gln Glu Ser Ala Leu Thr Thr Ser Pro Gly Glu
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Glu Lys Pro Asp His Leu Phe Thr Gly
           35                  40                  45
   Leu Ile Gly Gly Thr Asn Lys Arg Ala Pro Gly Val Pro Ala Arg Phe
       50                  55                  60
   Ser Gly Ser Leu Ile Gly Asp Lys Ala Ala Leu Thr Ile Thr Gly Ala
   65                  70                  75                  80
   Gln Thr Glu Asp Glu Ala Ile Tyr Phe Cys Ala Leu Trp Tyr Ser Asn
                   85                  90                  95
   Leu Trp Val Phe Gly Gly Gly Thr Lys Leu Thr Val Leu Ser Ser Ala
               100                 105                 110
   Ser Thr Lys Gly Pro Ser Val Phe Pro Leu Ala Pro Ser Ser Lys Ser
           115                 120                 125
   Thr Ser Gly Gly Thr Ala Ala Leu Gly Cys Leu Val Lys Asp Tyr Phe
       130                 135                 140
   Pro Glu Pro Val Thr Val Ser Trp Asn Ser Gly Ala Leu Thr Ser Gly
   145                 150                 155                 160
   Val His Thr Phe Pro Ala Val Leu Gln Ser Ser Gly Leu Tyr Ser Leu
                   165                 170                 175
   Ser Ser Val Val Thr Val Pro Ser Ser Ser Leu Gly Thr Gln Thr Tyr
               180                 185                 190
   Ile Cys Asn Val Asn His Lys Pro Ser Asn Thr Lys Val Asp Lys Lys
           195                 200                 205
   Val Glu Pro Lys Ser Cys
       210
<210> 216
   <211> 642
   <212> DNA
   <213> Artificial Sequence
<220>
   <223> CH2527(VL-CH1)
<400> 216
   caggccgtcg tgacccagga aagcgccctg acaacaagcc ctggcgagac agtgaccctg 60
   acctgcagat ctagcacagg cgccgtgacc accagcaact acgccaactg ggtgcaggaa 120
   aagcccgacc acctgttcac cggcctgatc ggcggcacca acaaaagggc tccaggcgtg 180
   ccagccagat tcagcggcag cctgattggc gataaggccg ccctgaccat cactggcgcc 240
   cagacagagg acgaggccat ctacttttgc gccctgtggt acagcaacct gtgggtgttc 300
   ggcggaggca ccaagctgac agtgctgagc agcgcttcca ccaaaggccc ttccgtgttt 360
   cctctggctc ctagctccaa gtccacctct ggaggcaccg ctgctctcgg atgcctcgtg 420
   aaggattatt ttcctgagcc tgtgacagtg tcctggaata gcggagcact gacctctgga 480
   gtgcatactt tccccgctgt gctgcagtcc tctggactgt acagcctgag cagcgtggtg 540
   acagtgccca gcagcagcct gggcacccag acctacatct gcaacgtgaa ccacaagccc 600
   agcaacacca aggtggacaa gaaggtggaa cccaagtctt gt 642
<210> 217
   <211> 684
   <212> PRT
   <213> Artificial Sequence
<220>
   <223> CH2527(VH-CL)-LC007(VH-CH1)-Fc(knob) P329G LALA
<400> 217
   Glu Val Gln Leu Val Glu Ser Gly Gly Gly Leu Val Gln Pro Lys Gly
   1 5 10 15
   Ser Leu Lys Leu Ser Cys Ala Ala Ser Gly Phe Thr Phe Asn Thr Tyr
   20 25 30
   Ala Met Asn Trp Val Arg Gln Ala Pro Gly Lys Gly Leu Glu Trp Val
   35 40 45
   Ala Arg Ile Arg Ser Lys Tyr Asn Asn Tyr Ala Thr Tyr Tyr Ala Asp
   50 55 60
   Ser Val Lys Asp Arg Phe Thr Ile Ser Arg Asp Asp Ser Gln Ser Ile
   65 70 75 80
   Leu Tyr Leu Gln Met Asn Asn Leu Lys Thr Glu Asp Thr Ala Met Tyr
   85 90 95
   Tyr Cys Val Arg His Gly Asn Phe Gly Asn Ser Tyr Val Ser Trp Phe
   100 105 110
   Ala Tyr Trp Gly Gln Gly Thr Leu Val Thr Val Ser Ala Ala Ser Val
   115 120 125
   Ala Ala Pro Ser Val Phe Ile Phe Pro Pro Ser Asp Glu Gln Leu Lys
   130 135 140
   Ser Gly Thr Ala Ser Val Val Cys Leu Leu Asn Asn Phe Tyr Pro Arg
   145 150 155 160
   Glu Ala Lys Val Gln Trp Lys Val Asp Asn Ala Leu Gln Ser Gly Asn
   165 170 175
   Ser Gln Glu Ser Val Thr Glu Gln Asp Ser Lys Asp Ser Thr Tyr Ser
   180 185 190
   Leu Ser Ser Thr Leu Thr Leu Ser Lys Ala Asp Tyr Glu Lys His Lys
   195 200 205
   Val Tyr Ala Cys Glu Val Thr His Gln Gly Leu Ser Ser Pro Val Thr
   210 215 220
   Lys Ser Phe Asn Arg Gly Glu Cys Gly Gly Gly Gly Ser Gly Gly Gly
   225 230 235 240
   Gly Ser Glu Val Gln Leu Gln Glu Ser Gly Pro Gly Leu Val Lys Pro
   245 250 255
   Ser Gln Ser Leu Ser Leu Thr Cys Ser Val Thr Gly Tyr Ser Ile Thr
   260 265 270
   Ser Gly Tyr Tyr Trp Asn Trp Ile Arg Gln Phe Pro Gly Asn Lys Leu
   275 280 285
   Glu Trp Met Gly Tyr Ile Thr Tyr Asp Gly Ser Asn Asn Tyr Asn Pro
   290 295 300
   Ser Leu Lys Asn Arg Ile Ser Ile Thr Arg Asp Thr Ser Lys Asn Gln
   305 310 315 320
   Phe Phe Leu Lys Leu Asn Ser Val Thr Thr Glu Asp Thr Ala Thr Tyr
   325 330 335
   Tyr Cys Ala Asp Phe Asp Tyr Trp Gly Gln Gly Thr Thr Leu Thr Val
   340 345 350
   Ser Ser Ala Ser Thr Lys Gly Pro Ser Val Phe Pro Leu Ala Pro Ser
   355 360 365
   Ser Lys Ser Thr Ser Gly Gly Thr Ala Ala Leu Gly Cys Leu Val Lys
   370 375 380
   Asp Tyr Phe Pro Glu Pro Val Thr Val Ser Trp Asn Ser Gly Ala Leu
   385 390 395 400
   Thr Ser Gly Val His Thr Phe Pro Ala Val Leu Gln Ser Ser Gly Leu
   405 410 415
   Tyr Ser Leu Ser Ser Val Val Thr Val Pro Ser Ser Ser Leu Gly Thr
   420 425 430
   Gln Thr Tyr Ile Cys Asn Val Asn His Lys Pro Ser Asn Thr Lys Val
   435 440 445
   Asp Lys Lys Val Glu Pro Lys Ser Cys Asp Lys Thr His Thr Cys Pro
   450 455 460
   Pro Cys Pro Ala Pro Glu Ala Ala Gly Gly Pro Ser Val Phe Leu Phe
   465 470 475 480
   Pro Pro Lys Pro Lys Asp Thr Leu Met Ile Ser Arg Thr Pro Glu Val
   485 490 495
   Thr Cys Val Val Val Asp Val Ser His Glu Asp Pro Glu Val Lys Phe
   500 505 510
   Asn Trp Tyr Val Asp Gly Val Glu Val His Asn Ala Lys Thr Lys Pro
   515 520 525
   Arg Glu Glu Gln Tyr Asn Ser Thr Tyr Arg Val Val Ser Val Leu Thr
   530 535 540
   Val Leu His Gln Asp Trp Leu Asn Gly Lys Glu Tyr Lys Cys Lys Val
   545 550 555 560
   Ser Asn Lys Ala Leu Gly Ala Pro Ile Glu Lys Thr Ile Ser Lys Ala
   565 570 575
   Lys Gly Gln Pro Arg Glu Pro Gln Val Tyr Thr Leu Pro Pro Cys Arg
   580 585 590
   Asp Glu Leu Thr Lys Asn Gln Val Ser Leu Trp Cys Leu Val Lys Gly
   595 600 605
   Phe Tyr Pro Ser Asp Ile Ala Val Glu Trp Glu Ser Asn Gly Gln Pro
   610 615 620
   Glu Asn Asn Tyr Lys Thr Thr Pro Pro Val Leu Asp Ser Asp Gly Ser
   625 630 635 640
   Phe Phe Leu Tyr Ser Lys Leu Thr Val Asp Lys Ser Arg Trp Gln Gln
   645 650 655
   Gly Asn Val Phe Ser Cys Ser Val Met His Glu Ala Leu His Asn His
   660 665 670
   Tyr Thr Gln Lys Ser Leu Ser Leu Ser Pro Gly Lys
   675 680
<210> 218
   <211> 2052
   <212> DNA
   <213> Artificial Sequence
<220>
   <223> CH2527(VH-CL)-LC007(VH-CH1)-Fc(knob) P329G LALA
<400> 218
   gaagtgcagc tggtggaaag cggcggaggc ctggtgcagc ctaagggctc tctgaagctg 60
   agctgtgccg ccagcggctt caccttcaac acctacgcca tgaactgggt gcgccaggcc 120
   cctggcaaag gcctggaatg ggtggcccgg atcagaagca agtacaacaa ttacgccacc 180
   tactacgccg acagcgtgaa ggaccggttc accatcagcc gggacgacag ccagagcatc 240
   ctgtacctgc agatgaacaa cctgaaaacc gaggacaccg ccatgtacta ctgcgtgcgg 300
   cacggcaact tcggcaacag ctatgtgtct tggtttgcct actggggcca gggcaccctc 360
   gtgacagtgt ctgctgctag cgtggctgca ccatctgtct tcatcttccc gccatctgat 420
   gagcagttga aatctggaac tgcctctgtt gtgtgcctgc tgaataactt ctatcccaga 480
   gaggccaaag tacagtggaa ggtggataac gccctccaat cgggtaactc ccaggagagt 540
   gtcacagagc aggacagcaa ggacagcacc tacagcctca gcagcaccct gacgctgagc 600
   aaagcagact acgagaaaca caaagtctac gcctgcgaag tcacccatca gggcctgagc 660
   tcgcccgtca caaagagctt caacagggga gagtgtggcg gaggcggatc cggcggaggg 720
   ggatctgagg tccagctgca ggagtcagga cctggcctcg tgaaaccttc tcagtctctg 780
   tctctcacct gctctgtcac tggctactcc atcaccagtg gttattactg gaactggatt 840
   cggcagtttc caggaaacaa gctggaatgg atgggctaca taacctacga cggtagcaat 900
   aactacaacc catctctcaa aaatcgaatc tccattactc gtgacacatc taagaaccag 960
   tttttcctga agttgaattc tgtgactact gaggacacag ctacatatta ctgtgcggac 1020
   tttgactact ggggccaagg caccactctc acagtctcca gcgctagcac caagggcccc 1080
   agcgtgttcc ccctggcacc cagcagcaag agcacatctg gcggaacagc cgctctgggc 1140
   tgtctggtga aagactactt ccccgagccc gtgaccgtgt cttggaactc tggcgccctg 1200
   accagcggcg tgcacacctt tccagccgtg ctgcagagca gcggcctgta ctccctgtcc 1260
   tccgtggtca ccgtgccctc tagctccctg ggaacacaga catatatctg taatgtcaat 1320
   cacaagcctt ccaacaccaa agtcgataag aaagtcgagc ccaagagctg cgacaaaact 1380
   cacacatgcc caccgtgccc agcacctgaa gctgcagggg gaccgtcagt cttcctcttc 1440
   cccccaaaac ccaaggacac cctcatgatc tcccggaccc ctgaggtcac atgcgtggtg 1500
   gtggacgtga gccacgaaga ccctgaggtc aagttcaact ggtacgtgga cggcgtggag 1560
   gtgcataatg ccaagacaaa gccgcgggag gagcagtaca acagcacgta ccgtgtggtc 1620
   agcgtcctca ccgtcctgca ccaggactgg ctgaatggca aggagtacaa gtgcaaggtc 1680
   tccaacaaag ccctcggcgc ccccatcgag aaaaccatct ccaaagccaa agggcagccc 1740
   cgagaaccac aggtgtacac cctgccccca tgccgggatg agctgaccaa gaaccaggtc 1800
   agcctgtggt gcctggtcaa aggcttctat cccagcgaca tcgccgtgga gtgggagagc 1860
   aatgggcagc cggagaacaa ctacaagacc acgcctcccg tgctggactc cgacggctcc 1920
   ttcttcctct acagcaagct caccgtggac aagagcaggt ggcagcaggg gaacgtcttc 1980
   tcatgctccg tgatgcatga ggctctgcac aaccactaca cgcagaagag cctctccctg 2040
   tctccgggta aa 2052
<210> 219
   <211> 685
   <212> PRT
   <213> Artificial Sequence
<220>
   <223> LC007(VH-CH1)-CH2527(VH-CL)-Fc(knob) P329G LALA
<400> 219
   Glu Val Gln Leu Gln Glu Ser Gly Pro Gly Leu Val Lys Pro Ser Gln
   1 5 10 15
   Ser Leu Ser Leu Thr Cys Ser Val Thr Gly Tyr Ser Ile Thr Ser Gly
   20 25 30
   Tyr Tyr Trp Asn Trp Ile Arg Gln Phe Pro Gly Asn Lys Leu Glu Trp
   35 40 45
   Met Gly Tyr Ile Thr Tyr Asp Gly Ser Asn Asn Tyr Asn Pro Ser Leu
   50 55 60
   Lys Asn Arg Ile Ser Ile Thr Arg Asp Thr Ser Lys Asn Gln Phe Phe
   65 70 75 80
   Leu Lys Leu Asn Ser Val Thr Thr Glu Asp Thr Ala Thr Tyr Tyr Cys
   85 90 95
   Ala Asp Phe Asp Tyr Trp Gly Gln Gly Thr Thr Leu Thr Val Ser Ser
   100 105 110
   Ala Ser Thr Lys Gly Pro Ser Val Phe Pro Leu Ala Pro Ser Ser Lys
   115 120 125
   Ser Thr Ser Gly Gly Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
   180 185 190
   Tyr Ile Cys Asn Val Asn His Lys Pro Ser Asn Thr Lys Val Asp Lys
   195 200 205
   Lys Val Glu Pro Lys Ser Cys Asp Gly Gly Gly Gly Ser Gly Gly Gly
   210 215 220
   Gly Ser Glu Val Gln Leu Val Glu Ser Gly Gly Gly Leu Val Gln Pro
   225 230 235 240
   Lys Gly Ser Leu Lys Leu Ser Cys Ala Ala Ser Gly Phe Thr Phe Asn
   245 250 255
   Thr Tyr Ala Met Asn Trp Val Arg Gln Ala Pro Gly Lys Gly Leu Glu
   260 265 270
   Trp Val Ala Arg Ile Arg Ser Lys Tyr Asn Asn Tyr Ala Thr Tyr Tyr
   275 280 285
   Ala Asp Ser Val Lys Asp Arg Phe Thr Ile Ser Arg Asp Asp Ser Gln
   290 295 300
   Ser Ile Leu Tyr Leu Gln Met Asn Asn Leu Lys Thr Glu Asp Thr Ala
   305 310 315 320
   Met Tyr Tyr Cys Val Arg His Gly Asn Phe Gly Asn Ser Tyr Val Ser
   325 330 335
   Trp Phe Ala Tyr Trp Gly Gln Gly Thr Leu Val Thr Val Ser Ala Ala
   340 345 350
   Ser Val Ala Ala Pro Ser Val Phe Ile Phe Pro Pro Ser Asp Glu Gln
   355 360 365
   Leu Lys Ser Gly Thr Ala Ser Val Val Cys Leu Leu Asn Asn Phe Tyr
   370 375 380
   Pro Arg Glu Ala Lys Val Gln Trp Lys Val Asp Asn Ala Leu Gln Ser
   385 390 395 400
   Gly Asn Ser Gln Glu Ser Val Thr Glu Gln Asp Ser Lys Asp Ser Thr
   405 410 415
   Tyr Ser Leu Ser Ser Thr Leu Thr Leu Ser Lys Ala Asp Tyr Glu Lys
   420 425 430
   His Lys Val Tyr Ala Cys Glu Val Thr His Gln Gly Leu Ser Ser Pro
   435 440 445
   Val Thr Lys Ser Phe Asn Arg Gly Glu Cys Asp Lys Thr His Thr Cys
       450                 455                 460
   Pro Pro Cys Pro Ala Pro Glu Ala Ala Gly Gly Pro Ser Val Phe Leu
   465                 470                 475                 480
   Phe Pro Pro Lys Pro Lys Asp Thr Leu Met Ile Ser Arg Thr Pro Glu
                   485                 490                 495
   Val Thr Cys Val Val Val Asp Val Ser His Glu Asp Pro Glu Val Lys
               500                 505                 510
   Phe Asn Trp Tyr Val Asp Gly Val Glu Val His Asn Ala Lys Thr Lys
           515                 520                 525
   Pro Arg Glu Glu Gln Tyr Asn Ser Thr Tyr Arg Val Val Ser Val Leu
       530                 535                 540
   Thr Val Leu His Gln Asp Trp Leu Asn Gly Lys Glu Tyr Lys Cys Lys
   545                 550                 555                 560
   Val Ser Asn Lys Ala Leu Gly Ala Pro Ile Glu Lys Thr Ile Ser Lys
                   565                 570                 575
   Ala Lys Gly Gln Pro Arg Glu Pro Gln Val Tyr Thr Leu Pro Pro Cys
               580                 585                 590
   Arg Asp Glu Leu Thr Lys Asn Gln Val Ser Leu Trp Cys Leu Val Lys
           595                 600                 605
   Gly Phe Tyr Pro Ser Asp Ile Ala Val Glu Trp Glu Ser Asn Gly Gln
       610                 615                 620
   Pro Glu Asn Asn Tyr Lys Thr Thr Pro Pro Val Leu Asp Ser Asp Gly
   625                 630                 635                 640
   Ser Phe Phe Leu Tyr Ser Lys Leu Thr Val Asp Lys Ser Arg Trp Gln
                   645                 650                 655
   Gln Gly Asn Val Phe Ser Cys Ser Val Met His Glu Ala Leu His Asn
               660                 665                 670
   His Tyr Thr Gln Lys Ser Leu Ser Leu Ser Pro Gly Lys
           675                 680                 685
<210> 220
   <211> 2055
   <212> DNA
   <213> Artificial Sequence
<220>
   <223> LC007(VH-CH1)-CH2527(VH-CL)-Fc(knob) P329G LALA
<400> 220
   gaggtgcagc tgcaggaatc tggccctggc ctggtcaagc caagccagag tctgagcctg      60
   acctgcagcg tgaccggcta cagcattacc agcggctact actggaactg gattcggcag     120
   ttccccggca ataagctgga atggatgggc tacatcacct acgacggcag caacaactac     180
   aaccccagcc tgaagaaccg gatcagcatc acccgggaca ccagcaagaa ccagttcttc     240
   ctgaagctga acagcgtgac caccgaggac accgccacat actattgcgc cgacttcgac     300
   tactggggcc agggcaccac cctgaccgtg tccagcgcca gcacaaaggg ccctagcgtg     360
   ttccctctgg cccccagcag caagagcaca agcggcggaa cagccgccct gggctgcctc     420
   gtgaaggact acttccccga gcccgtgaca gtgtcttgga acagcggagc cctgacaagc     480
   ggcgtgcaca ccttccctgc cgtgctgcag agcagcggcc tgtactccct gagcagcgtg     540
   gtcaccgtgc ctagcagcag cctgggcacc cagacctaca tctgcaacgt gaaccacaag     600
   cccagcaaca ccaaagtgga caagaaggtg gagcccaaga gctgtgatgg cggaggaggg     660
   tccggaggcg gaggatccga agtgcagctg gtggaaagcg gcggaggcct ggtgcagcct     720
   aagggctctc tgaagctgag ctgtgccgcc agcggcttca ccttcaacac ctacgccatg     780
   aactgggtgc gccaggcccc tggcaaaggc ctggaatggg tggcccggat cagaagcaag     840
   tacaacaatt acgccaccta ctacgccgac agcgtgaagg accggttcac catcagccgg     900
   gacgacagcc agagcatcct gtacctgcag atgaacaacc tgaaaaccga ggacaccgcc     960
   atgtactact gcgtgcggca cggcaacttc ggcaacagct atgtgtcttg gtttgcctac    1020
   tggggccagg gcaccctcgt gacagtgtct gctgctagcg tggccgctcc ctccgtgttt    1080
   atctttcccc catccgatga acagctgaaa agcggcaccg cctccgtcgt gtgtctgctg    1140
   aacaattttt accctaggga agctaaagtg cagtggaaag tggataacgc actgcagtcc    1200
   ggcaactccc aggaatctgt gacagaacag gactccaagg acagcaccta ctccctgtcc    1260
   tccaccctga cactgtctaa ggctgattat gagaaacaca aagtctacgc ctgcgaagtc    1320
   acccatcagg gcctgagctc gcccgtcaca aagagcttca acaggggaga gtgtgacaag    1380
   acccacacct gtcccccttg tcctgcccct gaagctgctg gcggcccttc tgtgttcctg     1440
   ttccccccaa agcccaagga caccctgatg atcagccgga cccccgaagt gacctgcgtg     1500
   gtggtggatg tgtcccacga ggaccctgaa gtgaagttca attggtacgt ggacggcgtg    1560
   gaagtgcaca acgccaagac aaagccgcgg gaggagcagt acaacagcac gtaccgtgtg    1620
   gtcagcgtcc tcaccgtcct gcaccaggac tggctgaatg gcaaggagta caagtgcaag    1680
   gtctccaaca aagccctcgg cgcccccatc gagaaaacca tctccaaagc caaagggcag    1740
   ccccgagaac cacaggtgta caccctgccc ccatgccggg atgagctgac caagaaccag    1800
   gtcagcctgt ggtgcctggt caaaggcttc tatcccagcg acatcgccgt ggagtgggag     1860
   agcaatgggc agccggagaa caactacaag accacgcctc ccgtgctgga ctccgacggc     1920
   tccttcttcc tctacagcaa gctcaccgtg gacaagagca ggtggcagca ggggaacgtc     1980
   ttctcatgct ccgtgatgca tgaggctctg cacaaccact acacgcagaa gagcctctcc     2040
   ctgtctccgg gtaaa                                                      2055
<210> 221
   <211> 218
   <212> PRT
   <213> Artificial Sequence
<220>
   <223> anti-CD33(VL-CL)
<400> 221
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Glu Ser Val Asp Asn Tyr
               20                  25                  30
   Gly Ile Ser Phe Met Asn Trp Phe Gln Gln Lys Pro Gly Lys Ala Pro
           35                  40                  45
   Lys Leu Leu Ile Tyr Ala Ala Ser Asn Gln Gly Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Asp Asp Phe Ala Thr Tyr Tyr Cys Gln Gln Ser Lys
                   85                  90                  95
   Glu Val Pro Trp Thr Phe Gly Gln Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 222
   <211> 654
   <212> DNA
   <213> Artificial Sequence
<220>
   <223> anti-CD33(VL-CL)
<400> 222
   gacatccaga tgacccagag ccccagcagc ctgagcgcca gcgtgggcga cagagtgacc      60
   atcacctgtc gggccagcga gagcgtggac aactacggca tcagcttcat gaactggttc     120
   cagcagaagc ccggcaaggc ccccaagctg ctgatctacg ccgccagcaa tcagggcagc     180
   ggcgtgccca gcagattcag cggctctggc agcggcaccg acttcaccct gaccatcagc     240
   agcctgcagc ccgacgactt cgccacctac tactgccagc agagcaaaga ggtgccctgg     300
   accttcggcc agggcaccaa ggtggaaatc aagcgtacgg tggctgcacc atctgtcttc     360
   atcttcccgc catctgatga gcagttgaaa tctggaactg cctctgttgt gtgcctgctg     420
   aataacttct atcccagaga ggccaaagta cagtggaagg tggataacgc cctccaatcg     480
   ggtaactccc aggagagtgt cacagagcag gacagcaagg acagcaccta cagcctcagc     540
   agcaccctga cgctgagcaa agcagactac gagaaacaca aagtctacgc ctgcgaagtc     600
   acccatcagg gcctgagctc gcccgtcaca aagagcttca acaggggaga gtgt           654
<210> 223
   <211> 668
   <212> PRT
   <213> Artificial Sequence
<220>
   <223> V9(VL-CH1)-anti-CD33(VH-CH1)-Fc(knob) P329G LALA
<400> 223
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Gln Gln Gly Asn Thr Leu Pro Trp
   85 90 95
   Thr Phe Gly Gln Gly Thr Lys Val Glu Ile Lys Ser Ser Ala Ser Thr
   100 105 110
   Lys Gly Pro Ser Val Phe Pro Leu Ala Pro Ser Ser Lys Ser Thr Ser
   115 120 125
   Gly Gly Thr Ala Ala Leu Gly Cys Leu Val Lys Asp Tyr Phe Pro Glu
   130 135 140
   Pro Val Thr Val Ser Trp Asn Ser Gly Ala Leu Thr Ser Gly Val His
   145 150 155 160
   Thr Phe Pro Ala Val Leu Gln Ser Ser Gly Leu Tyr Ser Leu Ser Ser
   165 170 175
   Val Val Thr Val Pro Ser Ser Ser Leu Gly Thr Gln Thr Tyr Ile Cys
   180 185 190
   Asn Val Asn His Lys Pro Ser Asn Thr Lys Val Asp Lys Lys Val Glu
   195 200 205
   Pro Lys Ser Cys Gly Gly Gly Gly Ser Gly Gly Gly Gly Ser Gln Val
   210 215 220
   Gln Leu Val Gln Ser Gly Ala Glu Val Lys Lys Pro Gly Ser Ser Val
   225 230 235 240
   Lys Val Ser Cys Lys Ala Ser Gly Tyr Thr Phe Thr Asp Tyr Asn Met
   245 250 255
   His Trp Val Arg Gln Ala Pro Gly Gln Gly Leu Glu Trp Ile Gly Tyr
   260 265 270
   Ile Tyr Pro Tyr Asn Gly Gly Thr Gly Tyr Asn Gln Lys Phe Lys Ser
   275 280 285
   Lys Ala Thr Ile Thr Ala Asp Glu Ser Thr Asn Thr Ala Tyr Met Glu
   290 295 300
   Leu Ser Ser Leu Arg Ser Glu Asp Thr Ala Val Tyr Tyr Cys Ala Arg
   305                 310                 315                 320
   Gly Arg Pro Ala Met Asp Tyr Trp Gly Gln Gly Thr Leu Val Thr Val
                   325                 330                 335
   Ser Ser Ala Ser Thr Lys Gly Pro Ser Val Phe Pro Leu Ala Pro Ser
               340                 345                 350
   Ser Lys Ser Thr Ser Gly Gly Thr Ala Ala Leu Gly Cys Leu Val Lys
           355                 360                 365
   Asp Tyr Phe Pro Glu Pro Val Thr Val Ser Trp Asn Ser Gly Ala Leu
       370                 375                 380
   Thr Ser Gly Val His Thr Phe Pro Ala Val Leu Gln Ser Ser Gly Leu
   385                 390                 395                 400
   Tyr Ser Leu Ser Ser Val Val Thr Val Pro Ser Ser Ser Leu Gly Thr
                   405                 410                 415
   Gln Thr Tyr Ile Cys Asn Val Asn His Lys Pro Ser Asn Thr Lys Val
               420                 425                 430
   Asp Lys Lys Val Glu Pro Lys Ser Cys Asp Lys Thr His Thr Cys Pro
           435                 440                 445
   Pro Cys Pro Ala Pro Glu Ala Ala Gly Gly Pro Ser Val Phe Leu Phe
       450                 455                 460
   Pro Pro Lys Pro Lys Asp Thr Leu Met Ile Ser Arg Thr Pro Glu Val
   465                 470                 475                 480
   Thr Cys Val Val Val Asp Val Ser His Glu Asp Pro Glu Val Lys Phe
                   485                 490                 495
   Asn Trp Tyr Val Asp Gly Val Glu Val His Asn Ala Lys Thr Lys Pro
               500                 505                 510
   Arg Glu Glu Gln Tyr Asn Ser Thr Tyr Arg Val Val Ser Val Leu Thr
           515                 520                 525
   Val Leu His Gln Asp Trp Leu Asn Gly Lys Glu Tyr Lys Cys Lys Val
       530                 535                 540
   Ser Asn Lys Ala Leu Gly Ala Pro Ile Glu Lys Thr Ile Ser Lys Ala
   545                 550                 555                 560
   Lys Gly Gln Pro Arg Glu Pro Gln Val Tyr Thr Leu Pro Pro Cys Arg
                   565                 570                 575
   Asp Glu Leu Thr Lys Asn Gln Val Ser Leu Trp Cys Leu Val Lys Gly
               580                 585                 590
   Phe Tyr Pro Ser Asp Ile Ala Val Glu Trp Glu Ser Asn Gly Gln Pro
           595                 600                 605
   Glu Asn Asn Tyr Lys Thr Thr Pro Pro Val Leu Asp Ser Asp Gly Ser
       610                 615                 620
   Phe Phe Leu Tyr Ser Lys Leu Thr Val Asp Lys Ser Arg Trp Gln Gln
   625                 630                 635                 640
   Gly Asn Val Phe Ser Cys Ser Val Met His Glu Ala Leu His Asn His
                   645                 650                 655
   Tyr Thr Gln Lys Ser Leu Ser Leu Ser Pro Gly Lys
               660                 665
<210> 224
   <211> 2004
   <212> DNA
   <213> Artificial Sequence
<220>
   <223> V9(VL-CH1)-anti-CD33(VH-CH1)-Fc(knob) P329G LALA
<400> 224
   gatatccaga tgacccagag ccccagctct ctgagcgcca gcgtgggcga cagagtgacc      60
   atcacctgtc gggccagcca ggacatcaga aactacctga actggtatca gcagaagccc     120
   ggcaaggccc ccaagctgct gatctactac accagcagac tggaaagcgg cgtgccctcc     180
   agattttccg gcagcggctc cggcaccgac tacaccctga ccatcagcag cctgcagccc     240
   gaggatttcg ccacatatta ctgccagcag ggcaataccc tgccctggac cttcggacag     300
   ggcacaaaag tggaaatcaa gagcagcgct tccaccaaag gcccttccgt gtttcctctg     360
   gctcctagct ccaagtccac ctctggaggc accgctgctc tcggatgcct cgtgaaggat     420
   tattttcctg agcctgtgac agtgtcctgg aatagcggag cactgacctc tggagtgcat     480
   actttccccg ctgtgctgca gtcctctgga ctgtacagcc tgagcagcgt ggtgacagtg     540
   cccagcagca gcctgggcac ccagacctac atctgcaacg tgaaccacaa gcccagcaac     600
   accaaggtgg acaagaaggt ggaacccaag tcttgtggcg gaggcggatc cggcggaggc     660
   ggatctcagg tgcagctggt gcagtctggc gccgaagtga agaaacccgg cagcagcgtg     720
   aaggtgtcct gcaaggccag cggctacacc ttcaccgact acaacatgca ctgggtccgc     780
   caggccccag gccagggact ggaatggatc ggctacatct acccctacaa cggcggcacc     840
   ggctacaacc agaagttcaa gagcaaggcc accatcaccg ccgacgagag caccaacacc     900
   gcctacatgg aactgagcag cctgcggagc gaggacaccg ccgtgtacta ctgcgccaga       960
   ggcagacccg ccatggacta ctggggccag ggcaccctgg tgacagtgtc cagcgccagc      1020
   acaaagggcc ccagcgtgtt ccccctggca cccagcagca agagcacatc tggcggaaca     1080
   gccgctctgg gctgtctggt gaaagactac ttccccgagc ccgtgaccgt gtcttggaac      1140
   tctggcgccc tgaccagcgg cgtgcacacc tttccagccg tgctgcagag cagcggcctg      1200
   tactccctgt cctccgtggt caccgtgccc tctagctccc tgggaacaca gacatatatc      1260
   tgtaatgtca atcacaagcc ttccaacacc aaagtcgata agaaagtcga gcccaagagc      1320
   tgcgacaaaa ctcacacatg cccaccgtgc ccagcacctg aagctgcagg gggaccgtca      1380
   gtcttcctct tccccccaaa acccaaggac accctcatga tctcccggac ccctgaggtc      1440
   acatgcgtgg tggtggacgt gagccacgaa gaccctgagg tcaagttcaa ctggtacgtg      1500
   gacggcgtgg aggtgcataa tgccaagaca aagccgcggg aggagcagta caacagcacg      1560
   taccgtgtgg tcagcgtcct caccgtcctg caccaggact ggctgaatgg caaggagtac      1620
   aagtgcaagg tctccaacaa agccctcggc gcccccatcg agaaaaccat ctccaaagcc      1680
   aaagggcagc cccgagaacc acaggtgtac accctgcccc catgccggga tgagctgacc      1740
   aagaaccagg tcagcctgtg gtgcctggtc aaaggcttct atcccagcga catcgccgtg      1800
   gagtgggaga gcaatgggca gccggagaac aactacaaga ccacgcctcc cgtgctggac      1860
   tccgacggct ccttcttcct ctacagcaag ctcaccgtgg acaagagcag gtggcagcag      1920
   gggaacgtct tctcatgctc cgtgatgcat gaggctctgc acaaccacta cacgcagaag      1980
   agcctctccc tgtctccggg taaa                                             2004
<210> 225
   <211> 446
   <212> PRT
   <213> Artificial Sequence
<220>
   <223> anti-CD33(VH-CH1)-Fc(hole) P329G LALA
<400> 225
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Thr Phe Thr Asp Tyr
               20                  25                  30
   Asn Met His Trp Val Arg Gln Ala Pro Gly Gln Gly Leu Glu Trp Ile
           35                  40                  45
   Gly Tyr Ile Tyr Pro Tyr Asn Gly Gly Thr Gly Tyr Asn Gln Lys Phe
       50                  55                  60
   Lys Ser Lys Ala Thr Ile Thr Ala Asp Glu Ser Thr Asn Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Arg Pro Ala Met Asp Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Ala Ala Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Gly Ala Pro Ile Glu Lys Thr Ile Ser
   325 330 335
   Lys Ala Lys Gly Gln Pro Arg Glu Pro Gln Val Cys Thr Leu Pro Pro
   340 345 350
   Ser Arg Asp Glu Leu Thr Lys Asn Gln Val Ser Leu Ser Cys Ala Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Val Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly Lys
   435 440 445
<210> 226
   <211> 1338
   <212> DNA
   <213> Artificial Sequence
<220>
   <223> anti-CD33(VH-CH1)-Fc(hole) P329G LALA
<400> 226
   caggtgcagc tggtgcagtc tggcgccgaa gtgaagaaac ccggcagcag cgtgaaggtg 60
   tcctgcaagg ccagcggcta caccttcacc gactacaaca tgcactgggt ccgccaggcc 120
   ccaggccagg gactggaatg gatcggctac atctacccct acaacggcgg caccggctac 180
   aaccagaagt tcaagagcaa ggccaccatc accgccgacg agagcaccaa caccgcctac 240
   atggaactga gcagcctgcg gagcgaggac accgccgtgt actactgcgc cagaggcaga 300
   cccgccatgg actactgggg ccagggcacc ctggtgacag tgtccagcgc tagcaccaag 360
   ggcccctccg tgttccccct ggcccccagc agcaagagca ccagcggcgg cacagccgct 420
   ctgggctgcc tggtcaagga ctacttcccc gagcccgtga ccgtgtcctg gaacagcgga 480
   gccctgacct ccggcgtgca caccttcccc gccgtgctgc agagttctgg cctgtatagc 540
   ctgagcagcg tggtcaccgt gccttctagc agcctgggca cccagaccta catctgcaac 600
   gtgaaccaca agcccagcaa caccaaggtg gacaagaagg tggagcccaa gagctgcgac 660
   aaaactcaca catgcccacc gtgcccagca cctgaagctg cagggggacc gtcagtcttc 720
   ctcttccccc caaaacccaa ggacaccctc atgatctccc ggacccctga ggtcacatgc 780
   gtggtggtgg acgtgagcca cgaagaccct gaggtcaagt tcaactggta cgtggacggc 840
   gtggaggtgc ataatgccaa gacaaagccg cgggaggagc agtacaacag cacgtaccgt 900
   gtggtcagcg tcctcaccgt cctgcaccag gactggctga atggcaagga gtacaagtgc 960
   aaggtctcca acaaagccct cggcgccccc atcgagaaaa ccatctccaa agccaaaggg 1020
   cagccccgag aaccacaggt gtgcaccctg cccccatccc gggatgagct gaccaagaac 1080
   caggtcagcc tctcgtgcgc agtcaaaggc ttctatccca gcgacatcgc cgtggagtgg 1140
   gagagcaatg ggcagccgga gaacaactac aagaccacgc ctcccgtgct ggactccgac 1200
   ggctccttct tcctcgtgag caagctcacc gtggacaaga gcaggtggca gcaggggaac 1260
   gtcttctcat gctccgtgat gcatgaggct ctgcacaacc actacacgca gaagagcctc 1320
   tccctgtctc cgggtaaa 1338
<210> 227
   <211> 219
   <212> PRT
   <213> Artificial Sequence
<220>
   <223> anti-CD20(VL-CL)
<400> 227
   Asp Ile Val Met Thr Gln Thr Pro Leu Ser Leu Pro Val Thr Pro Gly
   1               5                   10                  15
   Glu Pro Ala Ser Ile Ser Cys Arg Ser Ser Lys Ser Leu Leu His Ser
               20                  25                  30
   Asn Gly Ile Thr Tyr Leu Tyr Trp Tyr Leu Gln Lys Pro Gly Gln Ser
           35                  40                  45
   Pro Gln Leu Leu Ile Tyr Gln Met Ser Asn Leu Val Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Asn
                   85                  90                  95
   Leu Glu Leu Pro Tyr Thr Phe Gly Gly Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 228
   <211> 657
   <212> DNA
   <213> Artificial Sequence
<220>
   <223> anti-CD20(VL-CL)
<400> 228
   gatatcgtga tgacccagac tccactctcc ctgcccgtca cccctggaga gcccgccagc      60
   attagctgca ggtctagcaa gagcctcttg cacagcaatg gcatcactta tttgtattgg     120
   tacctgcaaa agccagggca gtctccacag ctcctgattt atcaaatgtc caaccttgtc     180
   tctggcgtcc ctgaccggtt ctccggatcc gggtcaggca ctgatttcac actgaaaatc     240
   agcagggtgg aggctgagga tgttggagtt tattactgcg ctcagaatct agaacttcct     300
   tacaccttcg gcggagggac caaggtggag atcaaacgta cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgt        657
<210> 229
   <211> 671
   <212> PRT
   <213> Artificial Sequence
<220>
   <223> V9(VL-CH1)-anti-CD20(VH-CH1)-Fc(knob) P329G LALA
<400> 229
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Tyr
               20                  25                  30
   Leu Asn Trp Tyr Gln Gln Lys Pro Gly Lys Ala Pro Lys Leu Leu Ile
           35                  40                  45
   Tyr Tyr Thr Ser Arg Leu Glu Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Gln Gln Gly Asn Thr Leu Pro Trp
                   85                  90                  95
   Thr Phe Gly Gln Gly Thr Lys Val Glu Ile Lys Ser Ser Ala Ser Thr
               100                 105                 110
   Lys Gly Pro Ser Val Phe Pro Leu Ala Pro Ser Ser Lys Ser Thr Ser
           115                 120                 125
   Gly Gly Thr Ala Ala Leu Gly Cys Leu Val Lys Asp Tyr Phe Pro Glu
       130                 135                 140
   Pro Val Thr Val Ser Trp Asn Ser Gly Ala Leu Thr Ser Gly Val His
   145                 150                 155                 160
   Thr Phe Pro Ala Val Leu Gln Ser Ser Gly Leu Tyr Ser Leu Ser Ser
                   165                 170                 175
   Val Val Thr Val Pro Ser Ser Ser Leu Gly Thr Gln Thr Tyr Ile Cys
               180                 185                 190
   Asn Val Asn His Lys Pro Ser Asn Thr Lys Val Asp Lys Lys Val Glu
           195                 200                 205
   Pro Lys Ser Cys Gly Gly Gly Gly Ser Gly Gly Gly Gly Ser Gln Val
       210                 215                 220
   Gln Leu Val Gln Ser Gly Ala Glu Val Lys Lys Pro Gly Ser Ser Val
   225                 230                 235                 240
   Lys Val Ser Cys Lys Ala Ser Gly Tyr Ala Phe Ser Tyr Ser Trp Ile
                   245                 250                 255
   Asn Trp Val Arg Gln Ala Pro Gly Gln Gly Leu Glu Trp Met Gly Arg
   260 265 270
   Ile Phe Pro Gly Asp Gly Asp Thr Asp Tyr Asn Gly Lys Phe Lys Gly
   275 280 285
   Arg Val Thr Ile Thr Ala Asp Lys Ser Thr Ser Thr Ala Tyr Met Glu
   290 295 300
   Leu Ser Ser Leu Arg Ser Glu Asp Thr Ala Val Tyr Tyr Cys Ala Arg
   305 310 315 320
   Asn Val Phe Asp Gly Tyr Trp Leu Val Tyr Trp Gly Gln Gly Thr Leu
   325 330 335
   Val Thr Val Ser Ser Ala Ser Thr Lys Gly Pro Ser Val Phe Pro Leu
   340 345 350
   Ala Pro Ser Ser Lys Ser Thr Ser Gly Gly Thr Ala Ala Leu Gly Cys
   355 360 365
   Leu Val Lys Asp Tyr Phe Pro Glu Pro Val Thr Val Ser Trp Asn Ser
   370 375 380
   Gly Ala Leu Thr Ser Gly Val His Thr Phe Pro Ala Val Leu Gln Ser
   385 390 395 400
   Ser Gly Leu Tyr Ser Leu Ser Ser Val Val Thr Val Pro Ser Ser Ser
   405 410 415
   Leu Gly Thr Gln Thr Tyr Ile Cys Asn Val Asn His Lys Pro Ser Asn
   420 425 430
   Thr Lys Val Asp Lys Lys Val Glu Pro Lys Ser Cys Asp Lys Thr His
   435 440 445
   Thr Cys Pro Pro Cys Pro Ala Pro Glu Ala Ala Gly Gly Pro Ser Val
   450 455 460
   Phe Leu Phe Pro Pro Lys Pro Lys Asp Thr Leu Met Ile Ser Arg Thr
   465 470 475 480
   Pro Glu Val Thr Cys Val Val Val Asp Val Ser His Glu Asp Pro Glu
   485 490 495
   Val Lys Phe Asn Trp Tyr Val Asp Gly Val Glu Val His Asn Ala Lys
   500 505 510
   Thr Lys Pro Arg Glu Glu Gln Tyr Asn Ser Thr Tyr Arg Val Val Ser
   515 520 525
   Val Leu Thr Val Leu His Gln Asp Trp Leu Asn Gly Lys Glu Tyr Lys
   530 535 540
   Cys Lys Val Ser Asn Lys Ala Leu Gly Ala Pro Ile Glu Lys Thr Ile
   545 550 555 560
   Ser Lys Ala Lys Gly Gln Pro Arg Glu Pro Gln Val Tyr Thr Leu Pro
   565 570 575
   Pro Cys Arg Asp Glu Leu Thr Lys Asn Gln Val Ser Leu Trp Cys Leu
   580 585 590
   Val Lys Gly Phe Tyr Pro Ser Asp Ile Ala Val Glu Trp Glu Ser Asn
   595 600 605
   Gly Gln Pro Glu Asn Asn Tyr Lys Thr Thr Pro Pro Val Leu Asp Ser
   610 615 620
   Asp Gly Ser Phe Phe Leu Tyr Ser Lys Leu Thr Val Asp Lys Ser Arg
   625 630 635 640
   Trp Gln Gln Gly Asn Val Phe Ser Cys Ser Val Met His Glu Ala Leu
   645 650 655
   His Asn His Tyr Thr Gln Lys Ser Leu Ser Leu Ser Pro Gly Lys
   660 665 670
<210> 230
   <211> 2013
   <212> DNA
   <213> Artificial Sequence
<220>
   <223> V9(VL-CH1)-anti-CD20(VH-CH1)-Fc(knob) P329G LALA
<400> 230
   gatatccaga tgacccagag ccccagctct ctgagcgcca gcgtgggcga cagagtgacc 60
   atcacctgtc gggccagcca ggacatcaga aactacctga actggtatca gcagaagccc 120
   ggcaaggccc ccaagctgct gatctactac accagcagac tggaaagcgg cgtgccctcc 180
   agattttccg gcagcggctc cggcaccgac tacaccctga ccatcagcag cctgcagccc 240
   gaggatttcg ccacatatta ctgccagcag ggcaataccc tgccctggac cttcggacag 300
   ggcacaaaag tggaaatcaa gagcagcgct tccaccaaag gcccttccgt gtttcctctg 360
   gctcctagct ccaagtccac ctctggaggc accgctgctc tcggatgcct cgtgaaggat 420
   tattttcctg agcctgtgac agtgtcctgg aatagcggag cactgacctc tggagtgcat 480
   actttccccg ctgtgctgca gtcctctgga ctgtacagcc tgagcagcgt ggtgacagtg 540
   cccagcagca gcctgggcac ccagacctac atctgcaacg tgaaccacaa gcccagcaac      600
   accaaggtgg acaagaaggt ggaacccaag tcttgtggcg gaggcggatc cggcggaggg      660
   ggatctcagg tgcaattggt gcagtctggc gctgaagtta agaagcctgg gagttcagtg      720
   aaggtctcct gcaaggcttc cggatacgcc ttcagctatt cttggatcaa ttgggtgcgg      780
   caggcgcctg gacaagggct cgagtggatg ggacggatct ttcccggcga tggggatact      840
   gactacaatg ggaaattcaa gggcagagtc acaattaccg ccgacaaatc cactagcaca      900
   gcctatatgg agctgagcag cctgagatct gaggacacgg ccgtgtatta ctgtgcaaga      960
   aatgtctttg atggttactg gcttgtttac tggggccagg gaaccctggt caccgtctcc     1020
   tcagctagca ccaagggccc cagcgtgttc cccctggcac ccagcagcaa gagcacatct     1080
   ggcggaacag ccgctctggg ctgtctggtg aaagactact tccccgagcc cgtgaccgtg     1140
   tcttggaact ctggcgccct gaccagcggc gtgcacacct ttccagccgt gctgcagagc     1200
   agcggcctgt actccctgtc ctccgtggtc accgtgccct ctagctccct gggaacacag     1260
   acatatatct gtaatgtcaa tcacaagcct tccaacacca aagtcgataa gaaagtcgag     1320
   cccaagagct gcgacaaaac tcacacatgc ccaccgtgcc cagcacctga agctgcaggg     1380
   ggaccgtcag tcttcctctt ccccccaaaa cccaaggaca ccctcatgat ctcccggacc     1440
   cctgaggtca catgcgtggt ggtggacgtg agccacgaag accctgaggt caagttcaac     1500
   tggtacgtgg acggcgtgga ggtgcataat gccaagacaa agccgcggga ggagcagtac     1560
   aacagcacgt accgtgtggt cagcgtcctc accgtcctgc accaggactg gctgaatggc     1620
   aaggagtaca agtgcaaggt ctccaacaaa gccctcggcg cccccatcga gaaaaccatc     1680
   tccaaagcca aagggcagcc ccgagaacca caggtgtaca ccctgccccc atgccgggat     1740
   gagctgacca agaaccaggt cagcctgtgg tgcctggtca aaggcttcta tcccagcgac     1800
   atcgccgtgg agtgggagag caatgggcag ccggagaaca actacaagac cacgcctccc     1860
   gtgctggact ccgacggctc cttcttcctc tacagcaagc tcaccgtgga caagagcagg     1920
   tggcagcagg ggaacgtctt ctcatgctcc gtgatgcatg aggctctgca caaccactac     1980
   acgcagaaga gcctctccct gtctccgggt aaa                                  2013
<210> 231
   <211> 449
   <212> PRT
   <213> Artificial Sequence
<220>
   <223> anti-CD20(VH-CH1)-Fc(hole) P329G LALA
<400> 231
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
   20 25 30
   Trp Ile Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser Ala Ser Thr Lys Gly Pro Ser Val Phe
   115 120 125
   Pro Leu Ala Pro Ser Ser Lys Ser Thr Ser Gly Gly Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Ile Cys Asn Val Asn His Lys Pro
   195 200 205
   Ser Asn Thr Lys Val Asp Lys Lys Val Glu Pro Lys Ser Cys Asp Lys
   210 215 220
   Thr His Thr Cys Pro Pro Cys Pro Ala Pro Glu Ala Ala Gly Gly Pro
   225 230 235 240
   Ser Val Phe Leu Phe Pro Pro Lys Pro Lys Asp Thr Leu Met Ile Ser
   245 250 255
   Arg Thr Pro Glu Val Thr Cys Val Val Val Asp Val Ser His Glu Asp
   260 265 270
   Pro Glu Val Lys Phe Asn Trp Tyr Val Asp Gly Val Glu Val His Asn
   275 280 285
   Ala Lys Thr Lys Pro Arg Glu Glu Gln Tyr Asn Ser Thr Tyr Arg Val
   290 295 300
   Val Ser Val Leu Thr Val Leu His Gln Asp Trp Leu Asn Gly Lys Glu
   305 310 315 320
   Tyr Lys Cys Lys Val Ser Asn Lys Ala Leu Gly Ala Pro Ile Glu Lys
   325 330 335
   Thr Ile Ser Lys Ala Lys Gly Gln Pro Arg Glu Pro Gln Val Cys Thr
   340 345 350
   Leu Pro Pro Ser Arg Asp Glu Leu Thr Lys Asn Gln Val Ser Leu Ser
   355 360 365
   Cys Ala Val Lys Gly Phe Tyr Pro Ser Asp Ile Ala Val Glu Trp Glu
   370 375 380
   Ser Asn Gly Gln Pro Glu Asn Asn Tyr Lys Thr Thr Pro Pro Val Leu
   385 390 395 400
   Asp Ser Asp Gly Ser Phe Phe Leu Val Ser Lys Leu Thr Val Asp Lys
   405 410 415
   Ser Arg Trp Gln Gln Gly Asn Val Phe Ser Cys Ser Val Met His Glu
   420 425 430
   Ala Leu His Asn His Tyr Thr Gln Lys Ser Leu Ser Leu Ser Pro Gly
   435 440 445
   Lys
<210> 232
   <211> 1347
   <212> DNA
   <213> Artificial Sequence
<220>
   <223> anti-CD20(VH-CH1)-Fc(hole) P329G LALA
<400> 232
   caggtgcaat tggtgcagtc tggcgctgaa gttaagaagc ctgggagttc agtgaaggtc 60
   tcctgcaagg cttccggata cgccttcagc tattcttgga tcaattgggt gcggcaggcg 120
   cctggacaag ggctcgagtg gatgggacgg atctttcccg gcgatgggga tactgactac 180
   aatgggaaat tcaagggcag agtcacaatt accgccgaca aatccactag cacagcctat 240
   atggagctga gcagcctgag atctgaggac acggccgtgt attactgtgc aagaaatgtc 300
   tttgatggtt actggcttgt ttactggggc cagggaaccc tggtcaccgt ctcctcagct 360
   agcaccaagg gcccatcggt cttccccctg gcaccctcct ccaagagcac ctctgggggc 420
   acagcggccc tgggctgcct ggtcaaggac tacttccccg aaccggtgac ggtgtcgtgg 480
   aactcaggcg ccctgaccag cggcgtgcac accttcccgg ctgtcctaca gtcctcagga 540
   ctctactccc tcagcagcgt ggtgaccgtg ccctccagca gcttgggcac ccagacctac 600
   atctgcaacg tgaatcacaa gcccagcaac accaaggtgg acaagaaagt tgagcccaaa 660
   tcttgtgaca aaactcacac atgcccaccg tgcccagcac ctgaagctgc agggggaccg 720
   tcagtcttcc tcttcccccc aaaacccaag gacaccctca tgatctcccg gacccctgag 780
   gtcacatgcg tggtggtgga cgtgagccac gaagaccctg aggtcaagtt caactggtac 840
   gtggacggcg tggaggtgca taatgccaag acaaagccgc gggaggagca gtacaacagc 900
   acgtaccgtg tggtcagcgt cctcaccgtc ctgcaccagg actggctgaa tggcaaggag 960
   tacaagtgca aggtctccaa caaagccctc ggcgccccca tcgagaaaac catctccaaa 1020
   gccaaagggc agccccgaga accacaggtg tgcaccctgc ccccatcccg ggatgagctg 1080
   accaagaacc aggtcagcct ctcgtgcgca gtcaaaggct tctatcccag cgacatcgcc 1140
   gtggagtggg agagcaatgg gcagccggag aacaactaca agaccacgcc tcccgtgctg 1200
   gactccgacg gctccttctt cctcgtgagc aagctcaccg tggacaagag caggtggcag 1260
   caggggaacg tcttctcatg ctccgtgatg catgaggctc tgcacaacca ctacacgcag 1320
   aagagcctct ccctgtctcc gggtaaa 1347
<210> 233
   <211> 11
   <212> PRT
   <213> Artificial Sequence
<220>
   <223> M4-3 ML2 HCDR1
<400> 233
   Gly Gly Ser Ile Thr Ser Gly Tyr Tyr Trp Asn
   1 5 10
<210> 234
   <211> 33
   <212> DNA
   <213> Artificial Sequence
<220>
   <223> M4-3 ML2 HCDR1
<400> 234
   ggcggcagca tcaccagcgg ctactactgg aac 33
<210> 235
   <211> 16
   <212> PRT
   <213> Artificial Sequence
<220>
   <223> M4-3 ML2 HCDR2
<400> 235
   Tyr Ile Thr Tyr Asp Gly Ser Asn Asn Tyr Asn Pro Ser Leu Lys Ser
   1 5 10 15
<210> 236
   <211> 48
   <212> DNA
   <213> Artificial Sequence
<220>
   <223> M4-3 ML2 HCDR2
<400> 236
   tacatcacct acgacggcag caacaactac aaccccagcc tgaagtcc 48
<210> 237
   <211> 3
   <212> PRT
   <213> Artificial Sequence
<220>
   <223> M4-3 ML2 HCDR3
<400> 237
   Phe Asp Tyr
   1
<210> 238
   <211> 9
   <212> DNA
   <213> Artificial Sequence
<220>
   <223> M4-3 ML2 HCDR3
<400> 238
   ttcgactac 9
<210> 239
   <211> 112
   <212> PRT
   <213> Artificial Sequence
<220>
   <223> M4-3 ML2 VH
<400> 239
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Gly Ser Ile Thr Ser Gly
               20                  25                  30
   Tyr Tyr Trp Asn Trp Ile Arg Gln His Pro Gly Lys Gly Leu Glu Trp
           35                  40                  45
   Ile Gly Tyr Ile Thr Tyr Asp Gly Ser Asn Asn Tyr Asn Pro Ser Leu
       50                  55                  60
   Lys Ser Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Asp Phe Asp Tyr Trp Gly Gln Gly Thr Leu Val Thr Val Ser Ser
               100                 105                 110
<210> 240
   <211> 336
   <212> DNA
   <213> Artificial Sequence
<220>
   <223> M4-3 ML2 VH
<400> 240
   caggtgcagc tgcaggaaag cggccctggc ctggtcaagc ccagccagac cctgagcctg       60
   acctgcaccg tgtccggcgg cagcatcacc agcggctact actggaactg gatccggcag      120
   caccccggca agggcctgga atggatcggc tacatcacct acgacggcag caacaactac      180
   aaccccagcc tgaagtccag agtgaccatc agccgggaca ccagcaagaa ccagttcagc      240
   ctgaagctgt ccagcgtgac agccgccgac accgccgtgt actactgcgc cgacttcgac      300
   tactggggcc agggcaccct ggtcaccgtg tccagc                                336
<210> 241
   <211> 11
   <212> PRT
   <213> Artificial Sequence
<220>
   <223> M4-3 ML2 LCDR1
<400> 241
   Arg Ala Ser Gln Gly Ile Arg Asn Tyr Leu Asn
   1 5 10
<210> 242
   <211> 33
   <212> DNA
   <213> Artificial Sequence
<220>
   <223> M4-3 ML2 LCDR1
<400> 242
   cgggccagcc agggcatccg gaactacctg aac 33
<210> 243
   <211> 7
   <212> PRT
   <213> Artificial Sequence
<220>
   <223> M4-3 ML2 LCDR2
<400> 243
   Tyr Thr Ser Ser Leu His Ser
   1 5
<210> 244
   <211> 21
   <212> DNA
   <213> Artificial Sequence
<220>
   <223> M4-3 ML2 LCDR2
<400> 244
   tacaccagca gcctgcacag c 21
<210> 245
   <211> 9
   <212> PRT
   <213> Artificial Sequence
<220>
   <223> M4-3 ML2 LCDR3
<400> 245
   Gln Gln Tyr Ser Lys Leu Pro Trp Thr
   1 5
<210> 246
   <211> 27
   <212> DNA
   <213> Artificial Sequence
<220>
   <223> M4-3 ML2 LCDR3
<400> 246
   cagcagtaca gcaagctgcc ctggacc 27
<210> 247
   <211> 107
   <212> PRT
   <213> Artificial Sequence
<220>
   <223> M4-3 ML2 VL
<400> 247
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Tyr
   20 25 30
   Leu Asn Trp Tyr Gln Gln Lys Pro Gly Lys Ala Pro Lys Leu Leu Ile
   35 40 45
   Tyr Tyr Thr Ser Ser Leu His Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Ser Lys Leu Pro Trp
   85 90 95
   Thr Phe Gly Gln Gly Thr Lys Val Glu Ile Lys
   100 105
<210> 248
   <211> 321
   <212> DNA
   <213> Artificial Sequence
<220>
   <223> M4-3 ML2 VL
<400> 248
   gacatccaga tgacccagag ccccagcagc ctgagcgcca gcgtgggcga cagagtgacc 60
   atcacctgcc gggccagcca gggcatccgg aactacctga actggtatca gcagaagccc 120
   ggcaaggccc ccaagctgct gatctactac accagcagcc tgcacagcgg cgtgcctagc 180
   cggtttagcg gcagcggctc cggcaccgac ttcaccctga ccattagctc cctgcagccc 240
   gaggacttcg ccacctacta ctgccagcag tacagcaagc tgccctggac cttcggccag 300
   ggaacaaagg tggagatcaa g 321
<210> 249
   <211> 5
   <212> PRT
   <213> Artificial Sequence
<220>
   <223> anti-CD3 HCDR1
<400> 249
   Thr Tyr Ala Met Asn
   1 5
<210> 250
   <211> 15
   <212> DNA
   <213> Artificial Sequence
<220>
   <223> anti-CD3 HCDR1
<400> 250
   acctacgcca tgaac 15
<210> 251
   <211> 19
   <212> PRT
   <213> Artificial Sequence
<220>
   <223> anti-CD3 HCDR2
<400> 251
   Arg Ile Arg Ser Lys Tyr Asn Asn Tyr Ala Thr Tyr Tyr Ala Asp Ser
   1 5 10 15
   Val Lys Asp
<210> 252
   <211> 57
   <212> DNA
   <213> Artificial Sequence
<220>
   <223> anti-CD3 HCDR2
<400> 252
   cggatcagaa gcaagtacaa caattacgcc acctactacg ccgacagcgt gaaggac 57
<210> 253
   <211> 14
   <212> PRT
   <213> Artificial Sequence
<220>
   <223> anti-CD3 HCDR3
<400> 253
   His Gly Asn Phe Gly Asn Ser Tyr Val Ser Trp Phe Ala Tyr
   1 5 10
<210> 254
   <211> 42
   <212> DNA
   <213> Artificial Sequence
<220>
   <223> anti-CD3 HCDR3
<400> 254
   cacggcaact tcggcaacag ctatgtgtct tggtttgcct ac 42
<210> 255
   <211> 125
   <212> PRT
   <213> Artificial Sequence
<220>
   <223> anti-CD3 VH
<400> 255
   Glu Val Lys Leu Leu Glu Ser Gly Gly Gly Leu Val Gln Pro Lys Gly
   1               5                   10                  15
   Ser Leu Lys Leu Ser Cys Ala Ala Ser Gly Phe Thr Phe Asn Thr Tyr
               20                  25                  30
   Ala Met Asn Trp Val Arg Gln Ala Pro Gly Lys Gly Leu Glu Trp Val
           35                  40                  45
   Ala Arg Ile Arg Ser Lys Tyr Asn Asn Tyr Ala Thr Tyr Tyr Ala Asp
       50                  55                  60
   Ser Val Lys Asp Arg Phe Thr Ile Ser Arg Asp Asp Ser Gln Ser Ile
   65                  70                  75                  80
   Leu Tyr Leu Gln Met Asn Asn Leu Lys Thr Glu Asp Thr Ala Met Tyr
                   85                  90                  95
   Tyr Cys Val Arg His Gly Asn Phe Gly Asn Ser Tyr Val Ser Trp Phe
               100                 105                 110
   Ala Tyr Trp Gly Gln Gly Thr Leu Val Thr Val Ser Ala
           115                 120                 125
<210> 256
   <211> 375
   <212> DNA
   <213> Artificial Sequence
<220>
   <223> anti-CD3 VH
<400> 256
   gaagtgcagc tggtggaaag cggcggaggc ctggtgcagc ctaagggctc tctgaagctg 60
   agctgtgccg ccagcggctt caccttcaac acctacgcca tgaactgggt gcgccaggcc 120
   cctggcaaag gcctggaatg ggtggcccgg atcagaagca agtacaacaa ttacgccacc 180
   tactacgccg acagcgtgaa ggaccggttc accatcagcc gggacgacag ccagagcatc 240
   ctgtacctgc agatgaacaa cctgaaaacc gaggacaccg ccatgtacta ctgcgtgcgg 300
   cacggcaact tcggcaacag ctatgtgtct tggtttgcct actggggcca gggcaccctc 360
   gtgacagtgt ctgct 375
<210> 257
   <211> 14
   <212> PRT
   <213> Artificial Sequence
<220>
   <223> anti-CD3 LCDR1
<400> 257
   Arg Ser Ser Thr Gly Ala Val Thr Thr Ser Asn Tyr Ala Asn
   1               5                   10
<210> 258
   <211> 42
   <212> DNA
   <213> Artificial Sequence
<220>
   <223> anti-CD3 LCDR1
<400> 258
   agatctagca caggcgccgt gaccaccagc aactacgcca ac 42
<210> 259
   <211> 7
   <212> PRT
   <213> Artificial Sequence
<220>
   <223> anti-CD3 LCDR2
<400> 259
   Gly Thr Asn Lys Arg Ala Pro
   1               5
<210> 260
   <211> 21
   <212> DNA
   <213> Artificial Sequence
<220>
   <223> anti-CD3 LCDR2
<400> 260
   ggcaccaaca aaagggctcc a 21
<210> 261
   <211> 9
   <212> PRT
   <213> Artificial Sequence
<220>
   <223> anti-CD3 LCDR3
<400> 261
   Ala Leu Trp Tyr Ser Asn Leu Trp Val
   1               5
<210> 262
   <211> 27
   <212> DNA
   <213> Artificial Sequence
<220>
   <223> anti-CD3 LCDR3
<400> 262
   gccctgtggt acagcaacct gtgggtg 27
<210> 263
   <211> 109
   <212> PRT
   <213> Artificial Sequence
<220>
   <223> anti-CD3 VL
<400> 263
   Gln Ala Val Val Thr Gln Glu Ser Ala Leu Thr Thr Ser Pro Gly Glu
   1               5                   10                  15
   Thr Val Thr Leu Thr Cys Arg Ser Ser Thr Gly Ala Val Thr Thr Ser
               20                  25                  30
   Asn Tyr Ala Asn Trp Val Gln Glu Lys Pro Asp His Leu Phe Thr Gly
           35                  40                  45
   Leu Ile Gly Gly Thr Asn Lys Arg Ala Pro Gly Val Pro Ala Arg Phe
       50                  55                  60
   Ser Gly Ser Leu Ile Gly Asp Lys Ala Ala Leu Thr Ile Thr Gly Ala
   65                  70                  75                  80
   Gln Thr Glu Asp Glu Ala Ile Tyr Phe Cys Ala Leu Trp Tyr Ser Asn
                   85                  90                  95
   Leu Trp Val Phe Gly Gly Gly Thr Lys Leu Thr Val Leu
               100                 105
<210> 264
   <211> 327
   <212> DNA
   <213> Artificial Sequence
<220>
   <223> anti-CD3 VL
<400> 264
   caggccgtcg tgacccagga aagcgccctg acaacaagcc ctggcgagac agtgaccctg        60
   acctgcagat ctagcacagg cgccgtgacc accagcaact acgccaactg ggtgcaggaa       120
   aagcccgacc acctgttcac cggcctgatc ggcggcacca acaaaagggc tccaggcgtg       180
   ccagccagat tcagcggcag cctgattggc gataaggccg ccctgaccat cactggcgcc       240
   cagacagagg acgaggccat ctacttttgc gccctgtggt acagcaacct gtgggtgttc       300
   ggcggaggca ccaagctgac agtgctg                                           327
<210> 265
   <211> 207
   <212> PRT
   <213> Homo sapiens
<400> 265
   Met Gln Ser Gly Thr His Trp Arg Val Leu Gly Leu Cys Leu Leu Ser
   1               5                   10                  15
   Val Gly Val Trp Gly Gln Asp Gly Asn Glu Glu Met Gly Gly Ile Thr
               20                  25                  30
   Gln Thr Pro Tyr Lys Val Ser Ile Ser Gly Thr Thr Val Ile Leu Thr
           35                  40                  45
   Cys Pro Gln Tyr Pro Gly Ser Glu Ile Leu Trp Gln His Asn Asp Lys
       50                  55                  60
   Asn Ile Gly Gly Asp Glu Asp Asp Lys Asn Ile Gly Ser Asp Glu Asp
   65                  70                  75                  80
   His Leu Ser Leu Lys Glu Phe Ser Glu Leu Glu Gln Ser Gly Tyr Tyr
                   85                  90                  95
   Val Cys Tyr Pro Arg Gly Ser Lys Pro Glu Asp Ala Asn Phe Tyr Leu
               100                 105                 110
   Tyr Leu Arg Ala Arg Val Cys Glu Asn Cys Met Glu Met Asp Val Met
           115                 120                 125
   Ser Val Ala Thr Ile Val Ile Val Asp Ile Cys Ile Thr Gly Gly Leu
       130                 135                 140
   Leu Leu Leu Val Tyr Tyr Trp Ser Lys Asn Arg Lys Ala Lys Ala Lys
   145                 150                 155                 160
   Pro Val Thr Arg Gly Ala Gly Ala Gly Gly Arg Gln Arg Gly Gln Asn
                   165                 170                 175
   Lys Glu Arg Pro Pro Pro Val Pro Asn Pro Asp Tyr Glu Pro Ile Arg
               180                 185                 190
   Lys Gly Gln Arg Asp Leu Tyr Ser Gly Leu Asn Gln Arg Arg Ile
           195                 200                 205
<210> 266
   <211> 198
   <212> PRT
   <213> Macaca fascicularis
<400> 266
   Met Gln Ser Gly Thr Arg Trp Arg Val Leu Gly Leu Cys Leu Leu Ser
   1               5                   10                  15
   Ile Gly Val Trp Gly Gln Asp Gly Asn Glu Glu Met Gly Ser Ile Thr
               20                  25                  30
   Gln Thr Pro Tyr Gln Val Ser Ile Ser Gly Thr Thr Val Ile Leu Thr
           35                  40                  45
   Cys Ser Gln His Leu Gly Ser Glu Ala Gln Trp Gln His Asn Gly Lys
       50                  55                  60
   Asn Lys Glu Asp Ser Gly Asp Arg Leu Phe Leu Pro Glu Phe Ser Glu
   65                  70                  75                  80
   Met Glu Gln Ser Gly Tyr Tyr Val Cys Tyr Pro Arg Gly Ser Asn Pro
                   85                  90                  95
   Glu Asp Ala Ser His His Leu Tyr Leu Lys Ala Arg Val Cys Glu Asn
               100                 105                 110
   Cys Met Glu Met Asp Val Met Ala Val Ala Thr Ile Val Ile Val Asp
           115                 120                 125
   Ile Cys Ile Thr Leu Gly Leu Leu Leu Leu Val Tyr Tyr Trp Ser Lys
       130                 135                 140
   Asn Arg Lys Ala Lys Ala Lys Pro Val Thr Arg Gly Ala Gly Ala Gly
   145                 150                 155                 160
   Gly Arg Gln Arg Gly Gln Asn Lys Glu Arg Pro Pro Pro Val Pro Asn
                   165                 170                 175
   Pro Asp Tyr Glu Pro Ile Arg Lys Gly Gln Gln Asp Leu Tyr Ser Gly
               180                 185                 190
   Leu Asn Gln Arg Arg Ile
           195

## Description

### Field of the Invention

The present invention generally relates to bispecific antigen binding molecules for activating T cells. In addition, the present invention relates to polynucleotides encoding such bispecific antigen binding molecules, to methods for producing the bispecific antigen binding molecules of the invention, and to such bispecific antigen binding molecules for use in the treatment of disease.

### Background

The selective destruction of an individual cell or a specific cell type is often desirable in a variety of clinical settings. For example, it is a primary goal of cancer therapy to specifically destroy tumor cells, while leaving healthy cells and tissues intact and undamaged.

An attractive way of achieving this is by inducing an immune response against the tumor, to make immune effector cells such as natural killer (NK) cells or cytotoxic T lymphocytes (CTLs) attack and destroy tumor cells. CTLs constitute the most potent effector cells of the immune system, however they cannot be activated by the effector mechanism mediated by the Fc domain of conventional therapeutic antibodies.

In this regard, bispecific antibodies designed to bind with one "arm" to a surface antigen on target cells, and with the second "arm" to an activating, invariant component of the T cell receptor (TCR) complex, have become of interest in recent years. The simultaneous binding of such an antibody to both of its targets will force a temporary interaction between target cell and T cell, causing activation of any cytotoxic T cell and subsequent lysis of the target cell. Hence, the immune response is re-directed to the target cells and is independent of peptide antigen presentation by the target cell or the specificity of the T cell as would be relevant for normal MHC-restricted activation of CTLs. In this context it is crucial that CTLs are only activated when a target cell is presenting the bispecific antibody to them, i.e. the immunological synapse is mimicked. Particularly desirable are bispecific antibodies that do not require lymphocyte preconditioning or co-stimulation in order to elicit efficient lysis of target cells.

Several bispecific antibody formats have been developed and their suitability for T cell mediated immunotherapy investigated. Out of these, the so-called BiTE (bispecific T cell engager) molecules have been very well characterized and already shown some promise in the clinic (reviewed in Nagorsen and Bäuerle, Exp Cell Res 317, 1255-1260 (2011)). BiTEs are tandem scFv molecules wherein two scFv molecules are fused by a flexible linker. Further bispecific formats being evaluated for T cell engagement include diabodies (Holliger et al., Prot Eng 9, 299-305 (1996)) and derivatives thereof, such as tandem diabodies (Kipriyanov et al., J Mol Biol 293, 41-66 (1999)). A more recent development are the so-called DART (dual affinity retargeting) molecules, which are based on the diabody format but feature a C-terminal disulfide bridge for additional stabilization (Moore et al., Blood 117, 4542-51 (2011)). The so-called triomabs, which are whole hybrid mouse/rat IgG molecules and also currently being evaluated in clinical trials, represent a larger sized format (reviewed in Seimetz et al., Cancer Treat Rev 36, 458-467 (2010)).

The variety of formats that are being developed shows the great potential attributed to T cell re-direction and activation in immunotherapy. The task of generating bispecific antibodies suitable therefor is, however, by no means trivial, but involves a number of challenges that have to be met related to efficacy, toxicity, applicability and produceability of the antibodies.

Small constructs such as, for example, BiTE molecules - while being able to efficiently crosslink effector and target cells - have a very short serum half life requiring them to be administered to patients by continuous infusion. IgG-like formats on the other hand - while having the great benefit of a long half life - suffer from toxicity associated with the native effector functions inherent to IgG molecules. Their immunogenic potential constitutes another unfavorable feature of IgG-like bispecific antibodies, especially non-human formats, for successful therapeutic development. Finally, a major challenge in the general development of bispecific antibodies has been the production of bispecific antibody constructs at a clinically sufficient quantity and purity, due to the mispairing of antibody heavy and light chains of different specificities upon co-expression, which decreases the yield of the correctly assembled construct and results in a number of non-functional side products from which the desired bispecific antibody may be difficult to separate.

Given the difficulties and disadvantages associated with currently available bispecific antibodies for T cell mediated immunotherapy, there remains a need for novel, improved formats of such molecules. The present invention provides bispecific antigen binding molecules designed for T cell activation and re-direction that combine good efficacy and produceability with low toxicity and favorable pharmacokinetic properties.

### Summary of the Invention

In a first aspect the present invention provides a T cell activating bispecific antigen binding molecule comprising a first and a second antigen binding moiety, one of which is a Fab molecule capable of specific binding to an activating T cell antigen and the other one of which is a Fab molecule capable of specific binding to a target cell antigen, and an Fc domain composed of a first and a second subunit capable of stable association; wherein the first antigen binding moiety is a crossover Fab molecule wherein either the variable or the constant regions of the Fab light chain and the Fab heavy chain are exchanged; wherein the first and the second antigen binding moiety are fused to each other, optionally via a peptide linker; and wherein the T cell activating bispecific antigen binding molecule comprises not more than one antigen binding moiety capable of specific binding to an activating T cell antigen (i.e. the T cell activating bispecific antigen binding molecule provides monovalent binding to the activating T cell antigen).

According to the invention, the first and the second antigen binding moiety of the T cell activating bispecific antigen binding molecule are fused to each other, optionally via a peptide linker. In one embodiment, the second antigen binding moiety is fused at the C-terminus of the Fab heavy chain to the N-terminus of the Fab heavy chain of the first antigen binding moiety. In another embodiment, the first antigen binding moiety is fused at the C-terminus of the Fab heavy chain to the N-terminus of the Fab heavy chain of the second antigen binding moiety. In yet another embodiment, the second antigen binding moiety is fused at the C-terminus of the Fab light chain to the N-terminus of the Fab light chain of the first antigen binding moiety. In embodiments wherein either (i) the second antigen binding moiety is fused at the C-terminus of the Fab heavy chain to the N-terminus of the Fab heavy chain of the first antigen binding moiety or (ii) the first antigen binding moiety is fused at the C-terminus of the Fab heavy chain to the N-terminus of the Fab heavy chain of the second antigen binding moiety, additionally the Fab light chain of the first antigen binding moiety and the Fab light chain of the second antigen binding moiety may be fused to each other, optionally via a peptide linker.

In one embodiment, wherein (i) the first antigen binding moiety is fused at the C-terminus of the Fab heavy chain to the N-terminus of the Fab heavy chain of the second antigen binding moiety or (ii) the second antigen binding moiety is fused at the C-terminus of the Fab light chain to the N-terminus of the Fab light chain of the first antigen binding moiety, the second antigen binding moiety of the T cell activating bispecific antigen binding molecule is fused at the C-terminus of the Fab heavy chain to the N-terminus of the first or the second subunit of the Fc domain. In another embodiment, wherein the second antigen binding moiety is fused at the C-terminus of the Fab heavy chain to the N-terminus of the Fab heavy chain of the first antigen binding moiety, the first antigen binding moiety is fused at the C-terminus of the Fab heavy chain to the N-terminus of the first or second subunit of the Fc domain.

In certain embodiments, the T cell activating bispecific antigen binding molecule comprises a third antigen binding moiety which is a Fab molecule capable of specific binding to a target cell antigen. In one such embodiment, the third antigen binding moiety is fused at the C-terminus of the Fab heavy chain to the N-terminus of the first or second subunit of the Fc domain. In a particular embodiment, the second and the third antigen binding moiety of the T cell activating antigen binding molecule are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety. In another particular embodiment, the first and the third antigen binding moiety of the T cell activating antigen binding molecule are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety. The components of the T cell activating bispecific antigen binding molecule may be fused directly or through suitable peptide linkers. In one embodiment, wherein the second and the third antigen binding moiety of the T cell activating antigen binding molecule are each fused at the C-terminus of the Fab heavy chain to the N-terminus of one of the subunits of the Fc domain, the second and the third antigen binding moiety and the Fc domain are part of an immunoglobulin molecule. In a particular embodiment the immunoglobulin molecule is an IgG class immunoglobulin.

In a particular embodiment, the Fc domain is an IgG Fc domain. In a specific embodiment, the Fc domain is an IgG₁ Fc domain. In another specific embodiment, the Fc domain is an IgG₄ Fc domain. In particular embodiments the Fc domain is a human Fc domain.

In particular embodiments the Fc domain comprises a modification promoting the association of the first and the second Fc domain subunit. In a specific such embodiment, an amino acid residue in the CH3 domain of the first subunit of the Fc domain is replaced with an amino acid residue having a larger side chain volume, thereby generating a protuberance within the CH3 domain of the first subunit which is positionable in a cavity within the CH3 domain of the second subunit, and an amino acid residue in the CH3 domain of the second subunit of the Fc domain is replaced with an amino acid residue having a smaller side chain volume, thereby generating a cavity within the CH3 domain of the second subunit within which the protuberance within the CH3 domain of the first subunit is positionable.

In a particular embodiment the Fc domain exhibits reduced binding affinity to an Fc receptor and/or reduced effector function, as compared to a native IgG₁ Fc domain. In one embodiment, the Fc domain comprises one or more amino acid substitution that reduces binding to an Fc receptor and/or effector function. In one embodiment, the one or more amino acid substitution in the Fc domain that reduces binding to an Fc receptor and/or effector function is at one or more position selected from the group of L234, L235, and P329 (EU numbering). In particular embodiments, each subunit of the Fc domain comprises three amino acid substitutions that reduce binding to an Fc receptor and/or effector function wherein said amino acid substitutions are L234A, L235A and P329G.

In one embodiment the Fc receptor is an Fcγ receptor. In one embodiment, the effector function is antibody-dependent cell-mediated cytotoxicity (ADCC).

In a particular embodiment, the activating T cell antigen is CD3. In one embodiment, the target cell antigen is selected from the group consisting of: Melanoma-associated Chondroitin Sulfate Proteoglycan (MCSP), Epidermal Growth Factor Receptor (EGFR), Carcinoembryonic Antigen (CEA), Fibroblast Activation Protein (FAP), CD19, CD20 and CD33.

According to another aspect of the invention there is provided an isolated polynucleotide encoding a T cell activating bispecific antigen binding molecule of the invention.

In another aspect is provided a method of producing the T cell activating bispecific antigen binding molecule of the invention, comprising the steps of a) culturing a host cell comprising the isolated polynucleotide of the invention, or a vector comprising the isolated polynucleotide of the invention, under conditions suitable for the expression of the T cell activating bispecific antigen binding molecule and b) recovering the T cell activating bispecific antigen binding molecule.

The invention further provides a pharmaceutical composition comprising the T cell activating bispecific antigen binding molecule of the invention and a pharmaceutically acceptable carrier.

Also encompassed by the invention are methods of using the T cell activating bispecific antigen binding molecule and pharmaceutical composition of the invention. In one aspect the invention provides a T cell activating bispecific antigen binding molecule or a pharmaceutical composition of the invention for use as a medicament. In one aspect is provided a T cell activating bispecific antigen binding molecule or a pharmaceutical composition according to the invention for use in the treatment of a disease in an individual in need thereof. In a specific embodiment the disease is cancer.

The invention also provides a T cell activating bispecific antigen binding molecule or a pharmaceutical composition of the invention for use in a method for inducing lysis of a target cell in an individual, comprising contacting a target cell with a T cell activating bispecific antigen binding molecule of the invention in the presence of a T cell, wherein the target cell is a tumor cell.

### Brief Description of the Drawings

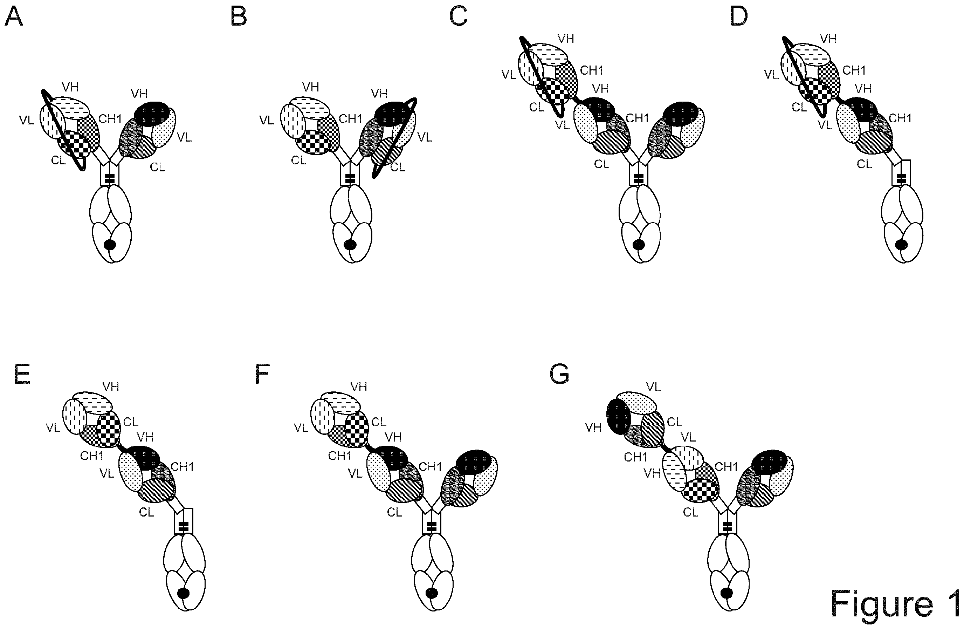

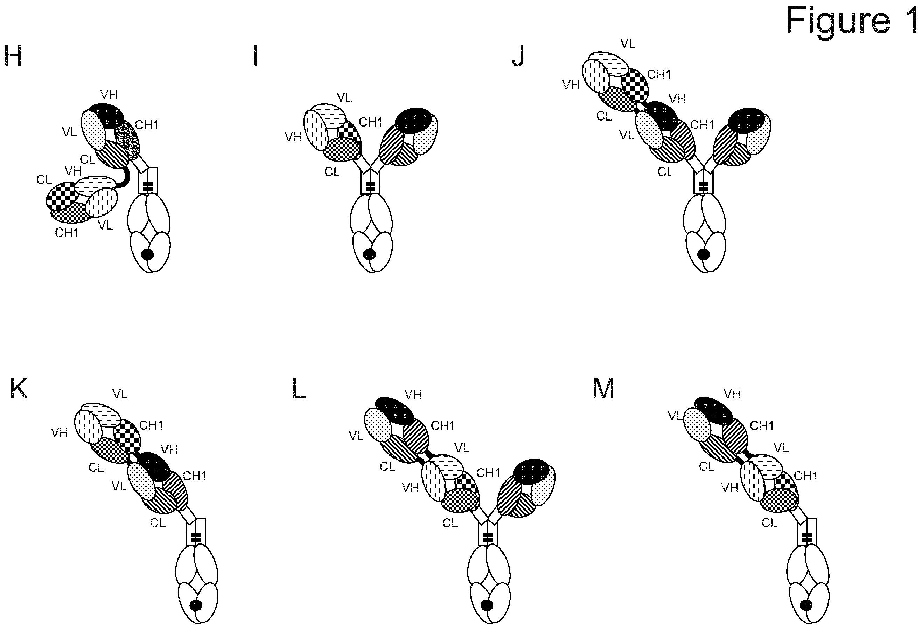

FIGURE 1. Exemplary configurations of the T cell activating bispecific antigen binding molecules disclosed herein. Illustration of (A) the "1+1 IgG scFab, one armed", and (B) the "1+1 IgG scFab, one armed inverted" molecule. In the "1+1 IgG scFab, one armed" molecule the light chain of the T cell targeting Fab is fused to the heavy chain by a linker, while the "1+1 IgG scFab, one armed inverted" molecule has the linker in the tumor targeting Fab. (C) Illustration of the "2+1 IgG scFab" molecule. (D) Illustration of the "1+1 IgG scFab" molecule. (E) Illustration of the "1+1 IgG Crossfab" molecule. (F) Illustration of the "2+1 IgG Crossfab" molecule. (G) Illustration of the "2+1 IgG Crossfab" molecule with alternative order of Crossfab and Fab components ("inverted"). (H) Illustration of the "1+1 IgG Crossfab light chain (LC) fusion" molecule. (I) Illustration of the "1+1 CrossMab" molecule. (J) Illustration of the "2+1 IgG Crossfab, linked light chain" molecule. (K) Illustration of the "1+1 IgG Crossfab, linked light chain" molecule. (L) Illustration of the "2+1 IgG Crossfab, inverted, linked light chain" molecule. (M) Illustration of the "1+1 IgG Crossfab, inverted, linked light chain" molecule. Black dot: optional modification in the Fc domain promoting heterodimerization.

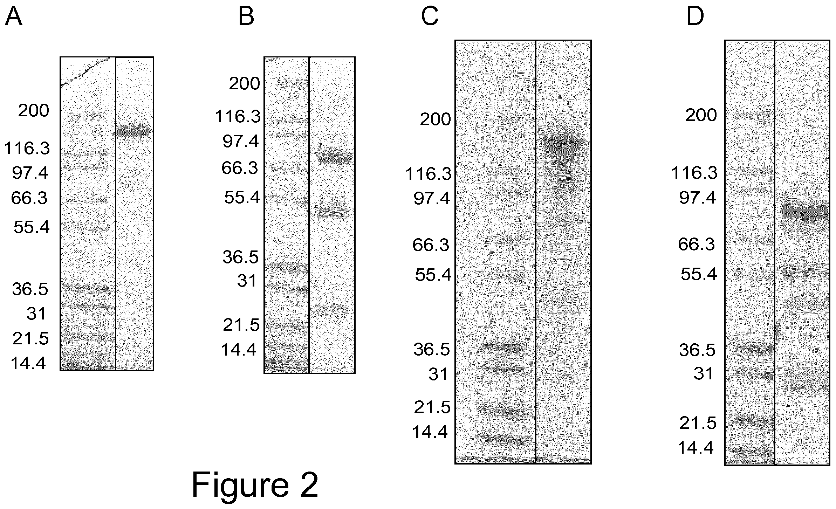

FIGURE 2. SDS PAGE (4-12% Bis/Tris, NuPage Invitrogen, Coomassie-stained) of "1+1 IgG scFab, one armed" (anti-MCSP/anti-huCD3) (see SEQ ID NOs 1, 3, 5), non reduced (A) and reduced (B), and of "1+1 IgG scFab, one armed inverted" (anti-MCSP/anti-huCD3) (see SEQ ID NOs 7, 9, 11), non reduced (C) and reduced (D).

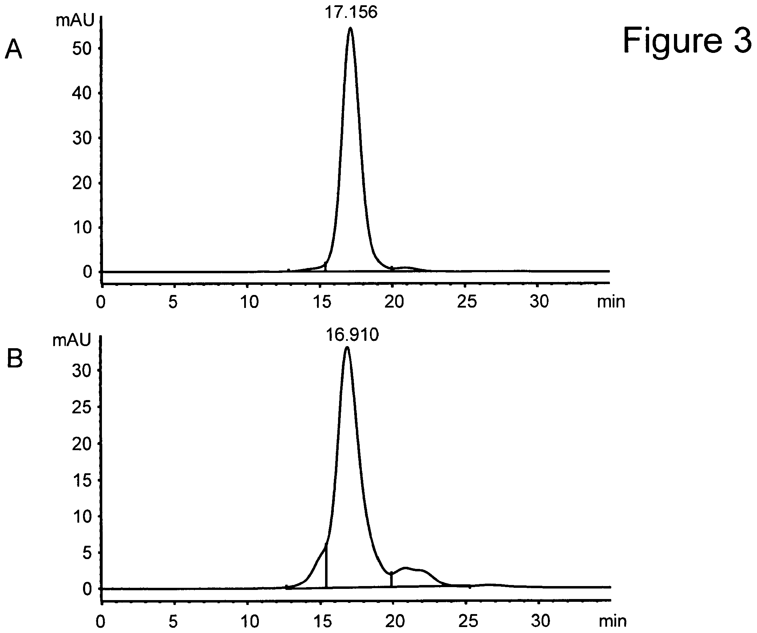

FIGURE 3. Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG scFab, one armed" (anti-MCSP/anti-huCD3) (see SEQ ID NOs 1, 3, 5) (A) and "1+1 IgG scFab, one armed inverted" (anti-MCSP/anti-huCD3) (see SEQ ID NOs 7, 9, 11) (B).

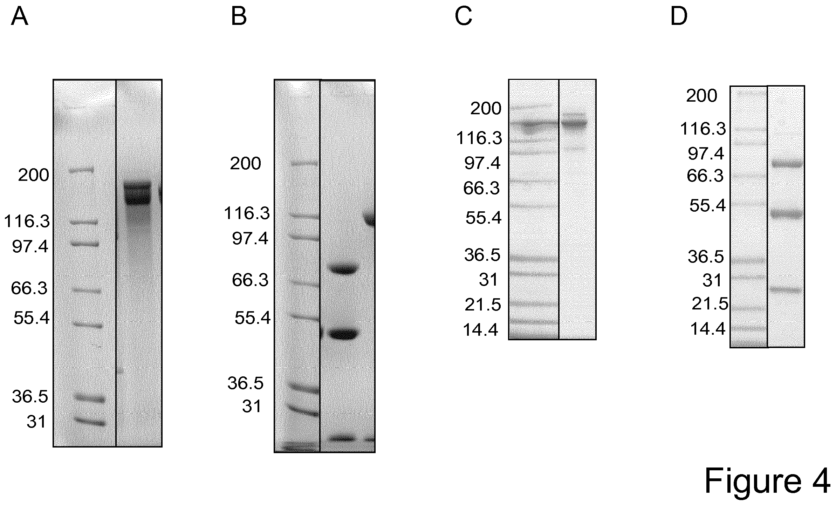

FIGURE 4. SDS PAGE (4-12% Bis/Tris, NuPage Invitrogen, Coomassie-stained) of "1+1 IgG scFab, one armed" (anti-EGFR/anti-huCD3) (see SEQ ID NOs 43, 45, 57), non reduced (A) and reduced (B), and of "1+1 IgG scFab, one armed inverted" (anti-EGFR/anti-huCD3) (see SEQ ID NOs 11, 49, 51), non reduced (C) and reduced (D).

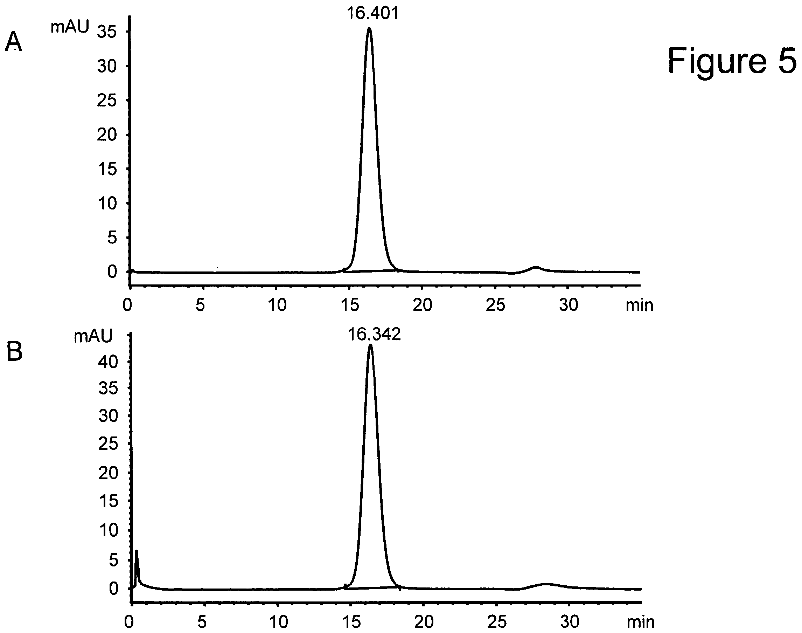

FIGURE 5. Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG scFab, one armed" (anti-EGFR/anti-huCD3) (see SEQ ID NOs 43, 45, 47) (A) and "1+1 IgG scFab, one armed inverted" (anti-EGFR/anti-huCD3) (see SEQ ID NOs 11, 49, 51) (B).

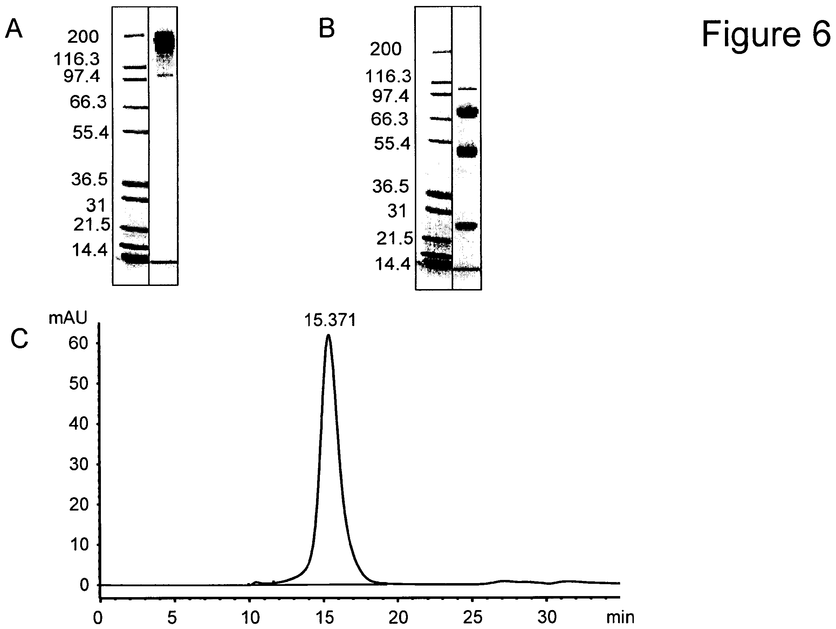

FIGURE 6. (A, B) SDS PAGE (4-12% Bis/Tris, NuPage Invitrogen, Coomassie-stained) of "1+1 IgG scFab, one armed inverted" (anti-FAP/anti-huCD3) (see SEQ ID NOs 11, 51, 55), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG scFab, one armed inverted" (anti-FAP/anti-huCD3).

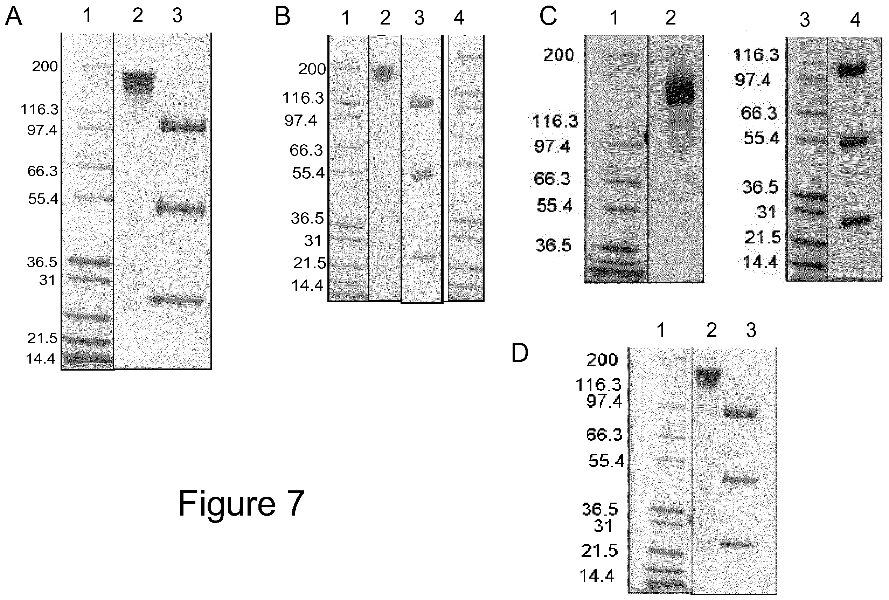

FIGURE 7. SDS PAGE (4-12% Bis/Tris, NuPage Invitrogen, Coomassie-stained) of (A) "2+1 IgG scFab, P329G LALA" (anti-MCSP/anti-huCD3) (see SEQ ID NOs 5, 21, 23), non reduced (lane 2) and reduced (lane 3); of (B) "2+1 IgG scFab, LALA" (anti-MCSP/anti-huCD3) (see SEQ ID NOs 5, 17, 19), non reduced (lane 2) and reduced (lane 3); of (C) "2+1 IgG scFab, wt" (anti-MCSP/anti-huCD3) (see SEQ ID NOs 5, 13, 15), non reduced (lane 2) and reduced (lane 3); and of (D) "2+1 IgG scFab, P329G LALA N297D" (anti-MCSP/anti-huCD3) (see SEQ ID NOs 5,25, 27), non reduced (lane 2) and reduced (lane 3).

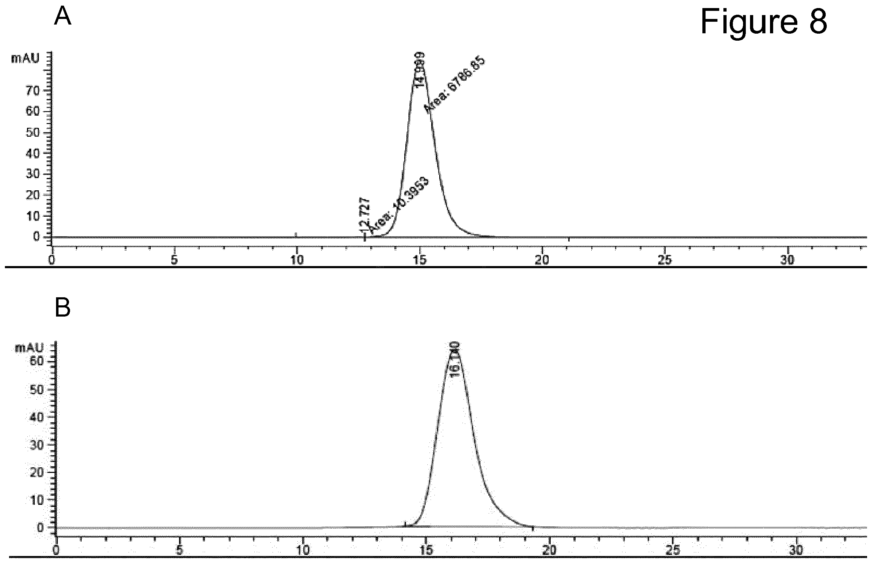

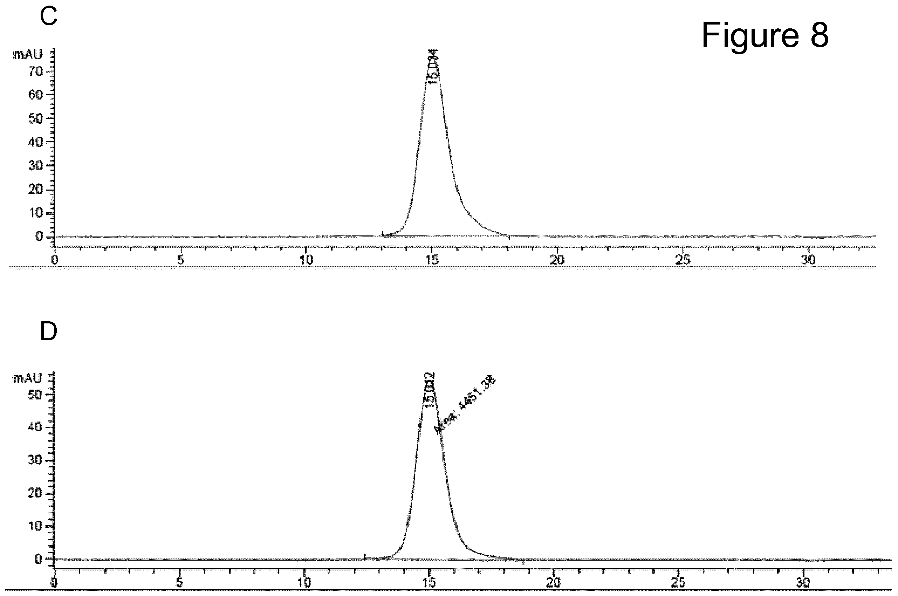

FIGURE 8. Analytical size exclusion chromatography (Superdex 200 10/300 GL GE Healthcare; 2 mM MOPS pH 7.3, 150 mM NaCl, 0.02% (w/v) NaCl; 50 µg sample injected) of (A) "2+1 IgG scFab, P329G LALA" (anti-MCSP/anti-huCD3) (see SEQ ID NOs 5, 21, 23); of (B) "2+1 IgG scFab, LALA" (anti-MCSP/anti-huCD3) (see SEQ ID NOs 5, 17, 19); of (C) "2+1 IgG scFab, wt" (anti-MCSP/anti-huCD3) (see SEQ ID NOs 5, 13, 15); and of (D) "2+1 IgG scFab, P329G LALA N297D" (anti-MCSP/anti-huCD3) (see SEQ ID NOs 5, 25, 27).

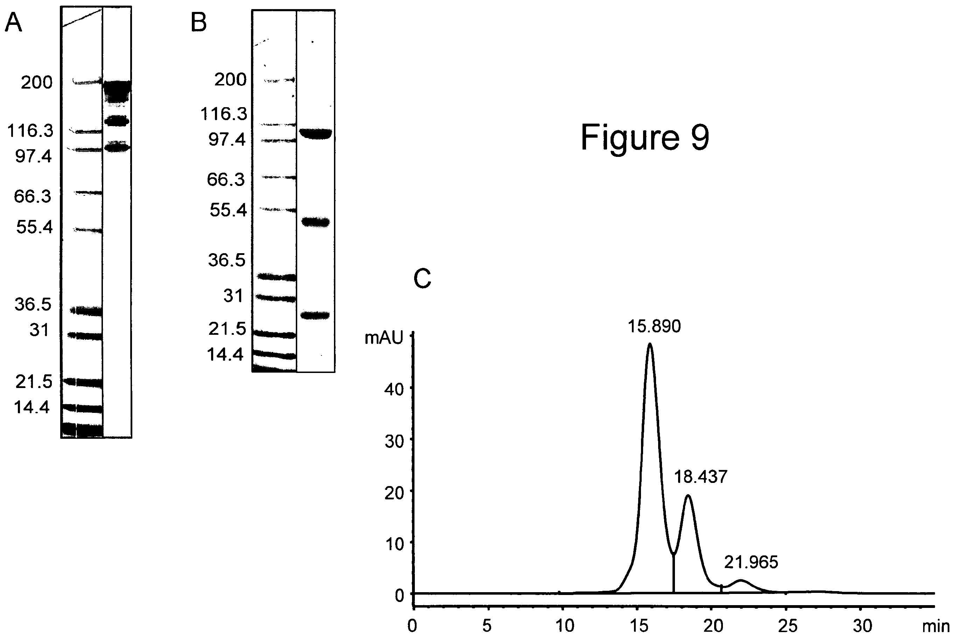

FIGURE 9. (A, B) SDS PAGE (4-12% Bis/Tris, NuPage Invitrogen, Coomassie-stained) of "2+1 IgG scFab, P329G LALA" (anti-EGFR/anti-huCD3) (see SEQ ID NOs 45, 47, 53), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG scFab, P329G LALA" (anti-EGFR/anti-huCD3).

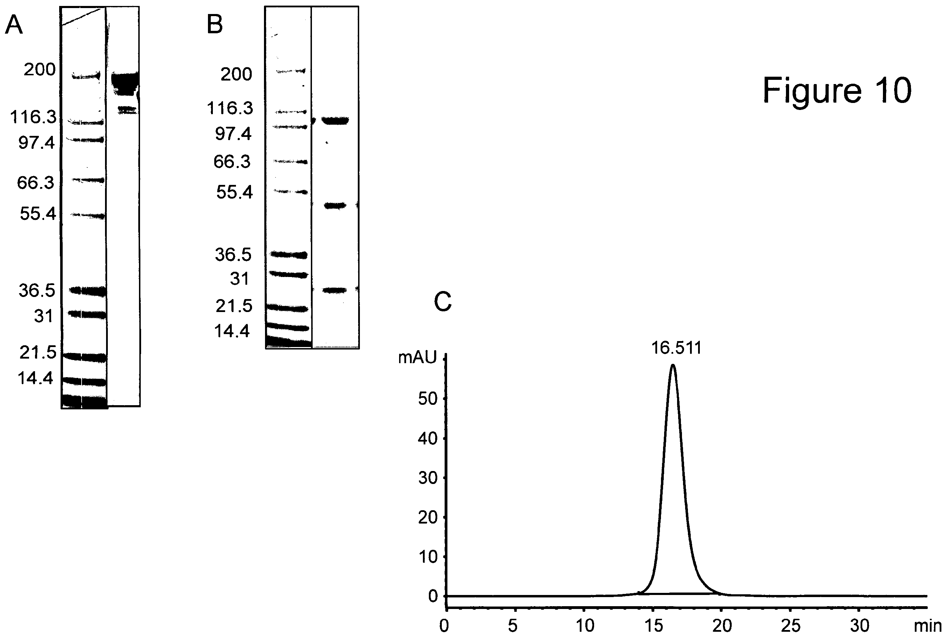

FIGURE 10. (A, B) SDS PAGE (4-12% Bis/Tris, NuPage Invitrogen, Coomassie-stained) of "2+1 IgG scFab, P329G LALA" (anti-FAP/anti-huCD3) (see SEQ ID NOs 57, 59, 61), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG scFab, P329G LALA" (anti-FAP/anti-huCD3).

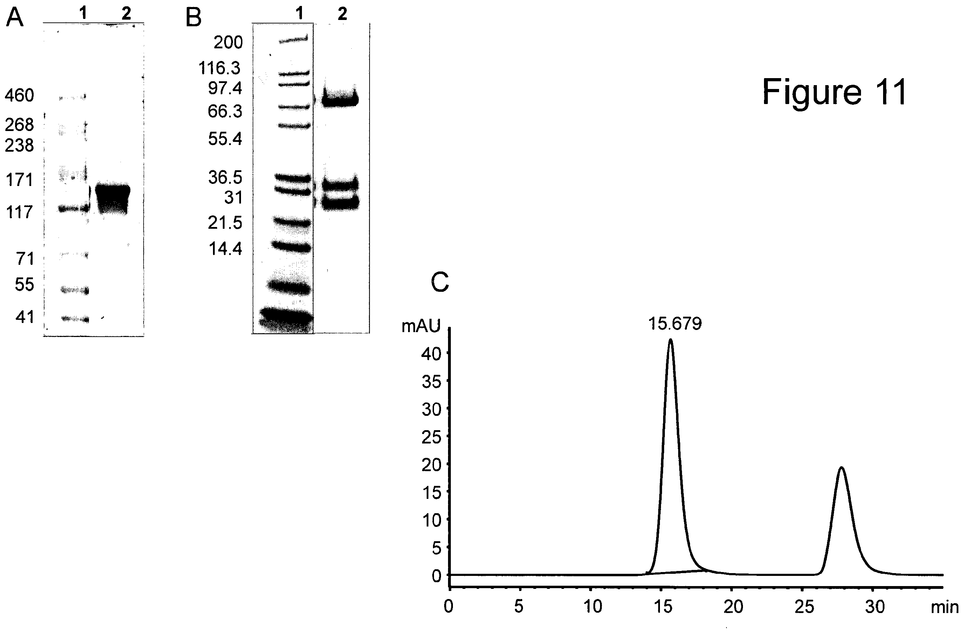

FIGURE 11. (A, B) SDS PAGE (4-12% Tris-Acetate (A) or 4-12% Bis/Tris (B), NuPage Invitrogen, Coomassie-stained) of "1+1 IgG Crossfab, Fc(hole) P329G LALA / Fc(knob) wt" (anti-MCSP/anti-huCD3) (see SEQ ID NOs 5, 29, 31, 33), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "1+1 IgG Crossfab, Fc(hole) P329G LALA / Fc(knob) wt" (anti-MCSP/anti-huCD3).

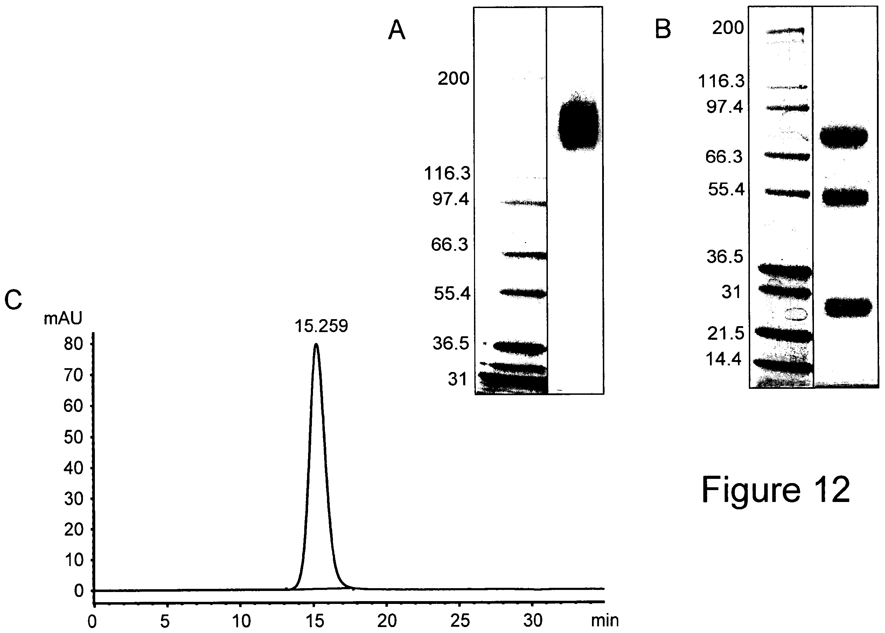

FIGURE 12. (A, B) SDS PAGE (4-12% Bis/Tris, NuPage Invitrogen, Coomassie-stained) of "2+1 IgG Crossfab" (anti-MCSP/anti-huCD3) (see SEQ ID NOs 3, 5, 29, 33), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG Crossfab" (anti-MCSP/anti-huCD3).

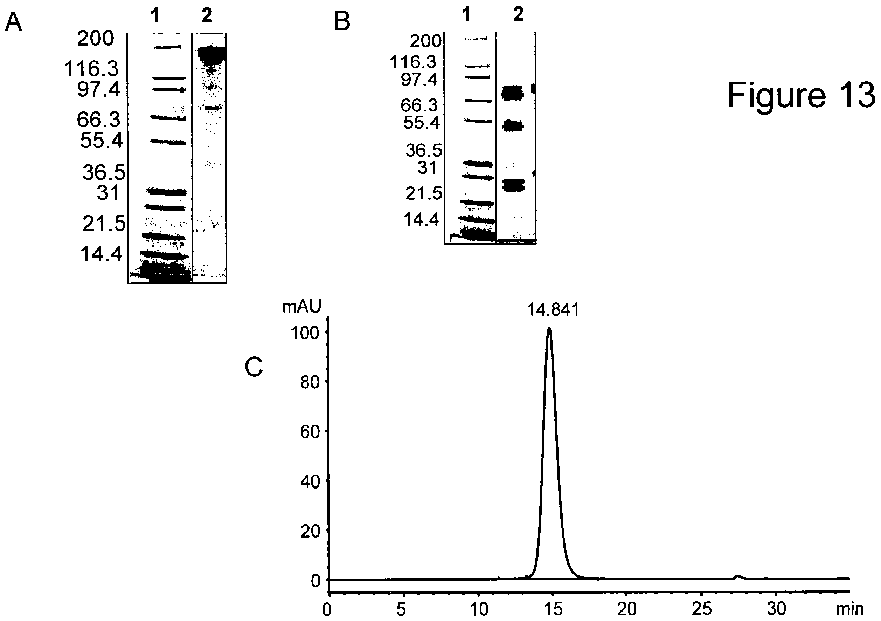

FIGURE 13. (A, B) SDS PAGE (4-12% Bis/Tris, NuPage Invitrogen, Coomassie-stained) of "2+1 IgG Crossfab" (anti-MCSP/anti-cyCD3) (see SEQ ID NOs 3, 5, 35, 37), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG Crossfab" (anti-MCSP/anti-cyCD3).

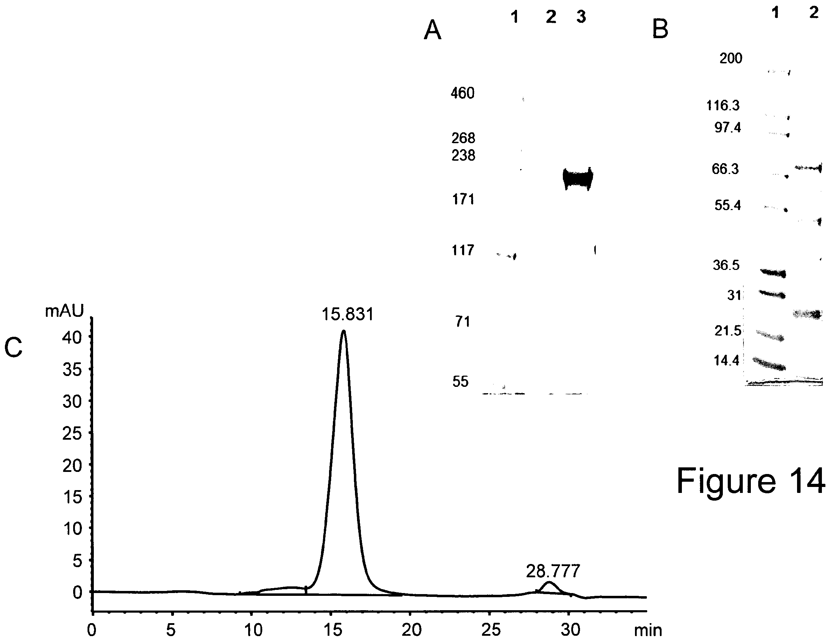

FIGURE 14. (A, B) SDS PAGE (4-12% Bis/Tris, NuPage Invitrogen, Coomassie-stained) of "2+1 IgG Crossfab, inverted" (anti-CEA/anti-huCD3) (see SEQ ID NOs 33, 63, 65, 67), non reduced (A) and reduced (B). (C) Analytical size exclusion chromatography (Superdex 200 10/300 GL GE Healthcare; 2 mM MOPS pH 7.3, 150 mM NaCl, 0.02% (w/v) NaCl; 50 µg sample injected) of "2+1 IgG Crossfab, inverted" (anti-CEA/anti-huCD3).

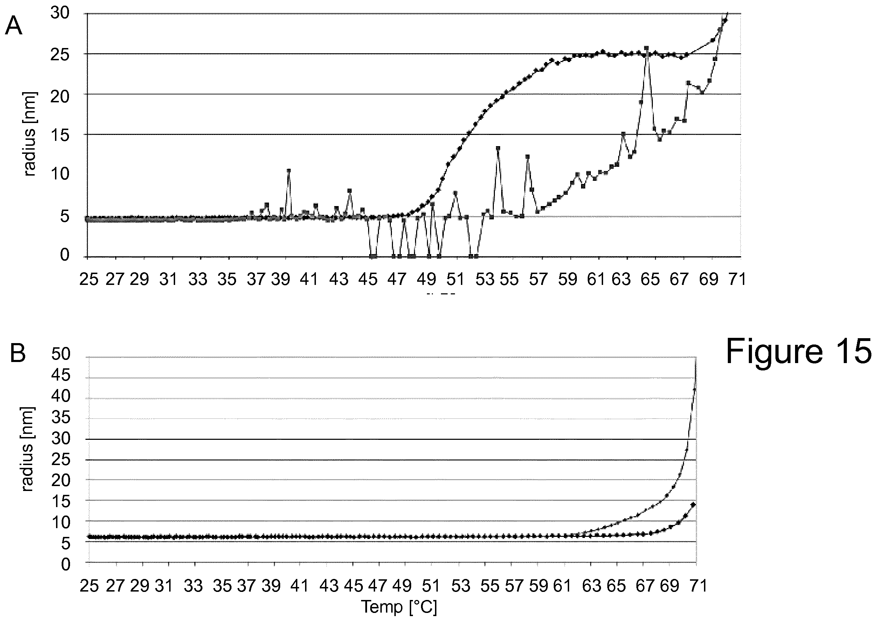

FIGURE 15. (A) Thermal stability of "(scFv)₂-Fc" and "(dsscFv)₂-Fc" (anti-MCSP (LC007)/anti-huCD3 (V9)). Dynamic Light Scattering, measured in a temperature ramp from 25-75°C at 0.05°C/min. Black curve: "(scFv)₂-Fc"; grey curve: "(dsscFv)₂-Fc". (B) Thermal stability of "2+1 IgG scFab" (see SEQ ID NOs 5, 21, 23) and "2+1 IgG Crossfab" (anti-MCSP/anti-huCD3) (see SEQ ID NOs 3, 5, 29, 33). Dynamic Light Scattering, measured in a temperature ramp from 25-75°C at 0.05°C/min. Black curve: "2+1 IgG scFab"; grey curve: "2+1 IgG Crossfab".

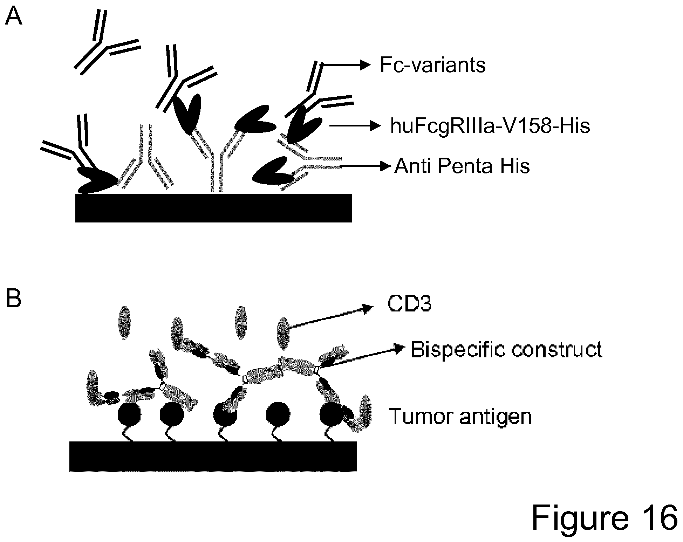

FIGURE 16. Biacore assay setup for (A) determination of interaction of various Fc-mutants with human FcyRIIIa, and for (B) simultaneous binding of T cell bespecific constructs with tumor target and human CD3γ(G₄S)₅CD3ε-AcTev-Fc(knob)-Avi/Fc(hole).
FIGURE 17. Simultaneous binding of T-cell bispecific constructs to the D3 domain of human MCSP and human CD3γ(G₄S)₅CD3ε-AcTev-Fc(knob)-Avi/Fc(hole). (A) "2+1 IgG Crossfab" (see SEQ ID NOs 3, 5, 29, 33), (B) "2+1 IgG scFab" (see SEQ ID NOs 5, 21, 23).

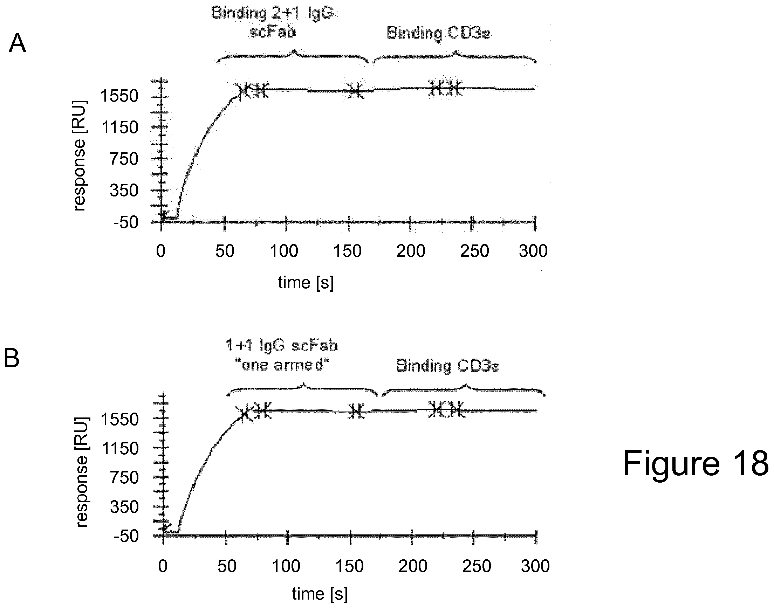

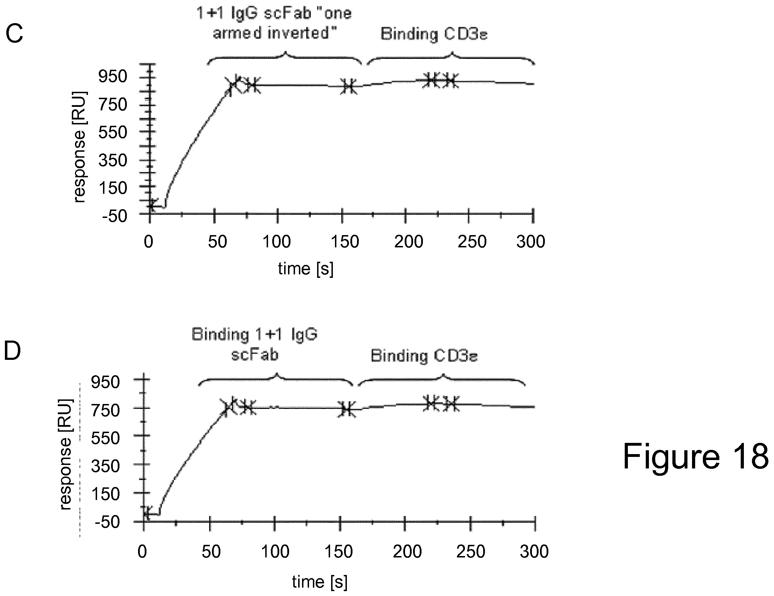

FIGURE 18. Simultaneous binding of T-cell bispecific constructs to human EGFR and human CD3γ(G₄S)₅CD3ε-AcTev-Fc(knob)-Avi/Fc(hole). (A) "2+1 IgG scFab" (see SEQ ID NOs 45, 47, 53), (B) "1+1 IgG scFab, one armed" (see SEQ ID NOs 43, 45, 47), (C) "1+1 IgG scFab, one armed inverted" (see SEQ ID NOs 11, 49, 51), and (D) "1+1 IgG scFab" (see SEQ ID NOs 47, 53, 213).

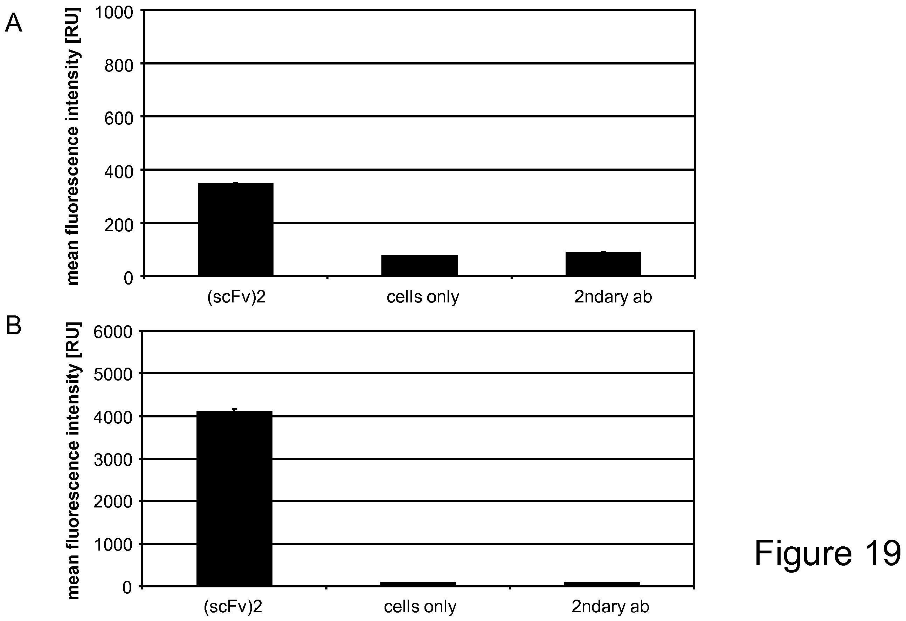

FIGURE 19. Binding of the "(scFv)₂" molecule (50 nM) to CD3 expressed on Jurkat cells (A), or to MCSP on Colo-38 cells (B) measured by FACS. Mean fluorescence intensity compared to untreated cells and cells stained with the secondary antibody only is depicted.

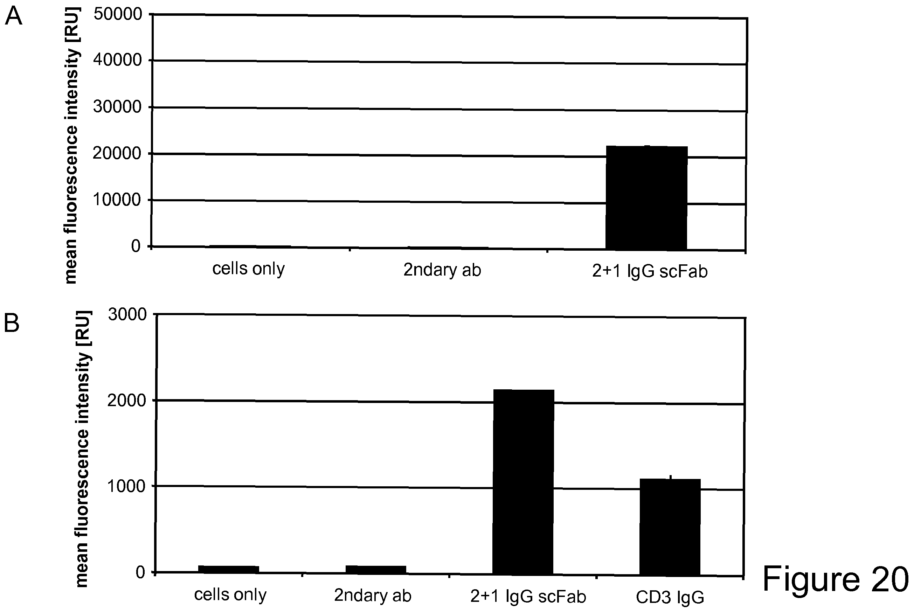

FIGURE 20. Binding of the "2+1 IgG scFab, LALA" (see SEQ ID NOs 5, 17, 19) construct (50 nM) to CD3 expressed on Jurkat cells (A), or to MCSP on Colo-38 cells (B) measured by FACS. Mean fluorescence intensity compared to cells treated with the reference anti-CD3 IgG (as indicated), untreated cells, and cells stained with the secondary antibody only is depicted.

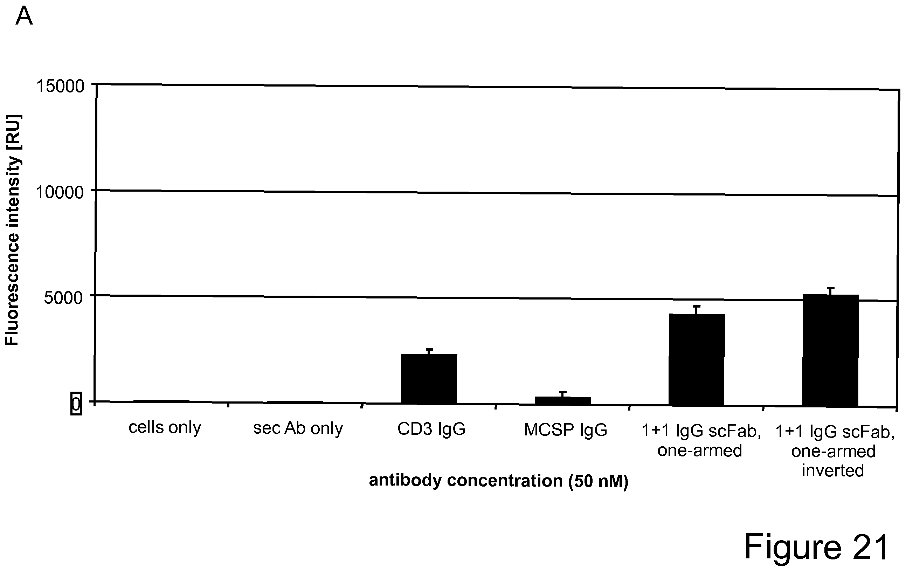

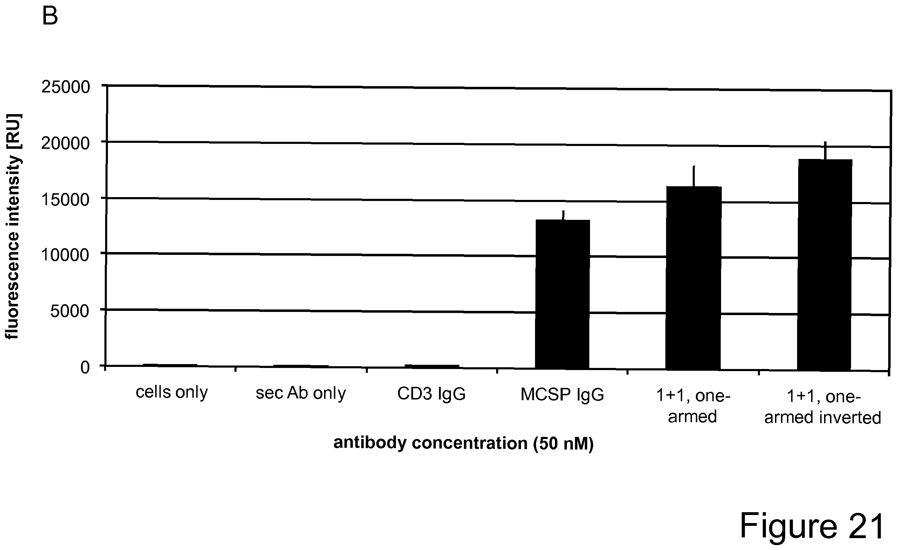

FIGURE 21. Binding of the "1+1 IgG scFab, one armed" (see SEQ ID NOs 1, 3, 5) and "1+1 IgG scFab, one armed inverted" (see SEQ ID NOs 7, 9, 11) constructs (50 nM) to CD3 expressed on Jurkat cells (A), or to MCSP on Colo-38 cells (B) measured by FACS. Mean fluorescence intensity compared to cells treated with the reference anti-CD3 or anti-MCSP IgG (as indicated), untreated cells, and cells stained with the secondary antibody only is depicted.

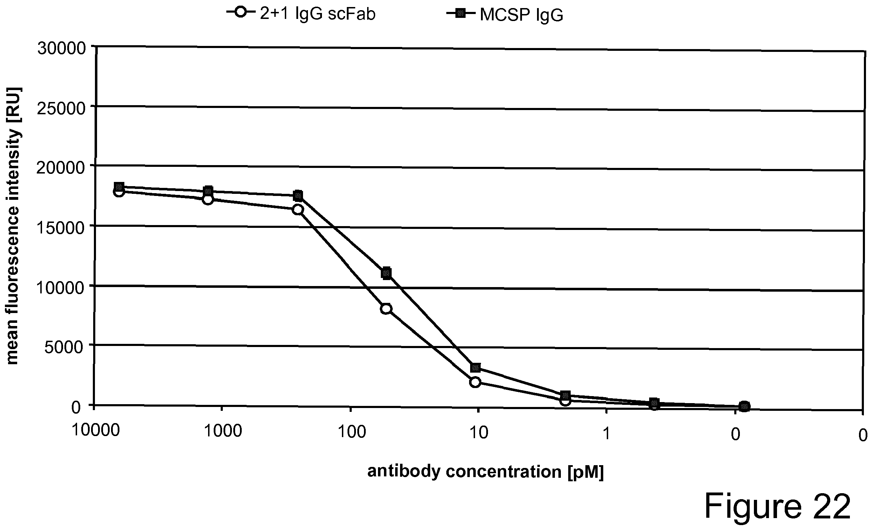

FIGURE 22. Dose dependent binding of the "2+1 IgG scFab, LALA" (see SEQ ID NOs 5, 17, 19) bispecific construct and the corresponding anti-MCSP IgG to MCSP on Colo-38 cells as measured by FACS.

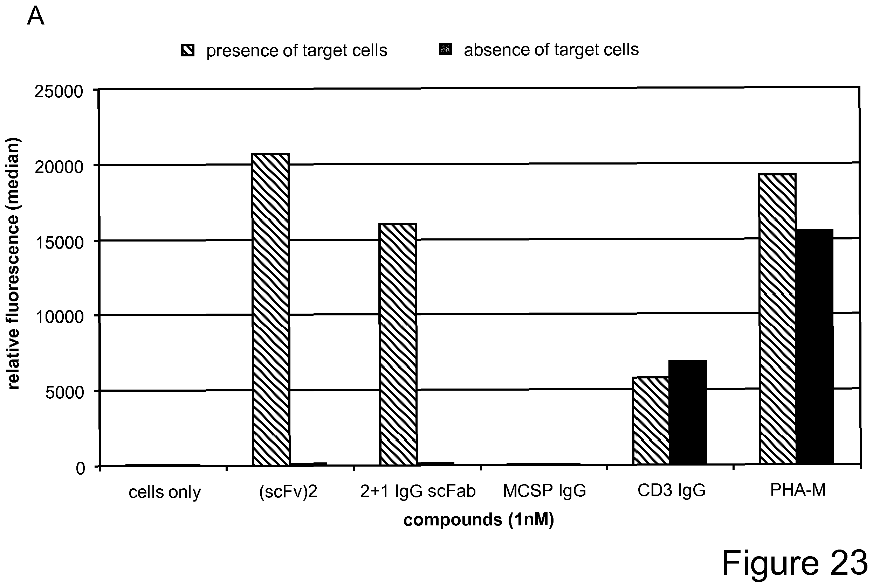

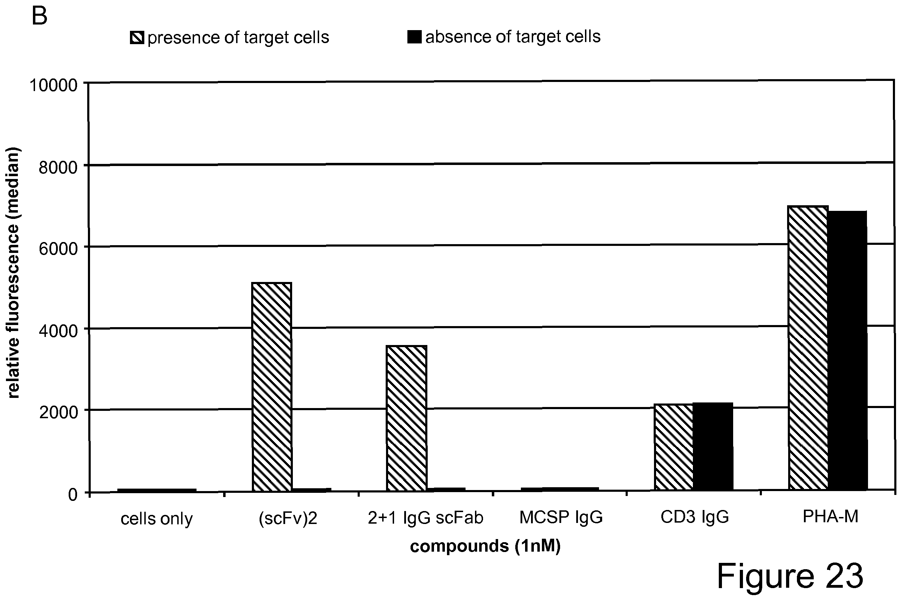

FIGURE 23. Surface expression level of different activation markers on human T cells after incubation with 1 nM of "2+1 IgG scFab, LALA" (see SEQ ID NOs 5, 17, 19) or "(scFv)₂" CD3-MCSP bispecific constructs in the presence or absence of Colo-38 tumor target cells, as indicated (E:T ratio of PBMCs to tumor cells = 10:1). Depicted is the expression level of the early activation marker CD69 (A), or the late activation marker CD25 (B) on CD8⁺ T cells after 15 or 24 hours incubation, respectively.

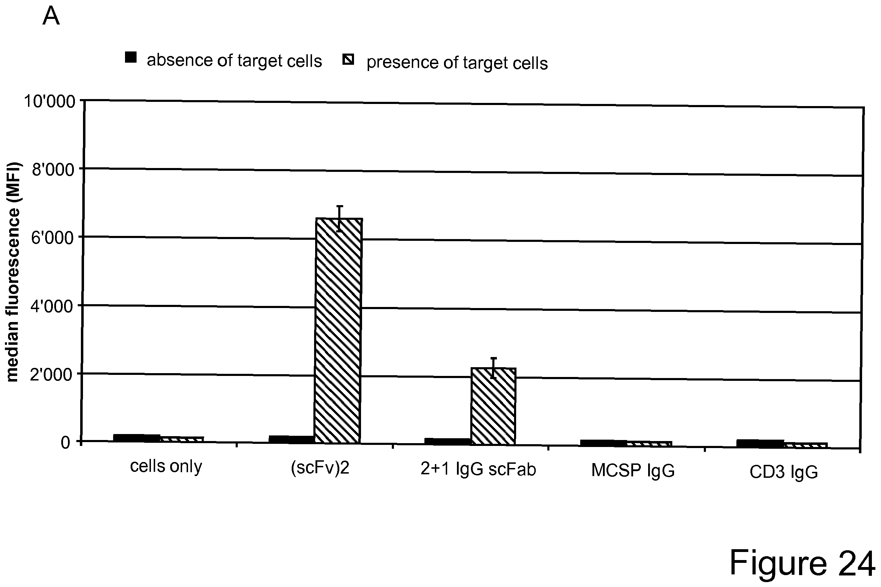

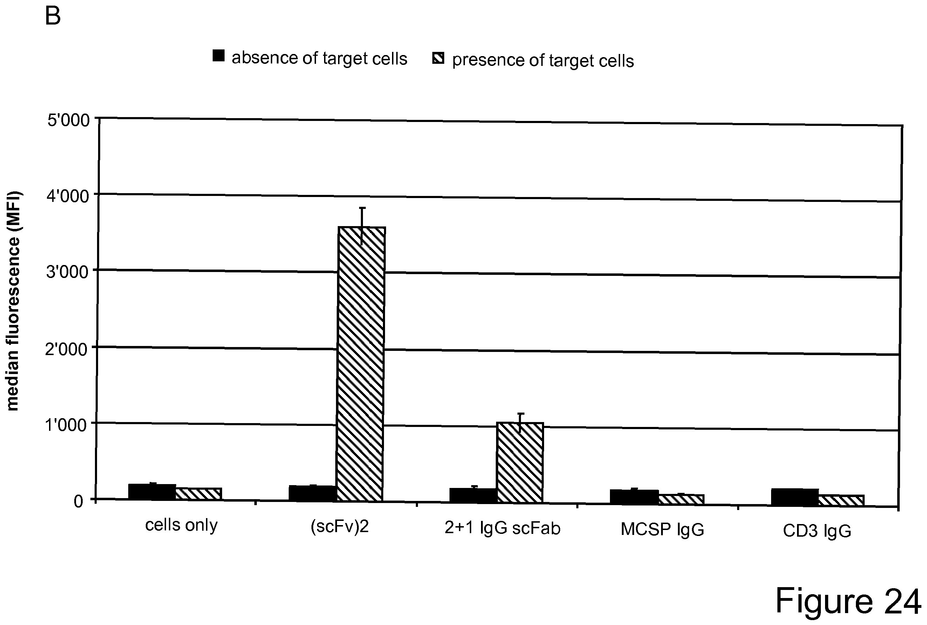

FIGURE 24. Surface expression level of the late activation marker CD25 on human T cells after incubation with 1 nM of "2+1 IgG scFab, LALA" (see SEQ ID NOs 5, 17, 19) or "(scFv)₂" CD3-MCSP bispecific constructs in the presence or absence of Colo-38 tumor target cells, as indicated (E:T ratio = 5:1). Depicted is the expression level of the late activation marker CD25 on CD8⁺ T cells (A) or on CD4⁺ T cells (B) after 5 days incubation.

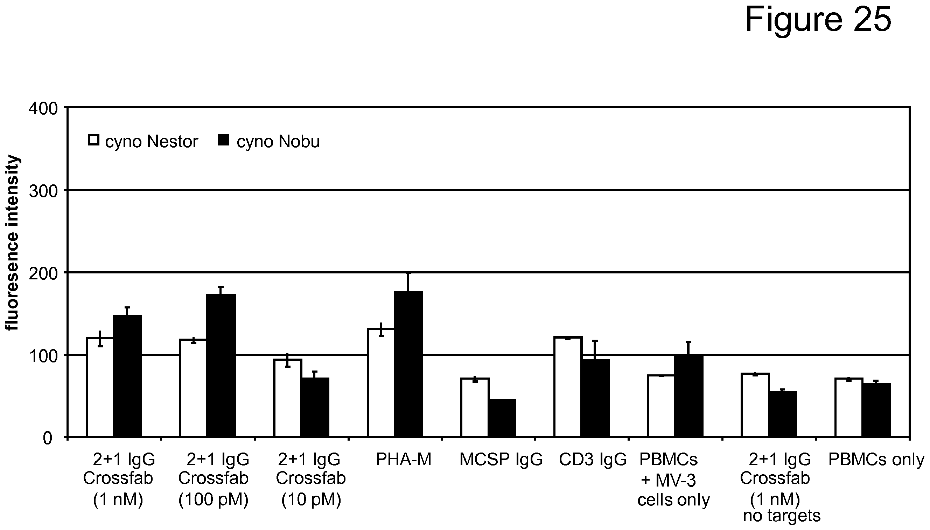

FIGURE 25. Surface expression level of the late activation marker CD25 on cynomolgus CD8⁺ T cells from two different animals (cyno Nestor, cyno Nobu) after 43 hours incubation with the indicated concentrations of the "2+1 IgG Crossfab" bispecific construct (targeting cynomolgus CD3 and human MCSP; see SEQ ID NOs 3, 5, 35, 37), in the presence or absence of human MCSP-expressing MV-3 tumor target cells (E:T ratio = 3:1). As controls, the reference IgGs (anti-cynomolgus CD3 IgG, anti-human MCSP IgG) or the unphysiologic stimulus PHA-M were used.

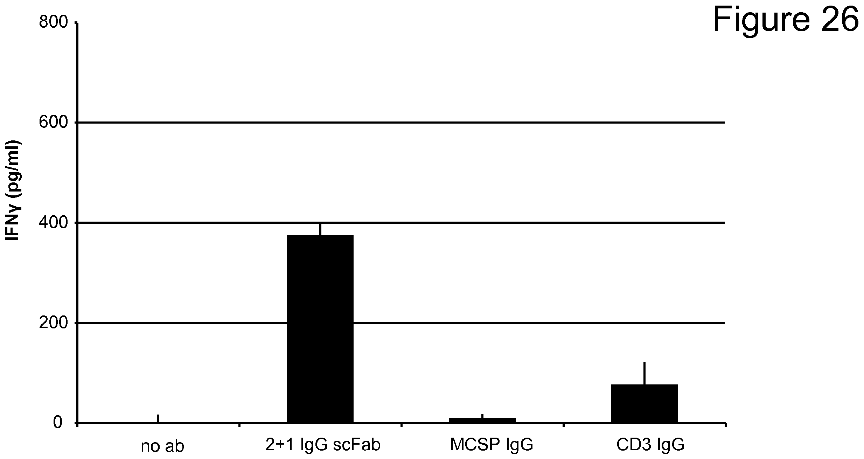

FIGURE 26. IFN-y levels, secreted by human pan T cells that were activated for 18.5 hours by the "2+1 IgG scFab, LALA" CD3-MCSP bispecific construct (see SEQ ID NOs 5, 17, 19) in the presence of U87MG tumor cells (E:T ratio = 5:1). As controls, the corresponding anti-CD3 and anti-MCSP IgGs were administered.

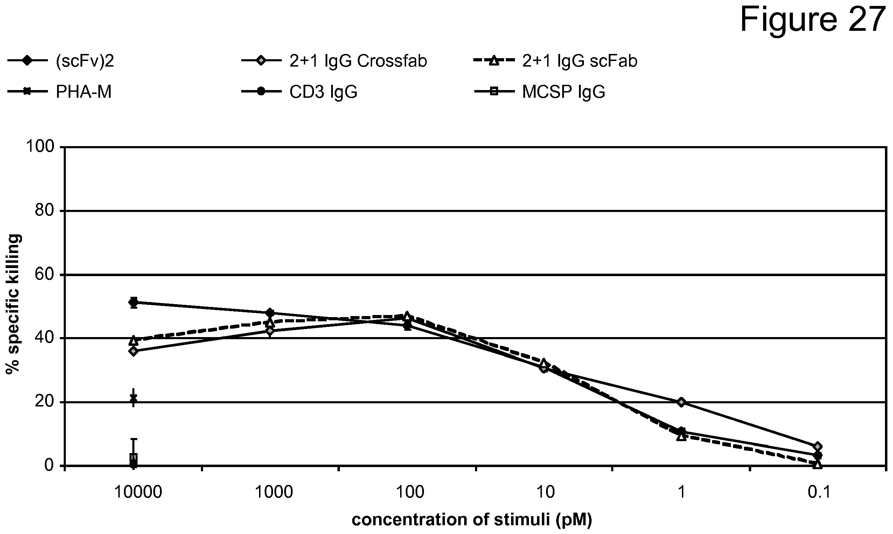

FIGURE 27. Killing (as measured by LDH release) of MDA-MB-435 tumor cells upon co-culture with human pan T cells (E:T ratio = 5:1) and activation for 20 hours by different concentrations of the "2+1 IgG scFab" (see SEQ ID NOs 5, 21, 23), "2+1 IgG Crossfab" (see SEQ ID NOs 3, 5, 29, 33) and "(scFv)₂" bispecific molecules and corresponding IgGs.

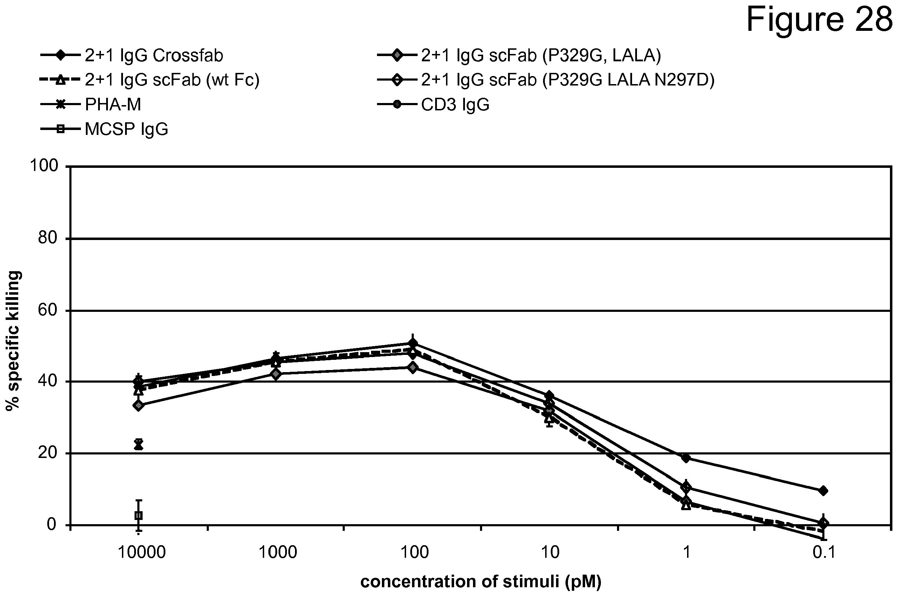

FIGURE 28. Killing (as measured by LDH release) of MDA-MB-435 tumor cells upon co-culture with human pan T cells (E:T ratio = 5:1), and activation for 20 hours by different concentrations of the bispecific constructs and corresponding IgGs. "2+1 IgG scFab" constructs differing in their Fc-domain (having either a wild-type Fc domain (see SEQ ID NOs 5, 13, 15), or a Fc-domain mutated to abolish (NK) effector cell function: P329G LALA (see SEQ ID NOs 5, 21, 23), P329G LALA N297D (see SEQ ID NOs 5, 25, 27)) and the "2+1 IgG Crossfab" (see SEQ ID NOs 3, 5, 29, 33) construct were compared.

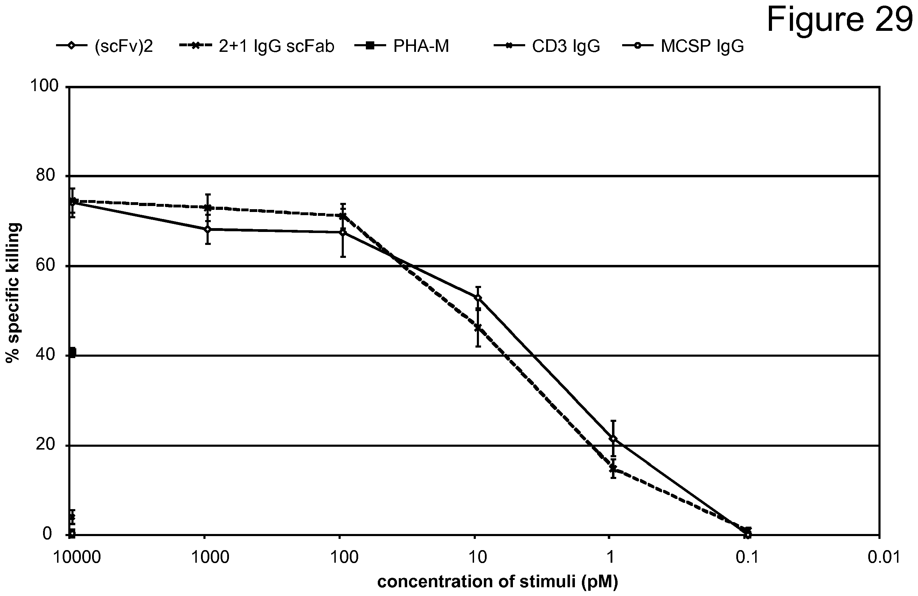

FIGURE 29. Killing (as measured by LDH release) of Colo-38 tumor cells upon co-culture with human pan T cells (E:T ratio = 5:1), treated with CD3-MCSP bispecific "2+1 IgG scFab, LALA" (see SEQ ID NOs 5, 17, 19) construct, "(scFv)₂" molecule or corresponding IgGs for 18.5 hours.

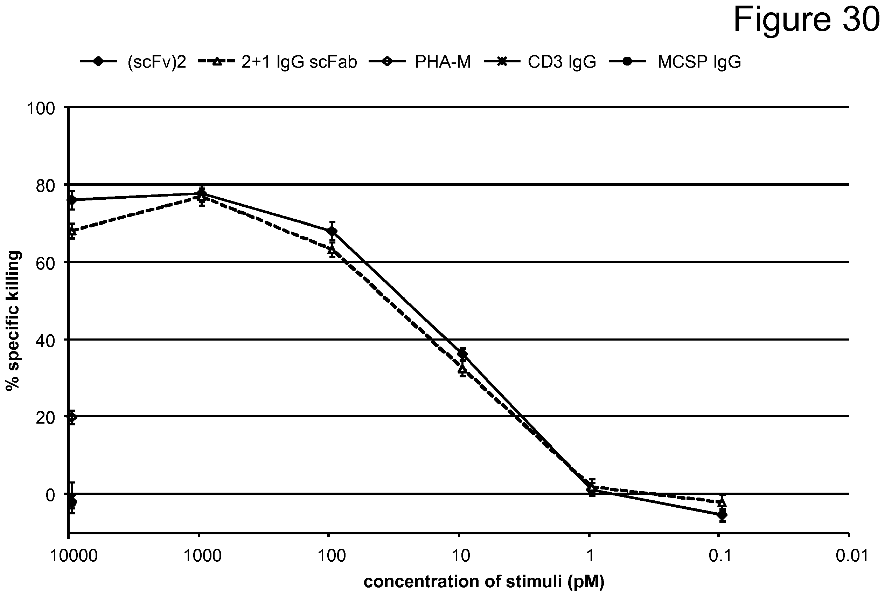

FIGURE 30. Killing (as measured by LDH release) of Colo-38 tumor cells upon co-culture with human pan T cells (E:T ratio = 5:1), treated with CD3-MCSP bispecific "2+1 IgG scFab, LALA" (see SEQ ID NOs 5, 17, 19) construct, the "(scFv)₂" molecule or corresponding IgGs for 18 hours.

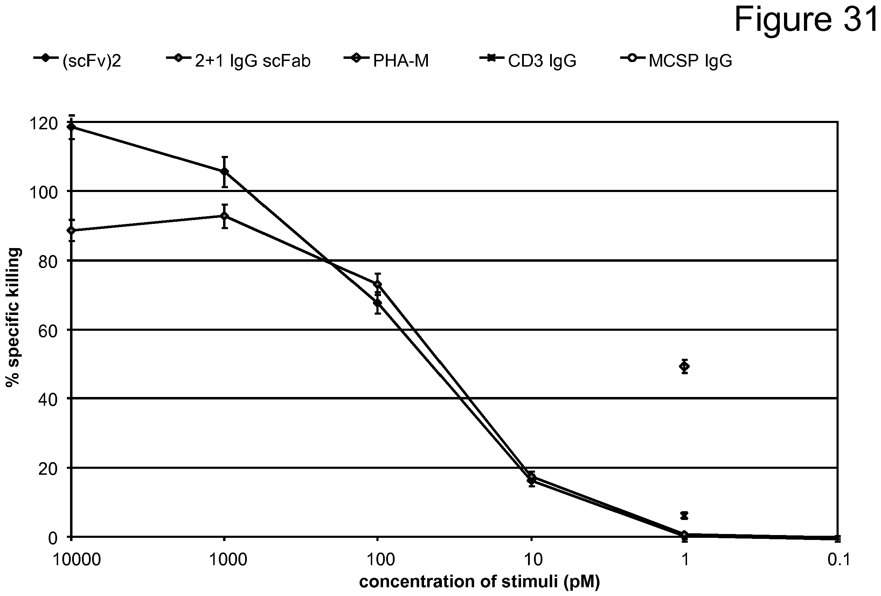

FIGURE 31. Killing (as measured by LDH release) of MDA-MB-435 tumor cells upon co-culture with human pan T cells (E:T ratio = 5:1), and activation for 23.5 hours by different concentrations of the CD3-MCSP bispecific "2+1 IgG scFab, LALA" (see SEQ ID NOs 5, 17, 19) construct, "(scFv)₂" molecule or corresponding IgGs.

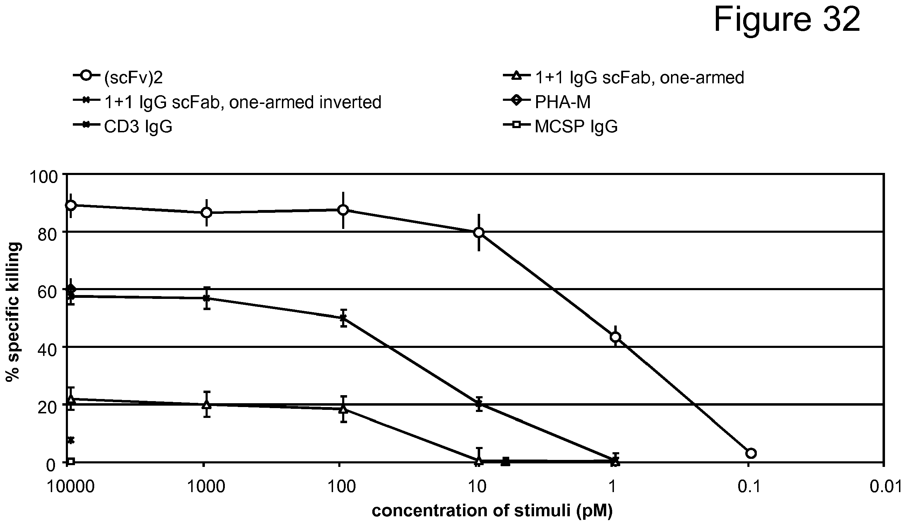

FIGURE 32. Killing (as measured by LDH release) of Colo-38 tumor cells upon co-culture with human pan T cells (E:T ratio = 5:1) and activation for 19 hours by different concentrations of the CD3-MCSP bispecific "1+1 IgG scFab, one armed" (see SEQ ID NOs 1, 3, 5), "1+1 IgG scFab, one armed inverted" (see SEQ ID NOs 7, 9, 11) or "(scFv)₂" constructs, or corresponding IgGs.

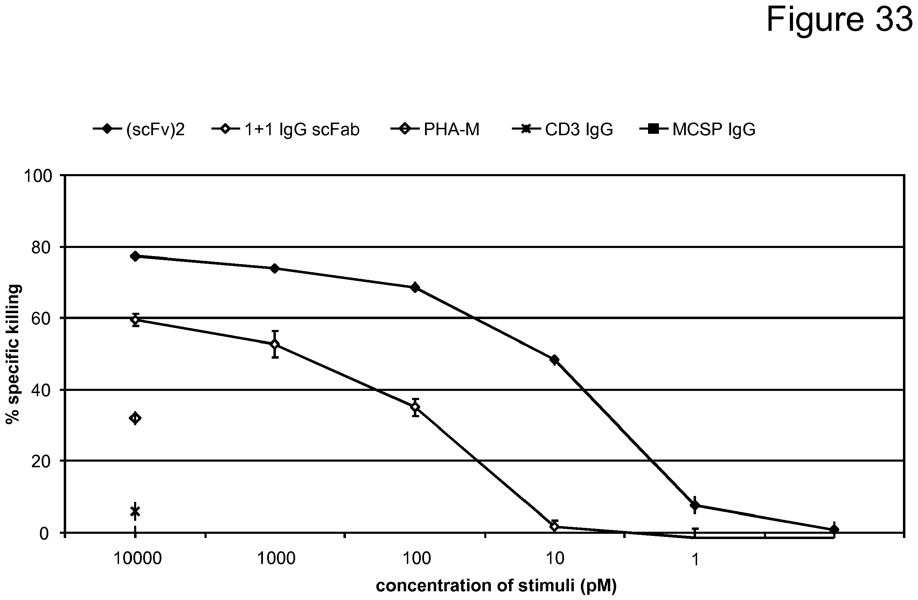

FIGURE 33. Killing (as measured by LDH release) of Colo-38 tumor cells upon co-culture with human pan T cells (E:T ratio = 5:1), treated with "1+1 IgG scFab" CD3-MCSP bispecific construct (see SEQ ID NOs 5, 21, 213) or "(scFv)₂" molecule for 20 hours.

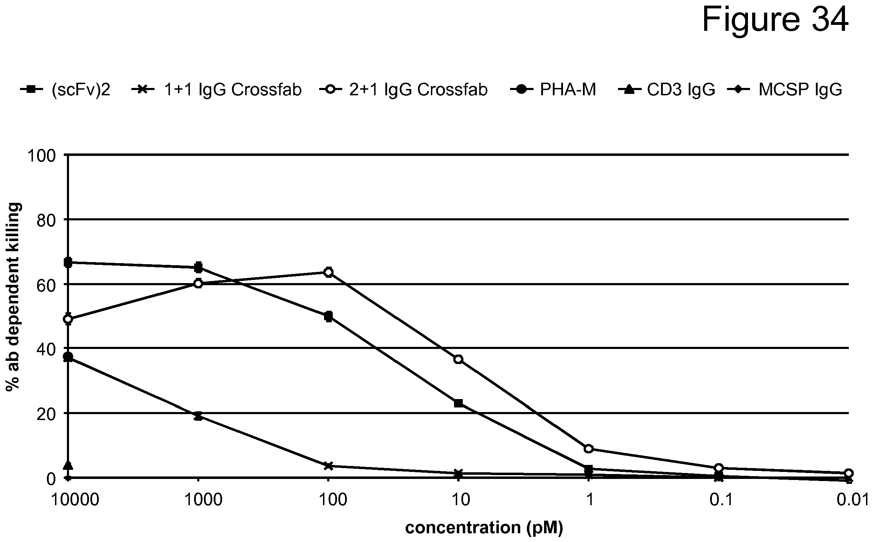

FIGURE 34. Killing (as measured by LDH release) of MDA-MB-435 tumor cells upon co-culture with human pan T cells (E:T ratio = 5:1), and activation for 21 hours by different concentrations of the bispecific constructs and corresponding IgGs. The CD3-MCSP bispecific "2+1 IgG Crossfab" (see SEQ ID NOs 3, 5, 29, 33) and "1+1 IgG Crossfab" (see SEQ ID NOs 5, 29, 31, 33) constructs, the "(scFv)₂" molecule and corresponding IgGs were compared.

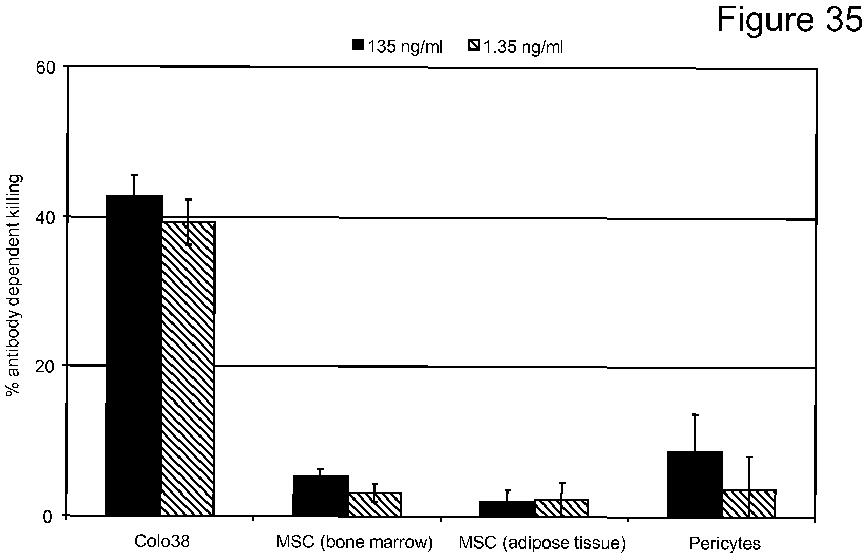

FIGURE 35. Killing (as measured by LDH release) of different target cells (MCSP-positive Colo-38 tumor target cells, mesenchymal stem cells derived from bone marrow or adipose tissue, or pericytes from placenta; as indicated) induced by the activation of human T cells by 135 ng/ml or 1.35 ng/ml of the "2+1 IgG Crossfab" CD3-MCSP bispecific construct (see SEQ ID NOs 3, 5, 29, 33) (E:T ratio = 25:1).

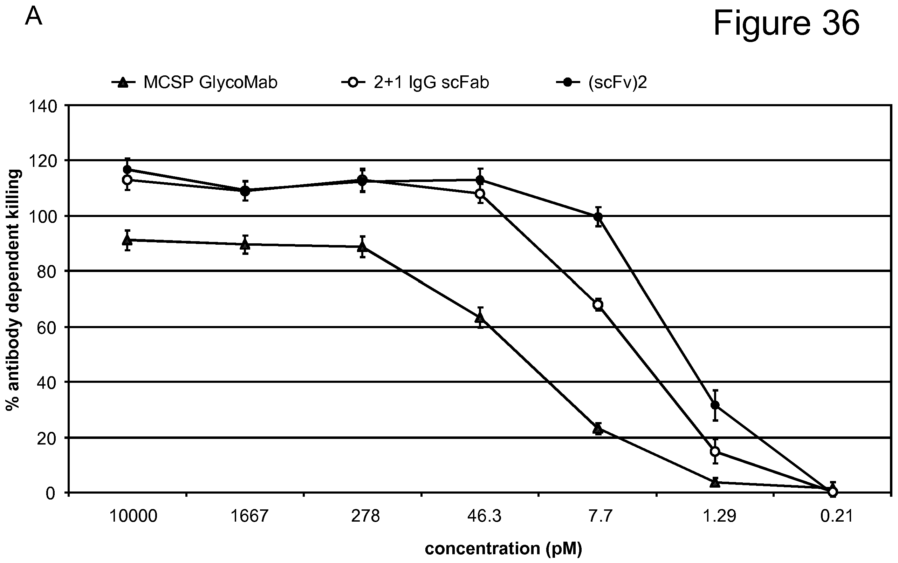

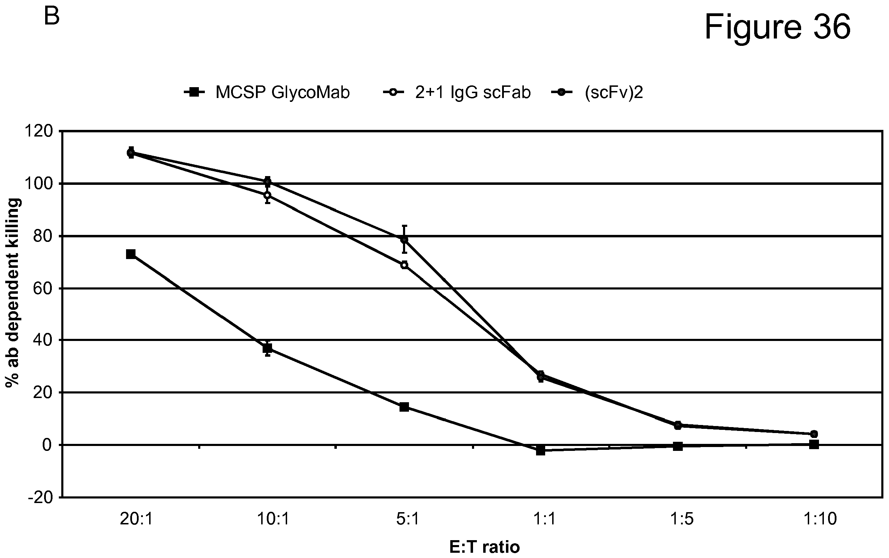

FIGURE 36. Killing (as measured by LDH release) of Colo-38 tumor target cells, measured after an overnight incubation of 21h, upon co-culture with human PBMCs and different CD3-MCSP bispecific constructs ("2+1 IgG scFab, LALA" (see SEQ ID NOs 5, 17, 19) and "(scFv)₂") or a glycoengineered anti-MCSP IgG (GlycoMab). The effector to target cell ratio was fixed at 25:1 (A), or varied as depicted (B). PBMCs were isolated from fresh blood (A) or from a Buffy Coat (B).

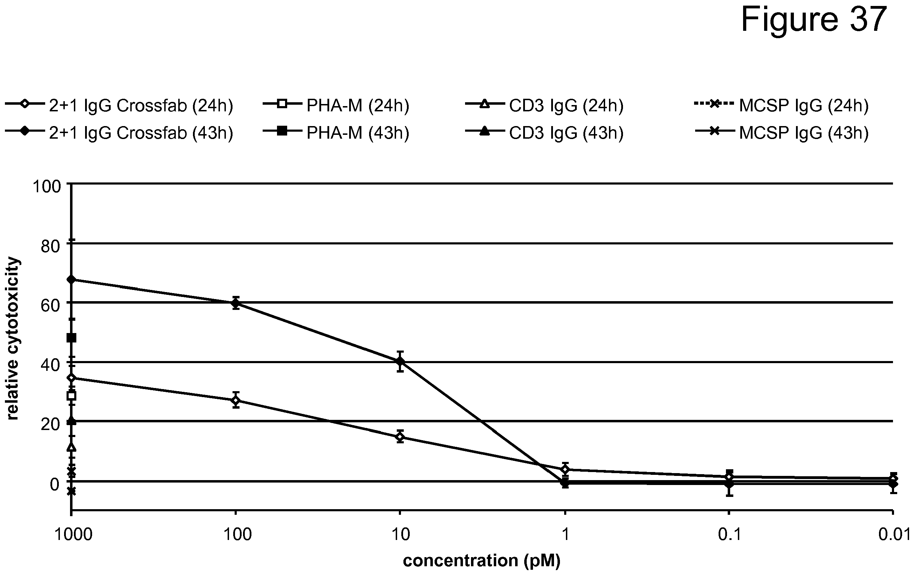

FIGURE 37. Time-dependent cytotoxic effect of the "2+1 IgG Crossfab" construct, targeting cynomolgus CD3 and human MCSP (see SEQ ID NOs 3, 5, 35, 37). Depicted is the LDH release from human MCSP-expressing MV-3 cells upon co-culture with primary cynomolgus PBMCs (E:T ratio = 3:1) for 24 h or 43 h. As controls, the reference IgGs (anti-cyno CD3 IgG and anti-human MCSP IgG) were used at the same molarity. PHA-M served as a control for (unphysiologic) T cell activation.

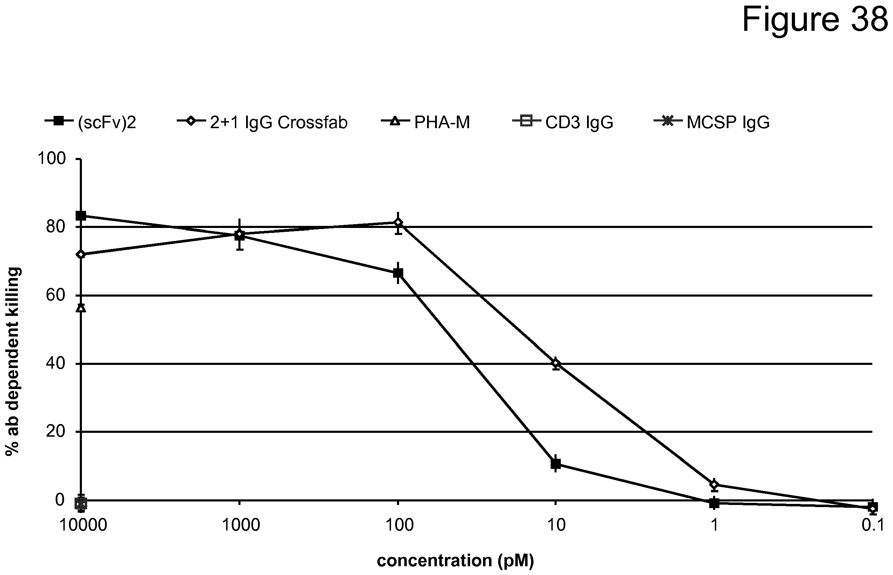

FIGURE 38. Killing (as measured by LDH release) of huMCSP-positive MV-3 melanoma cells upon co-culture with human PBMCs (E:T ratio = 10:1), treated with different CD3-MCSP bispecific constructs ("2+1 IgG Crossfab" (see SEQ ID NOs 3, 5, 29, 33) and "(scFv)₂") for -26 hours.

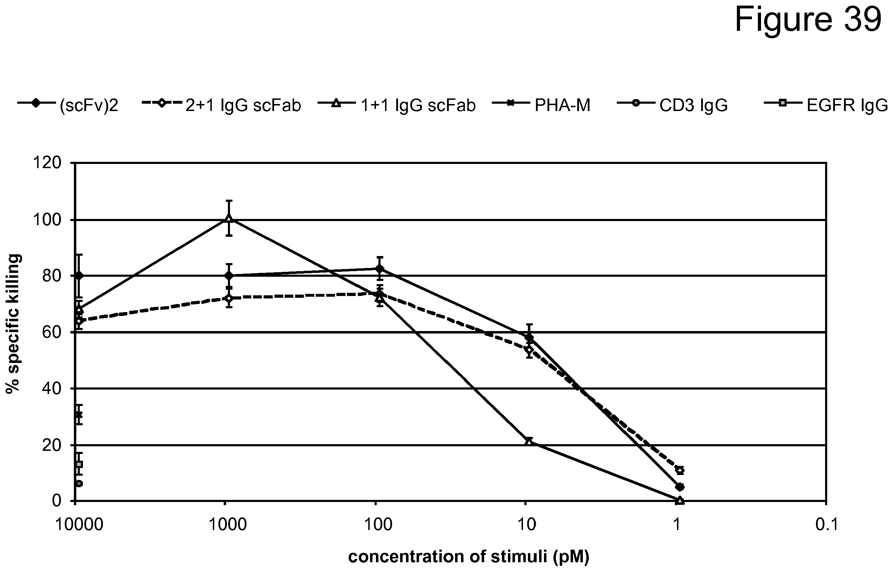

FIGURE 39. Killing (as measured by LDH release) of EGFR-positive LS-174T tumor cells upon co-culture with human pan T cells (E:T ratio = 5:1), treated with different CD3-EGFR bispecific constructs ("2+1 IgG scFab" (see SEQ ID NOs 45, 47, 53), "1+1 IgG scFab" (see SEQ ID NOs 47, 53, 213) and "(scFv)₂") or reference IgGs for 18 hours.

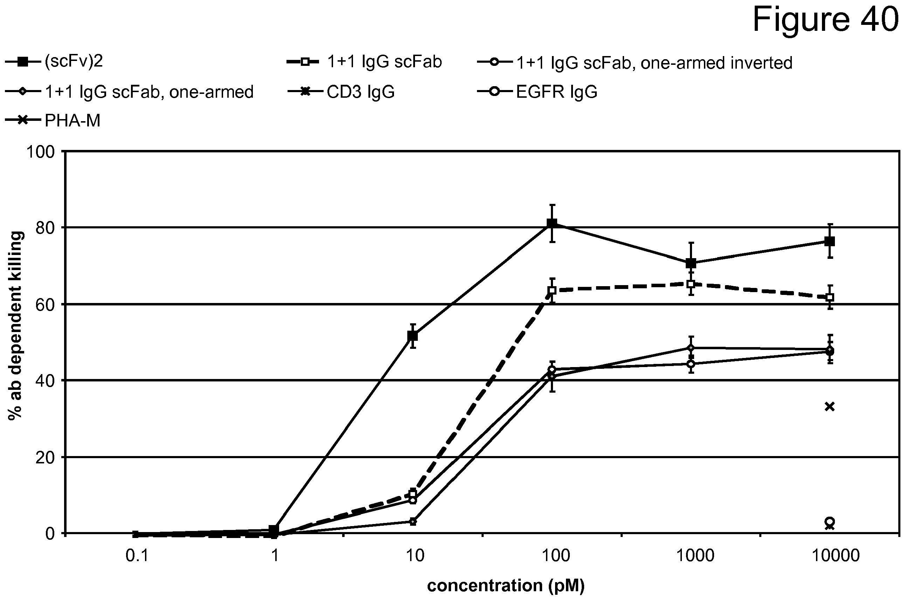

FIGURE 40. Killing (as measured by LDH release) of EGFR-positive LS-174T tumor cells upon co-culture with human pan T cells (E:T ratio = 5:1), treated with different CD3-EGFR bispecific constructs ("1+1 IgG scFab, one armed" (see SEQ ID NOs 43, 45, 47), "1+1 IgG scFab, one armed inverted" (see SEQ ID NOs 11, 49, 51), "1+1 IgG scFab" (see SEQ ID NOs 47, 53, 213) and "(scFv)₂") or reference IgGs for 21 hours.

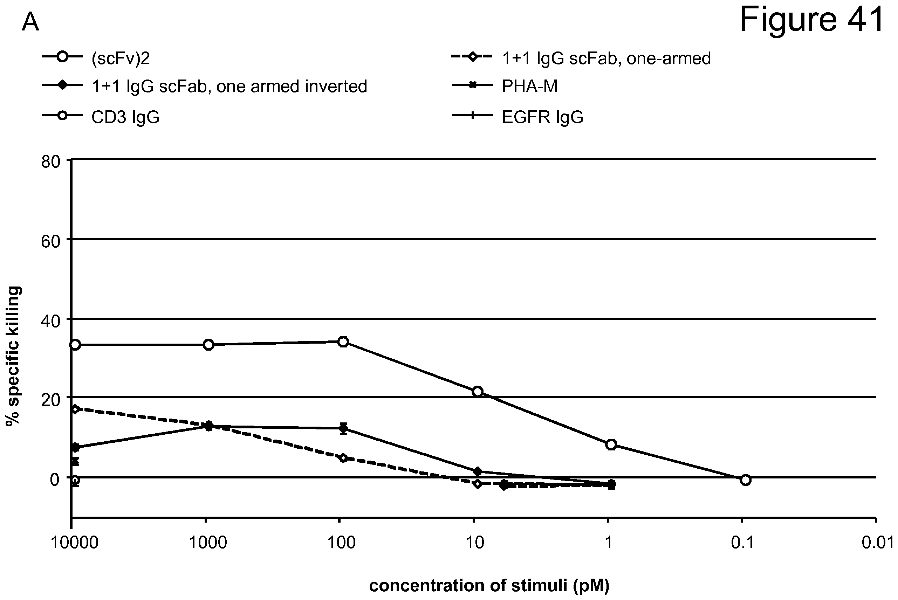

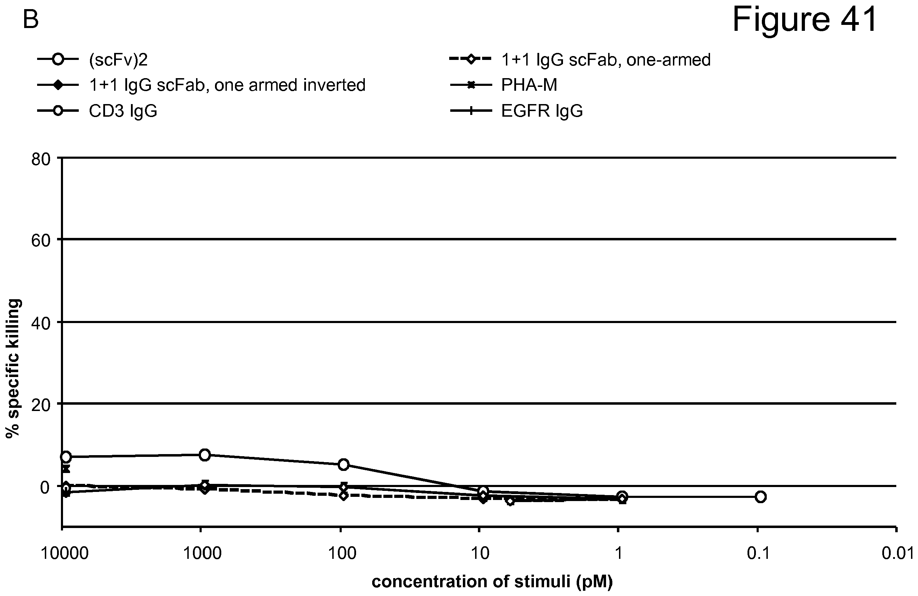

FIGURE 41. Killing (as measured by LDH release) of EGFR-positive LS-174T tumor cells upon co-culture with either human pan T cells (A) or human naive T cells (B), treated with different CD3-EGFR bispecific constructs ("1+1 IgG scFab, one armed" (see SEQ ID NOs 43, 45, 47), "1+1 IgG scFab, one armed inverted" (see SEQ ID NOs 11, 49, 51) and "(scFv)₂") or reference IgGs for 16 hours. The effector to target cell ratio was 5:1.

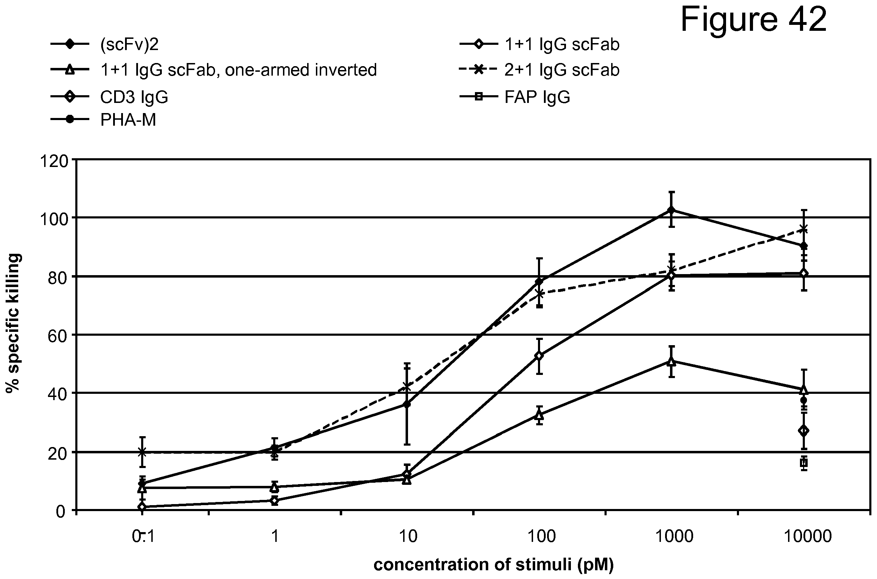

FIGURE 42. Killing (as measured by LDH release) of FAP-positive GM05389 fibroblasts upon co-culture with human pan T cells (E:T ratio = 5:1), treated with different CD3-FAP bispecific constructs ("1+1 IgG scFab, one armed inverted" (see SEQ ID NOs 11, 51, 55), "1+1 IgG scFab" (see SEQ ID NOs 57, 61, 213), "2+1 IgG scFab" (see SEQ ID NOs 57, 59, 61) and "(scFv)₂") for ∼18 hours.

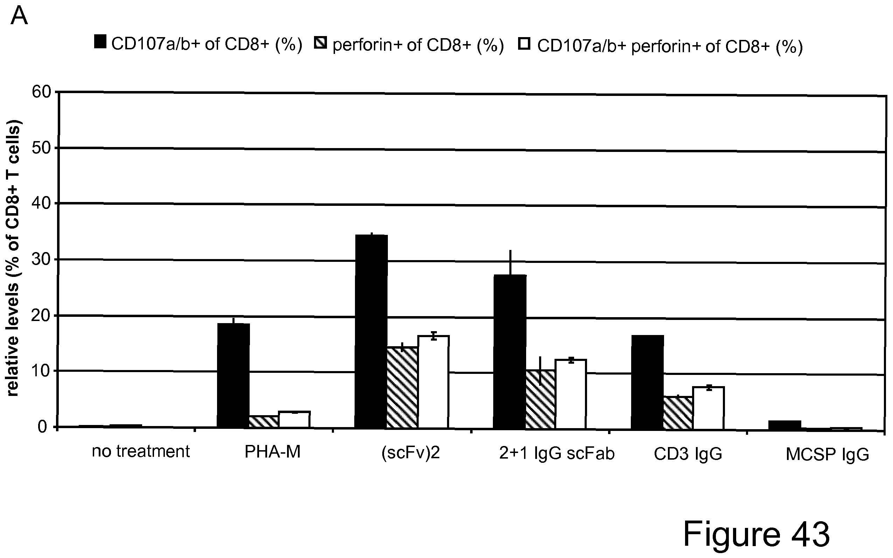

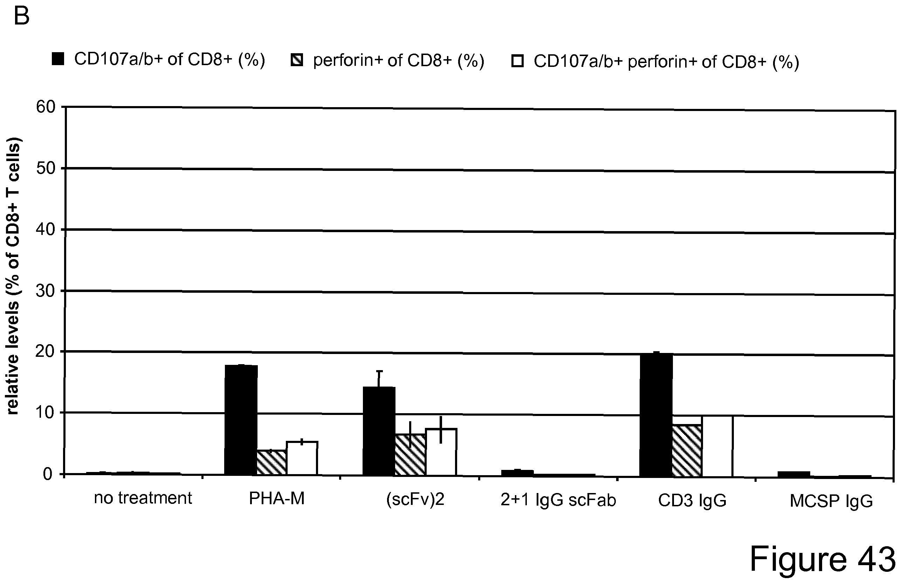

FIGURE 43. Flow cytrometric analysis of expression levels of CD107a/b, as well as perforin levels in CD8⁺ T cells that have been treated with different CD3-MCSP bispecific constructs ("2+1 IgG scFab, LALA" (see SEQ ID NOs 5, 17, 19) and "(scFv)₂") or corresponding control IgGs in the presence (A) or absence (B) of target cells for 6h. Human pan T cells were incubated with 9.43 nM of the different molecules in the presence or absence of Colo-38 tumor target cells at an effector to target ratio of 5:1. Monensin was added after the first hour of incubation to increase intracellular protein levels by preventing protein transport. Gates were set either on all CD107a/b positive, perforin-positive or double-positive cells, as depicted.

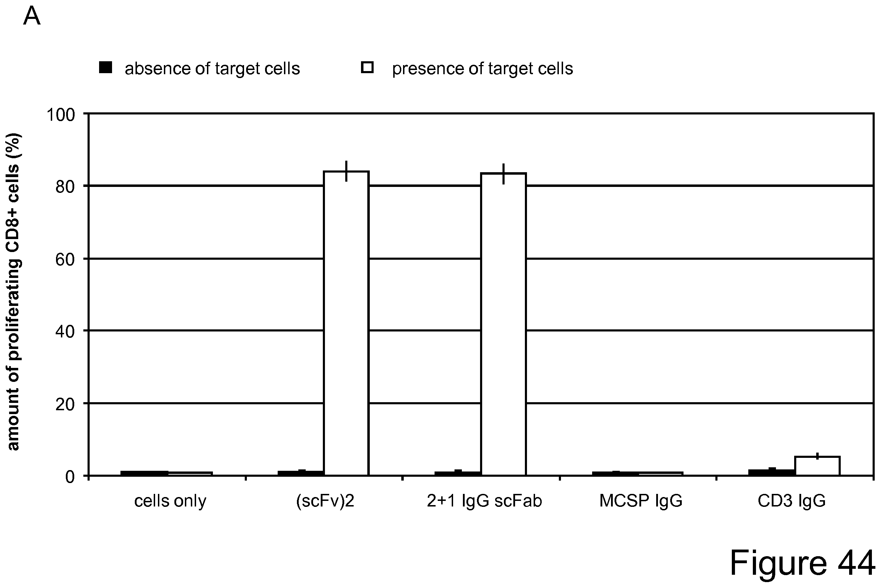

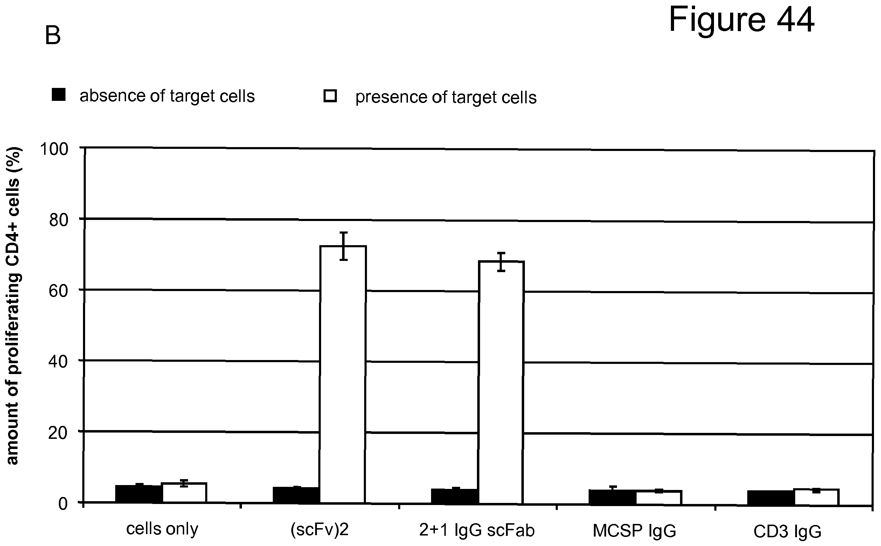

FIGURE 44. Relative proliferation of either CD8⁺ (A) or CD4⁺ (B) human T cells upon incubation with 1 nM of different CD3-MCSP bispecific constructs ("2+1 IgG scFab, LALA" (see SEQ ID NOs 5, 17, 19) or "(scFv)₂") or corresponding control IgGs in the presence or absence of Colo-38 tumor target cells at an effector to target cell ratio of 5:1. CFSE-labeled human pan T cells were characterized by FACS. The relative proliferation level was determined by setting a gate around the non-proliferating cells and using the cell number of this gate relative to the overall measured cell number as the reference.

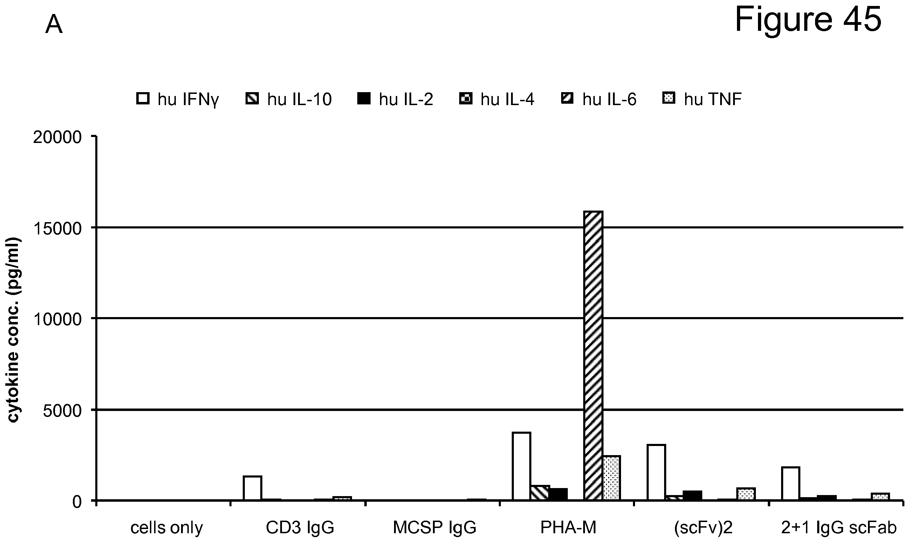

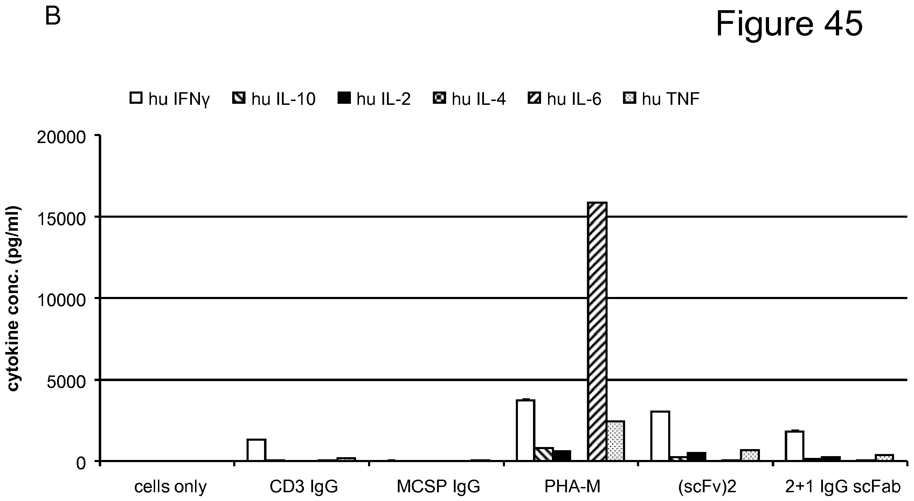

FIGURE 45. Levels of different cytokines measured in the supernatant of human PBMCs after treatment with 1 nM of different CD3-MCSP bispecific constructs ("2+1 IgG scFab, LALA" (see SEQ ID NOs 5, 17, 19) or "(scFv)₂") or corresponding control IgGs in the presence (A) or absence (B) of Colo-38 tumor cells for 24 hours. The effector to target cell ratio was 10:1.

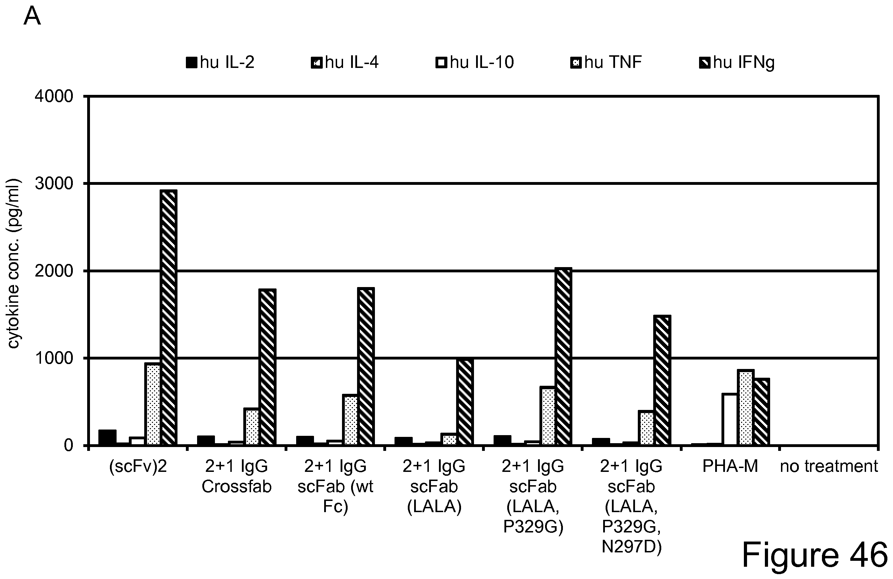

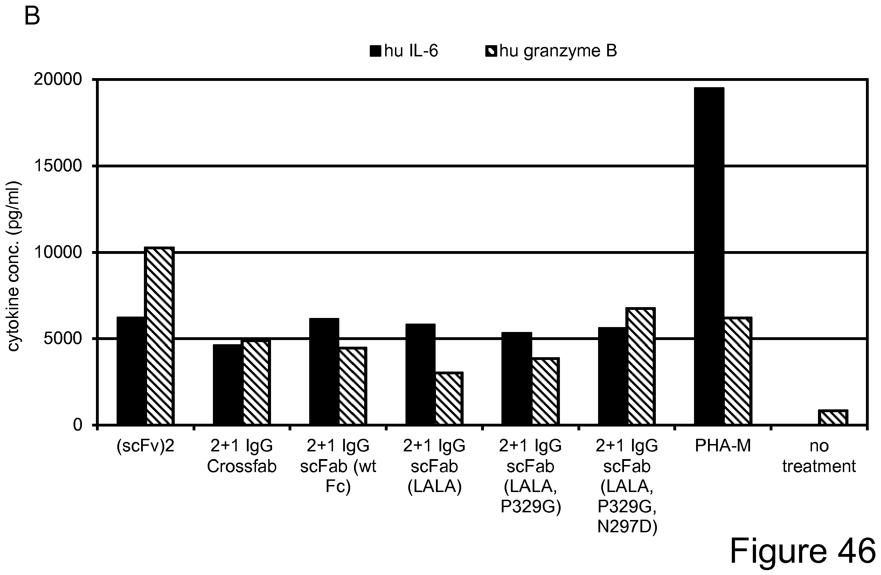

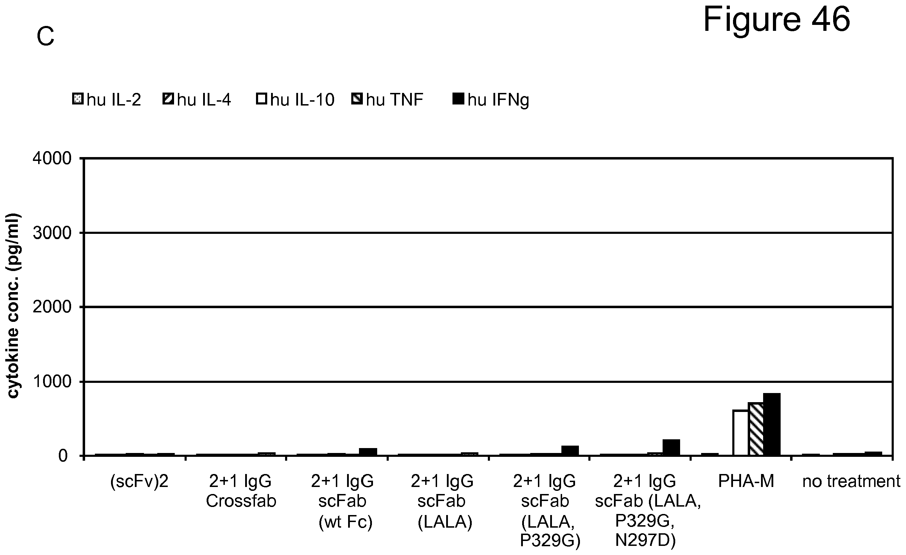

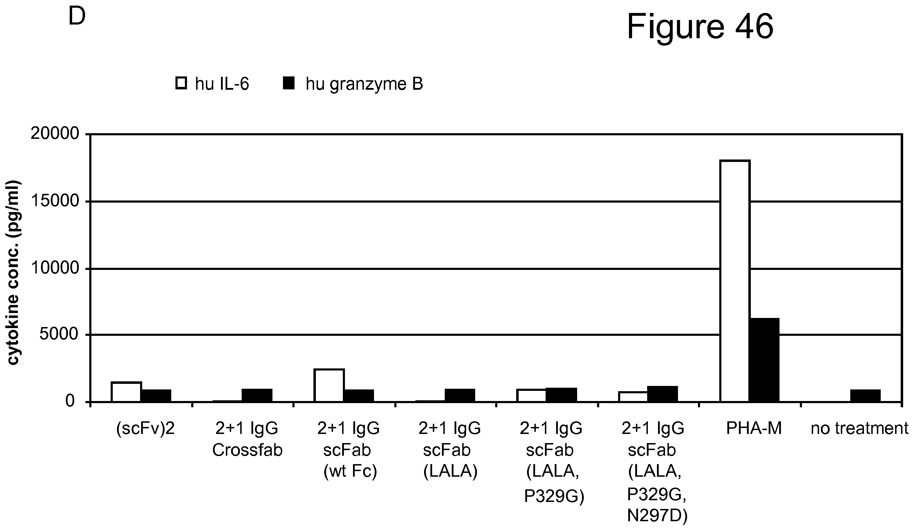

FIGURE 46. Levels of different cytokines measured in the supernatant of whole blood after treatment with 1 nM of different CD3-MCSP bispecific constructs ("2+1 IgG scFab", "2+1 IgG Crossfab" (see SEQ ID NOs 3, 5, 29, 33) or "(scFv)₂") or corresponding control IgGs in the presence (A, B) or absence (C, D) of Colo-38 tumor cells for 24 hours. Among the bispecific constructs were different "2+1 IgG scFab" constructs having either a wild-type Fc domain (see SEQ ID NOs 5, 13, 15), or an Fc domain mutated to abolish (NK) effector cell function (LALA (see SEQ ID NOs 5, 17, 19), P329G LALA (see SEQ ID NOs 5, 2, 23) and P329G LALA N297D (see SEQ ID NOs 5, 25, 27)).

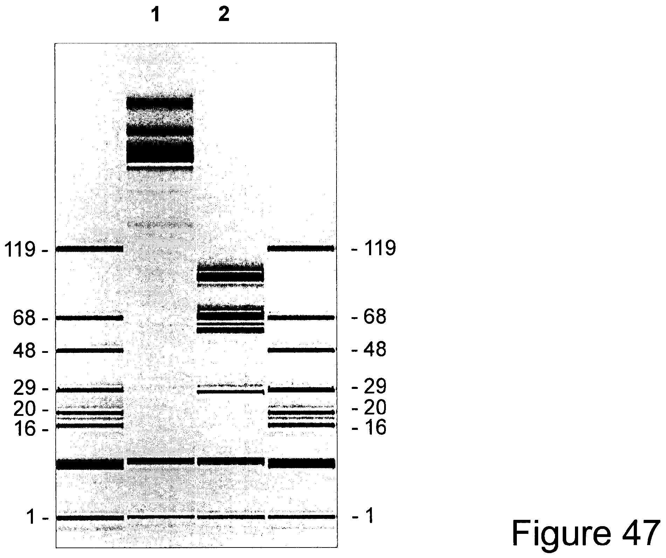

FIGURE 47. CE-SDS analyses. Electropherogram shown as SDS PAGE of 2+1 IgG Crossfab, linked light chain (see SEQ ID NOs 3, 5, 29, 179). (lane 1: reduced, lane 2: non-reduced).

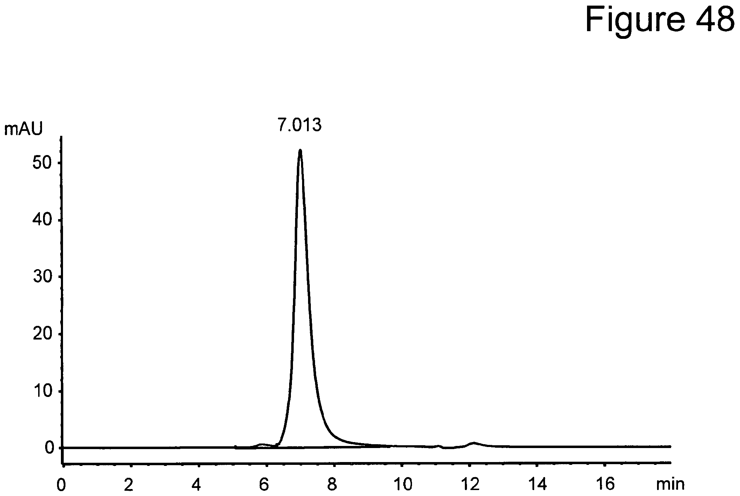

FIGURE 48. Analytical size exclusion chromatography of 2+1 IgG Crossfab, linked light chain (see SEQ ID NOs 3, 5, 29, 179) (final product). 20 µg sample were injected.

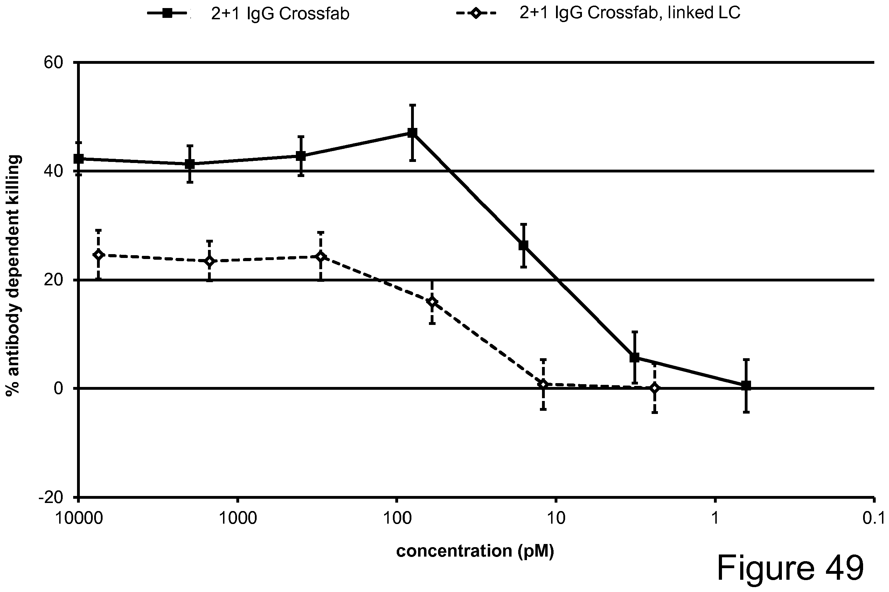

FIGURE 49. Killing (as measured by LDH release) of MCSP-positive MV-3 tumor cells upon co-culture by human PBMCs (E:T ratio = 10:1), treated with different CD3-MCSP bispecific constructs for ∼ 44 hours ("2+1 IgG Crossfab" (see SEQ ID NOs 3, 5, 29, 33) and "2+1 IgG Crossfab, linked LC" (see SEQ ID NOs 3, 5, 29, 179)). Human PBMCs were isolated from fresh blood of healthy volunteers.

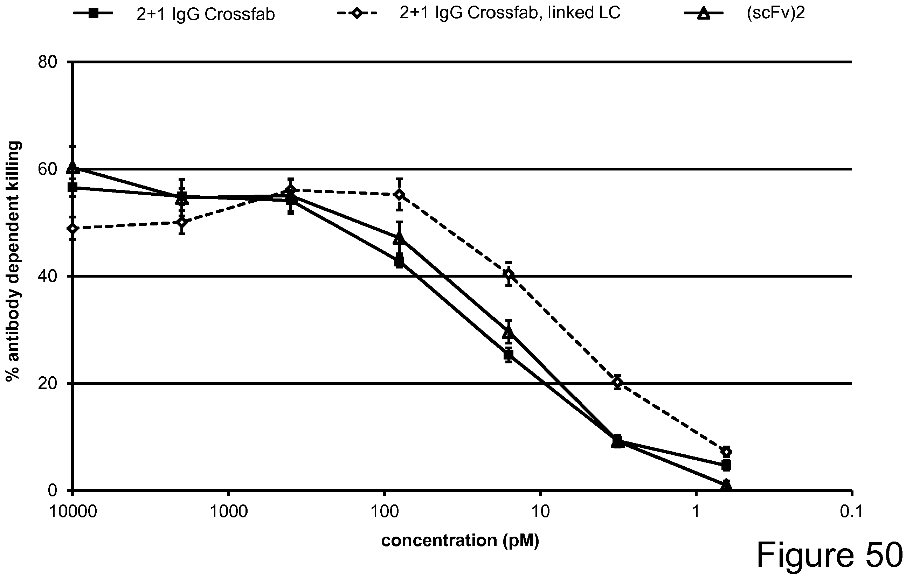

FIGURE 50. Killing (as measured by LDH release) of MCSP-positive Colo-38 tumor cells upon co-culture by human PBMCs (E:T ratio = 10:1), treated with different CD3-MCSP bispecific constructs for -22 hours ("2+1 IgG Crossfab" (see SEQ ID NOs 3, 5, 29, 33) and "2+1 IgG Crossfab, linked LC" (see SEQ ID NOs 3, 5, 29, 179)). Human PBMCs were isolated from fresh blood of healthy volunteers.

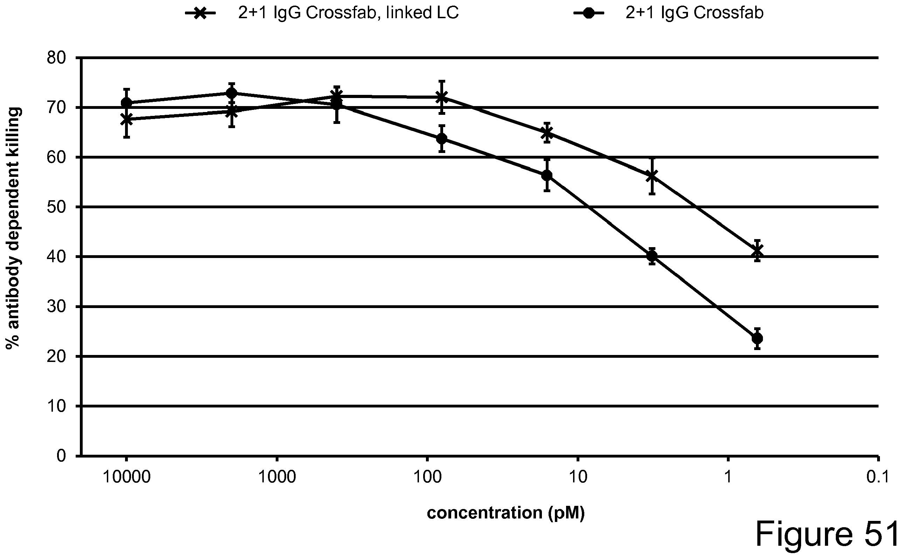

FIGURE 51. Killing (as measured by LDH release) of MCSP-positive Colo-38 tumor cells upon co-culture by human PBMCs (E:T ratio = 10:1), treated with different CD3-MCSP bispecific constructs for -22 hours ("2+1 IgG Crossfab" (see SEQ ID NOs 3, 5, 29, 33) and "2+1 IgG Crossfab, linked LC" (see SEQ ID NOs 3, 5, 29, 179)). Human PBMCs were isolated from fresh blood of healthy volunteers.

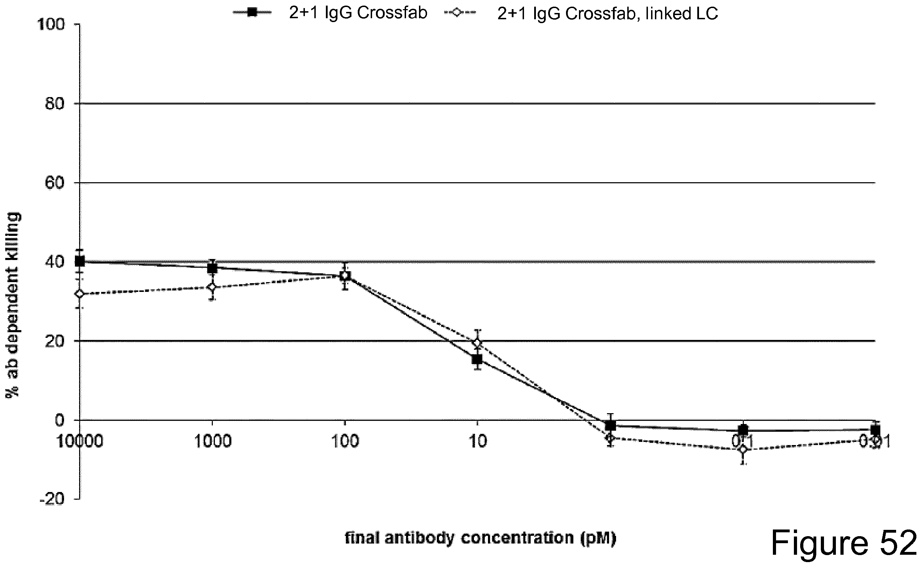

FIGURE 52. Killing (as measured by LDH release) of MCSP-positive WM266-4 cells upon co-culture by human PBMCs (E:T ratio = 10:1), treated with different CD3-MCSP bispecific constructs for -22 hours ("2+1 IgG Crossfab" (see SEQ ID NOs 3, 5, 29, 33) and "2+1 IgG Crossfab, linked LC" (see SEQ ID NOs 3, 5, 29, 179)). Human PBMCs were isolated from fresh blood of healthy volunteers.

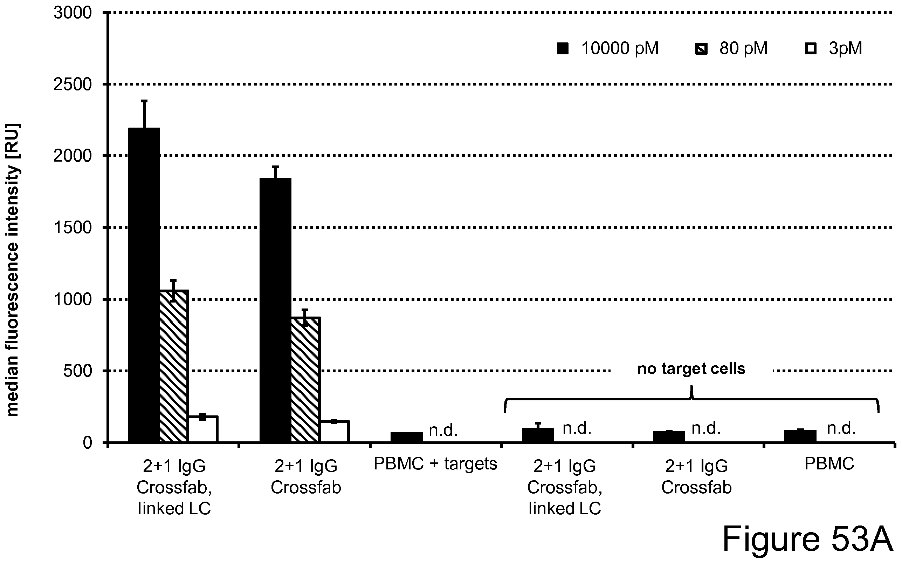

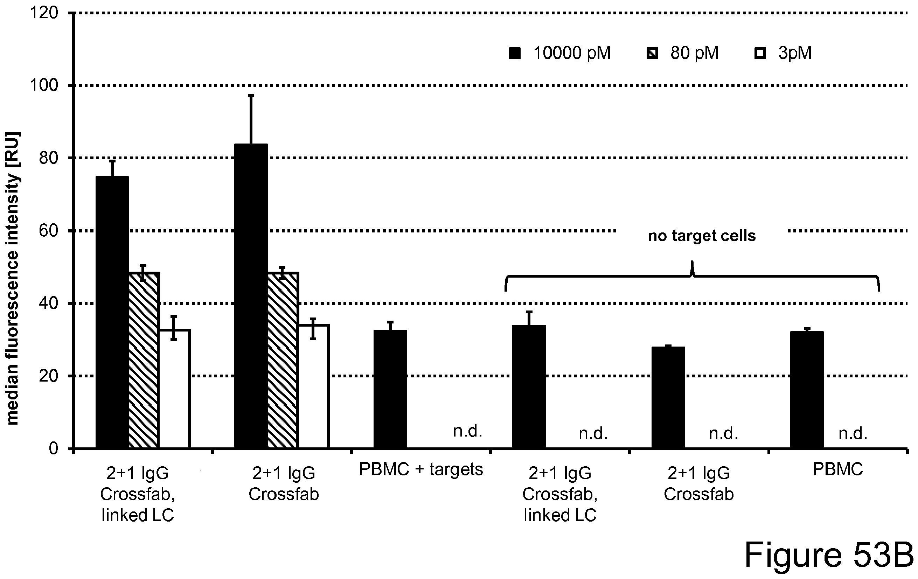

FIGURE 53. Surface expression level of the early activation marker CD69 (A) and the late activation marker CD25 (B) on human CD8⁺ T cells after 22 hours incubation with 10 nM, 80 pM or 3 pM of different CD3-MCSP bispecific constructs ("2+1 IgG Crossfab" (see SEQ ID NOs 3, 5, 29, 33) and "2+1 IgG Crossfab, linked LC" (see SEQ ID NOs 3, 5, 29, 179)) in the presence or absence of human MCSP-expressing Colo-38 tumor target cells (E:T ratio = 10:1).

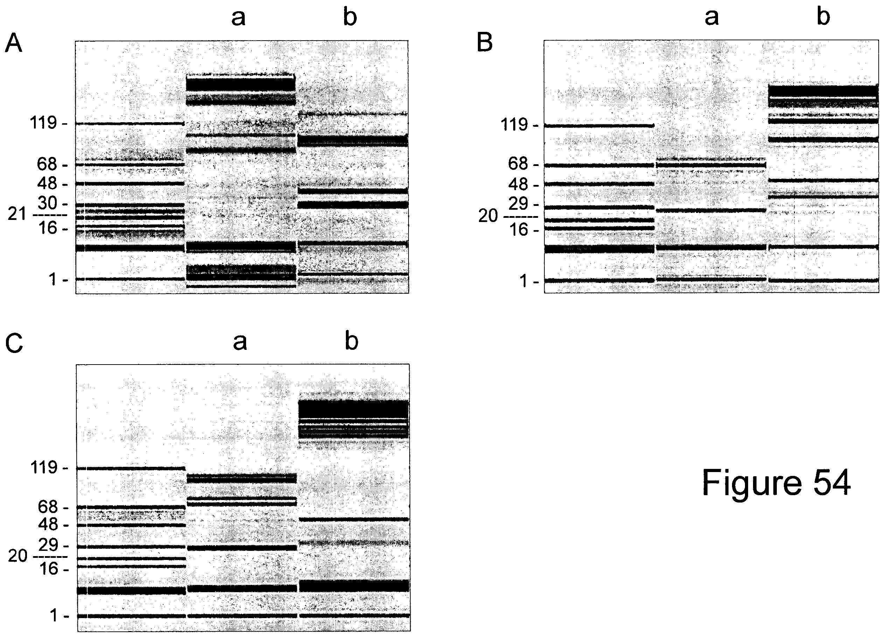

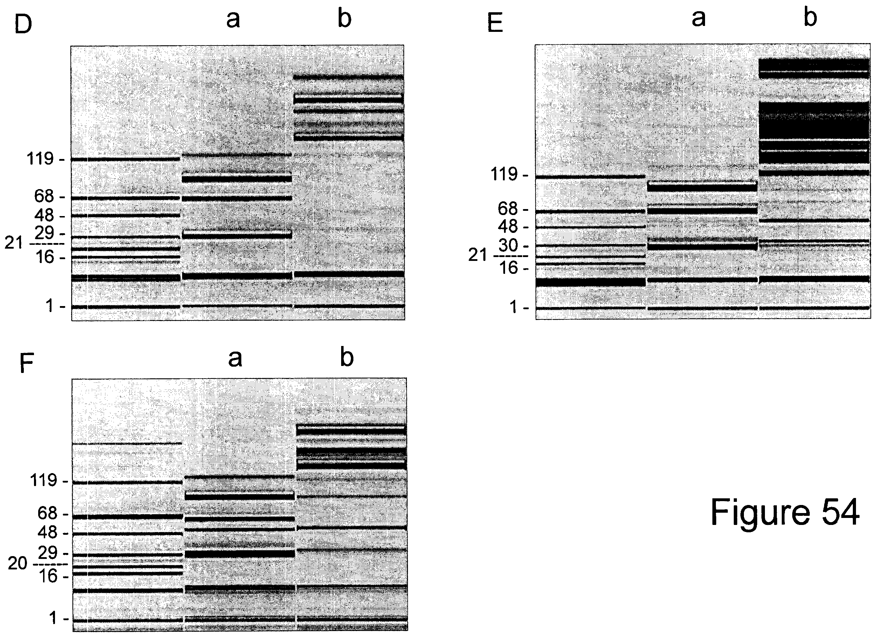

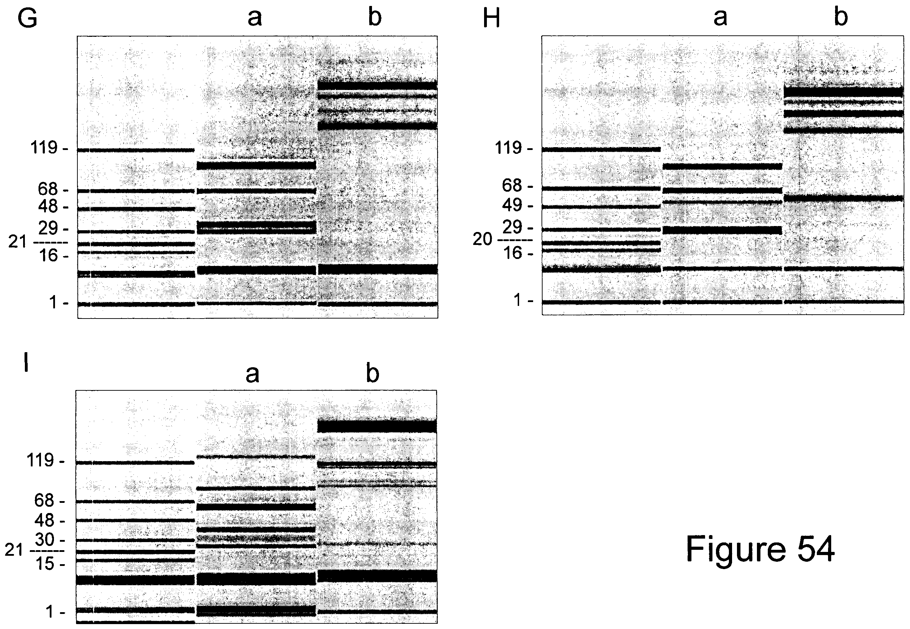

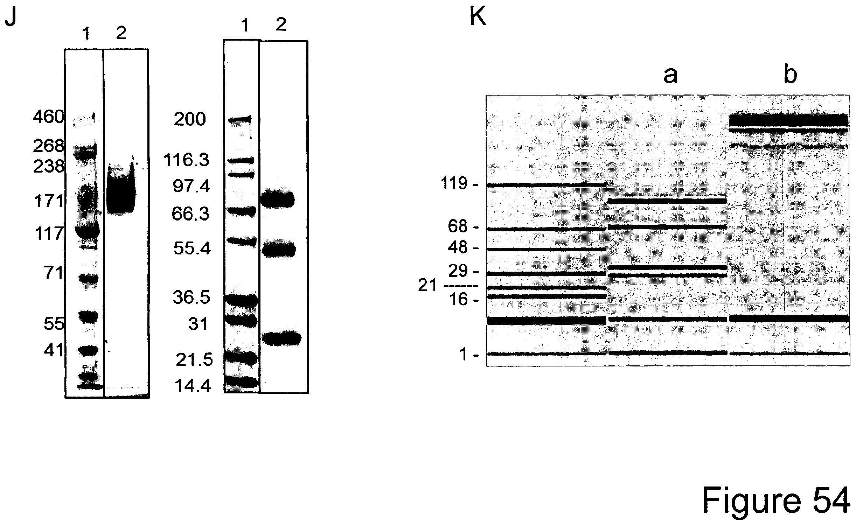

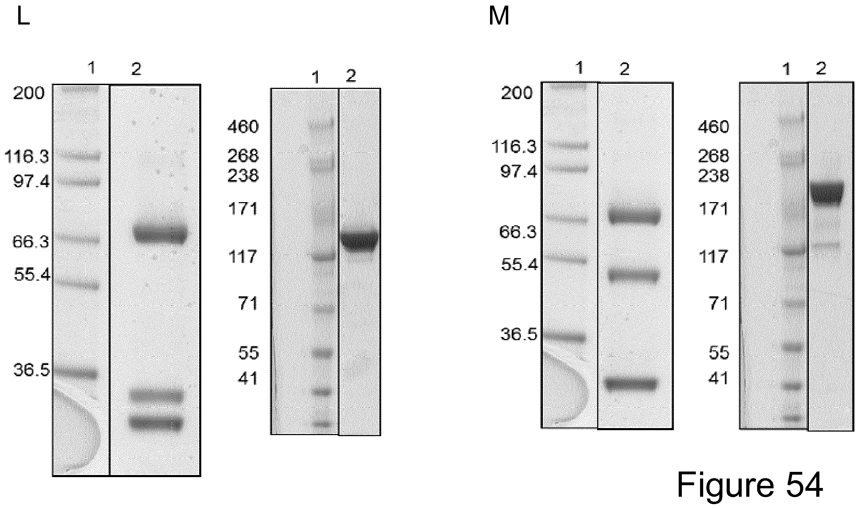

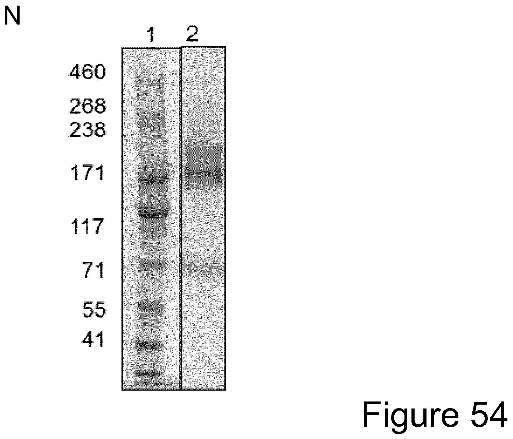

FIGURE 54. CE-SDS analyses. (A) Electropherogram shown as SDS-PAGE of 1+1 IgG Crossfab; VL/VH exchange (LC007/V9) (see SEQ ID NOs 5, 29, 33, 181): a) non-reduced, b) reduced. (B) Electropherogram shown as SDS-PAGE of 1+1 CrossMab; CL/CH1 exchange (LC007/V9) (see SEQ ID NOs 5, 23, 183, 185): a) reduced, b) non-reduced. (C) Electropherogram shown as SDS-PAGE of 2+1 IgG Crossfab, inverted; CL/CH1 exchange (LC007/V9) (see SEQ ID NOs 5, 23, 183, 187): a) reduced, b) non-reduced. (D) Electropherogram shown as SDS-PAGE of 2+1 IgG Crossfab; VL/VH exchange (M4-3 ML2/V9) (see SEQ ID NOs 33, 189, 191, 193): a) reduced, b) non-reduced. (E) Electropherogram shown as SDS-PAGE of 2+1 IgG Crossfab; CL/CH1 exchange (M4-3 ML2/V9) (see SEQ ID NOs 183, 189, 193, 195): a) reduced, b) non-reduced. (F) Electropherogram shown as SDS-PAGE of 2+1 IgG Crossfab, inverted; CL/CH1 exchange (CH1A1A/V9) (see SEQ ID NOs 65, 67, 183, 197): a) reduced, b) non-reduced. (G) Electropherogram shown as SDS-PAGE of 2+1 IgG Crossfab; CL/CH1 exchange (M4-3 ML2/H2C) (see SEQ ID NOs 189, 193, 199, 201): a) reduced, b) non-reduced. (H) Electropherogram shown as SDS-PAGE of 2+1 IgG Crossfab, inverted; CL/CH1 exchange (431/26/V9) (see SEQ ID NOs 183, 203, 205, 207): a) reduced, b) non-reduced. (I) Electropherogram shown as SDS-PAGE of "2+1 IgG Crossfab light chain fusion" (CH1A1A/V9) (see SEQ ID NOs 183, 209, 211, 213): a) reduced, b) non-reduced. (J) SDS PAGE (4-12% Bis/Tris, NuPage Invitrogen, Coomassie-stained) of "2+1 IgG Crossfab" (anti-MCSP/anti-huCD3) (see SEQ ID NOs 5, 23, 215, 217), non-reduced (left) and reduced (right). (K) Electropherogram shown as SDS-PAGE of "2+1 IgG Crossfab, inverted" (anti-MCSP/anti-huCD3) (see SEQ ID NOs 5, 23, 215, 219): a) reduced, b) non-reduced. (L) SDS PAGE (4-12% Bis/Tris, NuPage Invitrogen, Coomassie-stained) of "1+1 IgG Crossfab" (anti-CD33/anti-huCD3) (see SEQ ID NOs 33, 213, 221, 223), reduced (left) and non-reduced (right). (M) SDS PAGE (4-12% Bis/Tris, NuPage Invitrogen, Coomassie-stained) of "2+1 IgG Crossfab" (anti-CD33/anti-huCD3) (see SEQ ID NOs 33, 221, 223, 225), reduced (left) and non-reduced (right). (N) SDS PAGE (4-12% Bis/Tris, NuPage Invitrogen, Coomassie-stained) of "2+1 IgG Crossfab" (anti-CD20/anti-huCD3) (see SEQ ID NOs 33, 227, 229, 231), non-reduced.

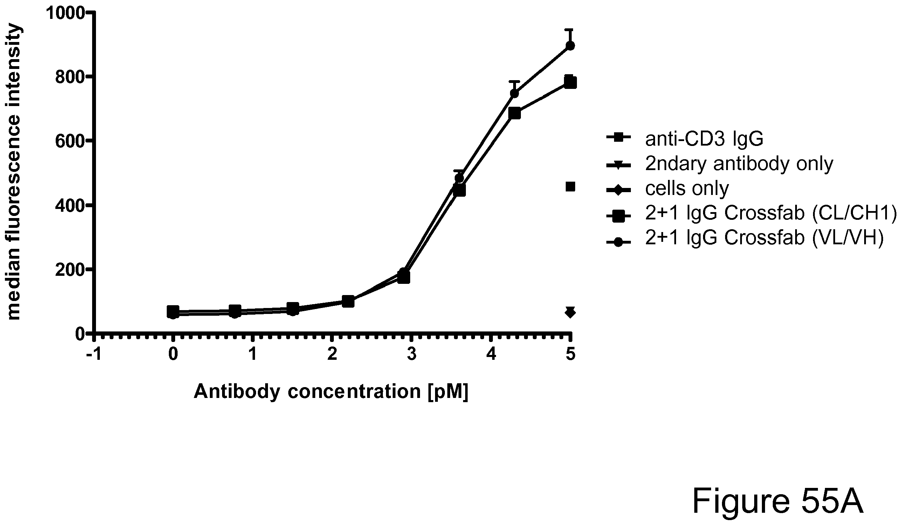

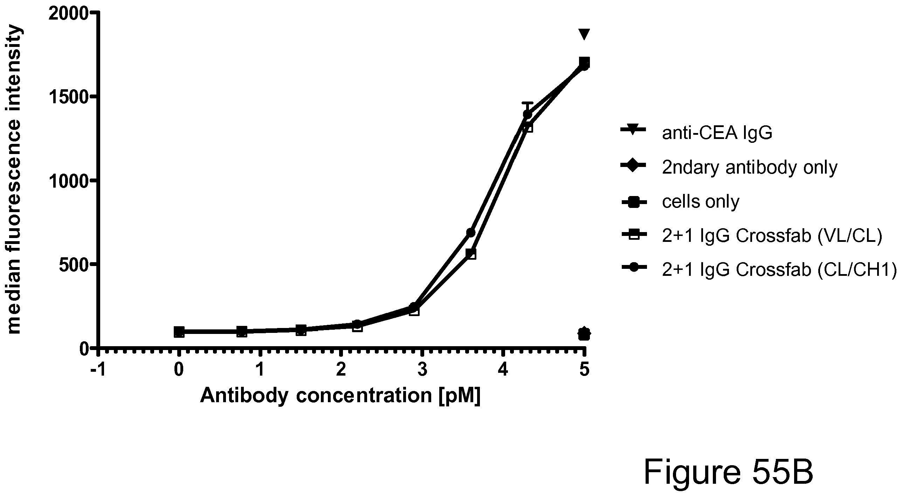

FIGURE 55. Binding of bispecific constructs (CEA/CD3 "2+1 IgG Crossfab, inverted (VL/VH)" (see SEQ ID NOs 33, 63, 65, 67) and "2+1 IgG Crossfab, inverted (CL/CH1)
   2 (see SEQ ID NOs 65, 67, 183, 197)) to human CD3, expressed by Jurkat cells (A), or to human CEA, expressed by LS-174T cells (B) as determined by FACS. As a control, the equivalent maximum concentration of the reference IgGs and the background staining due to the labeled 2ndary antibody (goat anti-human FITC-conjugated AffiniPure F(ab')2 Fragment, Fcγ Fragment-specific, Jackson Immuno Research Lab # 109-096-098) were assessed as well.

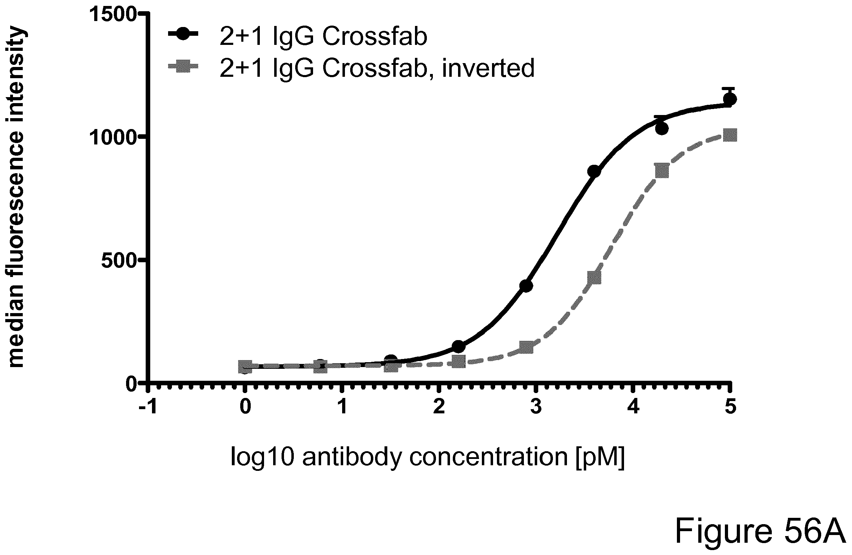

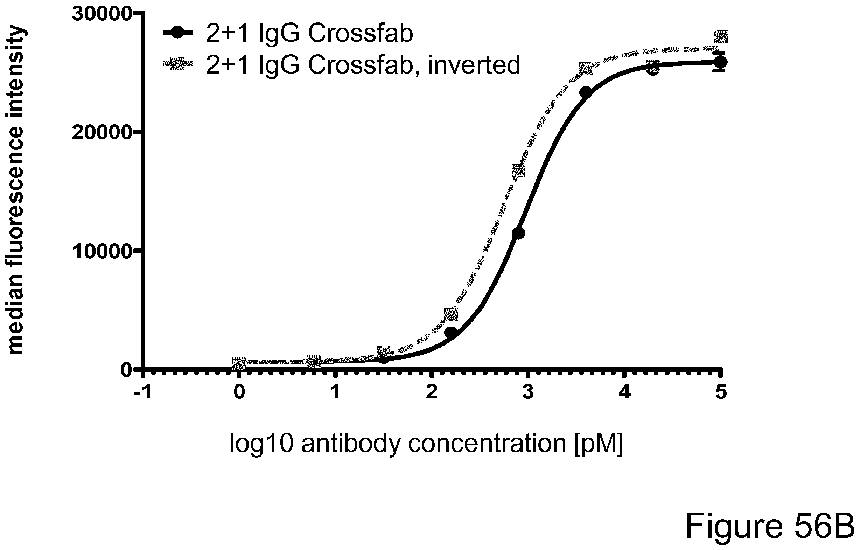

FIGURE 56. Binding of bispecific constructs constructs (MCSP/CD3 "2+1 IgG Crossfab" (see SEQ ID NOs 3, 5, 29, 33) and "2+1 IgG Crossfab, inverted" (see SEQ ID NOs 5, 23, 183, 187)) to human CD3, expressed by Jurkat cells (A), or to human MCSP, expressed by WM266-4 tumor cells (B) as determined by FACS.

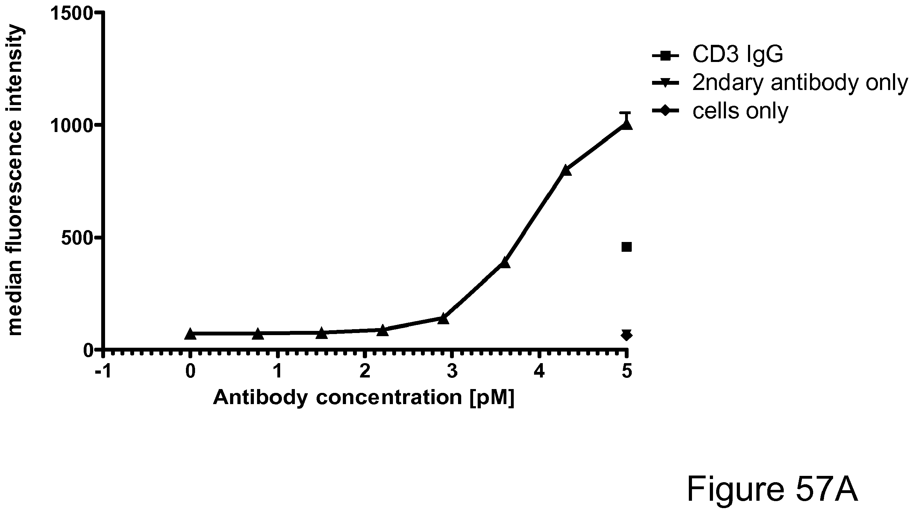

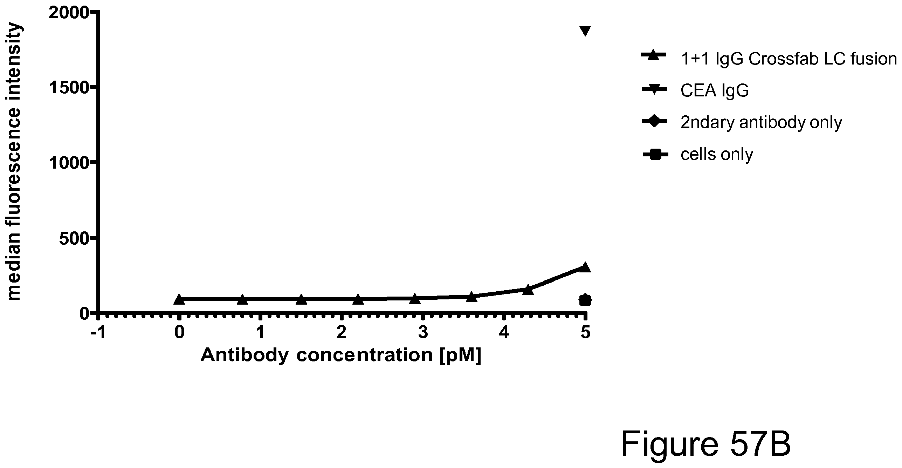

FIGURE 57. Binding of the "1+1 IgG Crossfab light chain fusion" (see SEQ ID NOs 183, 209, 211, 213) to human CD3, expressed by Jurkat cells (A), or to human CEA, expressed by LS-174T cells (B) as determined by FACS.

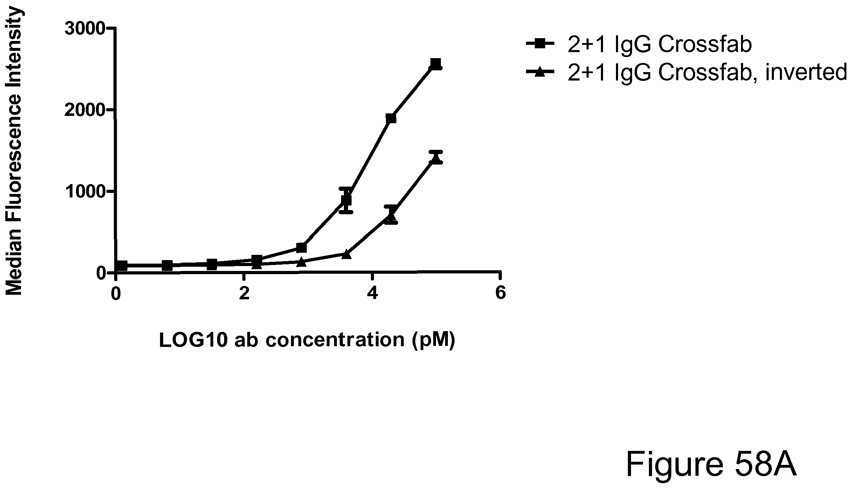

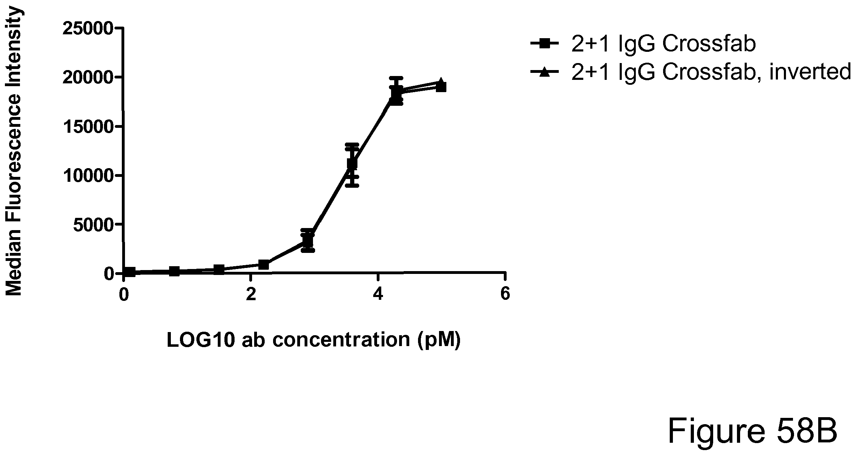

FIGURE 58. Binding of the "2+1 IgG Crossfab" (see SEQ ID NOs 5, 23, 215, 217) and the "2+1 IgG Crossfab, inverted" (see SEQ ID NOs 5, 23, 215, 219) constructs to human CD3, expressed by Jurkat cells (A), or human MCSP, expressed by WM266-4 tumor cells (B) as determined by FACS.

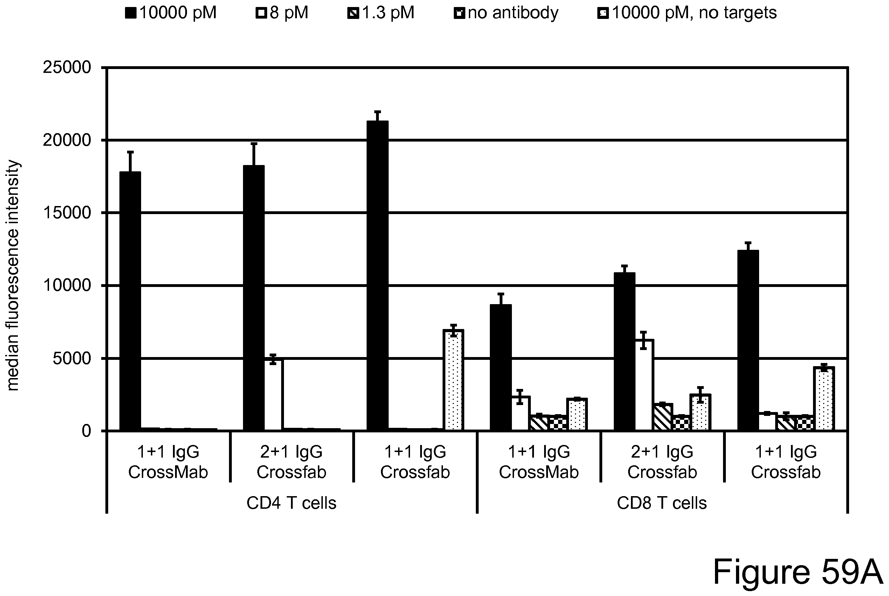

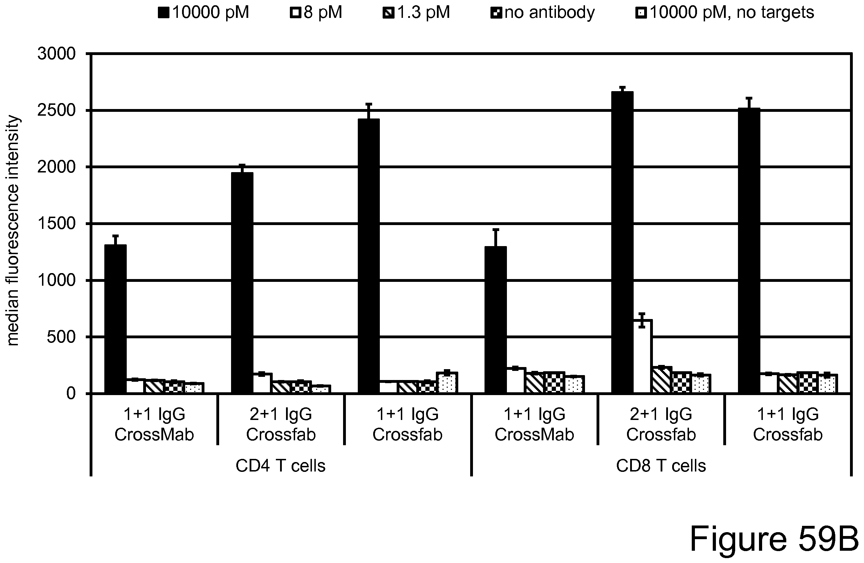

FIGURE 59. Surface expression level of the early activation marker CD69 (A) or the late activation marker CD25 (B) on human CD4⁺ or CD8⁺ T cells after 24 hours incubation with the indicated concentrations of the CD3/MCSP "1+1 CrossMab" (see SEQ ID NOs 5, 23, 183, 185), "1+1 IgG Crossfab" (see SEQ ID NOs 5, 29, 33, 181) and "2+1 IgG Crossfab" (see SEQ ID NOs 3, 5, 29, 33) constructs. The assay was performed in the presence or absence of MV-3 target cells, as indicated.

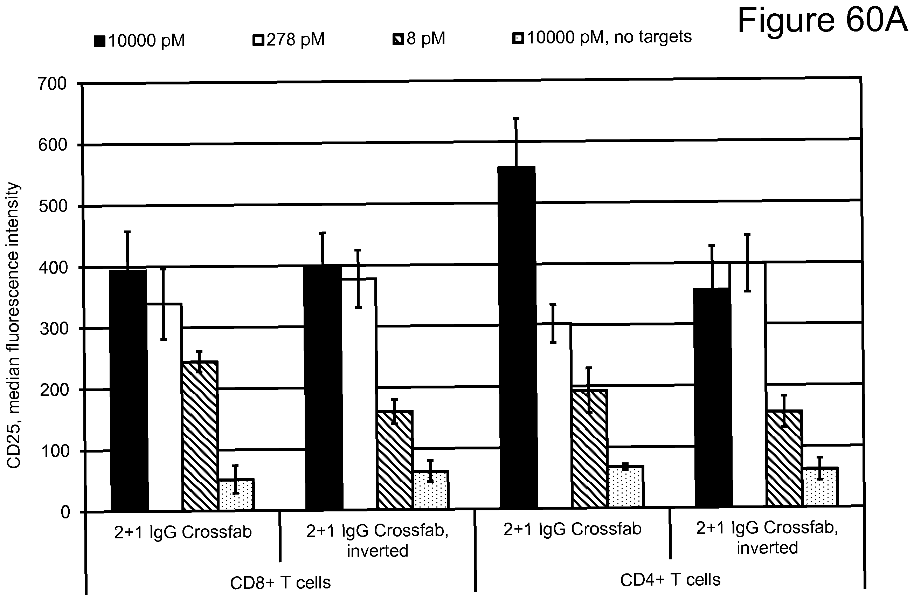

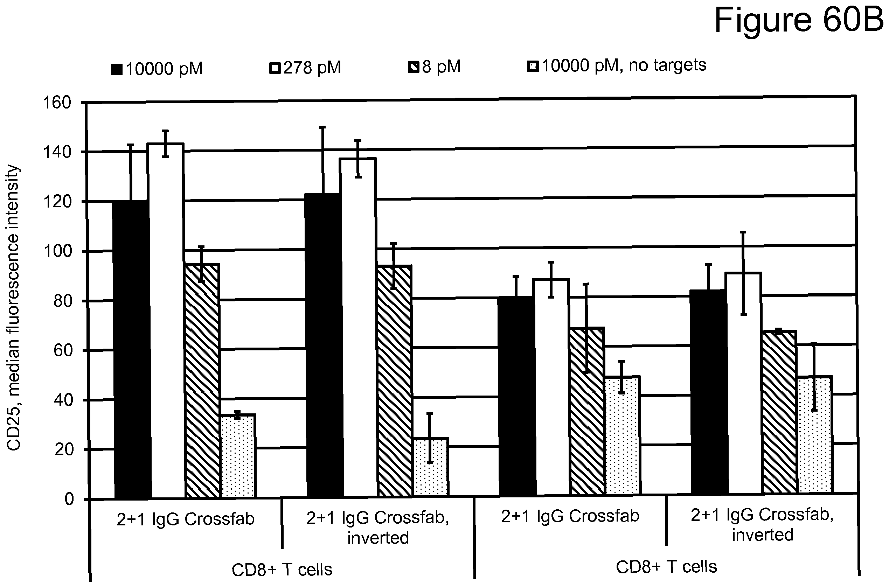

FIGURE 60. Surface expression level of the early activation marker CD25 on CD4⁺ or CD8⁺ T cells from two different cynomolgus monkeys (A and B) in the presence or absence of huMCSP-positive MV-3 tumor cells upon co-culture with cynomolgus PBMCs (E:T ratio = 3:1, normalized to CD3⁺ numbers), treated with the "2+1 IgG Crossfab" (see SEQ ID NOs 5, 23, 215, 217) and the "2+1 IgG Crossfab, inverted" (see SEQ ID NOs 5, 23, 215, 219) for ∼41 hours.

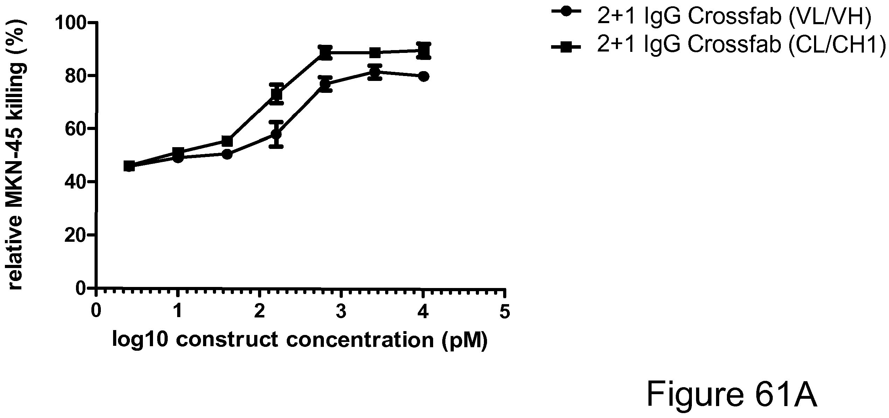

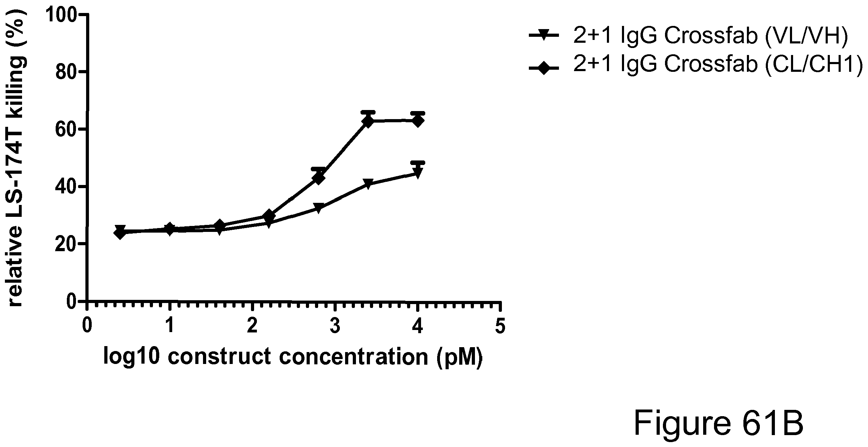

FIGURE 61. Killing (as measured by LDH release) of MKN-45 (A) or LS-174T (B) tumor cells upon co-culture with human PBMCs (E:T ratio = 10:1) and activation for 28 hours by different concentrations of the "2+1 IgG Crossfab, inverted (VL/VH)" (see SEQ ID NOs 33, 63, 65, 67) versus the "2+1 IgG Crossfab, inverted (CL/CH1)" (see SEQ ID NOs 65, 67, 183, 197) construct.

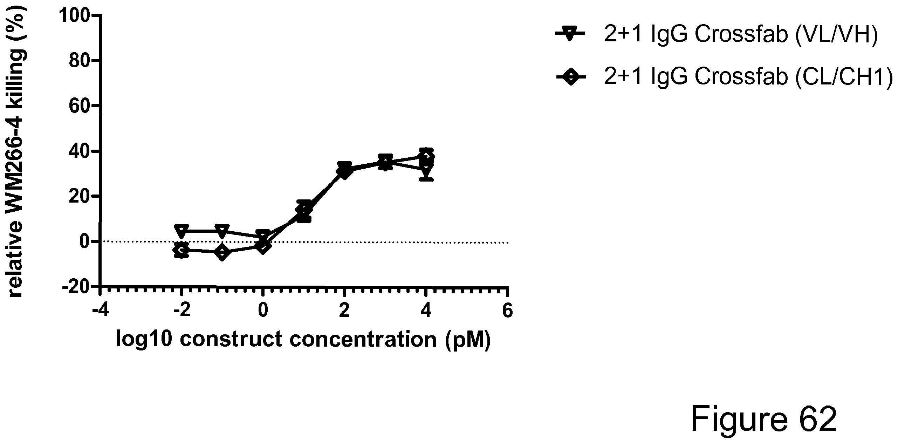

FIGURE 62. Killing (as measured by LDH release) of WM266-4 tumor cells upon co-culture with human PBMCs (E:T ratio = 10:1) and activation for 26 hours by different concentrations of the "2+1 IgG Crossfab (VL/VH)" (see SEQ ID NOs 33, 189, 191, 193) versus the "2+1 IgG Crossfab (CL/CH1)" (see SEQ ID NOs 183, 189, 193, 195) construct.

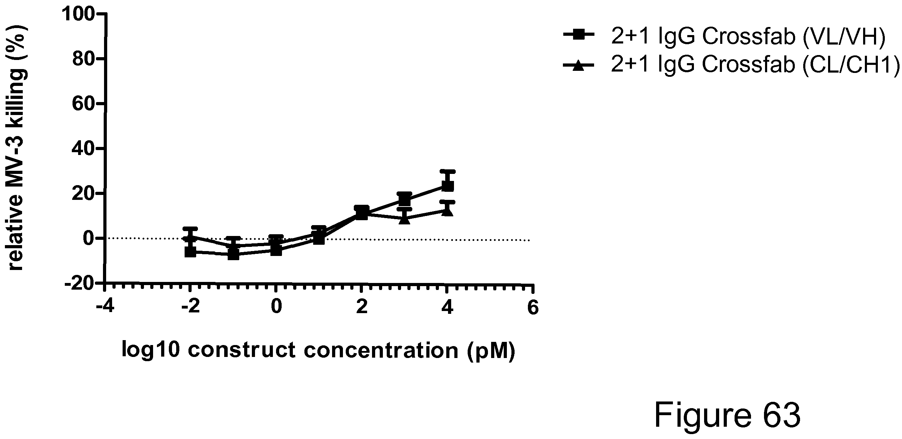

FIGURE 63. Killing (as measured by LDH release) of MV-3 tumor cells upon co-culture with human PBMCs (E:T ratio = 10:1) and activation for 27 hours by different concentrations of the "2+1 IgG Crossfab (VH/VL)" (see SEQ ID NOs 33, 189, 191, 193) versus the "2+1 IgG Crossfab (CL/CH1)" (see SEQ ID NOs 183, 189, 193, 195) constructs.

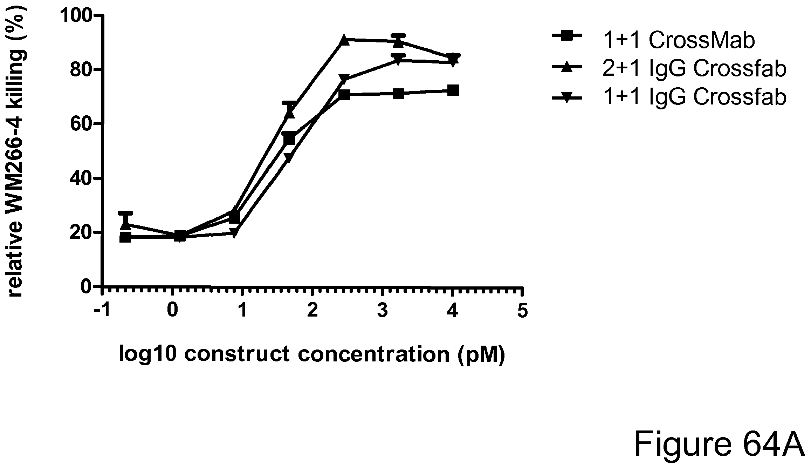

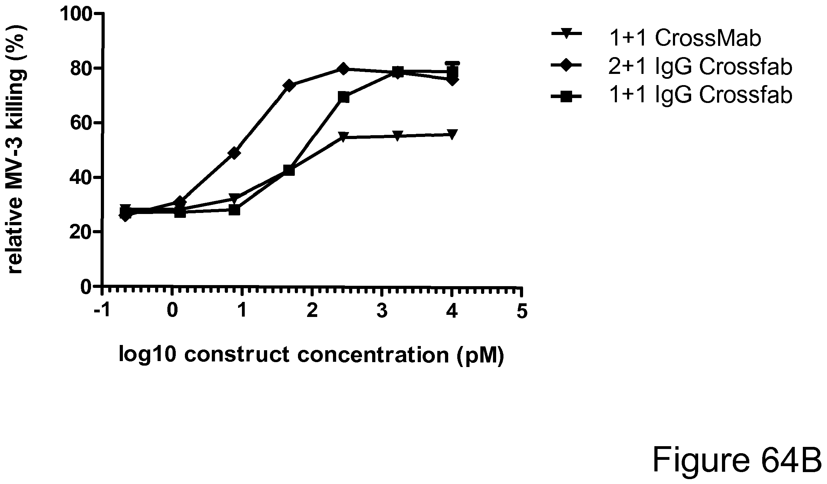

FIGURE 64. Killing (as measured by LDH release) of human MCSP-positive WM266-4 (A) or MV-3 (B) tumor cells upon co-culture with human PBMCs (E:T ratio = 10:1) and activation for 21 hours by different concentrations of the "2+1 IgG Crossfab" (see SEQ ID NOs 3, 5, 29, 33), the "1+1 CrossMab" (see SEQ ID NOs 5, 23, 183, 185), and the "1+1 IgG Crossfab" (see SEQ ID NOs 5, 29, 33, 181), as indicated.

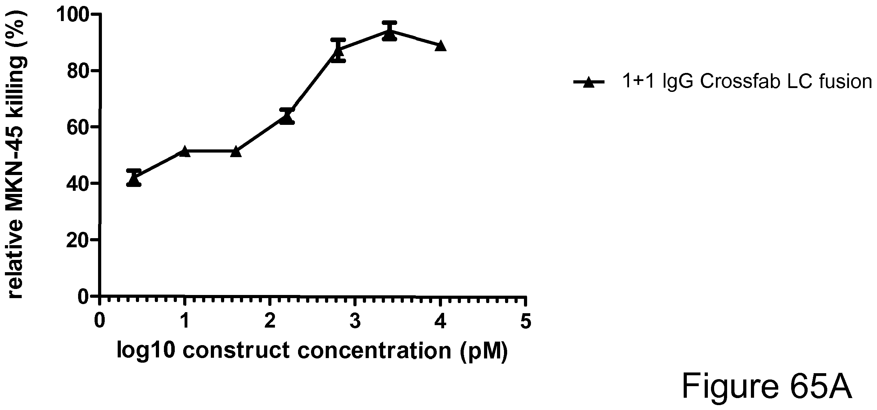

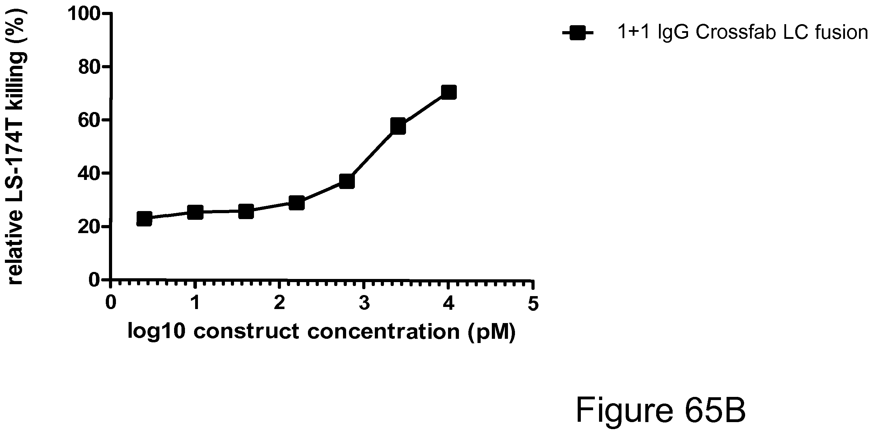

FIGURE 65. Killing (as measured by LDH release) of MKN-45 (A) or LS-174T (B) tumor cells upon co-culture with human PBMCs (E:T ratio = 10:1) and activation for 28 hours by different concentrations of the "1+1 IgG Crossfab LC fusion" (see SEQ ID NOs 183, 209, 211, 213).

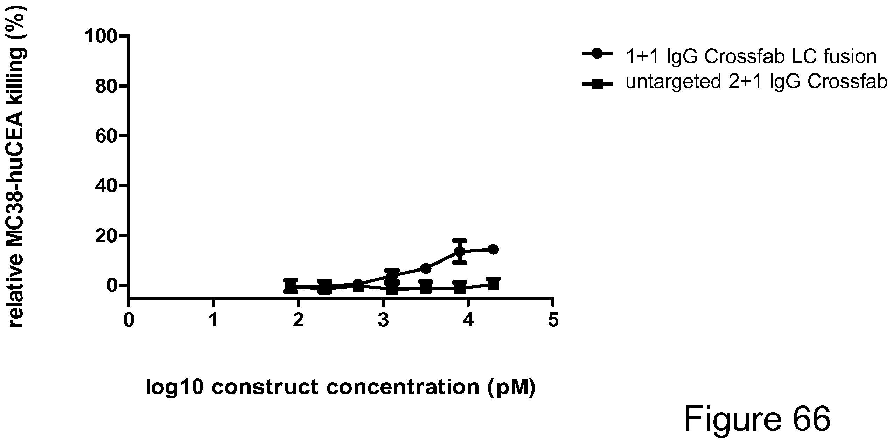

FIGURE 66. Killing (as measured by LDH release) of MC38-huCEA tumor cells upon co-culture with human PBMCs (E:T ratio = 10:1) and activation for 24 hours by different concentrations of the "1+1 IgG Crossfab LC fusion" (see SEQ ID NOs 183, 209, 211, 213) versus an untargeted "2+1 IgG Crossfab" reference.

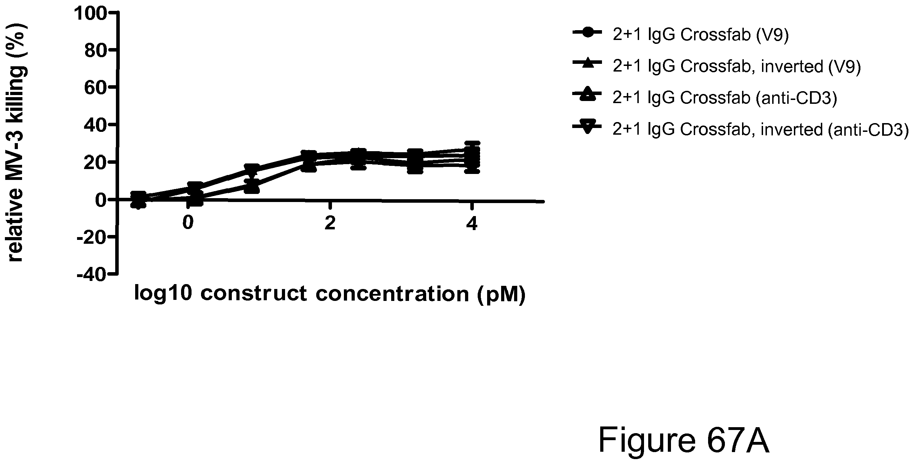

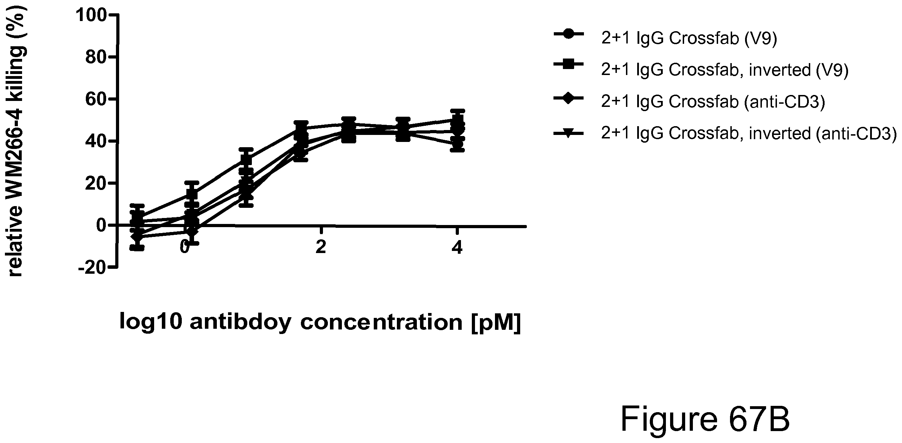

FIGURE 67. Killing (as measured by LDH release) of human MCSP-positive MV-3 (A) or WM266-4 (B) tumor cells upon co-culture with human PBMCs (E:T ratio = 10:1), treated with the "2+1 IgG Crossfab (V9)" (see SEQ ID NOs 3, 5, 29, 33) and the "2+1 IgG Crossfab, inverted (V9)" (see SEQ ID NOs 5, 23, 183, 187), the "2+1 IgG Crossfab (anti-CD3)" (see SEQ ID NOs 5, 23, 215, 217) and the "2+1 IgG Crossfab, inverted (anti-CD3)" (see SEQ ID NOs 5, 23, 215, 219) constructs.

### Detailed Description

### Definitions

Terms are used herein as generally used in the art, unless otherwise defined in the following.

As used herein, the term "antigen binding molecule" refers in its broadest sense to a molecule that specifically binds an antigenic determinant. Examples of antigen binding molecules are immunoglobulins and derivatives, e.g. fragments, thereof.

The term "bispecific" means that the antigen binding molecule is able to specifically bind to at least two distinct antigenic determinants. Typically, a bispecific antigen binding molecule comprises two antigen binding sites, each of which is specific for a different antigenic determinant. In certain embodiments the bispecific antigen binding molecule is capable of simultaneously binding two antigenic determinants, particularly two antigenic determinants expressed on two distinct cells.

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab molecule typically has a single antigen binding site.

As used herein, the term "antigen binding moiety" refers to a polypeptide molecule that specifically binds to an antigenic determinant. In one embodiment, an antigen binding moiety is able to direct the entity to which it is attached (e.g. a second antigen binding moiety) to a target site, for example to a specific type of tumor cell or tumor stroma bearing the antigenic determinant. In another embodiment an antigen binding moiety is able to activate signaling through its target antigen, for example a T cell receptor complex antigen. Antigen binding moieties include antibodies and fragments thereof as further defined herein. Particular antigen binding moieties include an antigen binding domain of an antibody, comprising an antibody heavy chain variable region and an antibody light chain variable region. In certain embodiments, the antigen binding moieties may comprise antibody constant regions as further defined herein and known in the art. Useful heavy chain constant regions include any of the five isotypes: α, δ, ε, γ, or µ. Useful light chain constant regions include any of the two isotypes: κ and λ.

As used herein, the term "antigenic determinant" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, on the surface of immune cells, free in blood serum, and/or in the extracellular matrix (ECM). The proteins referred to as antigens herein (e.g. MCSP, FAP, CEA, EGFR, CD33, CD3) can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants. Exemplary human proteins useful as antigens include, but are not limited to: Melanoma-associated Chondroitin Sulfate Proteoglycan (MCSP), also known as Chondroitin Sulfate Proteoglycan 4 (UniProt no. Q6UVK1 (version 70), NCBI RefSeq no. NP_001888.2); Fibroblast Activation Protein (FAP), also known as Seprase (Uni Prot nos. Q12884, Q86Z29, Q99998, NCBI Accession no. NP_004451); Carcinoembroynic antigen (CEA), also known as Carcinoembryonic antigen-related cell adhesion molecule 5 (UniProt no. P06731 (version 119), NCBI RefSeq no. NP_004354.2); CD33, also known as gp67 or Siglec-3 (UniProt no. P20138, NCBI Accession nos. NP_001076087, NP_001171079); Epidermal Growth Factor Receptor (EGFR), also known as ErbB-1 or Her1 (UniProt no. P0053, NCBI Accession nos. NP_958439, NP_958440), and CD3, particularly the epsilon subunit of CD3 (see UniProt no. P07766 (version 130), NCBI RefSeq no. NP_000724.1, SEQ ID NO: 265 for the human sequence; or UniProt no. Q95LI5 (version 49), NCBI GenBank no. BAB71849.1, SEQ ID NO: 266 for the cynomolgus [Macaca fascicularis] sequence). In certain embodiments the T cell activating bispecific antigen binding molecule disclosed herein binds to an epitope of an activating T cell antigen or a target cell antigen that is conserved among the activating T cell antigen or target antigen from different species.

By "specific binding" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance (SPR) technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding moiety to an unrelated protein is less than about 10% of the binding of the antigen binding moiety to the antigen as measured, e.g., by SPR. In certain embodiments, an antigen binding moiety that binds to the antigen, or an antigen binding molecule comprising that antigen binding moiety, has a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., an antigen binding moiety and an antigen, or a receptor and its ligand). The affinity of a molecule X for its partner Y can generally be represented by the dissociation constant (K_{D}), which is the ratio of dissociation and association rate constants (k_{off} and kₒₙ, respectively). Thus, equivalent affinities may comprise different rate constants, as long as the ratio of the rate constants remains the same. Affinity can be measured by well established methods known in the art, including those described herein. A particular method for measuring affinity is Surface Plasmon Resonance (SPR).

"Reduced binding", for example reduced binding to an Fc receptor, refers to a decrease in affinity for the respective interaction, as measured for example by SPR. For clarity the term includes also reduction of the affinity to zero (or below the detection limit of the analytic method), i.e. complete abolishment of the interaction. Conversely, "increased binding" refers to an increase in binding affinity for the respective interaction.

An "activating T cell antigen" as used herein refers to an antigenic determinant expressed on the surface of a T lymphocyte, particularly a cytotoxic T lymphocyte, which is capable of inducing T cell activation upon interaction with an antigen binding molecule. Specifically, interaction of an antigen binding molecule with an activating T cell antigen may induce T cell activation by triggering the signaling cascade of the T cell receptor complex. In a particular embodiment the activating T cell antigen is CD3.

"T cell activation" as used herein refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. The T cell activating bispecific antigen binding molecules disclosed herein are capable of inducing T cell activation. Suitable assays to measure T cell activation are known in the art described herein.

A "target cell antigen" as used herein refers to an antigenic determinant presented on the surface of a target cell, for example a cell in a tumor such as a cancer cell or a cell of the tumor stroma.

As used herein, the terms "first" and "second" with respect to antigen binding moieties etc., are used for convenience of distinguishing when there is more than one of each type of moiety. Use of these terms is not intended to confer a specific order or orientation of the T cell activating bispecific antigen binding molecule unless explicitly so stated.

A "Fab molecule" refers to a protein consisting of the VH and CH1 domain of the heavy chain (the "Fab heavy chain") and the VL and CL domain of the light chain (the "Fab light chain") of an immunoglobulin.

By "fused" is meant that the components (e.g. a Fab molecule and an Fc domain subunit) are linked by peptide bonds, either directly or via one or more peptide linkers.

As used herein, the term "single-chain" refers to a molecule comprising amino acid monomers linearly linked by peptide bonds. In certain embodiments, one of the antigen binding moieties is a single-chain Fab molecule, i.e. a Fab molecule wherein the Fab light chain and the Fab heavy chain are connected by a peptide linker to form a single peptide chain. In a particular such embodiment, the C-terminus of the Fab light chain is connected to the N-terminus of the Fab heavy chain in the single-chain Fab molecule.

By a "crossover" Fab molecule (also termed "Crossfab") is meant a Fab molecule wherein either the variable regions or the constant regions of the Fab heavy and light chain are exchanged, i.e. the crossover Fab molecule comprises a peptide chain composed of the light chain variable region and the heavy chain constant region, and a peptide chain composed of the heavy chain variable region and the light chain constant region. For clarity, in a crossover Fab molecule wherein the variable regions of the Fab light chain and the Fab heavy chain are exchanged, the peptide chain comprising the heavy chain constant region is referred to herein as the "heavy chain" of the crossover Fab molecule. Conversely, in a crossover Fab molecule wherein the constant regions of the Fab light chain and the Fab heavy chain are exchanged, the peptide chain comprising the heavy chain variable region is referred to herein as the "heavy chain" of the crossover Fab molecule.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five types, called α (IgA), δ (IgD), ε (IgE), γ (IgG), or µ (IgM), some of which may be further divided into subtypes, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ), based on the amino acid sequence of its constant domain. An immunoglobulin essentially consists of two Fab molecules and an Fc domain, linked via the immunoglobulin hinge region.

The term "antibody" herein is used in the broadest sense and encompasses various antibody structures, including but not limited to monoclonal antibodies, polyclonal antibodies, and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Plückthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046. Diabodies are antibody fragments with two antigen-binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Particularly, an antigen binding domain comprises an antibody light chain variable region (VL) and an antibody heavy chain variable region (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term "hypervariable region" or "HVR", as used herein, refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (L1, L2, L3). HVRs generally comprise amino acid residues from the hypervariable loops and/or from the complementarity determining regions (CDRs), the latter being of highest sequence variability and/or involved in antigen recognition. With the exception of CDR1 in VH, CDRs generally comprise the amino acid residues that form the hypervariable loops. Hypervariable regions (HVRs) are also referred to as "complementarity determining regions" (CDRs), and these terms are used herein interchangeably in reference to portions of the variable region that form the antigen binding regions. This particular region has been described by Kabat et al., U.S. Dept. of Health and Human Services, Sequences of Proteins of Immunological Interest (1983) and by Chothia et al., J Mol Biol 196:901-917 (1987), where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table 1 as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE 1. CDR Definitions¹**

| **CDR** | **Kabat** | **Chothia** | **AbM²** |
|---|---|---|---|
| V_{H} CDR1 | 31-35 | 26-32 | 26-35 |
| V_{H} CDR2 | 50-65 | 52-58 | 50-58 |
| V_{H} CDR3 | 95-102 | 95-102 | 95-102 |
| V_{L} CDR1 | 24-34 | 26-32 | 24-34 |
| V_{L} CDR2 | 50-56 | 50-52 | 50-56 |
| V_{L} CDR3 | 89-97 | 91-96 | 89-97 |

| | | | |
|---|---|---|---|
| ¹ Numbering of all CDR definitions in Table 1 is according to the numbering conventions set forth by Kabat et al. (see below). ² "AbM" with a lowercase "b" as used in Table 1 refers to the CDRs as defined by Oxford Molecular's "AbM" antibody modeling software. | | | |

Kabat et al. also defined a numbering system for variable region sequences that is applicable to any antibody. One of ordinary skill in the art can unambiguously assign this system of "Kabat numbering" to any variable region sequence, without reliance on any experimental data beyond the sequence itself. As used herein, "Kabat numbering" refers to the numbering system set forth by Kabat et al., U.S. Dept. of Health and Human Services, "Sequence of Proteins of Immunological Interest" (1983). Unless otherwise specified, references to the numbering of specific amino acid residue positions in an antibody variable region are according to the Kabat numbering system.

The polypeptide sequences of the sequence listing (i.e., SEQ ID NOs 1, 3, 5, 7, 9, 11, 13, 15 etc.) are not numbered according to the Kabat numbering system. However, it is well within the ordinary skill of one in the art to convert the numbering of the sequences of the Sequence Listing to Kabat numbering.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The "class" of an antibody or immunoglobulin refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991. A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g. antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

The term "effector functions" refers to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

As used herein, the terms "engineer, engineered, engineering", are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor, or increased association with another peptide. Amino acid sequence deletions and insertions include amino- and/or carboxy-terminal deletions and insertions of amino acids. Particular amino acid mutations are amino acid substitutions. For the purpose of altering e.g. the binding characteristics of an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc domain to glycine can be indicated as 329G, G329, G₃₂₉, P329G, or Pro329Gly.

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide disclosed herein may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the present disclosure, as are native or recombinant polypeptides which have been separated, fractionated, or partially or substantially purified by any suitable technique.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "polynucleotide" refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By "isolated" nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present disclosure. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include *in vivo* or *in vitro* RNA transcripts disclosed herein, as well as positive and negative strand forms, and double-stranded forms. Isolated polynucleotides or nucleic acids disclosed herein further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence disclosed herein, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence disclosed herein can be determined conventionally using known computer programs, such as the ones discussed above for polypeptides (e.g. ALIGN-2).

The term "expression cassette" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment.

Typically, the recombinant expression cassette portion of an expression vector includes, among other sequences, a nucleic acid sequence to be transcribed and a promoter. In certain embodiments, the expression cassette disclosed herein comprises polynucleotide sequences that encode bispecific antigen binding molecules disclosed herein or fragments thereof.

The term "vector" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector disclosed herein comprises an expression cassette.

Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector disclosed herein comprises an expression cassette that comprises polynucleotide sequences that encode bispecific antigen binding molecules disclosed herein or fragments thereof.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages.

Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the bispecific antigen binding molecules disclosed herein. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

An "activating Fc receptor" is an Fc receptor that following engagement by an Fc domain of an antibody elicits signaling events that stimulate the receptor-bearing cell to perform effector functions. Human activating Fc receptors include FcyRIIIa (CD16a), FcyRI (CD64), FcyRIIa (CD32), and FcaRI (CD89).

Antibody-dependent cell-mediated cytotoxicity (ADCC) is an immune mechanism leading to the lysis of antibody-coated target cells by immune effector cells. The target cells are cells to which antibodies or derivatives thereof comprising an Fc region specifically bind, generally via the protein part that is N-terminal to the Fc region. As used herein, the term "reduced ADCC" is defined as either a reduction in the number of target cells that are lysed in a given time, at a given concentration of antibody in the medium surrounding the target cells, by the mechanism of ADCC defined above, and/or an increase in the concentration of antibody in the medium surrounding the target cells, required to achieve the lysis of a given number of target cells in a given time, by the mechanism of ADCC. The reduction in ADCC is relative to the ADCC mediated by the same antibody produced by the same type of host cells, using the same standard production, purification, formulation and storage methods (which are known to those skilled in the art), but that has not been engineered. For example the reduction in ADCC mediated by an antibody comprising in its Fc domain an amino acid substitution that reduces ADCC, is relative to the ADCC mediated by the same antibody without this amino acid substitution in the Fc domain. Suitable assays to measure ADCC are well known in the art (see e.g. PCT publication no. WO 2006/082515 or PCT publication no. WO 2012/130831).

An "effective amount" of an agent refers to the amount that is necessary to result in a physiological change in the cell or tissue to which it is administered.

A "therapeutically effective amount" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

An "individual" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Particularly, the individual or subject is a human.

The term "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, T cell activating bispecific antigen binding molecules disclosed herein are used to delay development of a disease or to slow the progression of a disease.

The term "package insert" is used to refer to instructions customarily included in commercial packages of therapeutic products, that contain information about the indications, usage, dosage, administration, combination therapy, contraindications and/or warnings concerning the use of such therapeutic products.

### Detailed Description of the Embodiments

Provided herein is a T cell activating bispecific antigen binding molecule comprising a first and a second antigen binding moiety, one of which is a Fab molecule capable of specific binding to an activating T cell antigen and the other one of which is a Fab molecule capable of specific binding to a target cell antigen, and an Fc domain composed of a first and a second subunit capable of stable association;
wherein the first antigen binding moiety is
(a) a single chain Fab molecule wherein the Fab light chain and the Fab heavy chain are connected by a peptide linker, or
(b) a crossover Fab molecule wherein either the variable or the constant regions of the Fab light chain and the Fab heavy chain are exchanged.

### T cell activating bispecific antigen binding molecule formats

The components of the T cell activating bispecific antigen binding molecule can be fused to each other in a variety of configurations. Exemplary configurations are depicted in Figure 1.

In some embodiments, the second antigen binding moiety is fused at the C-terminus of the Fab heavy chain to the N-terminus of the first or the second subunit of the Fc domain.

In a particular such embodiment, the first antigen binding moiety is fused at the C-terminus of the Fab heavy chain to the N-terminus of the Fab heavy chain of the second antigen binding moiety. In a specific such embodiment, the T cell activating bispecific antigen binding molecule essentially consists of a first and a second antigen binding moiety, an Fc domain composed of a first and a second subunit, and optionally one or more peptide linkers, wherein the first antigen binding moiety is fused at the C-terminus of the Fab heavy chain to the N-terminus of the Fab heavy chain of the second antigen binding moiety, and the second antigen binding moiety is fused at the C-terminus of the Fab heavy chain to the N-terminus of the first or the second subunit of the Fc domain. In an even more specific embodiment, the first antigen binding moiety is a single chain Fab molecule. Alternatively, in a particular embodiment, the first antigen binding moiety is a crossover Fab molecule. Optionally, if the first antigen binding moiety is a crossover Fab molecule, the Fab light chain of the first antigen binding moiety and the Fab light chain of the second antigen binding moiety may additionally be fused to each other.

In an alternative such embodiment, the first antigen binding moiety is fused at the C-terminus of the Fab heavy chain to the N-terminus of the first or second subunit of the Fc domain. In a specific such embodiment, the T cell activating bispecific antigen binding molecule essentially consists of a first and a second antigen binding moiety, an Fc domain composed of a first and a second subunit, and optionally one or more peptide linkers, wherein the first and the second antigen binding moiety are each fused at the C-terminus of the Fab heavy chain to the N-terminus of one of the subunits of the Fc domain. In an even more specific embodiment, the first antigen binding moiety is a single chain Fab molecule. Alternatively, in a particular embodiment, the first antigen binding moiety is a crossover Fab molecule.

In yet another such embodiment, the second antigen binding moiety is fused at the C-terminus of the Fab light chain to the N-terminus of the Fab light chain of the first antigen binding moiety. In a specific such embodiment, the T cell activating bispecific antigen binding molecule essentially consists of a first and a second antigen binding moiety, an Fc domain composed of a first and a second subunit, and optionally one or more peptide linkers, wherein the first antigen binding moiety is fused at the N-terminus of the Fab light chain to the C-terminus of the Fab light chain of the second antigen binding moiety, and the second antigen binding moiety is fused at the C-terminus of the Fab heavy chain to the N-terminus of the first or the second subunit of the Fc domain. In an even more specific embodiment, the first antigen binding moiety is a crossover Fab molecule.

In other embodiments, the first antigen binding moiety is fused at the C-terminus of the Fab heavy chain to the N-terminus of the first or second subunit of the Fc domain.
In a particular such embodiment, the second antigen binding moiety is fused at the C-terminus of the Fab heavy chain to the N-terminus of the Fab heavy chain of the first antigen binding moiety. In a specific such embodiment, the T cell activating bispecific antigen binding molecule essentially consists of a first and a second antigen binding moiety, an Fc domain composed of a first and a second subunit, and optionally one or more peptide linkers, wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first or the second subunit of the Fc domain. In an even more specific embodiment, the first antigen binding moiety is a crossover Fab molecule. Optionally, the Fab light chain of the first antigen binding moiety and the Fab light chain of the second antigen binding moiety may additionally be fused to each other.

In particular of these embodiments, the first antigen binding moiety is capable of specific binding to an activating T cell antigen. In other embodiments, the first antigen binding moiety is capable of specific binding to a target cell antigen.
The antigen binding moieties may be fused to the Fc domain or to each other directly or through a peptide linker, comprising one or more amino acids, typically about 2-20 amino acids. Peptide linkers are known in the art and are described herein. Suitable, non-immunogenic peptide linkers include, for example, (G₄S)ₙ, (SG₄)ₙ, (G₄S)ₙ or G₄(SG₄)ₙ peptide linkers. "n" is generally a number between 1 and 10, typically between 2 and 4. A particularly suitable peptide linker for fusing the Fab light chains of the first and the second antigen binding moiety to each other is (G₄S)₂. An exemplary peptide linker suitable for connecting the Fab heavy chains of the first and the second antigen binding moiety is EPKSC(D)-(G₄S)₂ (SEQ ID NOs 150 and 151). Additionally, linkers may comprise (a portion of) an immunoglobulin hinge region. Particularly where an antigen binding moiety is fused to the N-terminus of an Fc domain subunit, it may be fused via an immunoglobulin hinge region or a portion thereof, with or without an additional peptide linker.
A T cell activating bispecific antigen binding molecule with a single antigen binding moiety capable of specific binding to a target cell antigen (for example as shown in Figure 1A, 1B, 1D, IE, 1H, 1I, 1K or 1M) is useful, particularly in cases where internalization of the target cell antigen is to be expected following binding of a high affinity antigen binding moiety. In such cases, the presence of more than one antigen binding moiety specific for the target cell antigen may enhance internalization of the target cell antigen, thereby reducing its availablity.

In many other cases, however, it will be advantageous to have a T cell activating bispecific antigen binding molecule comprising two or more antigen binding moieties specific for a target cell antigen (see examples in shown in Figure 1C, IF, 1G, 1J or 1L), for example to optimize targeting to the target site or to allow crosslinking of target cell antigens.

Accordingly, in certain embodiments, the T cell activating bispecific antigen binding molecule disclosed herein further comprises a third antigen binding moiety which is a Fab molecule capable of specific binding to a target cell antigen. In one embodiment, the third antigen binding moiety is capable of specific binding to the same target cell antigen as the first or second antigen binding moiety. In a particular embodiment, the first antigen binding moiety is capable of specific binding to an activating T cell antigen, and the second and third antigen binding moieties are capable of specific binding to a target cell antigen.

In one embodiment, the third antigen binding moiety is fused at the C-terminus of the Fab heavy chain to the N-terminus of the first or second subunit of the Fc domain. In a particular embodiment, the second and the third antigen binding moiety are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety. In one such embodiment the first antigen binding moiety is a single chain Fab molecule. In a particular such embodiment the first antigen binding moiety is a crossover Fab molecule. Optionally, if the first antigen binding moiety is a crossover Fab molecule, the Fab light chain of the first antigen binding moiety and the Fab light chain of the second antigen binding moiety may additionally be fused to each other.

The second and the third antigen binding moiety may be fused to the Fc domain directly or through a peptide linker. In a particular embodiment the second and the third antigen binding moiety are each fused to the Fc domain through an immunoglobulin hinge region. In a specific embodiment, the immunoglobulin hinge region is a human IgG₁ hinge region. In one embodiment the second and the third antigen binding moiety and the Fc domain are part of an immunoglobulin molecule. In a particular embodiment the immunoglobulin molecule is an IgG class immunoglobulin. In an even more particular embodiment the immunoglobulin is an IgG₁ subclass immunoglobulin. In another embodiment the immunoglobulin is an IgG₄ subclass immunoglobulin. In a further particular embodiment the immunoglobulin is a human immunoglobulin. In other embodiments the immunoglobulin is a chimeric immunoglobulin or a humanized immunoglobulin. In one embodiment, the T cell activating bispecific antigen binding molecule essentially consists of an immunoglobulin molecule capable of specific binding to a target cell antigen, and an antigen binding moiety capable of specific binding to an activating T cell antigen wherein the antigen binding moiety is a single chain Fab molecule or a crossover Fab molecule, particularly a crossover Fab molecule, fused to the N-terminus of one of the immunoglobulin heavy chains, optionally via a peptide linker.
In an alternative embodiment, the first and the third antigen binding moiety are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety. In a specific such embodiment, the T cell activating bispecific antigen binding molecule essentially consists of a first, a second and a third antigen binding moiety, an Fc domain composed of a first and a second subunit, and optionally one or more peptide linkers, wherein the second antigen binding moiety is fused at the C-terminus of the Fab heavy chain to the N-terminus of the Fab heavy chain of the first antigen binding moiety, and the first antigen binding moiety is fused at the C-terminus of the Fab heavy chain to the N-terminus of the first subunit of the Fc domain, and wherein the third antigen binding moiety is fused at the C-terminus of the Fab heavy chain to the N-terminus of the second subunit of the Fc domain. In a particular such embodiment the first antigen binding moiety is a crossover Fab molecule. Optionally, the Fab light chain of the first antigen binding moiety and the Fab light chain of the second antigen binding moiety may additionally be fused to each other.

In some of the T cell activating bispecific antigen binding molecule disclosed herein, the Fab light chain of the first antigen binding moiety and the Fab light chain of the second antigen binding moiety are fused to each other, optionally via a linker peptide. Depending on the configuration of the first and the second antigen binding moiety, the Fab light chain of the first antigen binding moiety may be fused at its C-terminus to the N-terminus of the Fab light chain of the second antigen binding moiety, or the Fab light chain of the second antigen binding moiety may be fused at its C-terminus to the N-terminus of the Fab light chain of the first antigen binding moiety. Fusion of the Fab light chains of the first and the second antigen binding moiety further reduces mispairing of unmatched Fab heavy and light chains, and also reduces the number of plasmids needed for expression of some of the T cell activating bispecific antigen binding molecules disclosed herein.

In certain embodiments the T cell activating bispecific antigen binding molecule comprises a polypeptide wherein a first Fab light chain shares a carboxy-terminal peptide bond with a peptide linker, which in turn shares a carboxy-terminal peptide bond with a first Fab heavy chain, which in turn shares a carboxy-terminal peptide bond with an Fc domain subunit (VL-CL-linker-VH-CH1-CH2-CH2(-CH4)), and a polypeptide wherein a second Fab heavy chain shares a carboxy-terminal peptide bond with an Fc domain subunit (VH-CH1-CH2-CH3(-CH4)). In some embodiments the T cell activating bispecific antigen binding molecule further comprises a second Fab light chain polypeptide (VL-CL). In certain embodiments the polypeptides are covalently linked, e.g., by a disulfide bond.

In some embodiments, the T cell activating bispecific antigen binding molecule comprises a polypeptide wherein a first Fab light chain shares a carboxy-terminal peptide bond with a peptide linker, which in turn shares a carboxy-terminal peptide bond with a first Fab heavy chain, which in turn shares a carboxy-terminal peptide bond with a second Fab heavy chain, which in turn shares a carboxy-terminal peptide bond with an Fc domain subunit (VL-CL-linker-VH-CH1-VH-CH1-CH2-CH3(-CH4)). In one of these embodiments that T cell activating bispecific antigen binding molecule further comprises a second Fab light chain polypeptide (VL-CL). The T cell activating bispecific antigen binding molecule according to these embodiments may further comprise (i) an Fc domain subunit polypeptide (CH2-CH3(-CH4)), or (ii) a polypeptide wherein a third Fab heavy chain shares a carboxy-terminal peptide bond with an Fc domain subunit (VH-CH1-CH2-CH3(-CH4)) and a third Fab light chain polypeptide (VL-CL). In certain embodiments the polypeptides are covalently linked, e.g., by a disulfide bond.

In certain embodiments the T cell activating bispecific antigen binding molecule comprises a polypeptide wherein a first Fab light chain variable region shares a carboxy-terminal peptide bond with a first Fab heavy chain constant region (i.e. a crossover Fab heavy chain, wherein the heavy chain variable region is replaced by a light chain variable region), which in turn shares a carboxy-terminal peptide bond with an Fc domain subunit (VL-CH1-CH2-CH2(-CH4)), and a polypeptide wherein a second Fab heavy chain shares a carboxy-terminal peptide bond with an Fc domain subunit (VH-CH1-CH2-CH3(-CH4)). In some embodiments the T cell activating bispecific antigen binding molecule further comprises a polypeptide wherein a Fab heavy chain variable region shares a carboxy-terminal peptide bond with a Fab light chain constant region (VH-CL) and a Fab light chain polypeptide (VL-CL). In certain embodiments the polypeptides are covalently linked, e.g., by a disulfide bond.

In alternative embodiments the T cell activating bispecific antigen binding molecule comprises a polypeptide wherein a first Fab heavy chain variable region shares a carboxy-terminal peptide bond with a first Fab light chain constant region (i.e. a crossover Fab heavy chain, wherein the heavy chain constant region is replaced by a light chain constant region), which in turn shares a carboxy-terminal peptide bond with an Fc domain subunit (VH-CL-CH2-CH2(-CH4)), and a polypeptide wherein a second Fab heavy chain shares a carboxy-terminal peptide bond with an Fc domain subunit (VH-CH1-CH2-CH3(-CH4)). In some embodiments the T cell activating bispecific antigen binding molecule further comprises a polypeptide wherein a Fab light chain variable region shares a carboxy-terminal peptide bond with a Fab heavy chain constant region (VL-CH1) and a Fab light chain polypeptide (VL-CL). In certain embodiments the polypeptides are covalently linked, e.g., by a disulfide bond.

In some embodiments, the T cell activating bispecific antigen binding molecule comprises a polypeptide wherein a first Fab light chain variable region shares a carboxy-terminal peptide bond with a first Fab heavy chain constant region (i.e. a crossover Fab heavy chain, wherein the heavy chain variable region is replaced by a light chain variable region), which in turn shares a carboxy-terminal peptide bond with a second Fab heavy chain, which in turn shares a carboxy-terminal peptide bond with an Fc domain subunit (VL-CH1-VH-CH1-CH2-CH3(-CH4)). In other embodiments, the T cell activating bispecific antigen binding molecule comprises a polypeptide wherein a first Fab heavy chain variable region shares a carboxy-terminal peptide bond with a first Fab light chain constant region (i.e. a crossover Fab heavy chain, wherein the heavy chain constant region is replaced by a light chain constant region), which in turn shares a carboxy-terminal peptide bond with a second Fab heavy chain, which in turn shares a carboxy-terminal peptide bond with an Fc domain subunit (VH-CL-VH-CH1-CH2-CH3(-CH4)). In still other embodiments, the T cell activating bispecific antigen binding molecule comprises a polypeptide wherein a second Fab heavy chain shares a carboxy-terminal peptide bond with a first Fab light chain variable region which in turn shares a carboxy-terminal peptide bond with a first Fab heavy chain constant region (i.e. a crossover Fab heavy chain, wherein the heavy chain variable region is replaced by a light chain variable region), which in turn shares a carboxy-terminal peptide bond with an Fc domain subunit (VH-CH1-VL-CH1-CH2-CH3(-CH4)). In other embodiments, the T cell activating bispecific antigen binding molecule comprises a polypeptide wherein a second Fab heavy chain shares a carboxy-terminal peptide bond with a first Fab heavy chain variable region which in turn shares a carboxy-terminal peptide bond with a first Fab light chain constant region (i.e. a crossover Fab heavy chain, wherein the heavy chain constant region is replaced by a light chain constant region), which in turn shares a carboxy-terminal peptide bond with an Fc domain subunit (VH-CH1-VH-CL-CH2-CH3(-CH4)).
In some of these embodiments the T cell activating bispecific antigen binding molecule further comprises a crossover Fab light chain polypeptide, wherein a Fab heavy chain variable region shares a carboxy-terminal peptide bond with a Fab light chain constant region (VH-CL), and a Fab light chain polypeptide (VL-CL). In others of these embodiments the T cell activating bispecific antigen binding molecule further comprises a crossover Fab light chain polypeptide, wherein a Fab light chain variable region shares a carboxy-terminal peptide bond with a Fab heavy chain constant region (VL-CH1), and a Fab light chain polypeptide (VL-CL). In still others of these embodiments the T cell activating bispecific antigen binding molecule further comprises a polypeptide wherein a Fab light chain variable region shares a carboxy-terminal peptide bond with a Fab heavy chain constant region which in turn shares a carboxy-terminal peptide bond with a Fab light chain polypeptide (VL-CH1-VL-CL), a polypeptide wherein a Fab heavy chain variable region shares a carboxy-terminal peptide bond with a Fab light chain constant region which in turn shares a carboxy-terminal peptide bond with a Fab light chain polypeptide (VH-CL-VL-CL), a polypeptide wherein a Fab light chain polypeptide shares a carboxy-terminal peptide bond with a Fab light chain variable region which in turn shares a carboxy-terminal peptide bond with a Fab heavy chain constant region (VL-CL-VL-CH1), or a polypeptide wherein a Fab light chain polypeptide shares a carboxy-terminal peptide bond with a Fab heavy chain variable region which in turn shares a carboxy-terminal peptide bond with a Fab light chain constant region (VL-CL-VH-CL).
The T cell activating bispecific antigen binding molecule according to these embodiments may further comprise (i) an Fc domain subunit polypeptide (CH2-CH3(-CH4)), or (ii) a polypeptide wherein a third Fab heavy chain shares a carboxy-terminal peptide bond with an Fc domain subunit (VH-CH1-CH2-CH3(-CH4)) and a third Fab light chain polypeptide (VL-CL). In certain embodiments the polypeptides are covalently linked, e.g., by a disulfide bond.

In one embodiment, the T cell activating bispecific antigen binding molecule comprises a polypeptide wherein a second Fab light chain shares a carboxy-terminal peptide bond with a first Fab light chain variable region which in turn shares a carboxy-terminal peptide bond with a first Fab heavy chain constant region (i.e. a crossover Fab light chain, wherein the light chain constant region is replaced by a heavy chain constant region) (VL-CL-VL-CH1), a polypeptide wherein a second Fab heavy chain shares a carboxy-terminal peptide bond with an Fc domain subunit (VH-CH1-CH2-CH3(-CH4)), and a polypeptide wherein a first Fab heavy chain variable region shares a carboxy-terminal peptide bond with a first Fab light chain constant region (VH-CL). In another embodiment, the T cell activating bispecific antigen binding molecule comprises a polypeptide wherein a second Fab light chain shares a carboxy-terminal peptide bond with a first Fab heavy chain variable region which in turn shares a carboxy-terminal peptide bond with a first Fab light chain constant region (i.e. a crossover Fab light chain, wherein the light chain variable region is replaced by a heavy chain variable region) (VL-CL-VH-CL), a polypeptide wherein a second Fab heavy chain shares a carboxy-terminal peptide bond with an Fc domain subunit (VH-CH1-CH2-CH3(-CH4)), and a polypeptide wherein a first Fab light chain variable region shares a carboxy-terminal peptide bond with a first Fab heavy chain constant region (VL-CH1). The T cell activating bispecific antigen binding molecule according to these embodiments may further comprise (i) an Fc domain subunit polypeptide (CH2-CH3(-CH4)), or (ii) a polypeptide wherein a third Fab heavy chain shares a carboxy-terminal peptide bond with an Fc domain subunit (VH-CH1-CH2-CH3(-CH4)) and a third Fab light chain polypeptide (VL-CL). In certain embodiments the polypeptides are covalently linked, e.g., by a disulfide bond.

According to any of the above embodiments, components of the T cell activating bispecific antigen binding molecule (e.g. antigen binding moiety, Fc domain) may be fused directly or through various linkers, particularly peptide linkers comprising one or more amino acids, typically about 2-20 amino acids, that are described herein or are known in the art. Suitable, non-immunogenic peptide linkers include, for example, (G₄S)ₙ, (SG₄)ₙ, (G₄S)ₙ or G₄(SG₄)ₙ peptide linkers, wherein n is generally a number between 1 and 10, typically between 2 and 4.

### Fc domain

The Fc domain of the T cell activating bispecific antigen binding molecule consists of a pair of polypeptide chains comprising heavy chain domains of an immunoglobulin molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other. In one embodiment the T cell activating bispecific antigen binding molecule disclosed herein comprises not more than one Fc domain.

In one embodiment the Fc domain of the T cell activating bispecific antigen binding molecule is an IgG Fc domain. In a particular embodiment the Fc domain is an IgG₁ Fc domain. In another embodiment the Fc domain is an IgG₄ Fc domain. In a more specific embodiment, the Fc domain is an IgG₄ Fc domain comprising an amino acid substitution at position S228 (EU numbering), particularly the amino acid substitution S228P. This amino acid substitution reduces in vivo Fab arm exchange of IgG₄ antibodies (see Stubenrauch et al., Drug Metabolism and Disposition 38, 84-91 (2010)). In a further particular embodiment the Fc domain is human. An exemplary sequence of a human IgG₁ Fc region is given in SEQ ID NO: 149.

### Fc domain modifications promoting heterodimerization

T cell activating bispecific antigen binding molecules disclosed herein comprise different antigen binding moieties, fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of T cell activating bispecific antigen binding molecules in recombinant production, it will thus be advantageous to introduce in the Fc domain of the T cell activating bispecific antigen binding molecule a modification promoting the association of the desired polypeptides.

Accordingly, in particular embodiments the Fc domain of the T cell activating bispecific antigen binding molecule disclosed herein comprises a modification promoting the association of the first and the second subunit of the Fc domain. The site of most extensive protein-protein interaction between the two subunits of a human IgG Fc domain is in the CH3 domain of the Fc domain. Thus, in one embodiment said modification is in the CH3 domain of the Fc domain.

In a specific embodiment said modification is a so-called "knob-into-hole" modification, comprising a "knob" modification in one of the two subunits of the Fc domain and a "hole" modification in the other one of the two subunits of the Fc domain.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first subunit of the Fc domain of the T cell activating bispecific antigen binding molecule an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

The protuberance and cavity can be made by altering the nucleic acid encoding the polypeptides, e.g. by site-specific mutagenesis, or by peptide synthesis.

In a specific embodiment, in the CH3 domain of the first subunit of the Fc domain the threonine residue at position 366 is replaced with a tryptophan residue (T366W), and in the CH3 domain of the second subunit of the Fc domain the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A).

In yet a further embodiment, in the first subunit of the Fc domain additionally the serine residue at position 354 is replaced with a cysteine residue (S354C), and in the second subunit of the Fc domain additionally the tyrosine residue at position 349 is replaced by a cysteine residue (Y349C). Introduction of these two cysteine residues results in formation of a disulfide bridge between the two subunits of the Fc domain, further stabilizing the dimer (Carter, J Immunol Methods 248, 7-15 (2001)).

In a particular embodiment the antigen binding moiety capable of binding to an activating T cell antigen is fused (optionally via the antigen binding moiety capable of binding to a target cell antigen) to the first subunit of the Fc domain (comprising the "knob" modification). Without wishing to be bound by theory, fusion of the antigen binding moiety capable of binding to an activating T cell antigen to the knob-containing subunit of the Fc domain will (further) minimize the generation of antigen binding molecules comprising two antigen binding moieties capable of binding to an activating T cell antigen (steric clash of two knob-containing polypeptides).

In an alternative embodiment a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable.

### Fc domain modifications reducing Fc receptor binding and/or effector function

The Fc domain confers to the T cell activating bispecific antigen binding molecule favorable pharmacokinetic properties, including a long serum half-life which contributes to good accumulation in the target tissue and a favorable tissue-blood distribution ratio. At the same time it may, however, lead to undesirable targeting of the T cell activating bispecific antigen binding molecule to cells expressing Fc receptors rather than to the preferred antigen-bearing cells. Moreover, the co-activation of Fc receptor signaling pathways may lead to cytokine release which, in combination with the T cell activating properties and the long half-life of the antigen binding molecule, results in excessive activation of cytokine receptors and severe side effects upon systemic administration. Activation of (Fc receptor-bearing) immune cells other than T cells may even reduce efficacy of the T cell activating bispecific antigen binding molecule due to the potential destruction of T cells e.g. by NK cells.

Accordingly, in particular embodiments the Fc domain of the T cell activating bispecific antigen binding molecules disclosed herein exhibits reduced binding affinity to an Fc receptor and/or reduced effector function, as compared to a native IgG₁ Fc domain. In one such embodiment the Fc domain (or the T cell activating bispecific antigen binding molecule comprising said Fc domain) exhibits less than 50%, preferably less than 20%, more preferably less than 10% and most preferably less than 5% of the binding affinity to an Fc receptor, as compared to a native IgG₁ Fc domain (or a T cell activating bispecific antigen binding molecule comprising a native IgG₁ Fc domain), and/or less than 50%, preferably less than 20%, more preferably less than 10% and most preferably less than 5% of the effector function, as compared to a native IgG₁ Fc domain domain (or a T cell activating bispecific antigen binding molecule comprising a native IgG₁ Fc domain). In one embodiment, the Fc domain domain (or the T cell activating bispecific antigen binding molecule comprising said Fc domain) does not substantially bind to an Fc receptor and/or induce effector function. In a particular embodiment the Fc receptor is an Fcγ receptor. In one embodiment the Fc receptor is a human Fc receptor. In one embodiment the Fc receptor is an activating Fc receptor. In a specific embodiment the Fc receptor is an activating human Fcγ receptor, more specifically human FcyRIIIa, FcyRI or FcyRIIa, most specifically human FcyRIIIa. In one embodiment the effector function is one or more selected from the group of CDC, ADCC, ADCP, and cytokine secretion. In a particular embodiment the effector function is ADCC. In one embodiment the Fc domain domain exhibits substantially similar binding affinity to neonatal Fc receptor (FcRn), as compared to a native IgG₁ Fc domain domain. Substantially similar binding to FcRn is achieved when the Fc domain (or the T cell activating bispecific antigen binding molecule comprising said Fc domain) exhibits greater than about 70%, particularly greater than about 80%, more particularly greater than about 90% of the binding affinity of a native IgG₁ Fc domain (or the T cell activating bispecific antigen binding molecule comprising a native IgG₁ Fc domain) to FcRn.

In certain embodiments the Fc domain is engineered to have reduced binding affinity to an Fc receptor and/or reduced effector function, as compared to a non-engineered Fc domain. In particular embodiments, the Fc domain of the T cell activating bispecific antigen binding molecule comprises one or more amino acid mutation that reduces the binding affinity of the Fc domain to an Fc receptor and/or effector function. Typically, the same one or more amino acid mutation is present in each of the two subunits of the Fc domain. In one embodiment the amino acid mutation reduces the binding affinity of the Fc domain to an Fc receptor. In one embodiment the amino acid mutation reduces the binding affinity of the Fc domain to an Fc receptor by at least 2-fold, at least 5-fold, or at least 10-fold. In embodiments where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one embodiment the T cell activating bispecific antigen binding molecule comprising an engineered Fc domain exhibits less than 20%, particularly less than 10%, more particularly less than 5% of the binding affinity to an Fc receptor as compared to a T cell activating bispecific antigen binding molecule comprising a non-engineered Fc domain. In a particular embodiment the Fc receptor is an Fcγ receptor. In some embodiments the Fc receptor is a human Fc receptor. In some embodiments the Fc receptor is an activating Fc receptor. In a specific embodiment the Fc receptor is an activating human Fcγ receptor, more specifically human FcyRIIIa, FcyRI or FcyRIIa, most specifically human FcyRIIIa. Preferably, binding to each of these receptors is reduced. In some embodiments binding affinity to a complement component, specifically binding affinity to C1q, is also reduced. In one embodiment binding affinity to neonatal Fc receptor (FcRn) is not reduced. Substantially similar binding to FcRn, i.e. preservation of the binding affinity of the Fc domain to said receptor, is achieved when the Fc domain (or the T cell activating bispecific antigen binding molecule comprising said Fc domain) exhibits greater than about 70% of the binding affinity of a non-engineered form of the Fc domain (or the T cell activating bispecific antigen binding molecule comprising said non-engineered form of the Fc domain) to FcRn. The Fc domain, or T cell activating bispecific antigen binding molecules disclosed herein comprising said Fc domain, may exhibit greater than about 80% and even greater than about 90% of such affinity. In certain embodiments the Fc domain of the T cell activating bispecific antigen binding molecule is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced crosslinking of target-bound antibodies, reduced dendritic cell maturation, or reduced T cell priming. In one embodiment the reduced effector function is one or more selected from the group of reduced CDC, reduced ADCC, reduced ADCP, and reduced cytokine secretion. In a particular embodiment the reduced effector function is reduced ADCC. In one embodiment the reduced ADCC is less than 20% of the ADCC induced by a non-engineered Fc domain (or a T cell activating bispecific antigen binding molecule comprising a non-engineered Fc domain).

In one embodiment the amino acid mutation that reduces the binding affinity of the Fc domain to an Fc receptor and/or effector function is an amino acid substitution. In one embodiment the Fc domain comprises an amino acid substitution at a position selected from the group of E233, L234, L235, N297, P331 and P329. In a more specific embodiment the Fc domain comprises an amino acid substitution at a position selected from the group of L234, L235 and P329. In some embodiments the Fc domain comprises the amino acid substitutions L234A and L235A. In one such embodiment, the Fc domain is an IgG₁ Fc domain, particularly a human IgG₁ Fc domain. In one embodiment the Fc domain comprises an amino acid substitution at position P329. In a more specific embodiment the amino acid substitution is P329A or P329G, particularly P329G. In one embodiment the Fc domain comprises an amino acid substitution at position P329 and a further amino acid substitution at a position selected from E233, L234, L235, N297 and P331. In a more specific embodiment the further amino acid substitution is E233P, L234A, L235A, L235E, N297A, N297D or P331S. In particular embodiments the Fc domain comprises amino acid substitutions at positions P329, L234 and L235. In more particular embodiments the Fc domain comprises the amino acid mutations L234A, L235A and P329G ("P329G LALA"). In one such embodiment, the Fc domain is an IgG₁ Fc domain, particularly a human IgG₁ Fc domain. The "P329G LALA" combination of amino acid substitutions almost completely abolishes Fcγ receptor binding of a human IgG₁ Fc domain, as described in PCT publication no.WO 2012/130831. WO 2012/130831 also describes methods of preparing such mutant Fc domains and methods for determining its properties such as Fc receptor binding or effector functions. IgG₄ antibodies exhibit reduced binding affinity to Fc receptors and reduced effector functions as compared to IgG₁ antibodies. Hence, in some embodiments the Fc domain of the T cell activating bispecific antigen binding molecules disclosed herein is an IgG₄ Fc domain, particularly a human IgG₄ Fc domain. In one embodiment the IgG₄ Fc domain comprises amino acid substitutions at position S228, specifically the amino acid substitution S228P. To further reduce its binding affinity to an Fc receptor and/or its effector function, in one embodiment the IgG₄ Fc domain comprises an amino acid substitution at position L235, specifically the amino acid substitution L235E. In another embodiment, the IgG₄ Fc domain comprises an amino acid substitution at position P329, specifically the amino acid substitution P329G. In a particular embodiment, the IgG₄ Fc domain comprises amino acid substitutions at positions S228, L235 and P329, specifically amino acid substitutions S228P, L235E and P329G. Such IgG₄ Fc domain mutants and their Fcγ receptor binding properties are described in PCT publication no. WO 2012/130831.

In a particular embodiment the Fc domain exhibiting reduced binding affinity to an Fc receptor and/or reduced effector function, as compared to a native IgG₁ Fc domain, is a human IgG₁ Fc domain comprising the amino acid substitutions L234A, L235A and optionally P329G, or a human IgG₄ Fc domain comprising the amino acid substitutions S228P, L235E and optionally P329G.

In certain embodiments N-glycosylation of the Fc domain has been eliminated. In one such embodiment the Fc domain comprises an amino acid mutation at position N297, particularly an amino acid substitution replacing asparagine by alanine (N297A) or aspartic acid (N297D).

In addition to the Fc domains described hereinabove and in PCT publication no. WO 2012/130831, Fc domains with reduced Fc receptor binding and/or effector function also include those with substitution of one or more of Fc domain residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. A suitable such binding assay is described herein. Alternatively, binding affinity of Fc domains or cell activating bispecific antigen binding molecules comprising an Fc domain for Fc receptors may be evaluated using cell lines known to express particular Fc receptors, such as human NK cells expressing FcγIIIa receptor.

Effector function of an Fc domain, or a T cell activating bispecific antigen binding molecule comprising an Fc domain, can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96® non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

In some embodiments, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the T cell activating bispecific antigen binding molecule is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

### Antigen Binding Moieties

The antigen binding molecule disclosed herein is bispecific, i.e. it comprises at least two antigen binding moieties capable of specific binding to two distinct antigenic determinants. According to the present diclosure, the antigen binding moieties are Fab molecules (i.e. antigen binding domains composed of a heavy and a light chain, each comprising a variable and a constant region). In one embodiment said Fab molecules are human. In another embodiment said Fab molecules are humanized. In yet another embodiment said Fab molecules comprise human heavy and light chain constant regions.

At least one of the antigen binding moieties is a single chain Fab molecule or a crossover Fab molecule. Such modifications prevent mispairing of heavy and light chains from different Fab molecules, thereby improving the yield and purity of the T cell activating bispecific antigen binding molecule disclosed herein in recombinant production. In a particular single chain Fab molecule useful for the T cell activating bispecific antigen binding molecule disclosed herein, the C-terminus of the Fab light chain is connected to the N-terminus of the Fab heavy chain by a peptide linker. The peptide linker allows arrangement of the Fab heavy and light chain to form a functional antigen binding moiety. Peptide linkers suitable for connecting the Fab heavy and light chain include, for example, (G₄S)₆-GG (SEQ ID NO: 152) or (SG₃)₂-(SEG₃)₄-(SG₃)-SG (SEQ ID NO: 153). In a particular crossover Fab molecule useful for the T cell activating bispecific antigen binding molecule disclosed herein, the constant regions of the Fab light chain and the Fab heavy chain are exchanged. In another crossover Fab molecule useful for the T cell activating bispecific antigen binding molecule disclosed herein, the variable regions of the Fab light chain and the Fab heavy chain are exchanged.

In a particular embodiment, the T cell activating bispecific antigen binding molecule is capable of simultaneous binding to a target cell antigen, particularly a tumor cell antigen, and an activating T cell antigen. In one embodiment, the T cell activating bispecific antigen binding molecule is capable of crosslinking a T cell and a target cell by simultaneous binding to a target cell antigen and an activating T cell antigen. In an even more particular embodiment, such simultaneous binding results in lysis of the target cell, particularly a tumor cell. In one embodiment, such simultaneous binding results in activation of the T cell. In other embodiments, such simultaneous binding results in a cellular response of a T lymphocyte, particularly a cytotoxic T lymphocyte, selected from the group of: proliferation, differentiation, cytokine secretion, cytotoxic effector molecule release, cytotoxic activity, and expression of activation markers. In one embodiment, binding of the T cell activating bispecific antigen binding molecule to the activating T cell antigen without simultaneous binding to the target cell antigen does not result in T cell activation.

In one embodiment, the T cell activating bispecific antigen binding molecule is capable of redirecting cytotoxic activity of a T cell to a target cell. In a particular embodiment, said re-direction is independent of MHC-mediated peptide antigen presentation by the target cell and and/or specificity of the T cell.

Particularly, a T cell according to any of the embodiments of the present disclosure is a cytotoxic T cell. In some embodiments the T cell is a CD4⁺ or a CD8⁺ T cell, particularly a CD8⁺ T cell.

### Activating T cell antigen binding moiety

The T cell activating bispecific antigen binding molecule disclosed herein comprises at least one antigen binding moiety capable of binding to an activating T cell antigen (also referred to herein as an "activating T cell antigen binding moiety"). In a particular embodiment, the T cell activating bispecific antigen binding molecule comprises not more than one antigen binding moiety capable of specific binding to an activating T cell antigen. In one embodiment the T cell activating bispecific antigen binding molecule provides monovalent binding to the activating T cell antigen. The activating T cell antigen binding moiety can either be a conventional Fab molecule or a modified Fab molecule, i.e. a single chain or crossover Fab molecule. In embodiments where there is more than one antigen binding moiety capable of specific binding to a target cell antigen comprised in the T cell activating bispecific antigen binding molecule, the antigen binding moiety capable of specific binding to an activating T cell antigen preferably is a modified Fab molecule.

In a particular embodiment the activating T cell antigen is CD3, particularly human CD3 (SEQ ID NO: 265) or cynomolgus CD3 (SEQ ID NO: 266), most particularly human CD3. In a particular embodiment the activating T cell antigen binding moiety is cross-reactive for (i.e. specifically binds to) human and cynomolgus CD3. In some embodiments, the activating T cell antigen is the epsilon subunit of CD3.

In one embodiment, the activating T cell antigen binding moiety can compete with monoclonal antibody H2C (described in PCT publication no. WO2008/119567) for binding an epitope of CD3. In another embodiment, the activating T cell antigen binding moiety can compete with monoclonal antibody V9 (described in Rodrigues et al., Int J Cancer Suppl 7, 45-50 (1992) and US patent no. 6,054,297) for binding an epitope of CD3. In yet another embodiment, the activating T cell antigen binding moiety can compete with monoclonal antibody FN18 (described in Nooij et al., Eur J Immunol 19, 981-984 (1986)) for binding an epitope of CD3. In a particular embodiment, the activating T cell antigen binding moiety can compete with monoclonal antibody SP34 (described in Pessano et al., EMBO J 4, 337-340 (1985)) for binding an epitope of CD3. In one embodiment, the activating T cell antigen binding moiety binds to the same epitope of CD3 as monoclonal antibody SP34. In one embodiment, the activating T cell antigen binding moiety comprises the heavy chain CDR1 of SEQ ID NO: 163, the heavy chain CDR2 of SEQ ID NO: 165, the heavy chain CDR3 of SEQ ID NO: 167, the light chain CDR1 of SEQ ID NO: 171, the light chain CDR2 of SEQ ID NO: 173, and the light chain CDR3 of SEQ ID NO: 175. In a further embodiment, the activating T cell antigen binding moiety comprises a heavy chain variable region sequence that is at least about 80%, 85%, 90%, 95%, 96%, 97%, 98%, 99% or 100% identical to SEQ ID NO: 169 and a light chain variable region sequence that is at least about 80%, 85%, 90%, 95%, 96%, 97%, 98%, 99% or 100% identical to SEQ ID NO: 177, or variants thereof that retain functionality.

In a particular embodiment, the activating T cell antigen binding moiety comprises the heavy chain CDR1 of SEQ ID NO: 249, the heavy chain CDR2 of SEQ ID NO: 251, the heavy chain CDR3 of SEQ ID NO: 253, the light chain CDR1 of SEQ ID NO: 257, the light chain CDR2 of SEQ ID NO: 259, and the light chain CDR3 of SEQ ID NO: 261. In one embodiment, the activating T cell antigen binding moiety can compete for binding an epitope of CD3 with an antigen binding moiety comprising the heavy chain CDR1 of SEQ ID NO: 249, the heavy chain CDR2 of SEQ ID NO: 251, the heavy chain CDR3 of SEQ ID NO: 253, the light chain CDR1 of SEQ ID NO: 257, the light chain CDR2 of SEQ ID NO: 259, and the light chain CDR3 of SEQ ID NO: 261. In one embodiment, the activating T cell antigen binding moiety binds to the same epitope of CD3 as an antigen binding moiety comprising the heavy chain CDR1 of SEQ ID NO: 249, the heavy chain CDR2 of SEQ ID NO: 251, the heavy chain CDR3 of SEQ ID NO: 253, the light chain CDR1 of SEQ ID NO: 257, the light chain CDR2 of SEQ ID NO: 259, and the light chain CDR3 of SEQ ID NO: 261. In a further embodiment, the activating T cell antigen binding moiety comprises a heavy chain variable region sequence that is at least about 80%, 85%, 90%, 95%, 96%, 97%, 98%, 99% or 100% identical to SEQ ID NO: 255 and a light chain variable region sequence that is at least about 80%, 85%, 90%, 95%, 96%, 97%, 98%, 99% or 100% identical to SEQ ID NO: 263, or variants thereof that retain functionality. In one embodiment, the activating T cell antigen binding moiety can compete for binding an epitope of CD3 with an antigen binding moiety comprising the heavy chain variable region sequence of SEQ ID NO: 255 and the light chain variable region sequence of SEQ ID NO: 263. In one embodiment, the activating T cell antigen binding moiety binds to the same epitope of CD3 as an antigen binding moiety comprising the heavy chain variable region sequence of SEQ ID NO: 255 and the light chain variable region sequence of SEQ ID NO: 263. In another embodiment, the activating T cell antigen binding moiety comprises a humanized version of the heavy chain variable region sequence of SEQ ID NO: 255 and a humanized version of the light chain variable region sequence of SEQ ID NO: 263. In one embodiment, the activating T cell antigen binding moiety comprises the heavy chain CDR1 of SEQ ID NO: 249, the heavy chain CDR2 of SEQ ID NO: 251, the heavy chain CDR3 of SEQ ID NO: 253, the light chain CDR1 of SEQ ID NO: 257, the light chain CDR2 of SEQ ID NO: 259, the light chain CDR3 of SEQ ID NO: 261, and human heavy and light chain variable region framework sequences.

### Target cell antigen binding moiety

The T cell activating bispecific antigen binding molecule disclosed herein comprises at least one antigen binding moiety capable of binding to a target cell antigen (also referred to herein as an "target cell antigen binding moiety"). In certain embodiments, the T cell activating bispecific antigen binding molecule comprises two antigen binding moieties capable of binding to a target cell antigen. In a particular such embodiment, each of these antigen binding moieties specifically binds to the same antigenic determinant. In one embodiment, the T cell activating bispecific antigen binding molecule comprises an immunoglobulin molecule capable of specific binding to a target cell antigen. In one embodiment the T cell activating bispecific antigen binding molecule comprises not more than two antigen binding moieties capable of binding to a target cell antigen. The target cell antigen binding moiety is generally a Fab molecule that binds to a specific antigenic determinant and is able to direct the T cell activating bispecific antigen binding molecule to a target site, for example to a specific type of tumor cell that bears the antigenic determinant.

In certain embodiments the target cell antigen binding moiety is directed to an antigen associated with a pathological condition, such as an antigen presented on a tumor cell or on a virus-infected cell. Suitable antigens are cell surface antigens, for example, but not limited to, cell surface receptors. In particular embodiments the antigen is a human antigen. In a specific embodiment the target cell antigen is selected from the group of Fibroblast Activation Protein (FAP), Melanoma-associated Chondroitin Sulfate Proteoglycan (MCSP), Epidermal Growth Factor Receptor (EGFR), Carcinoembryonic Antigen (CEA),CD19, CD20 and CD33.

In particular embodiments the T cell activating bispecific antigen binding molecule comprises at least one antigen binding moiety that is specific for Melanoma-associated Chondroitin Sulfate Proteoglycan (MCSP). In one embodiment the T cell activating bispecific antigen binding molecule comprises at least one, typically two or more antigen binding moieties that can compete with monoclonal antibody LC007 (see SEQ ID NOs 75 and 83, and European patent application publication no. EP 2748195) for binding to an epitope of MCSP. In one embodiment, the antigen binding moiety that is specific for MCSP comprises the heavy chain CDR1 of SEQ ID NO: 69, the heavy chain CDR2 of SEQ ID NO: 71, the heavy chain CDR3 of SEQ ID NO: 73, the light chain CDR1 of SEQ ID NO: 77, the light chain CDR2 of SEQ ID NO: 79, and the light chain CDR3 of SEQ ID NO: 81. In a further embodiment, the antigen binding moiety that is specific for MCSP comprises a heavy chain variable region sequence that is at least about 80%, 85%, 90%, 95%, 96%, 97%, 98%, 99% or 100% identical to SEQ ID NO: 75 and a light chain variable region sequence that is at least about 80%, 85%, 90%, 95%, 96%, 97%, 98%, 99% or 100% identical to SEQ ID NO: 83, or variants thereof that retain functionality. In particular embodiments the T cell activating bispecific antigen binding molecule comprises at least one, typically two or more antigen binding moieties that can compete with monoclonal antibody M4-3 ML2 (see SEQ ID NOs 239 and 247, and European patent application publication no. EP 2748195) for binding to an epitope of MCSP. In one embodiment, the antigen binding moiety that is specific for MCSP binds to the same epitope of MCSP as monoclonal antibody M4-3 ML2. In one embodiment, the antigen binding moiety that is specific for MCSP comprises the heavy chain CDR1 of SEQ ID NO: 233, the heavy chain CDR2 of SEQ ID NO: 235, the heavy chain CDR3 of SEQ ID NO: 237, the light chain CDR1 of SEQ ID NO: 241, the light chain CDR2 of SEQ ID NO: 243, and the light chain CDR3 of SEQ ID NO: 245. In a further embodiment, the antigen binding moiety that is specific for MCSP comprises a heavy chain variable region sequence that is at least about 80%, 85%, 90%, 95%, 96%, 97%, 98%, 99% or 100%, particularly about 98%, 99% or 100%, identical to SEQ ID NO: 239 and a light chain variable region sequence that is at least about 80%, 85%, 90%, 95%, 96%, 97%, 98%, 99% or 100%, particularly about 98%, 99% or 100%, identical to SEQ ID NO: 247, or variants thereof that retain functionality. In one embodiment, the antigen binding moiety that is specific for MCSP comprises the heavy and light chain variable region sequences of an affinity matured version of monoclonal antibody M4-3 ML2. In one embodiment, the antigen binding moiety that is specific for MCSP comprises the heavy chain variable region sequence of SEQ ID NO: 239 with one, two, three, four, five, six or seven, particularly two, three, four or five, amino acid substitutions; and the light chain variable region sequence of SEQ ID NO: 247 with one, two, three, four, five, six or seven, particularly two, three, four or five, amino acid substitutions. Any amino acid residue within the variable region sequences may be substituted by a different amino acid, including amino acid residues within the CDR regions, provided that binding to MCSP, particularly human MCSP, is preserved. Preferred variants are those having a binding affinity for MCSP at least equal (or stronger) to the binding affinity of the antigen binding moiety comprising the unsubstituted variable region sequences.

In one embodiment the T cell activating bispecific antigen binding molecule comprises the polypeptide sequence of SEQ ID NO: 1, the polypeptide sequence of SEQ ID NO: 3 and the polypeptide sequence of SEQ ID NO: 5, or variants thereof that retain functionality. In a further embodiment the T cell activating bispecific antigen binding molecule comprises the polypeptide sequence of SEQ ID NO: 7, the polypeptide sequence of SEQ ID NO: 9 and the polypeptide sequence of SEQ ID NO: 11, or variants thereof that retain functionality. In yet another embodiment the T cell activating bispecific antigen binding molecule comprises the polypeptide sequence of SEQ ID NO: 13, the polypeptide sequence of SEQ ID NO: 15 and the polypeptide sequence of SEQ ID NO: 5, or variants thereof that retain functionality. In yet another embodiment the T cell activating bispecific antigen binding molecule comprises the polypeptide sequence of SEQ ID NO: 17, the polypeptide sequence of SEQ ID NO: 19 and the polypeptide sequence of SEQ ID NO: 5, or variants thereof that retain functionality. In another embodiment the T cell activating bispecific antigen binding molecule comprises the polypeptide sequence of SEQ ID NO: 21, the polypeptide sequence of SEQ ID NO: 23 and the polypeptide sequence of SEQ ID NO: 5, or variants thereof that retain functionality. In still another embodiment the T cell activating bispecific antigen binding molecule comprises the polypeptide sequence of SEQ ID NO: 25, the polypeptide sequence of SEQ ID NO: 27 and the polypeptide sequence of SEQ ID NO: 5, or variants thereof that retain functionality. In another embodiment the T cell activating bispecific antigen binding molecule comprises the polypeptide sequence of SEQ ID NO: 29, the polypeptide sequence of SEQ ID NO: 31, the polypeptide sequence of SEQ ID NO: 33, and the polypeptide sequence of SEQ ID NO: 5, or variants thereof that retain functionality. In another embodiment the T cell activating bispecific antigen binding molecule comprises the polypeptide sequence of SEQ ID NO: 29, the polypeptide sequence of SEQ ID NO: 3, the polypeptide sequence of SEQ ID NO: 33, and the polypeptide sequence of SEQ ID NO: 5, or variants thereof that retain functionality. In another embodiment the T cell activating bispecific antigen binding molecule comprises the polypeptide sequence of SEQ ID NO: 35, the polypeptide sequence of SEQ ID NO: 3, the polypeptide sequence of SEQ ID NO: 37, and the polypeptide sequence of SEQ ID NO: 5, or variants thereof that retain functionality. In another embodiment the T cell activating bispecific antigen binding molecule comprises the polypeptide sequence of SEQ ID NO: 39, the polypeptide sequence of SEQ ID NO: 3, the polypeptide sequence of SEQ ID NO: 41, and the polypeptide sequence of SEQ ID NO: 5, or variants thereof that retain functionality. In yet another embodiment the T cell activating bispecific antigen binding molecule comprises the polypeptide sequence of SEQ ID NO: 29, the polypeptide sequence of SEQ ID NO: 3, the polypeptide sequence of SEQ ID NO: 5 and the polypeptide sequence of SEQ ID NO: 179, or variants thereof that retain functionality. In one embodiment the T cell activating bispecific antigen binding molecule comprises the polypeptide sequence of SEQ ID NO: 5, the polypeptide sequence of SEQ ID NO: 29, the polypeptide sequence of SEQ ID NO: 33 and the polypeptide sequence of SEQ ID NO: 181, or variants thereof that retain functionality. In one embodiment the T cell activating bispecific antigen binding molecule comprises the polypeptide sequence of SEQ ID NO: 5, the polypeptide sequence of SEQ ID NO: 23, the polypeptide sequence of SEQ ID NO: 183 and the polypeptide sequence of SEQ ID NO: 185, or variants thereof that retain functionality. In one embodiment the T cell activating bispecific antigen binding molecule comprises the polypeptide sequence of SEQ ID NO: 5, the polypeptide sequence of SEQ ID NO: 23, the polypeptide sequence of SEQ ID NO: 183 and the polypeptide sequence of SEQ ID NO: 187, or variants thereof that retain functionality. In one embodiment the T cell activating bispecific antigen binding molecule comprises the polypeptide sequence of SEQ ID NO: 33, the polypeptide sequence of SEQ ID NO: 189, the polypeptide sequence of SEQ ID NO: 191 and the polypeptide sequence of SEQ ID NO: 193, or variants thereof that retain functionality. In one embodiment the T cell activating bispecific antigen binding molecule comprises the polypeptide sequence of SEQ ID NO: 183, the polypeptide sequence of SEQ ID NO: 189, the polypeptide sequence of SEQ ID NO: 193 and the polypeptide sequence of SEQ ID NO: 195, or variants thereof that retain functionality. In one embodiment the T cell activating bispecific antigen binding molecule comprises the polypeptide sequence of SEQ ID NO: 189, the polypeptide sequence of SEQ ID NO: 193, the polypeptide sequence of SEQ ID NO: 199 and the polypeptide sequence of SEQ ID NO: 201, or variants thereof that retain functionality. In one embodiment the T cell activating bispecific antigen binding molecule comprises the polypeptide sequence of SEQ ID NO: 5, the polypeptide sequence of SEQ ID NO: 23, the polypeptide sequence of SEQ ID NO: 215 and the polypeptide sequence of SEQ ID NO: 217, or variants thereof that retain functionality. In one embodiment the T cell activating bispecific antigen binding molecule comprises the polypeptide sequence of SEQ ID NO: 5, the polypeptide sequence of SEQ ID NO: 23, the polypeptide sequence of SEQ ID NO: 215 and the polypeptide sequence of SEQ ID NO: 219, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 234, SEQ ID NO: 236, SEQ ID NO: 238, SEQ ID NO: 240, SEQ ID NO: 242, SEQ ID NO: 244, SEQ ID NO: 246, SEQ ID NO: 248, SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 180, SEQ ID NO: 182, SEQ ID NO: 184, SEQ ID NO: 186, SEQ ID NO: 188, SEQ ID NO: 190, SEQ ID NO: 192, SEQ ID NO: 194, SEQ ID NO: 196, SEQ ID NO: 200, SEQ ID NO: 202, SEQ ID NO: 216, SEQ ID NO: 218 and SEQ ID NO: 220.

In one embodiment the T cell activating bispecific antigen binding molecule comprises at least one antigen binding moiety that is specific for Epidermal Growth Factor Receptor (EGFR). In another embodiment the T cell activating bispecific antigen binding molecule comprises at least one, typically two or more antigen binding moieties that can compete with monoclonal antibody GA201 for binding to an epitope of EGFR. See PCT publication WO 2006/082515. In one embodiment, the antigen binding moiety that is specific for EGFR comprises the heavy chain CDR1 of SEQ ID NO: 85, the heavy chain CDR2 of SEQ ID NO: 87, the heavy chain CDR3 of SEQ ID NO: 89, the light chain CDR1 of SEQ ID NO: 93, the light chain CDR2 of SEQ ID NO: 95, and the light chain CDR3 of SEQ ID NO: 97. In a further embodiment, the antigen binding moiety that is specific for EGFR comprises a heavy chain variable region sequence that is at least about 80%, 85%, 90%, 95%, 96%, 97%, 98%, 99% or 100% identical to SEQ ID NO: 91 and a light chain variable region sequence that is at least about 80%, 85%, 90%, 95%, 96%, 97%, 98%, 99% or 100% identical to SEQ ID NO: 99, or variants thereof that retain functionality.

In yet another embodiment the T cell activating bispecific antigen binding molecule comprises the polypeptide sequence of SEQ ID NO: 43, the polypeptide sequence of SEQ ID NO: 45 and the polypeptide sequence of SEQ ID NO: 47, or variants thereof that retain functionality. In a further embodiment the T cell activating bispecific antigen binding molecule comprises the polypeptide sequence of SEQ ID NO: 49, the polypeptide sequence of SEQ ID NO: 51 and the polypeptide sequence of SEQ ID NO: 11, or variants thereof that retain functionality. In yet another embodiment the T cell activating bispecific antigen binding molecule comprises the polypeptide sequence of SEQ ID NO: 53, the polypeptide sequence of SEQ ID NO: 45 and the polypeptide sequence of SEQ ID NO: 47, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 44, SEQ ID NO: 46, SEQ ID NO: 48, SEQ ID NO: 50, SEQ ID NO: 52, SEQ ID NO: 54 and SEQ ID NO: 12.

In one embodiment the T cell activating bispecific antigen binding molecule comprises at least one antigen binding moiety that is specific for Fibroblast Activation Protein (FAP). In another embodiment the T cell activating bispecific antigen binding molecule comprises at least one, typically two or more antigen binding moieties that can compete with monoclonal antibody 3F2 for binding to an epitope of FAP. See PCT publication WO 2012/020006. In one embodiment, the antigen binding moiety that is specific for FAP comprises the heavy chain CDR1 of SEQ ID NO: 101, the heavy chain CDR2 of SEQ ID NO: 103, the heavy chain CDR3 of SEQ ID NO: 105, the light chain CDR1 of SEQ ID NO: 109, the light chain CDR2 of SEQ ID NO: 111, and the light chain CDR3 of SEQ ID NO: 113. In a further embodiment, the antigen binding moiety that is specific for FAP comprises a heavy chain variable region sequence that is at least about 80%, 85%, 90%, 95%, 96%, 97%, 98%, 99% or 100% identical to SEQ ID NO: 107 and a light chain variable region sequence that is at least about 80%, 85%, 90%, 95%, 96%, 97%, 98%, 99% or 100% identical to SEQ ID NO: 115, or variants thereof that retain functionality.

In yet another embodiment the T cell activating bispecific antigen binding molecule comprises the polypeptide sequence of SEQ ID NO: 55, the polypeptide sequence of SEQ ID NO: 51 and the polypeptide sequence of SEQ ID NO: 11, or variants thereof that retain functionality. In a further embodiment the T cell activating bispecific antigen binding molecule comprises the polypeptide sequence of SEQ ID NO: 57, the polypeptide sequence of SEQ ID NO: 59 and the polypeptide sequence of SEQ ID NO: 61, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 116, SEQ ID NO: 56, SEQ ID NO: 58, SEQ ID NO: 60, SEQ ID NO: 62, SEQ ID NO: 52 and SEQ ID NO: 12.

In particular embodiments the T cell activating bispecific antigen binding molecule comprises at least one antigen binding moiety that is specific for Carcinoembryonic Antigen (CEA). In one embodiment the T cell activating bispecific antigen binding molecule comprises at least one, typically two or more antigen binding moieties that can compete with monoclonal antibody BW431/26 (described in European patent no. EP 160 897, and Bosslet et al., Int J Cancer 36, 75-84 (1985)) for binding to an epitope of CEA. In one embodiment the T cell activating bispecific antigen binding molecule comprises at least one, typically two or more antigen binding moieties that can compete with monoclonal antibody CH1A1A (see SEQ ID NOs 123 and 131) for binding to an epitope of CEA. See PCT publication number WO 2011/023787. In one embodiment, the antigen binding moiety that is specific for CEA binds to the same epitope of CEA as monoclonal antibody CH1A1A. In one embodiment, the antigen binding moiety that is specific for CEA comprises the heavy chain CDR1 of SEQ ID NO: 117, the heavy chain CDR2 of SEQ ID NO: 119, the heavy chain CDR3 of SEQ ID NO: 121, the light chain CDR1 of SEQ ID NO: 125, the light chain CDR2 of SEQ ID NO: 127, and the light chain CDR3 of SEQ ID NO: 129. In a further embodiment, the antigen binding moiety that is specific for CEA comprises a heavy chain variable region sequence that is at least about 80%, 85%, 90%, 95%, 96%, 97%, 98%, 99% or 100%, particularly about 98%, 99% or 100%, identical to SEQ ID NO: 123 and a light chain variable region sequence that is at least about 80%, 85%, 90%, 95%, 96%, 97%, 98%, 99% or 100%, particularly about 98%, 99% or 100%, identical to SEQ ID NO: 131, or variants thereof that retain functionality. In one embodiment, the antigen binding moiety that is specific for CEA comprises the heavy and light chain variable region sequences of an affinity matured version of monoclonal antibody CH1A1A. In one embodiment, the antigen binding moiety that is specific for CEA comprises the heavy chain variable region sequence of SEQ ID NO: 123 with one, two, three, four, five, six or seven, particularly two, three, four or five, amino acid substitutions; and the light chain variable region sequence of SEQ ID NO: 131 with one, two, three, four, five, six or seven, particularly two, three, four or five, amino acid substitutions. Any amino acid residue within the variable region sequences may be substituted by a different amino acid, including amino acid residues within the CDR regions, provided that binding to CEA, particularly human CEA, is preserved. Preferred variants are those having a binding affinity for CEA at least equal (or stronger) to the binding affinity of the antigen binding moiety comprising the unsubstituted variable region sequences.

In one embodiment the T cell activating bispecific antigen binding molecule comprises the polypeptide sequence of SEQ ID NO: 63, the polypeptide sequence of SEQ ID NO: 65, the polypeptide sequence of SEQ ID NO: 67 and the polypeptide sequence of SEQ ID NO: 33, or variants thereof that retain functionality. In one embodiment the T cell activating bispecific antigen binding molecule comprises the polypeptide sequence of SEQ ID NO: 65, the polypeptide sequence of SEQ ID NO: 67, the polypeptide sequence of SEQ ID NO: 183 and the polypeptide sequence of SEQ ID NO: 197, or variants thereof that retain functionality. In one embodiment the T cell activating bispecific antigen binding molecule comprises the polypeptide sequence of SEQ ID NO: 183, the polypeptide sequence of SEQ ID NO: 203, the polypeptide sequence of SEQ ID NO: 205 and the polypeptide sequence of SEQ ID NO: 207, or variants thereof that retain functionality. In one embodiment the T cell activating bispecific antigen binding molecule comprises the polypeptide sequence of SEQ ID NO: 183, the polypeptide sequence of SEQ ID NO: 209, the polypeptide sequence of SEQ ID NO: 211 and the polypeptide sequence of SEQ ID NO: 213, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 64, SEQ ID NO: 66, SEQ ID NO: 68, SEQ ID NO: 34, SEQ ID NO: 184, SEQ ID NO: 198, SEQ ID NO: 204, SEQ ID NO: 206, SEQ ID NO: 208, SEQ ID NO: 210, SEQ ID NO: 212 and SEQ ID NO: 214.

In one embodiment the T cell activating bispecific antigen binding molecule comprises at least one antigen binding moiety that is specific for CD33. In one embodiment, the antigen binding moiety that is specific for CD33 comprises the heavy chain CDR1 of SEQ ID NO: 133, the heavy chain CDR2 of SEQ ID NO: 135, the heavy chain CDR3 of SEQ ID NO: 137, the light chain CDR1 of SEQ ID NO: 141, the light chain CDR2 of SEQ ID NO: 143, and the light chain CDR3 of SEQ ID NO: 145. In a further embodiment, the antigen binding moiety that is specific for CD33 comprises a heavy chain variable region sequence that is at least about 80%, 85%, 90%, 95%, 96%, 97%, 98%, 99% or 100% identical to SEQ ID NO: 139 and a light chain variable region sequence that is at least about 80%, 85%, 90%, 95%, 96%, 97%, 98%, 99% or 100% identical to SEQ ID NO: 147, or variants thereof that retain functionality.

In one embodiment the T cell activating bispecific antigen binding molecule comprises the polypeptide sequence of SEQ ID NO: 33, the polypeptide sequence of SEQ ID NO: 213, the polypeptide sequence of SEQ ID NO: 221 and the polypeptide sequence of SEQ ID NO: 223, or variants thereof that retain functionality. In one embodiment the T cell activating bispecific antigen binding molecule comprises the polypeptide sequence of SEQ ID NO: 33, the polypeptide sequence of SEQ ID NO: 221, the polypeptide sequence of SEQ ID NO: 223 and the polypeptide sequence of SEQ ID NO: 225, or variants thereof that retain functionality.

In a specific embodiment the T cell activating bispecific antigen binding molecule comprises a polypeptide sequence encoded by a polynucleotide sequence that is at least about 80%, 85%, 90%, 95%, 96%, 97%, 98%, 99% or 100% identical to a sequence selected from the group of SEQ ID NO: 134, SEQ ID NO: 136, SEQ ID NO: 138, SEQ ID NO: 140, SEQ ID NO: 142, SEQ ID NO: 144, SEQ ID NO: 146, SEQ ID NO: 148, SEQ ID NO: 34, SEQ ID NO: 214, SEQ ID NO: 222, SEQ ID NO: 224 and SEQ ID NO: 226.

### Polynucleotides

Further provided herein are isolated polynucleotides encoding a T cell activating bispecific antigen binding molecule as described herein or a fragment thereof.

Polynucleotides disclosed herein include those that are at least about 80%, 85%, 90%, 95%, 96%, 97%, 98%, 99%, or 100% identical to the sequences set forth in SEQ ID NOs 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262 and 264, including functional fragments or variants thereof.

The polynucleotides encoding T cell activating bispecific antigen binding molecules disclosed herein may be expressed as a single polynucleotide that encodes the entire T cell activating bispecific antigen binding molecule or as multiple (e.g., two or more) polynucleotides that are co-expressed. Polypeptides encoded by polynucleotides that are co-expressed may associate through, e.g., disulfide bonds or other means to form a functional T cell activating bispecific antigen binding molecule. For example, the light chain portion of an antigen binding moiety may be encoded by a separate polynucleotide from the portion of the T cell activating bispecific antigen binding molecule comprising the heavy chain portion of the antigen binding moiety, an Fc domain subunit and optionally (part of) another antigen binding moiety. When co-expressed, the heavy chain polypeptides will associate with the light chain polypeptides to form the antigen binding moiety. In another example, the portion of the T cell activating bispecific antigen binding molecule comprising one of the two Fc domain subunits and optionally (part of) one or more antigen binding moieties could be encoded by a separate polynucleotide from the portion of the T cell activating bispecific antigen binding molecule comprising the the other of the two Fc domain subunits and optionally (part of) an antigen binding moiety. When co-expressed, the Fc domain subunits will associate to form the Fc domain.

In certain embodiments, an isolated polynucleotide disclosed herein encodes a fragment of a T cell activating bispecific antigen binding molecule comprising a first and a second antigen binding moiety, and an Fc domain consisting of two subunits, wherein the first antigen binding moiety is a single chain Fab molecule. In one embodiment, an isolated polynucleotide disclosed herein encodes the first antigen binding moiety and a subunit of the Fc domain. In a more specific embodiment the isolated polynucleotide encodes a polypeptide wherein a single chain Fab molecule shares a carboxy-terminal peptide bond with an Fc domain subunit. In another embodiment, an isolated polynucleotide disclosed herein encodes the heavy chain of the second antigen binding moiety and a subunit of the Fc domain. In a more specific embodiment the isolated polynucleotide encodes a polypeptide wherein a Fab heavy chain shares a carboxy terminal peptide bond with an Fc domain subunit. In yet another embodiment, an isolated polynucleotide disclosed herein encodes the first antigen binding moiety, the heavy chain of the second antigen binding moiety and a subunit of the Fc domain. In a more specific embodiment, the isolated polynucleotide encodes a polypeptide wherein a single chain Fab molecule shares a carboxy-terminal peptide bond with a Fab heavy chain, which in turn shares a carboxy-terminal peptide bond with an Fc domain subunit.

In certain embodiments, an isolated polynucleotide disclosed herein encodes a fragment of a T cell activating bispecific antigen binding molecule comprising a first and a second antigen binding moiety, and an Fc domain consisting of two subunits, wherein the first antigen binding moiety is a crossover Fab molecule. In one embodiment, an isolated polynucleotide disclosed herein encodes the heavy chain of the first antigen binding moiety and a subunit of the Fc domain. In a more specific embodiment the isolated polynucleotide encodes a polypeptide wherein Fab light chain variable region shares a carboxy terminal peptide bond with a Fab heavy chain constant region, which in turn shares a carboxy-terminal peptide bond with an Fc domain subunit. In another specific embodiment the isolated polynucleotide encodes a polypeptide wherein Fab heavy chain variable region shares a carboxy terminal peptide bond with a Fab light chain constant region, which in turn shares a carboxy-terminal peptide bond with an Fc domain subunit. In another embodiment, an isolated polynucleotide disclosed herein encodes the heavy chain of the second antigen binding moiety and a subunit of the Fc domain. In a more specific embodiment the isolated polynucleotide encodes a polypeptide wherein a Fab heavy chain shares a carboxy terminal peptide bond with an Fc domain subunit. In yet another embodiment, an isolated polynucleotide disclosed herein encodes the heavy chain of the first antigen binding moiety, the heavy chain of the second antigen binding moiety and a subunit of the Fc domain. In a more specific embodiment, the isolated polynucleotide encodes a polypeptide wherein a Fab light chain variable region shares a carboxy-terminal peptide bond with a Fab heavy chain constant region, which in turn shares a carboxy-terminal peptide bond with a Fab heavy chain, which in turn shares a carboxy-terminal peptide bond with an Fc domain subunit. In another specific embodiment, the isolated polynucleotide encodes a polypeptide wherein a Fab heavy chain variable region shares a carboxy-terminal peptide bond with a Fab light chain constant region, which in turn shares a carboxy-terminal peptide bond with a Fab heavy chain, which in turn shares a carboxy-terminal peptide bond with an Fc domain subunit. In yet another specific embodiment the isolated polynucleotide encodes a polypeptide wherein a Fab heavy chain shares a carboxy-terminal peptide bond with a Fab light chain variable region, which in turn shares a carboxy-terminal peptide bond with a Fab heavy chain constant region, which in turn shares a carboxy-terminal peptide bond with an Fc domain subunit. In still another specific embodiment the isolated polynucleotide encodes a polypeptide wherein a Fab heavy chain shares a carboxy-terminal peptide bond with a Fab heavy chain variable region, which in turn shares a carboxy-terminal peptide bond with a Fab light chain constant region, which in turn shares a carboxy-terminal peptide bond with an Fc domain subunit.

In further embodiments, an isolated polynucleotide disclosed herein encodes the heavy chain of a third antigen binding moiety and a subunit of the Fc domain. In a more specific embodiment the isolated polynucleotide encodes a polypeptide wherein a Fab heavy chain shares a carboxy terminal peptide bond with an Fc domain subunit.

In further embodiments, an isolated polynucleotide disclosed herein encodes the light chain of an antigen binding moiety. In some embodiments, the isolated polynucleotide encodes a polypeptide wherein a Fab light chain variable region shares a carboxy-terminal peptide bond with a Fab heavy chain constant region. In other embodiments, the isolated polynucleotide encodes a polypeptide wherein a Fab heavy chain variable region shares a carboxy-terminal peptide bond with a Fab light chain constant region. In still other embodiments, an isolated polynucleotide disclosed herein encodes the light chain of the first antigen binding moiety and the light chain of the second antigen binding moiety. In a more specific embodiment, the isolated polynucleotide encodes a polypeptide wherein a Fab heavy chain variable region shares a carboxy-terminal peptide bond with a Fab light chain constant region, which in turn shares a carboxy-terminal peptide bond with a Fab light chain. In another specific embodiment the isolated polynucleotide encodes a polypeptide wherein a Fab light chain shares a carboxy-terminal peptide bond with a Fab heavy chain variable region, which in turn shares a carboxy-terminal peptide bond with a Fab light chain constant region. In yet another specific embodiment, the isolated polynucleotide encodes a polypeptide wherein a Fab light chain variable region shares a carboxy-terminal peptide bond with a Fab heavy chain constant region, which in turn shares a carboxy-terminal peptide bond with a Fab light chain. In yet another specific embodiment the isolated polynucleotide encodes a polypeptide wherein a Fab light chain shares a carboxy-terminal peptide bond with a Fab light chain variable region, which in turn shares a carboxy-terminal peptide bond with a Fab heavy chain constant region.

In another embodiment, the present disclosure is directed to an isolated polynucleotide encoding a T cell activating bispecific antigen binding molecule disclosed herein or a fragment thereof, wherein the polynucleotide comprises a sequence that encodes a variable region sequence as shown in SEQ ID NOs 75, 83, 91, 99, 107, 115, 123, 131, 139, 147, 169, 177, 239, 247, 255 and 263. In another embodiment, the present disclosure is directed to an isolated polynucleotide encoding a T cell activating bispecific antigen binding molecule or fragment thereof, wherein the polynucleotide comprises a sequence that encodes a polypeptide sequence as shown in SEQ ID NOs 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 179, 181, 183, 185, 187, 189, 191, 193, 195, 197, 199, 201, 203, 205, 207, 209, 211, 213, 215, 217, 219, 221, 223, 225, 227, 229 and 231. In another embodiment, the present disclosure is directed to an isolated polynucleotide encoding a T cell activating bispecific antigen binding molecule disclosed herein or a fragment thereof, wherein the polynucleotide comprises a sequence that is at least about 80%, 85%, 90%, 95%, 96%, 97%, 98%, or 99% identical to a nucleotide sequence shown in SEQ ID NOs 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262 or 264. In another embodiment, the present disclosure is directed to an isolated polynucleotide encoding a T cell activating bispecific antigen binding molecule disclosed herein or a fragment thereof, wherein the polynucleotide comprises a nucleic acid sequence shown in SEQ ID NOs 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262 or 264. In another embodiment, the present disclosure is directed to an isolated polynucleotide encoding a T cell activating bispecific antigen binding molecule disclosed herein or a fragment thereof, wherein the polynucleotide comprises a sequence that encodes a variable region sequence that is at least about 80%, 85%, 90%, 95%, 96%, 97%, 98%, or 99% identical to an amino acid sequence in SEQ ID NOs 75, 83, 91, 99, 107, 115, 123, 131, 139, 147, 169, 177, 239, 247, 255 or 263. In another embodiment, the present disclosure is directed to an isolated polynucleotide encoding a T cell activating bispecific antigen binding molecule or fragment thereof, wherein the polynucleotide comprises a sequence that encodes a polypeptide sequence that is at least 80%, 85%, 90%, 95%, 96%, 97%, 98%, or 99% identical to an amino acid sequence in SEQ ID NOs 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 179, 181, 183, 185, 187, 189, 191, 193, 195, 197, 199, 201, 203, 205, 207, 209, 211, 213, 215, 217, 219, 221, 223, 225, 227, 229 or 231. The present disclosure encompasses an isolated polynucleotide encoding a T cell activating bispecific antigen binding molecule disclosed herein or a fragment thereof, wherein the polynucleotide comprises a sequence that encodes the variable region sequence of SEQ ID NOs 75, 83, 91, 99, 107, 115, 123, 131, 139, 147, 169, 177, 239, 247, 255 or 263 with conservative amino acid substitutions. The disclosure also encompasses an isolated polynucleotide encoding a T cell activating bispecific antigen binding molecule disclosed herein or fragment thereof, wherein the polynucleotide comprises a sequence that encodes the polypeptide sequence of SEQ ID NOs 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 179, 181, 183, 185, 187, 189, 191, 193, 195, 197, 199, 201, 203, 205, 207, 209, 211, 213, 215, 217, 219, 221, 223, 225, 227, 229 or 231 with conservative amino acid substitutions.

In certain embodiments the polynucleotide or nucleic acid is DNA. In other embodiments, a polynucleotide disclosed herein is RNA, for example, in the form of messenger RNA (mRNA). RNA disclosed herein may be single stranded or double stranded.

### Recombinant Methods

T cell activating bispecific antigen binding molecules disclosed herein may be obtained, for example, by solid-state peptide synthesis (e.g. Merrifield solid phase synthesis) or recombinant production. For recombinant production one or more polynucleotide encoding the T cell activating bispecific antigen binding molecule (fragment), e.g., as described above, is isolated and inserted into one or more vectors for further cloning and/or expression in a host cell. Such polynucleotide may be readily isolated and sequenced using conventional procedures. In one embodiment a vector, preferably an expression vector, comprising one or more of the polynucleotides disclosed herein is provided. Methods which are well known to those skilled in the art can be used to construct expression vectors containing the coding sequence of a T cell activating bispecific antigen binding molecule (fragment) along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the T cell activating bispecific antigen binding molecule (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector disclosed herein may encode one or more polypeptides, which are post- or co-translationally separated into the final proteins via proteolytic cleavage. In addition, a vector, polynucleotide, or nucleic acid disclosed herein may encode heterologous coding regions, either fused or unfused to a polynucleotide encoding the T cell activating bispecific antigen binding molecule (fragment) disclosed herein, or variant or derivative thereof. Heterologous coding regions include without limitation specialized elements or motifs, such as a secretory signal peptide or a heterologous functional domain. An operable association is when a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions disclosed herein may be associated with additional coding regions which encode secretory or signal peptides, which direct the secretion of a polypeptide encoded by a polynucleotide disclosed herein. For example, if secretion of the T cell activating bispecific antigen binding molecule is desired, DNA encoding a signal sequence may be placed upstream of the nucleic acid encoding a T cell activating bispecific antigen binding molecule disclosed herein or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain embodiments, the native signal peptide, *e.g.* an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β-glucuronidase. Exemplary amino acid and polynucleotide sequences of secretory signal peptides are given in SEQ ID NOs 154-162.

DNA encoding a short protein sequence that could be used to facilitate later purification (e.g. a histidine tag) or assist in labeling the T cell activating bispecific antigen binding molecule may be included within or at the ends of the T cell activating bispecific antigen binding molecule (fragment) encoding polynucleotide.

In a further embodiment, a host cell comprising one or more polynucleotides disclosed herein is provided. In certain embodiments a host cell comprising one or more vectors disclosed herein is provided. The polynucleotides and vectors may incorporate any of the features, singly or in combination, described herein in relation to polynucleotides and vectors, respectively. In one such embodiment a host cell comprises (e.g. has been transformed or transfected with) a vector comprising a polynucleotide that encodes (part of) a T cell activating bispecific antigen binding molecule disclosed herein. As used herein, the term "host cell" refers to any kind of cellular system which can be engineered to generate the T cell activating bispecific antigen binding molecules disclosed herein or fragments thereof. Host cells suitable for replicating and for supporting expression of T cell activating bispecific antigen binding molecules are well known in the art. Such cells may be transfected or transduced as appropriate with the particular expression vector and large quantities of vector containing cells can be grown for seeding large scale fermenters to obtain sufficient quantities of the T cell activating bispecific antigen binding molecule for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr⁻ CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one embodiment, the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell).

Standard technologies are known in the art to express foreign genes in these systems. Cells expressing a polypeptide comprising either the heavy or the light chain of an antigen binding domain such as an antibody, may be engineered so as to also express the other of the antibody chains such that the expressed product is an antibody that has both a heavy and a light chain.

In one embodiment, a method of producing a T cell activating bispecific antigen binding molecule as disclosed herein is provided, wherein the method comprises culturing a host cell comprising a polynucleotide encoding the T cell activating bispecific antigen binding molecule, as provided herein, under conditions suitable for expression of the T cell activating bispecific antigen binding molecule, and recovering the T cell activating bispecific antigen binding molecule from the host cell (or host cell culture medium).

The components of the T cell activating bispecific antigen binding molecule are genetically fused to each other. T cell activating bispecific antigen binding molecule can be designed such that its components are fused directly to each other or indirectly through a linker sequence. The composition and length of the linker may be determined in accordance with methods well known in the art and may be tested for efficacy. Examples of linker sequences between different components of T cell activating bispecific antigen binding molecules are found in the sequences provided herein. Additional sequences may also be included to incorporate a cleavage site to separate the individual components of the fusion if desired, for example an endopeptidase recognition sequence.

In certain embodiments the one or more antigen binding moieties of the T cell activating bispecific antigen binding molecules comprise at least an antibody variable region capable of binding an antigenic determinant. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty).

Any animal species of antibody, antibody fragment, antigen binding domain or variable region can be used in the T cell activating bispecific antigen binding molecules disclosed herein. Non-limiting antibodies, antibody fragments, antigen binding domains or variable regions useful herein can be of murine, primate, or human origin. If the T cell activating bispecific antigen binding molecule is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments.

In certain embodiments, the antigen binding moieties useful herein are engineered to have enhanced binding affinity according to, for example, the methods disclosed in U.S. Pat. Appl. Publ. No. 2004/0132066. The ability of the T cell activating bispecific antigen binding molecule disclosed herein to bind to a specific antigenic determinant can be measured either through an enzyme-linked immunosorbent assay (ELISA) or other techniques familiar to one of skill in the art, e.g. surface plasmon resonance technique (analyzed on a BIACORE T100 system) (Liljeblad, et al., Glyco J 17, 323-329 (2000)), and traditional binding assays (Heeley, Endocr Res 28, 217-229 (2002)). Competition assays may be used to identify an antibody, antibody fragment, antigen binding domain or variable domain that competes with a reference antibody for binding to a particular antigen, e.g. an antibody that competes with the V9 antibody for binding to CD3. In certain embodiments, such a competing antibody binds to the same epitope (e.g. a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen (e.g. CD3) is incubated in a solution comprising a first labeled antibody that binds to the antigen (e.g. V9 antibody) and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

T cell activating bispecific antigen binding molecules prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the T cell activating bispecific antigen binding molecule binds. For example, for affinity chromatography purification of T cell activating bispecific antigen binding molecules disclosed herein, a matrix with protein A or protein G may be used. Sequential Protein A or G affinity chromatography and size exclusion chromatography can be used to isolate a T cell activating bispecific antigen binding molecule essentially as described in the Examples. The purity of the T cell activating bispecific antigen binding molecule can be determined by any of a variety of well known analytical methods including gel electrophoresis, high pressure liquid chromatography, and the like. For example, the heavy chain fusion proteins expressed as described in the Examples were shown to be intact and properly assembled as demonstrated by reducing SDS-PAGE (see e.g. Figure 2). Three bands were resolved at approximately Mr 25,000, Mr 50,000 and Mr 75,000, corresponding to the predicted molecular weights of the T cell activating bispecific antigen binding molecule light chain, heavy chain and heavy chain/light chain fusion protein.

### Assays

T cell activating bispecific antigen binding molecules provided herein may be identified, screened for, or characterized for their physical/chemical properties and/or biological activities by various assays known in the art.

### Affinity assays

The affinity of the T cell activating bispecific antigen binding molecule for an Fc receptor or a target antigen can be determined in accordance with the methods set forth in the Examples by surface plasmon resonance (SPR), using standard instrumentation such as a BIAcore instrument (GE Healthcare), and receptors or target proteins such as may be obtained by recombinant expression. Alternatively, binding of T cell activating bispecific antigen binding molecules for different receptors or target antigens may be evaluated using cell lines expressing the particular receptor or target antigen, for example by flow cytometry (FACS). A specific illustrative and exemplary embodiment for measuring binding affinity is described in the following and in the Examples below.

According to one embodiment, K_{D} is measured by surface plasmon resonance using a BIACORE® T100 machine (GE Healthcare) at 25 °C.

To analyze the interaction between the Fc-portion and Fc receptors, His-tagged recombinant Fc-receptor is captured by an anti-Penta His antibody (Qiagen) immobilized on CM5 chips and the bispecific constructs are used as analytes. Briefly, carboxymethylated dextran biosensor chips (CM5, GE Healthcare) are activated with N-ethyl-N'-(3-dimethylaminopropyl)-carbodiimide hydrochloride (EDC) and N-hydroxysuccinimide (NHS) according to the supplier's instructions.

Anti Penta-His antibody is diluted with 10 mM sodium acetate, pH 5.0, to 40 µg/ml before injection at a flow rate of 5 µl/min to achieve approximately 6500 response units (RU) of coupled protein. Following the injection of the ligand, 1 M ethanolamine is injected to block unreacted groups. Subsequently the Fc-receptor is captured for 60 s at 4 or 10 nM. For kinetic measurements, four-fold serial dilutions of the bispecific construct (range between 500 nM and 4000 nM) are injected in HBS-EP (GE Healthcare, 10 mM HEPES, 150 mM NaCl, 3 mM EDTA, 0.05 % Surfactant P20, pH 7.4) at 25 °C at a flow rate of 30 µl/min for 120 s.

To determine the affinity to the target antigen, bispecific constructs are captured by an anti human Fab specific antibody (GE Healthcare) that is immobilized on an activated CM5-sensor chip surface as described for the anti Penta-His antibody. The final amount of coupled protein is is approximately 12000 RU. The bispecific constructs are captured for 90 s at 300 nM. The target antigens are passed through the flow cells for 180 s at a concentration range from 250 to 1000 nM with a flowrate of 30 µl/min. The dissociation is monitored for 180 s.

Bulk refractive index differences are corrected for by subtracting the response obtained on reference flow cell. The steady state response was used to derive the dissociation constant K_{D} by non-linear curve fitting of the Langmuir binding isotherm. Association rates (kₒₙ) and dissociation rates (k_{off}) are calculated using a simple one-to-one Langmuir binding model (BIACORE® T100 Evaluation Software version 1.1.1) by simultaneously fitting the association and dissociation sensorgrams. The equilibrium dissociation constant (K_{D}) is calculated as the ratio k_{off}/kₒₙ. See, e.g., Chen et al., J Mol Biol 293, 865-881 (1999).

### Activity assays

Biological activity of the T cell activating bispecific antigen binding molecules disclosed herein can be measured by various assays as described in the Examples. Biological activities may for example include the induction of proliferation of T cells, the induction of signaling in T cells, the induction of expression of activation markers in T cells, the induction of cytokine secretion by T cells, the induction of lysis of target cells such as tumor cells, and the induction of tumor regression and/or the improvement of survival.

### Compositions, Formulations, and Routes of Administration

In a further aspect, provided herein are pharmaceutical compositions comprising any of the T cell activating bispecific antigen binding molecules provided herein, e.g., for use in any of the below therapeutic methods. In one embodiment, a pharmaceutical composition comprises any of the T cell activating bispecific antigen binding molecules provided herein and a pharmaceutically acceptable carrier. In another embodiment, a pharmaceutical composition comprises any of the T cell activating bispecific antigen binding molecules provided herein and at least one additional therapeutic agent, e.g., as described below.

Further provided is a method of producing a T cell activating bispecific antigen binding molecule as disclosed herein in a form suitable for administration in vivo, the method comprising (a) obtaining a T cell activating bispecific antigen binding molecule as disclosed herein, and (b) formulating the T cell activating bispecific antigen binding molecule with at least one pharmaceutically acceptable carrier, whereby a preparation of T cell activating bispecific antigen binding molecule is formulated for administration in vivo.

Pharmaceutical compositions disclosed herein comprise a therapeutically effective amount of one or more T cell activating bispecific antigen binding molecule dissolved or dispersed in a pharmaceutically acceptable carrier. The phrases "pharmaceutical or pharmacologically acceptable" refers to molecular entities and compositions that are generally non-toxic to recipients at the dosages and concentrations employed, i.e. do not produce an adverse, allergic or other untoward reaction when administered to an animal, such as, for example, a human, as appropriate. The preparation of a pharmaceutical composition that contains at least one T cell activating bispecific antigen binding molecule and optionally an additional active ingredient will be known to those of skill in the art in light of the present disclosure, as exemplified by Remington's Pharmaceutical Sciences, 18th Ed. Mack Printing Company, 1990. Moreover, for animal (e.g., human) administration, it will be understood that preparations should meet sterility, pyrogenicity, general safety and purity standards as required by FDA Office of Biological Standards or corresponding authorities in other countries. Preferred compositions are lyophilized formulations or aqueous solutions. As used herein, "pharmaceutically acceptable carrier" includes any and all solvents, buffers, dispersion media, coatings, surfactants, antioxidants, preservatives (e.g. antibacterial agents, antifungal agents), isotonic agents, absorption delaying agents, salts, preservatives, antioxidants, proteins, drugs, drug stabilizers, polymers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

The composition may comprise different types of carriers depending on whether it is to be administered in solid, liquid or aerosol form, and whether it need to be sterile for such routes of administration as injection. T cell activating bispecific antigen binding molecules disclosed herein (and any additional therapeutic agent) can be administered intravenously, intradermally, intraarterially, intraperitoneally, intralesionally, intracranially, intraarticularly, intraprostatically, intrasplenically, intrarenally, intrapleurally, intratracheally, intranasally, intravitreally, intravaginally, intrarectally, intratumorally, intramuscularly, intraperitoneally, subcutaneously, subconjunctivally, intravesicularlly, mucosally, intrapericardially, intraumbilically, intraocularally, orally, topically, locally, by inhalation (e.g. aerosol inhalation), injection, infusion, continuous infusion, localized perfusion bathing target cells directly, via a catheter, via a lavage, in cremes, in lipid compositions (e.g. liposomes), or by other method or any combination of the forgoing as would be known to one of ordinary skill in the art (see, for example, Remington's Pharmaceutical Sciences, 18th Ed. Mack Printing Company). Parenteral administration, in particular intravenous injection, is most commonly used for administering polypeptide molecules such as the T cell activating bispecific antigen binding molecules disclosed herein.

Parenteral compositions include those designed for administration by injection, e.g. subcutaneous, intradermal, intralesional, intravenous, intraarterial intramuscular, intrathecal or intraperitoneal injection. For injection, the T cell activating bispecific antigen binding molecules disclosed herein may be formulated in aqueous solutions, preferably in physiologically compatible buffers such as Hanks' solution, Ringer's solution, or physiological saline buffer. The solution may contain formulatory agents such as suspending, stabilizing and/or dispersing agents. Alternatively, the T cell activating bispecific antigen binding molecules may be in powder form for constitution with a suitable vehicle, e.g., sterile pyrogen-free water, before use. Sterile injectable solutions are prepared by incorporating the T cell activating bispecific antigen binding molecules disclosed herein in the required amount in the appropriate solvent with various of the other ingredients enumerated below, as required. Sterility may be readily accomplished, e.g., by filtration through sterile filtration membranes. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein. Suitable pharmaceutically acceptable carriers include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Aqueous injection suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions. Additionally, suspensions of the active compounds may be prepared as appropriate oily injection suspensions. Suitable lipophilic solvents or vehicles include fatty oils such as sesame oil, or synthetic fatty acid esters, such as ethyl cleats or triglycerides, or liposomes.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences (18th Ed. Mack Printing Company, 1990). Sustained-release preparations may be prepared. Suitable examples of sustained-release preparations include semipermeable matrices of solid hydrophobic polymers containing the polypeptide, which matrices are in the form of shaped articles, e.g. films, or microcapsules. In particular embodiments, prolonged absorption of an injectable composition can be brought about by the use in the compositions of agents delaying absorption, such as, for example, aluminum monostearate, gelatin or combinations thereof.

In addition to the compositions described previously, the T cell activating bispecific antigen binding molecules may also be formulated as a depot preparation. Such long acting formulations may be administered by implantation (for example subcutaneously or intramuscularly) or by intramuscular injection. Thus, for example, the T cell activating bispecific antigen binding molecules may be formulated with suitable polymeric or hydrophobic materials (for example as an emulsion in an acceptable oil) or ion exchange resins, or as sparingly soluble derivatives, for example, as a sparingly soluble salt.

Pharmaceutical compositions comprising the T cell activating bispecific antigen binding molecules disclosed herein may be manufactured by means of conventional mixing, dissolving, emulsifying, encapsulating, entrapping or lyophilizing processes. Pharmaceutical compositions may be formulated in conventional manner using one or more physiologically acceptable carriers, diluents, excipients or auxiliaries which facilitate processing of the proteins into preparations that can be used pharmaceutically. Proper formulation is dependent upon the route of administration chosen.

The T cell activating bispecific antigen binding molecules may be formulated into a composition in a free acid or base, neutral or salt form. Pharmaceutically acceptable salts are salts that substantially retain the biological activity of the free acid or base. These include the acid addition salts, e.g., those formed with the free amino groups of a proteinaceous composition, or which are formed with inorganic acids such as for example, hydrochloric or phosphoric acids, or such organic acids as acetic, oxalic, tartaric or mandelic acid. Salts formed with the free carboxyl groups can also be derived from inorganic bases such as for example, sodium, potassium, ammonium, calcium or ferric hydroxides; or such organic bases as isopropylamine, trimethylamine, histidine or procaine. Pharmaceutical salts tend to be more soluble in aqueous and other protic solvents than are the corresponding free base forms.

### Therapeutic Methods and Compositions

Any of the T cell activating bispecific antigen binding molecules provided herein may be used in therapeutic methods. T cell activating bispecific antigen binding molecules as disclosed herein can be used as immunotherapeutic agents, for example in the treatment of cancers.

For use in therapeutic methods, T cell activating bispecific antigen binding molecules disclosed herein would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one aspect, T cell activating bispecific antigen binding molecules as disclosed herein for use as a medicament are provided. In further aspects, T cell activating bispecific antigen binding molecules as disclosed herein for use in treating a disease are provided. In certain embodiments, T cell activating bispecific antigen binding molecules as disclosed herein for use in a method of treatment are provided. In one embodiment, a T cell activating bispecific antigen binding molecule as described herein is provided for use in the treatment of a disease in an individual in need thereof. In certain embodiments, a T cell activating bispecific antigen binding molecule is provided for use in a method of treating an individual having a disease comprising administering to the individual a therapeutically effective amount of the T cell activating bispecific antigen binding molecule. In certain embodiments the disease to be treated is a proliferative disorder. In a particular embodiment the disease is cancer. In certain embodiments the method further comprises administering to the individual a therapeutically effective amount of at least one additional therapeutic agent, e.g., an anti-cancer agent if the disease to be treated is cancer. In further embodiments, a T cell activating bispecific antigen binding molecule as described herein is provided for use in inducing lysis of a target cell, particularly a tumor cell. In certain embodiments, a T cell activating bispecific antigen binding molecule is provided for use in a method of inducing lysis of a target cell, particularly a tumor cell, in an individual comprising administering to the individual an effective amount of the T cell activating bispecific antigen binding molecule to induce lysis of a target cell. An "individual" according to any of the above embodiments is a mammal, preferably a human.

In a further aspect, provided herein is the use of a T cell activating bispecific antigen binding molecule as disclosed herein in the manufacture or preparation of a medicament. In one embodiment the medicament is for the treatment of a disease in an individual in need thereof. In a further embodiment, the medicament is for use in a method of treating a disease comprising administering to an individual having the disease a therapeutically effective amount of the medicament. In certain embodiments the disease to be treated is a proliferative disorder. In a particular embodiment the disease is cancer. In one embodiment, the method further comprises administering to the individual a therapeutically effective amount of at least one additional therapeutic agent, e.g., an anti-cancer agent if the disease to be treated is cancer. In a further embodiment, the medicament is for inducing lysis of a target cell, particularly a tumor cell. In still a further embodiment, the medicament is for use in a method of inducing lysis of a target cell, particularly a tumor cell, in an individual comprising administering to the individual an effective amount of the medicament to induce lysis of a target cell. An "individual" according to any of the above embodiments may be a mammal, preferably a human.

In a further aspect, provided herein is a method for treating a disease. In one embodiment, the method comprises administering to an individual having such disease a therapeutically effective amount of a T cell activating bispecific antigen binding molecule as disclosed herein. In one embodiment a composition is administered to said individual, comprising the T cell activating bispecific antigen binding molecule disclosed herein in a pharmaceutically acceptable form. In certain embodiments the disease to be treated is a proliferative disorder. In a particular embodiment the disease is cancer. In certain embodiments the method further comprises administering to the individual a therapeutically effective amount of at least one additional therapeutic agent, e.g., an anti-cancer agent if the disease to be treated is cancer. An "individual" according to any of the above embodiments may be a mammal, preferably a human.

In a further aspect, provided herein is a method for inducing lysis of a target cell, particularly a tumor cell. In one embodiment the method comprises contacting a target cell with a T cell activating bispecific antigen binding molecule as disclosed herein in the presence of a T cell, particularly a cytotoxic T cell. In a further aspect, a method for inducing lysis of a target cell, particularly a tumor cell, in an individual is provided. In one such embodiment, the method comprises administering to the individual an effective amount of a T cell activating bispecific antigen binding molecule to induce lysis of a target cell. In one embodiment, an "individual" is a human.

In certain embodiments the disease to be treated is a proliferative disorder, particularly cancer. Non-limiting examples of cancers include bladder cancer, brain cancer, head and neck cancer, pancreatic cancer, lung cancer, breast cancer, ovarian cancer, uterine cancer, cervical cancer, endometrial cancer, esophageal cancer, colon cancer, colorectal cancer, rectal cancer, gastric cancer, prostate cancer, blood cancer, skin cancer, squamous cell carcinoma, bone cancer, and kidney cancer. Other cell proliferation disorders that can be treated using a T cell activating bispecific antigen binding molecule disclosed herein include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Also included are pre-cancerous conditions or lesions and cancer metastases. In certain embodiments the cancer is chosen from the group consisting of renal cell cancer, skin cancer, lung cancer, colorectal cancer, breast cancer, brain cancer, head and neck cancer. A skilled artisan readily recognizes that in many cases the T cell activating bispecific antigen binding molecule may not provide a cure but may only provide partial benefit. In some embodiments, a physiological change having some benefit is also considered therapeutically beneficial. Thus, in some embodiments, an amount of T cell activating bispecific antigen binding molecule that provides a physiological change is considered an "effective amount" or a "therapeutically effective amount". The subject, patient, or individual in need of treatment is typically a mammal, more specifically a human.

In some embodiments, an effective amount of a T cell activating bispecific antigen binding molecule disclosed herein is administered to a cell. In other embodiments, a therapeutically effective amount of a T cell activating bispecific antigen binding molecule disclosed herein is administered to an individual for the treatment of disease.

For the prevention or treatment of disease, the appropriate dosage of a T cell activating bispecific antigen binding molecule disclosed herein (when used alone or in combination with one or more other additional therapeutic agents) will depend on the type of disease to be treated, the route of administration, the body weight of the patient, the type of T cell activating bispecific antigen binding molecule, the severity and course of the disease, whether the T cell activating bispecific antigen binding molecule is administered for preventive or therapeutic purposes, previous or concurrent therapeutic interventions, the patient's clinical history and response to the T cell activating bispecific antigen binding molecule, and the discretion of the attending physician. The practitioner responsible for administration will, in any event, determine the concentration of active ingredient(s) in a composition and appropriate dose(s) for the individual subject. Various dosing schedules including but not limited to single or multiple administrations over various time-points, bolus administration, and pulse infusion are contemplated herein.

The T cell activating bispecific antigen binding molecule is suitably administered to the patient at one time or over a series of treatments. Depending on the type and severity of the disease, about 1 µg/kg to 15 mg/kg (e.g. 0.1 mg/kg - 10 mg/kg) of T cell activating bispecific antigen binding molecule can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the T cell activating bispecific antigen binding molecule would be in the range from about 0.005 mg/kg to about 10 mg/kg. In other non-limiting examples, a dose may also comprise from about 1 microgram/kg body weight, about 5 microgram/kg body weight, about 10 microgram/kg body weight, about 50 microgram/kg body weight, about 100 microgram/kg body weight, about 200 microgram/kg body weight, about 350 microgram/kg body weight, about 500 microgram/kg body weight, about 1 milligram/kg body weight, about 5 milligram/kg body weight, about 10 milligram/kg body weight, about 50 milligram/kg body weight, about 100 milligram/kg body weight, about 200 milligram/kg body weight, about 350 milligram/kg body weight, about 500 milligram/kg body weight, to about 1000 mg/kg body weight or more per administration, and any range derivable therein. In non-limiting examples of a derivable range from the numbers listed herein, a range of about 5 mg/kg body weight to about 100 mg/kg body weight, about 5 microgram/kg body weight to about 500 milligram/kg body weight, etc., can be administered, based on the numbers described above.

Thus, one or more doses of about 0.5 mg/kg, 2.0 mg/kg, 5.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the T cell activating bispecific antigen binding molecule). An initial higher loading dose, followed by one or more lower doses may be administered. However, other dosage regimens may be useful. The progress of this therapy is easily monitored by conventional techniques and assays.

The T cell activating bispecific antigen binding molecules disclosed herein will generally be used in an amount effective to achieve the intended purpose. For use to treat or prevent a disease condition, the T cell activating bispecific antigen binding molecules disclosed herein, or pharmaceutical compositions thereof, are administered or applied in a therapeutically effective amount. Determination of a therapeutically effective amount is well within the capabilities of those skilled in the art, especially in light of the detailed disclosure provided herein.

For systemic administration, a therapeutically effective dose can be estimated initially from *in vitro* assays, such as cell culture assays. A dose can then be formulated in animal models to achieve a circulating concentration range that includes the IC₅₀ as determined in cell culture. Such information can be used to more accurately determine useful doses in humans.

Initial dosages can also be estimated from *in vivo* data, *e.g.,* animal models, using techniques that are well known in the art. One having ordinary skill in the art could readily optimize administration to humans based on animal data.

Dosage amount and interval may be adjusted individually to provide plasma levels of the T cell activating bispecific antigen binding molecules which are sufficient to maintain therapeutic effect. Usual patient dosages for administration by injection range from about 0.1 to 50 mg/kg/day, typically from about 0.5 to 1 mg/kg/day. Therapeutically effective plasma levels may be achieved by administering multiple doses each day. Levels in plasma may be measured, for example, by HPLC.

In cases of local administration or selective uptake, the effective local concentration of the T cell activating bispecific antigen binding molecules may not be related to plasma concentration. One having skill in the art will be able to optimize therapeutically effective local dosages without undue experimentation.

A therapeutically effective dose of the T cell activating bispecific antigen binding molecules described herein will generally provide therapeutic benefit without causing substantial toxicity. Toxicity and therapeutic efficacy of a T cell activating bispecific antigen binding molecule can be determined by standard pharmaceutical procedures in cell culture or experimental animals.

Cell culture assays and animal studies can be used to determine the LD₅₀ (the dose lethal to 50% of a population) and the ED₅₀ (the dose therapeutically effective in 50% of a population). The dose ratio between toxic and therapeutic effects is the therapeutic index, which can be expressed as the ratio LD₅₀/ED₅₀. T cell activating bispecific antigen binding molecules that exhibit large therapeutic indices are preferred. In one embodiment, the T cell activating bispecific antigen binding molecule disclosed herein exhibits a high therapeutic index. The data obtained from cell culture assays and animal studies can be used in formulating a range of dosages suitable for use in humans. The dosage lies preferably within a range of circulating concentrations that include the ED₅₀ with little or no toxicity. The dosage may vary within this range depending upon a variety of factors, e.g., the dosage form employed, the route of administration utilized, the condition of the subject, and the like. The exact formulation, route of administration and dosage can be chosen by the individual physician in view of the patient's condition (see, e.g., Fingl et al., 1975, in: The Pharmacological Basis of Therapeutics, Ch. 1, p. 1).

The attending physician for patients treated with T cell activating bispecific antigen binding molecules disclosed herein would know how and when to terminate, interrupt, or adjust administration due to toxicity, organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient.

### Other Agents and Treatments

The T cell activating bispecific antigen binding molecules disclosed herein may be administered in combination with one or more other agents in therapy. For instance, a T cell activating bispecific antigen binding molecule disclosed herein may be co-administered with at least one additional therapeutic agent. The term "therapeutic agent" encompasses any agent administered to treat a symptom or disease in an individual in need of such treatment. Such additional therapeutic agent may comprise any active ingredients suitable for the particular indication being treated, preferably those with complementary activities that do not adversely affect each other. In certain embodiments, an additional therapeutic agent is an immunomodulatory agent, a cytostatic agent, an inhibitor of cell adhesion, a cytotoxic agent, an activator of cell apoptosis, or an agent that increases the sensitivity of cells to apoptotic inducers. In a particular embodiment, the additional therapeutic agent is an anti-cancer agent, for example a microtubule disruptor, an antimetabolite, a topoisomerase inhibitor, a DNA intercalator, an alkylating agent, a hormonal therapy, a kinase inhibitor, a receptor antagonist, an activator of tumor cell apoptosis, or an antiangiogenic agent.

Such other agents are suitably present in combination in amounts that are effective for the purpose intended. The effective amount of such other agents depends on the amount of T cell activating bispecific antigen binding molecule used, the type of disorder or treatment, and other factors discussed above. The T cell activating bispecific antigen binding molecules are generally used in the same dosages and with administration routes as described herein, or about from 1 to 99% of the dosages described herein, or in any dosage and by any route that is empirically/clinically determined to be appropriate.

Such combination therapies noted above encompass combined administration (where two or more therapeutic agents are included in the same or separate compositions), and separate administration, in which case, administration of the T cell activating bispecific antigen binding molecule disclosed herein can occur prior to, simultaneously, and/or following, administration of the additional therapeutic agent and/or adjuvant. T cell activating bispecific antigen binding molecules disclosed herein can also be used in combination with radiation therapy.

### Articles of Manufacture

In another aspect, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a T cell activating bispecific antigen binding molecule as disclosed herein. The label or package insert indicates that the composition is used for treating the condition of choice. Moreover, the article of manufacture may comprise (a) a first container with a composition contained therein, wherein the composition comprises a T cell activating bispecific antigen binding molecule as disclosed herein; and (b) a second container with a composition contained therein, wherein the composition comprises a further cytotoxic or otherwise therapeutic agent. The article of manufacture in this embodiment may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Examples

The following are examples of methods and compositions disclosed herein. It is understood that various other embodiments may be practiced, given the general description provided above.

### General methods

### Recombinant DNA Techniques

Standard methods were used to manipulate DNA as described in Sambrook et al., Molecular cloning: A laboratory manual; Cold Spring Harbor Laboratory Press, Cold Spring Harbor, New York, 1989. The molecular biological reagents were used according to the manufacturers' instructions. General information regarding the nucleotide sequences of human immunoglobulins light and heavy chains is given in: Kabat, E.A. et al., (1991) Sequences of Proteins of Immunological Interest, 5th ed., NIH Publication No. 91-3242.

### DNA Sequencing

DNA sequences were determined by double strand sequencing.

### Gene Synthesis

Desired gene segments where required were either generated by PCR using appropriate templates or were synthesized by Geneart AG (Regensburg, Germany) from synthetic oligonucleotides and PCR products by automated gene synthesis. In cases where no exact gene sequence was available, oligonucleotide primers were designed based on sequences from closest homologues and the genes were isolated by RT-PCR from RNA originating from the appropriate tissue. The gene segments flanked by singular restriction endonuclease cleavage sites were cloned into standard cloning / sequencing vectors. The plasmid DNA was purified from transformed bacteria and concentration determined by UV spectroscopy. The DNA sequence of the subcloned gene fragments was confirmed by DNA sequencing. Gene segments were designed with suitable restriction sites to allow sub-cloning into the respective expression vectors. All constructs were designed with a 5'-end DNA sequence coding for a leader peptide which targets proteins for secretion in eukaryotic cells. SEQ ID NOs 154-162 give exemplary leader peptides and polynucleotide sequences encoding them, respectively.

### Isolation of primary human pan T cells from PBMCs

Peripheral blood mononuclear cells (PBMCs) were prepared by Histopaque density centrifugation from enriched lymphocyte preparations (buffy coats) obtained from local blood banks or from fresh blood from healthy human donors. Briefly, blood was diluted with sterile PBS and carefully layered over a Histopaque gradient (Sigma, H8889). After centrifugation for 30 minutes at 450 x g at room temperature (brake switched off), part of the plasma above the PBMC containing interphase was discarded. The PBMCs were transferred into new 50 ml Falcon tubes and tubes were filled up with PBS to a total volume of 50 ml. The mixture was centrifuged at room temperature for 10 minutes at 400 x g (brake switched on). The supernatant was discarded and the PBMC pellet washed twice with sterile PBS (centrifugation steps at 4°C for 10 minutes at 350 x g). The resulting PBMC population was counted automatically (ViCell) and stored in RPMI1640 medium, containing 10% FCS and 1% L-alanyl-L-glutamine (Biochrom, K0302) at 37°C, 5% CO₂ in the incubator until assay start.

T cell enrichment from PBMCs was performed using the Pan T Cell Isolation Kit II (Miltenyi Biotec #130-091-156), according to the manufacturer's instructions. Briefly, the cell pellets were diluted in 40 µl cold buffer per 10 million cells (PBS with 0.5% BSA, 2 mM EDTA, sterile filtered) and incubated with 10 µl Biotin-Antibody Cocktail per 10 million cells for 10 min at 4°C. 30 µl cold buffer and 20 µl Anti-Biotin magnetic beads per 10 million cells were added, and the mixture incubated for another 15 min at 4°C. Cells were washed by adding 10-20x the current volume and a subsequent centrifugation step at 300 x g for 10 min. Up to 100 million cells were resuspended in 500 µl buffer. Magnetic separation of unlabeled human pan T cells was performed using LS columns (Miltenyi Biotec #130-042-401) according to the manufacturer's instructions. The resulting T cell population was counted automatically (ViCell) and stored in AIM-V medium at 37°C, 5% CO₂ in the incubator until assay start (not longer than 24 h).

### Isolation of primary human naive T cells from PBMCs

Peripheral blood mononuclar cells (PBMCs) were prepared by Histopaque density centrifugation from enriched lymphocyte preparations (buffy coats) obtained from local blood banks or from fresh blood from healthy human donors. T-cell enrichment from PBMCs was performed using the Naive CD8⁺ T cell isolation Kit from Miltenyi Biotec (#130-093-244), according to the manufacturer's instructions, but skipping the last isolation step of CD8⁺ T cells (also see description for the isolation of primary human pan T cells).

### Isolation of murine pan T cells from splenocytes

Spleens were isolated from C57BL/6 mice, transferred into a GentleMACS C-tube (Miltenyi Biotech #130-093-237) containing MACS buffer (PBS + 0.5% BSA + 2 mM EDTA) and dissociated with the GentleMACS Dissociator to obtain single-cell suspensions according to the manufacturer's instructions. The cell suspension was passed through a pre-separation filter to remove remaining undissociated tissue particles. After centrifugation at 400 x g for 4 min at 4°C, ACK Lysis Buffer was added to lyse red blood cells (incubation for 5 min at room temperature). The remaining cells were washed with MACS buffer twice, counted and used for the isolation of murine pan T cells. The negative (magnetic) selection was performed using the Pan T Cell Isolation Kit from Miltenyi Biotec (#130-090-861), following the manufacturer's instructions. The resulting T cell population was automatically counted (ViCell) and immediately used for further assays.

### Isolation of primary cynomolgus PBMCs from heparinized blood

Peripheral blood mononuclar cells (PBMCs) were prepared by density centrifugation from fresh blood from healthy cynomolgus donors, as follows: Heparinized blood was diluted 1:3 with sterile PBS, and Lymphoprep medium (Axon Lab #1114545) was diluted to 90% with sterile PBS. Two volumes of the diluted blood were layered over one volume of the diluted density gradient and the PBMC fraction was separated by centrifugation for 30 min at 520 x g, without brake, at room temperature. The PBMC band was transferred into a fresh 50 ml Falcon tube and washed with sterile PBS by centrifugation for 10 min at 400 x g at 4°C. One low-speed centrifugation was performed to remove the platelets (15 min at 150 x g, 4°C), and the resulting PBMC population was automatically counted (ViCell) and immediately used for further assays.

### Target cells

For the assessment of MCSP-targeting bispecific antigen binding molecules, the following tumor cell lines were used: the human melanoma cell line WM266-4 (ATCC #CRL-1676), derived from a metastatic site of a malignant melanoma and expressing high levels of human MCSP; and the human melanoma cell line MV-3 (a kind gift from The Radboud University Nijmegen Medical Centre), expressing medium levels of human MCSP.

For the assessment of CEA-targeting bispecific antigen binding molecules, the following tumor cell lines were used: the human gastric cancer cell line MKN45 (DSMZ #ACC 409), expressing very high levels of human CEA; the human female Caucasian colon adenocarcinoma cell line LS-174T (ECACC #87060401), expressing medium to low levels of human CEA; the human epithelioid pancreatic carcinoma cell line Panc-1 (ATCC #CRL-1469), expressing (very) low levels of human CEA; and a murine colon carcinoma cell line MC38-huCEA, that was engineered in-house to stably express human CEA.

In addition, a human T cell leukaemia cell line, Jurkat (ATCC #TIB-152), was used to assess binding of different bispecific constructs to human CD3 on cells.

### Example 1

### Preparation, purification and characterization of bispecific antigen binding molecules

The heavy and light chain variable region sequences were subcloned in frame with either the constant heavy chain or the constant light chain pre-inserted into the respective recipient mammalian expression vector. The antibody expression was driven by an MPSV promoter and a synthetic polyA signal sequence is located at the 3' end of the CDS. In addition each vector contained an EBV OriP sequence.

The molecules were produced by co-transfecting HEK293 EBNA cells with the mammalian expression vectors. Exponentially growing HEK293 EBNA cells were transfected using the calcium phosphate method. Alternatively, HEK293 EBNA cells growing in suspension were transfected using polyethylenimine (PEI). For preparation of "1+1 IgG scFab, one armed / one armed inverted" constructs, cells were transfected with the corresponding expression vectors in a 1:1:1 ratio ("vector heavy chain" : "vector light chain" : "vector heavy chain-scFab"). For preparation of "2+1 IgG scFab" constructs, cells were transfected with the corresponding expression vectors in a 1:2:1 ratio ("vector heavy chain" : "vector light chain" : "vector heavy chain-scFab"). For preparation of "1+1 IgG Crossfab" constructs, cells were transfected with the corresponding expression vectors in a 1:1:1:1 ratio ("vector second heavy chain" : "vector first light chain" : "vector light chain Crossfab" : "vector first heavy chain-heavy chain Crossfab"). For preparation of "2+1 IgG Crossfab" constructs cells were transfected with the corresponding expression vectors in a 1:2:1:1 ratio ("vector second heavy chain" : "vector light chain" : "vector first heavy chain-heavy chain Crossfab)" : "vector light chain Crossfab". For preparation of the "2+1 IgG Crossfab, linked light chain" construct, cells were transfected with the corresponding expression vectors in a 1:1:1:1 ratio ("vector heavy chain" : "vector light chain" : "vector heavy chain (CrossFab-Fab-Fc)" : "vector linked light chain"). For preparation of the "1+1 CrossMab" construct, cells were transfected with the corresponding expression vectors in a 1:1:1:1 ratio ("vector first heavy chain" : "vector second heavy chain" : "vector first light chain" : "vector second light chain"). For preparation of the "1+1 IgG Crossfab light chain fusion " construct, cells were transfected with the corresponding expression vectors in a 1:1:1:1 ratio ("vector first heavy chain" : "vector second heavy chain" : "vector light chain Crossfab" : "vector second light chain").

For transfection using calcium phosphate cells were grown as adherent monolayer cultures in T-flasks using DMEM culture medium supplemented with 10 % (v/v) FCS, and transfected when they were between 50 and 80 % confluent. For the transfection of a T150 flask, 15 million cells were seeded 24 hours before transfection in 25 ml DMEM culture medium supplemented with FCS (at 10% v/v final), and cells were placed at 37°C in an incubator with a 5% CO₂ atmosphere overnight. For each T150 flask to be transfected, a solution of DNA, CaCl₂ and water was prepared by mixing 94 µg total plasmid vector DNA divided in the corresponding ratio, water to a final volume of 469 µl and 469 µl of a 1 M CaCl₂ solution. To this solution, 938 µl of a 50 mM HEPES, 280 mM NaCl, 1.5 mM Na₂HPO₄ solution at pH 7.05 were added, mixed immediately for 10 s and left to stand at room temperature for 20 s. The suspension was diluted with 10 ml of DMEM supplemented with 2 % (v/v) FCS, and added to the T150 in place of the existing medium. Subsequently, additional 13 ml of transfection medium were added. The cells were incubated at 37°C, 5% CO₂ for about 17 to 20 hours, then medium was replaced with 25 ml DMEM, 10 % FCS. The conditioned culture medium was harvested approximately 7 days post-media exchange by centrifugation for 15 min at 210 x g, sterile filtered (0.22 • m filter), supplemented with sodium azide to a final concentration of 0.01 % (w/v), and kept at 4°C.

For transfection using polyethylenimine (PEI) HEK293 EBNA cells were cultivated in suspension in serum free CD CHO culture medium. For the production in 500 ml shake flasks, 400 million HEK293 EBNA cells were seeded 24 hours before transfection. For transfection cells were centrifuged for 5 min at 210 x g, and supernatant was replaced by 20 ml pre-warmed CD CHO medium. Expression vectors were mixed in 20 ml CD CHO medium to a final amount of 200 µg DNA. After addition of 540 µl PEI, the mixture was vortexed for 15 s and subsequently incubated for 10 min at room temperature. Afterwards cells were mixed with the DNA/PEI solution, transferred to a 500 ml shake flask and incubated for 3 hours at 37°C in an incubator with a 5% CO₂ atmosphere. After the incubation time 160 ml F17 medium was added and cells were cultivated for 24 hours. One day after transfection 1 mM valproic acid and 7% Feed 1 (Lonza) were added. After a cultivation of 7 days, supernatant was collected for purification by centrifugation for 15 min at 210 x g, the solution was sterile filtered (0.22 µm filter), supplemented with sodium azide to a final concentration of 0.01 % w/v, and kept at 4°C. The secreted proteins were purified from cell culture supernatants by Protein A affinity chromatography, followed by a size exclusion chromatography step.

For affinity chromatography supernatant was loaded on a HiTrap ProteinA HP column (CV = 5 ml, GE Healthcare) equilibrated with 25 ml 20 mM sodium phosphate, 20 mM sodium citrate, pH 7.5 or 40 ml 20 mM sodium phosphate, 20 mM sodium citrate, 0.5 M sodium chloride, pH 7.5. Unbound protein was removed by washing with at least ten column volumes 20 mM sodium phosphate, 20 mM sodium citrate, 0.5 M sodium chloride pH 7.5, followed by an additional wash step using six column volumes 10 mM sodium phosphate, 20 mM sodium citrate, 0.5 M sodium chloride pH 5.45. Subsequently, the column was washed with 20 ml 10 mM MES, 100 mM sodium chloride, pH 5.0, and target protein was eluted in six column volumes 20 mM sodium citrate, 100 mM sodium chloride, 100 mM glycine, pH 3.0. Alternatively, target protein was eluted using a gradient over 20 column volumes from 20 mM sodium citrate, 0.5 M sodium chloride, pH 7.5 to 20 mM sodium citrate, 0.5 M sodium chloride, pH 2.5. The protein solution was neutralized by adding 1/10 of 0.5 M sodium phosphate, pH 8. The target protein was concentrated and filtrated prior to loading on a HiLoad Superdex 200 column (GE Healthcare) equilibrated with 25 mM potassium phosphate, 125 mM sodium chloride, 100 mM glycine solution of pH 6.7. For the purification of 1+1 IgG Crossfab the column was equilibrated with 20 mM histidine, 140 mM sodium chloride solution of pH 6.0.

The protein concentration of purified protein samples was determined by measuring the optical density (OD) at 280 nm, using the molar extinction coefficient calculated on the basis of the amino acid sequence. Purity and molecular weight of the bispecific constructs were analyzed by SDS-PAGE in the presence and absence of a reducing agent (5 mM 1,4-dithiotreitol) and staining with Coomassie (SimpleBlue™ SafeStain from Invitrogen) using the NuPAGE® PreCast gel system (Invitrogen, USA) was used according to the manufacturer's instructions (4-12% Tris-Acetate gels or 4-12% Bis-Tris). Alternatively, purity and molecular weight of molecules were analyzed by CE-SDS analyses in the presence and absence of a reducing agent, using the Caliper LabChip GXII system (Caliper Lifescience) according to the manufacturer's instructions. The aggregate content of the protein samples was analyzed using a Superdex 200 10/300GL analytical size-exclusion chromatography column (GE Healthcare) in 2 mM MOPS, 150 mM NaCl, 0.02% (w/v) NaN₃, pH 7.3 running buffer at 25°C. Alternatively, the aggregate content of antibody samples was analyzed using a TSKgel G3000 SW XL analytical size-exclusion column (Tosoh) in 25 mM K₂HPO₄, 125 mM NaCl, 200 mM L-arginine monohydrocloride, 0.02% (w/v) NaN₃, pH 6.7 running buffer at 25°C.

Figures 2-14 show the results of the SDS PAGE and analytical size exclusion chromatography and Table 2A shows the yields, aggregate content after Protein A, and final monomer content of the preparations of the different bispecific constructs.

Figure 47 shows the result of the CE-SDS analyses of the anti-CD3/anti-MCSP bispecific "2+1 IgG Crossfab, linked light chain" construct (see SEQ ID NOs 3, 5, 29 and 179). 2 µg sample was used for analyses. Figure 48 shows the result of the analytical size exclusion chromatography of the final product (20 µg sample injected).

Figure 54 shows the results of the CE-SDS and SDS PAGE analyses of various constructs, and Table 2A shows the yields, aggregate content after Protein A and final monomer content of the preparations of the different bispecific constructs.

**TABLE 2A. Yields, aggregate content after Protein A and final monomer content.**

| **Construct** | **Yield [mg/l]** | **Aggregate content after Protein A [%]** | **HMW [%]** | **LMW [%]** | **Monomer [%]** |
|---|---|---|---|---|---|
| **MCSP** | | | | | |
| 2+1 IgG Crossfab; VH/VL exchange (LC007/V9) (SEQ ID NOs 3, 5, 29, 33) | 12.8 | 2.2 | 0 | 0 | 100 |
| 2+1 IgG Crossfab; VH/VL exchange (LC007/FN18) (SEQ ID NOs 3, 5, 35, 37) | 3.2 | 5.7 | 0.4 | 0 | 99.6 |
| 2+1 IgG scFab, P329G LALA (SEQ ID NOs 5, 21, 23) | 11.9 | 23 | 0.3 | 0 | 99.7 |
| 2+1 IgG scFab, LALA (SEQ ID NOs 5, 17, 19) | 9 | 23 | 0 | 0 | 100 |
| 2+1 IgG scFab, P329G LALA N297D (SEQ ID NOs 5, 25, 27) | 12.9 | 32.7 | 0 | 0 | 100 |
| 2+1 IgG scFab, wt (SEQ ID NOs 5, 13, 15) | 15.5 | 31.8 | 0 | 0 | 100 |
| 1+1 IgG scFab (SEQ ID NOs 5, 21, 213) | 7 | 24.5 | 0 | 0 | 100 |
| 1+1 IgG scFab "one armed" (SEQ ID NOs 1, 3, 5) | 7.6 | 43.7 | 2.3 | 0 | 97.7 |
| 1+1 IgG scFab "one armed inverted" (SEQ ID NOs 7, 9, 11) | 1 | 27 | 7.1 | 9.1 | 83.8 |
| 1+1 IgG Crossfab; VH/VL exchange (LC007/V9) (SEQ ID NOs 5, 29, 31, 33) | 9.8 | 0 | 0 | 0 | 100 |
| 2+1 IgG Crossfab, linked light chain; VL/VH exchange (LC007/V9) (SEQ ID NOs 3, 5, 29, 179) | 0.54 | 40 | 1.4 | 0 | 98.6 |
| 1+1 IgG Crossfab; VL/VH exchange (LC007/V9) (SEQ ID NOs 5, 29, 33, 181) | 6.61 | 8.5 | 0 | 0 | 100 |
| 1+1 CrossMab; CL/CH1 exchange (LC00/V9) (SEQ ID NOs 5, 23, 183, 185) | 6.91 | 10.5 | 1.3 | 1.7 | 97 |
| 2+1 IgG Crossfab, inverted; CL/CH1 exchange (LC007/V9) (SEQ ID NOs 5, 23, 183, 187) | 9.45 | 6.1 | 0.8 | 0 | 99.2 |
| 2+1 IgG Crossfab; VL/VH exchange (M4-3 ML2/V9) (SEQ ID NOs 33, 189, 191, 193) | 36.6 | 0 | 9.5 | 35.3 | 55.2 |
| 2+1 IgG Crossfab; CL/CH1 exchange (M4-3 ML2/V9) (SEQ ID NOs 183, 189, 193, 195) | 2.62 | 12 | 2.8 | 0 | 97.2 |
| 2+1 IgG Crossfab; CL/CH1 exchange (M4-3 ML2/H2C) (SEQ ID NOs 189, 193, 199, 201) | 29.75 | 0 | 0 | 0 | 100 |
| 2+1 IgG Crossfab; CL/CH1 exchange (LC007/anti-CD3) (SEQ ID NOs 5, 23, 215, 217) | 1.2 | 0 | 1.25 | 1.65 | 97.1 |
| 2+1 IgG Crossfab, inverted; CL/CH1 exchange (LC007/anti-CD3) (SEQ ID NOs 5, 23, 215, 219) | 7.82 | 0.5 | 0 | 0 | 100 |

| **EGFR** | | | | | |
|---|---|---|---|---|---|
| 2+1 IgG scFab (SEQ ID NOs 45, 47, 53) | 5.2 | 53 | 0 | 30 | 70 |
| 1+1 IgG scFab (SEQ ID NOs 47, 53, 213) | 3.4 | 66.6 | 0 | 1.6 | 98.4 |
| 1+1 IgG scFab "one armed" (SEQ ID NOs 43, 45, 47) | 9.05 | 60.8 | 0 | 0 | 100 |
| 1+1 IgG scFab "one armed inverted" (SEQ ID NOs 11, 49, 51) | 3.87 | 58.8 | 0 | 0 | 100 |

| **FAP** | | | | | |
|---|---|---|---|---|---|
| 2+1 IgG scFab (SEQ ID NOs 57, 59, 61) | 12.57 | 53 | 0 | 0 | 100 |
| 1+1 IgG scFab (SEQ ID NOs 57, 61, 213) | 17.95 | 41 | 0.4 | 0 | 99.6 |
| 1+1 IgG scFab "one armed inverted" (SEQ ID NOs 11, 51, 55) | 2.44 | 69 | 0.6 | 0 | 99.4 |

| **CEA** | | | | | |
|---|---|---|---|---|---|
| 2+1 IgG Crossfab, inverted; VL/VH exchange (CH1A1A/V9) (SEQ ID NOs 33, 63, 65, 67) | 0.34 | 13 | 4.4 | 0 | 95.6 |
| 2+1 IgG Crossfab, inverted; CL/CH1 exchange (CH1A1A/V9) (SEQ ID NOs 65, 67, 183, 197) | 12.7 | 43 | 0 | 0 | 100 |
| 2+1 IgG Crossfab, inverted; CL/CH1 exchange (431/26/V9) (SEQ ID NOs 183, 203, 205, 207) | 7.1 | 20 | 0 | 0 | 100 |
| 1+1 IgG-Crossfab light chain fusion (CH1A1A/V9) (SEQ ID NOs 183, 209, 211, 213) | 7.85 | 27 | 4.3 | 3.2 | 92.5 |

As controls, bispecific antigen binding molecules were generated in the prior art tandem scFv format ("(scFv)₂") and by fusing a tandem scFv to an Fc domain ("(scFv)₂-Fc"). The molecules were produced in HEK293-EBNA cells and purified by Protein A affinity chromatography followed by a size exclusion chromatographic step in an analogous manner as described above. Due to high aggregate formation, some of the samples had to be further purified by applying eluted and concentrated samples from the HiLoad Superdex 200 column (GE Healthcare) to a Superdex 10/300 GL column (GE Healthcare) equilibrated with 20 mM histidine, 140 mM sodium chloride, pH 6.7 in order to obtain protein with high monomer content. Subsequently, protein concentration, purity and molecular weight, and aggregate content were determined as described above.

Yields, aggregate content after the first purification step, and final monomer content for the control molecules is shown in Table 2B. Comparison of the aggregate content after the first purification step (Protein A) indicates the superior stability of the IgG Crossfab and IgG scFab constructs compared to the "(scFv)₂-Fc" and the disulfide bridge-stabilized "(dsscFv)₂-Fc" molecules.

**TABLE 2B. Yields, aggregate content after Protein A and final monomer content.**

| **Construct** | **Yield [mg/l]** | **Aggregates after ProteinA [%]** | **Final** | | |
|---|---|---|---|---|---|
| | | | **HMW [%]** | **LMW [%]** | **Monomer [%]** |
| (scFv)₂-Fc (antiMCSP/anti huCD3) | 76.5 | 40 | 0.5 | 0 | 99.5 |
| (dsscFv)₂-Fc (antiMCSP/anti huCD3) | 2.65 | 48 | 7.3 | 8.0 | 84.7 |

Thermal stability of the proteins was monitored by Dynamic Light Scattering (DLS). 30 • g of filtered protein sample with a protein concentration of 1 mg/ml was applied in duplicate to a Dynapro plate reader (Wyatt Technology Corporation; USA). The temperature was ramped from 25 to75°C at 0.05°C/min, with the radius and total scattering intensity being collected. The results are shown in Figure 15 and Table 2C. For the "(scFv)2-Fc" (antiMCSP/anti huCD3) molecule two aggregation points were observed, at 49°C and 68°C. The "(dsscFv)₂-Fc" construct has an increased aggregation temperature (57°C) as a result of the introduced disulfide bridge (Figure 15A, Table 2C). Both, the "2+1 IgG scFab" and the "2+1 IgG Crossfab" constructs are aggregating at temperatures higher than 60°C, demonstrating their superior thermal stability as compared to the "(scFv)₂-Fc" and "(dsscFv)₂-Fc" formats (Figure 15B, Table 2C).

**TABLE 2C. Thermal stability determined by dynamic light scattering.**

| **Construct** | **T_{agg} [**°**C]** |
|---|---|
| 2+1 IgG scFab (LC007/V9) | 68 |
| 2+1 IgG Crossfab (LC007/V9) | 65 |
| Fc-(scFv)2 (LC007/V9) | 49/68 |
| Fc-(dsscFv)2 (LC007/V9) | 57 |

### Example 2

### Surface Plasmon resonance analysis of Fc receptor and target antigen binding

### Method

All surface plasmon resonance (SPR) experiments are performed on a Biacore T100 at 25°C with HBS-EP as running buffer (0.01 M HEPES pH 7.4, 0.15 M NaCl, 3 mM EDTA, 0.005% Surfactant P20, Biacore, Freiburg/Germany).

### Analysis of FcR binding of different Fc-variants

The assay setup is shown in Figure 16A. For analyzing interaction of different Fc-variants with human FcyRIIIa-V158 and murine FcyRIV direct coupling of around 6,500 resonance units (RU) of the anti-Penta His antibody (Qiagen) is performed on a CM5 chip at pH 5.0 using the standard amine coupling kit (Biacore, Freiburg/Germany). HuFcγRIIIa-V158-K6H6 and muFcγRIV-aviHis-biotin are captured for 60 s at 4 and 10 nM respectively.

Constructs with different Fc-mutations are passed through the flow cells for 120 s at a concentration of 1000 nM with a flow rate of 30 µl/min. The dissociation is monitored for 220 s. Bulk refractive index differences are corrected for by subtracting the response obtained in a reference flow cell. Here, the Fc-variants are flown over a surface with immobilized anti-Penta His antibody but on which HBS-EP has been injected rather than HuFcγRIIIa-V158-K6H6 or muFcγRIV-aviHis-biotin. Affinity for human FcγRIIIa-V158 and murine FcyRIV was determined for wild-type Fc using a concentration range from 500 - 4000 nM.

The steady state response was used to derive the dissociation constant K_{D} by non-linear curve fitting of the Langmuir binding isotherm. Kinetic constants were derived using the Biacore T100 Evaluation Software (vAA, Biacore AB, Uppsala/Sweden), to fit rate equations for 1:1 Langmuir binding by numerical integration.

### Result

The interaction of Fc variants with human FcγRIIIa and murine FcyRIV was monitored by surface plasmon resonance. Binding to captured huFcγRIIIa-V158-K6H6 and muFcγRIV-aviHis-biotin is significantly reduced for all analyzed Fc mutants as compared to the construct with a wild-type (wt) Fc domain.

The Fc mutants with the lowest binding to the human Fcγ-receptor were P329G L234A L235A (LALA) and P329G LALA N297D. The LALA mutation alone was not enough to abrogate binding to huFcγRIIIa-V158-K6H6. The Fc variant carrying only the LALA mutation had a residual binding affinity to human FcγRIIIa of 2.100 nM, while the wt Fc bound the human FcγRIIIa receptor with an affinity of 600 nM (Table 3). Both K_{D} values were derived by 1:1 binding model, using a single concentration.

Affinity to human FcγRIIIa-V158 and murine FcγRIV could only be analyzed for wt Fc. K_{D} values are listed in Table 3. Binding to the murine FcγRIV was almost completely eliminated for all analyzed Fc mutants.

**TABLE 3. Affinity of Fc-variants to the human FcγRIIIa-V158 and murine FcγRIV.**

| **K_{D} in nM** **T = 25°C** | **human FcγRIIIa-V158** | | **murine FcγRIV** | |
|---|---|---|---|---|
| | **kinetic** | **steady state** | **kinetic** | **steady state** |
| Fc-wt (SEQ ID NOs 5, 13, 15) | 600* (1200) | 3470 | 576 | 1500 |
| Fc-LALA (SEQ ID NOs 5, 17, 19) | 2130* | n.d. | n.d. | |
| Fc-P329G LALA (SEQ ID NOs 5, 21, 23) | n.d. | | n.d. | |
| Fc-P329G LALA N297D (SEQ ID NOs 5, 25, 27) | n.d. | | n.d. | |

| | | | | |
|---|---|---|---|---|
| *determined using one concentration (1000 nM) | | | | |

### Analysis of simultaneous binding to tumor antigen and CD3

Analysis of simultaneous binding of the T-cell bispecific constructs to the tumor antigen and the human CD3ε was performed by direct coupling of 1650 resonance units (RU) of biotinylated D3 domain of MCSP on a sensor chip SA using the standard coupling procedure. Human EGFR was immobilized using standard amino coupling procedure. 8000 RU were immobilized on a CM5 sensor chip at pH 5.5. The assay setup is shown in Figure 16B.

Different T-cell bispecific constructs were captured for 60 s at 200 nM. Human CD3γ(G₄S)₅CD3ε-AcTev-Fc(knob)-Avi/Fc(hole) was subsequently passed at a concentration of 2000 nM and a flow rate of 40 µl/min for 60 s. Bulk refractive index differences were corrected for by subtracting the response obtained on a reference flow cell where the recombinant CD3ε was flown over a surface with immobilized D3 domain of MCSP or EGFR without captured T-cell bispecific constructs.

### Result

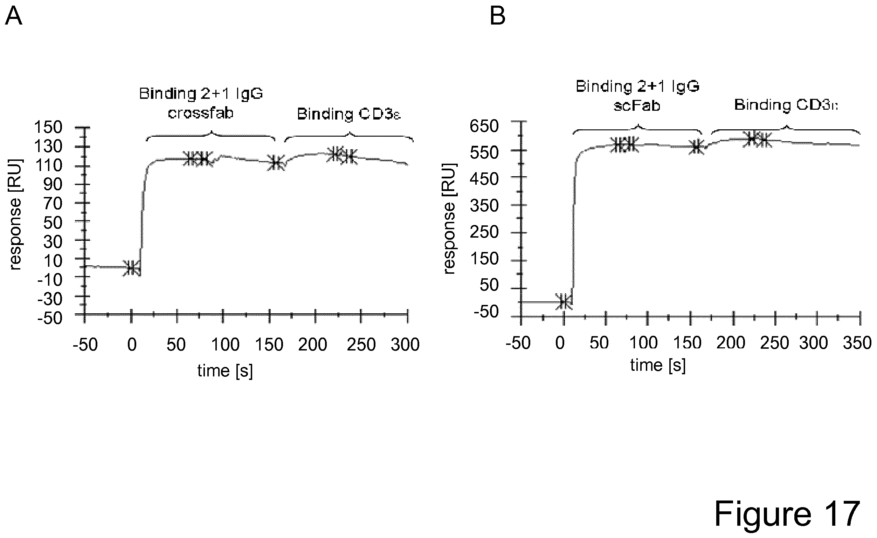

Simultaneous binding to both tumor antigen and human CD3ε was analyzed by surface plasmon resonance (Figure 17, Figure 18). All constructs were able to bind the tumor antigen and the CD3 simultaneously. For most of the constructs the binding level (RU) after injection of human CD3ε was higher than the binding level achieved after injection of the construct alone reflecting that both tumor antigen and the human CD3ε were bound to the construct.

### Example 3

### Binding of bispecific constructs to the respective target antigen on cells

Binding of the different bispecific constructs to CD3 on Jurkat cells (ATCC #TIB-152), and the respective tumor antigen on target cells, was determined by FACS. Briefly, cells were harvested, counted and checked for viability. 0.15 - 0.2 million cells per well (in PBS containing 0.1% BSA; 90 µl) were plated in a round-bottom 96-well plate and incubated with the indicated concentration of the bispecific constructs and corresponding IgG controls (10 µl) for 30 min at 4°C. For a better comparison, all constructs and IgG controls were normalized to same molarity. After the incubation, cells were centrifuged (5 min, 350 x g), washed with 150 µl PBS containing 0.1% BSA, resuspended and incubated for further 30 min at 4°C with 12 µl/well of a FITC-or PE-conjugated secondary antibody. Bound constructs were detected using a FACSCantoII (Software FACS Diva). The "(scFv)₂" molecule was detected using a FITC-conjugated anti-His antibody (Lucerna, #RHIS-45F-Z). For all other molecules, a FITC- or PE-conjugated AffiniPure F(ab')2 Fragment goat anti-human IgG Fcγ Fragment Specific (Jackson Immuno Research Lab # 109-096-098 / working solution 1:20, or #109-116-170 / working solution 1:80, respectively) was used. Cells were washed by addition of 120 µl/well PBS containing 0.1% BSA and centrifugation at 350 x g for 5 min. A second washing step was performed with 150 µl/well PBS containing 0.1% BSA. Unless otherwise indicated, cells were fixed with 100 µl/well fixation buffer (BD #554655) for 15 min at 4°C in the dark, centrifuged for 6 min at 400 x g and kept in 200 µl/well PBS containing 0.1% BSA until the samples were measured with FACS CantoII. EC50 values were calculated using the GraphPad Prism software. In a first experiment, different bispecific constructs targeting human MCSP and human CD3 were analyzed by flow cytometry for binding to human CD3 expressed on Jurkat, human T cell leukaemia cells, or to human MCSP on Colo-38 human melanoma cells.

Results are presented in Figure 19-21, which show the mean fluorescence intensity of cells that were incubated with the bispecific molecule, control IgG, the secondary antibody only, or left untreated.

As shown in Figure 19, for both antigen binding moieties of the "(scFv)₂" molecule, i.e. CD3 (Figure 191A) and MCSP (Figure 19B), a clear binding signal is observed compared to the control samples.

The "2+1 IgG scFab" molecule (SEQ ID NOs 5, 17, 19) shows good binding to huMCSP on Colo-38 cells (Figure 20A). The CD3 moiety binds CD3 slightly better than the reference anti-human CD3 IgG (Figure 20B).

As depicted in Figure 21A, the two "1+1" constructs show comparable binding signals to human CD3 on cells. The reference anti-human CD3 IgG gives a slightly weaker signal. In addition, both constructs tested ("1+1 IgG scFab, one-armed" (SEQ ID NOs 1, 3, 5) and "1+1 IgG scFab, one-armed inverted" (SEQ ID NOs 7, 9, 11)) show comparable binding to human MCSP on cells (Figure 21B). The binding signal obtained with the reference anti-human MCSP IgG is slightly weaker.

In another experiment, the purified "2+1 IgG scFab" bispecific construct (SEQ ID NOs 5, 17, 19) and the corresponding anti human MCSP IgG were analyzed by flow cytometry for dose-dependent binding to human MCSP on Colo-38 human melanoma cells, to determine whether the bispecific construct binds to MCSP via one or both of its "arms". As depicted in Figure 22, the "2+1 IgG scFab" construct shows the same binding pattern as the MCSP IgG.

In yet another experiment, the binding of CD3/CEA "2+1 IgG Crossfab, inverted" bispecific constructs with either a VL/VH (see SEQ ID NOs 33, 63, 65, 67) or a CL/CH1 exchange (see SEQ ID NOs 66, 67, 183, 197) in the Crossfab fragment to human CD3, expressed by Jurkat cells, or to human CEA, expressed by LS-174T cells, was assessed. As a control, the equivalent maximum concentration of the corresponding IgGs and the background staining due to the labeled 2ndary antibody (goat anti-human FITC-conjugated AffiniPure F(ab')2 Fragment, Fcγ Fragment-specific, Jackson Immuno Research Lab # 109-096-098) were assessed as well. As illustrated in Figure 55, both constructs show good binding to human CEA, as well as to human CD3 on cells. The calculated EC50 values were 4.6 and 3.9 nM (CD3), and 9.3 and 6.7 nM (CEA) for the "2+1 IgG Crossfab, inverted (VL/VH)" and the "2+1 IgG Crossfab, inverted (CL/CH1)" constructs, respectively.

In another experiment, the binding of CD3/MCSP "2+1 IgG Crossfab" (see SEQ ID NOs 3, 5, 29, 33) and "2+1 IgG Crossfab, inverted" (see SEQ ID NOs 5, 23, 183, 187) constructs to human CD3, expressed by Jurkat cells, or to human MCSP, expressed by WM266-4 cells, was assessed. Figure 56 shows that, while binding of both constructs to MCSP on cells was comparably good, the binding of the "inverted" construct to CD3 was reduced compared to the other construct. The calculated EC50 values were 6.1 and 1.66 nM (CD3), and 0.57 and 0.95 nM (MCSP) for the "2+1 IgG Crossfab, inverted" and the "2+1 IgG Crossfab" constructs, respectively.

In a further experiment, binding of the "1+1 IgG Crossfab light chain (LC) fusion" construct (SEQ ID NOs 183, 209, 211, 213) to human CD3, expressed by Jurkat cells, and to human CEA, expressed by LS-174T cells was determined. As a control, the equivalent maximum concentration of the corresponding anti-CD3 and anti-CEA IgGs and the background staining due to the labeled 2ndary antibody (goat anti-human FITC-conjugated AffiniPure F(ab')2 Fragment, Fcγ Fragment-specific, Jackson Immuno Research Lab #109-096-098) were assessed as well. As depicted in Figure 57, the binding of the "1+1 IgG Crossfab LC fusion" to CEA appears to be greatly reduced, whereas the binding to CD3 was at least comparable to the reference IgG.

In a final experiment, binding of the "2+1 IgG Crossfab" (SEQ ID NOs 5, 23, 215, 217) and the "2+1 IgG Crossfab, inverted" (SEQ ID NOs 5, 23, 215, 219) constructs to human CD3, expressed by Jurkat cells, and to human MCSP, expressed by WM266-4 tumor cells was determined. As depicted in Figure 58 the binding to human CD3 was reduced for the "2+1 IgG Crossfab, inverted" compared to the other construct, but the binding to human MCSP was comparably good. The calculated EC50 values were 10.3 and 32.0 nM (CD3), and 3.1 and 3.4 nM (MCSP) for the "2+1 IgG Crossfab" and the "2+1 IgG Crossfab, inverted" construct, respectively.

### Example 4

### FACS analysis of surface activation markers on primary human T cells upon engagement of bispecific constructs

The purified huMCSP-huCD3-targeting bispecific "2+1 IgG scFab" (SEQ ID NOs 5, 17, 19) and "(scFv)₂" molecules were tested by flow cytometry for their potential to up-regulate the early surface activation marker CD69, or the late activation marker CD25 on CD8⁺ T cells in the presence of human MCSP-expressing tumor cells.

Briefly, MCSP-positive Colo-38 cells were harvested with Cell Dissociation buffer, counted and checked for viability. Cells were adjusted to 0.3 x 10⁶ (viable) cells per ml in AIM-V medium, 100 µl of this cell suspension per well were pipetted into a round-bottom 96-well plate (as indicated). 50 µl of the (diluted) bispecific construct were added to the cell-containing wells to obtain a final concentration of 1 nM. Human PBMC effector cells were isolated from fresh blood of a healthy donor and adjusted to 6 x 10⁶ (viable) cells per ml in AIM-V medium. 50 µl of this cell suspension was added per well of the assay plate (see above) to obtain a final E:T ratio of 10:1. To analyze whether the bispecific constructs are able to activate T cells exclusively in the presence of target cells expressing the tumor antigen huMCSP, wells were included that contained 1 nM of the respective bispecific molecules, as well as PBMCs, but no target cells. After incubation for 15 h (CD69), or 24 h (CD25) at 37°C, 5% CO₂, cells were centrifuged (5 min, 350 x g) and washed twice with 150 µl/well PBS containing 0.1% BSA. Surface staining for CD8 (mouse IgG1,κ; clone HIT8a; BD #555635), CD69 (mouse IgG1; clone L78; BD #340560) and CD25 (mouse IgG1,κ; clone M-A251; BD #555434) was performed at 4°C for 30 min, according to the supplier's suggestions. Cells were washed twice with 150 µl/well PBS containing 0.1% BSA and fixed for 15 min at 4°C, using 100 µl/well fixation buffer (BD #554655). After centrifugation, the samples were resuspended in 200 µl/well PBS with 0.1% BSA and analyzed using a FACS CantoII machine (Software FACS Diva).

Figure 23 depicts the expression level of the early activation marker CD69 (A), or the late activation marker CD25 (B) on CD8⁺ T cells after 15 hours or 24 hours incubation, respectively. Both constructs induce up-regulation of both activation markers exclusively in the presence of target cells. The "(scFv)₂" molecule seems to be slightly more active in this assay than the "2+1 IgG scFab" construct.

The purified huMCSP-huCD3-targeting bispecific "2+1 IgG scFab" and "(scFv)₂" molecules were further tested by flow cytometry for their potential to up-regulate the late activation marker CD25 on CD8⁺ T cells or CD4⁺ T cells in the presence of human MCSP-expressing tumor cells. Experimental procedures were as described above, using human pan T effector cells at an E:T ratio of 5:1 and an incubation time of five days.

Figure 24 shows that both constructs induce up-regulation of CD25 exclusively in the presence of target cells on both, CD8⁺ (A) as well as CD4⁺ (B) T cells. The "2+1 IgG scFab" construct seems to induce less up-regulation of CD25 in this assay, compared to the "(scFv)₂" molecule. In general, the up-regulation of CD25 is more pronounced on CD8⁺ than on CD4⁺ T cells.

In another experiment, purified "2+1 IgG Crossfab" targeting cynomolgus CD3 and human MCSP (SEQ ID NOs 3, 5, 35, 37) was analyzed for its potential to up-regulate the surface activation marker CD25 on CD8⁺ T cells in the presence of tumor target cells. Briefly, human MCSP-expressing MV-3 tumor target cells were harvested with Cell Dissociation Buffer, washed and resuspendend in DMEM containing 2% FCS and 1% GlutaMax. 30 000 cells per well were plated in a round-bottom 96-well plate and the respective antibody dilution was added at the indicated concentrations (Figure 25). The bispecific construct and the different IgG controls were adjusted to the same molarity. Cynomolgus PBMC effector cells, isolated from blood of two healthy animals, were added to obtain a final E:T ratio of 3:1. After an incubation for 43 h at 37°C, 5% CO₂, the cells were centrifuged at 350 x g for 5 min and washed twice with PBS, containing 0.1% BSA. Surface staining for CD8 (Miltenyi Biotech #130-080-601) and CD25 (BD #557138) was performed according to the supplier's suggestions. Cells were washed twice with 150 µl/well PBS containing 0.1% BSA and fixed for 15 min at 4°C, using 100 µl/well fixation buffer (BD #554655). After centrifugation, the samples were resuspended in 200 µl/well PBS with 0.1% BSA and analyzed using a FACS CantoII machine (Software FACS Diva).

As depicted in Figure 25, the bispecific construct induces concentration-dependent up-regulation of CD25 on CD8⁺ T cells only in the presence of target cells. The anti cyno CD3 IgG (clone FN-18) is also able to induce up-regulation of CD25 on CD8⁺ T cells, without being crosslinked (see data obtained with cyno Nestor). There is no hyperactivation of cyno T cells with the maximal concentration of the bispecific construct (in the absence of target cells).

In another experiment, the CD3-MCSP "2+1 IgG Crossfab, linked light chain" (see SEQ ID NOs 3, 5, 29, 179) was compared to the CD3-MCSP "2+1 IgG Crossfab" (see SEQ ID NOs 3, 5, 29, 33) for its potential to up-regulate the early activation marker CD69 or the late activation marker CD25 on CD8⁺ T cells in the presence of tumor target cells. Primary human PBMCs (isolated as described above) were incubated with the indicated concentrations of bispecific constructs for at least 22 h in the presence or absence of MCSP-positive Colo38 target cells. Briefly, 0.3 million primary human PBMCs were plated per well of a flat-bottom 96-well plate, containing the MCSP-positive target cells (or medium). The final effector to target cell (E:T) ratio was 10:1. The cells were incubated with the indicated concentration of the bispecific constructs and controls for the indicated incubation times at 37°C, 5% CO₂. The effector cells were stained for CD8, and CD69 or CD25 and analyzed by FACS CantoII.

Figure 53 shows the result of this experiment. There were no significant differences detected for CD69 (A) or CD25 up-regulation (B) between the two 2+1 IgG Crossfab molecules (with or without the linked light chain).

In yet another experiment, the CD3/MCSP "2+1 IgG Crossfab" (see SEQ ID NOs 3, 5, 29, 33) and "1+1 IgG Crossfab" (see SEQ ID NOs 5, 29, 33, 181) constructs were compared to the "1+1 CrossMab" construct (see SEQ ID NOs 5, 23, 183, 185) for their potential to up-regulate CD69 or CD25 on CD4⁺ or CD8⁺ T cells in the presence of tumor target cells. The assay was performed as described above, in the presence of absence of human MCSP expressing MV-3 tumor cells, with an incubation time of 24 h.

As shown in Figure 59, the "1+1 IgG Crossfab" and "2+1 IgG Crossfab" constructs induced more pronounced upregulation of activation markers than the "1+1 CrossMab" molecule.

In a final experiment, the CD3/MCSP "2+1 IgG Crossfab" (see SEQ ID NOs 5, 23, 215, 217) and "2+1 IgG Crossfab, inverted" (see SEQ ID NOs 5, 23, 215, 219) constructs were assessed for their potential to up-regulate CD25 on CD4⁺ or CD8⁺ T cells from two different cynomolgus monkeys in the presence of tumor target cells. The assay was performed as described above, in the presence of absence of human MCSP expressing MV-3 tumor cells, with an E:T ratio of 3:1 and an incubation time of about 41 h.

As shown in Figure 60, both constructs were able to up-regulate CD25 on CD4⁺ and CD8⁺ T cells in a concentration-dependent manner, without significant difference between the two formats. Control samples without antibody and without target cells gave a comparable signal to the samples with antibody but no targets (not shown).

### Example 5

### Interferon-γ secretion upon activation of human pan T cells with CD3 bispecific constructs

Purified "2+1 IgG scFab" targeting human MCSP and human CD3 (SEQ ID NOs 5, 17, 19) was analyzed for its potential to induce T cell activation in the presence of human MCSP-positive U-87MG cells, measured by the release of human interferon (IFN)-y into the supernatant. As controls, anti-human MCSP and anti-human CD3 IgGs were used, adjusted to the same molarity. Briefly, huMCSP-expressing U-87MG glioblastoma astrocytoma target cells (ECACC 89081402) were harvested with Cell Dissociation Buffer, washed and resuspendend in AIM-V medium (Invitrogen #12055-091). 20 000 cells per well were plated in a round-bottom 96-well-plate and the respective antibody dilution was added to obtain a final concentration of 1 nM. Human pan T effector cells, isolated from Buffy Coat, were added to obtain a final E:T ratio of 5:1. After an overnight incubation of 18.5 h at 37°C, 5% CO₂, the assay plate was centrifuged for 5 min at 350 x g and the supernatant was transferred into a fresh 96-well plate. Human IFN-γ levels in the supernatant were measured by ELISA, according to the manufacturer's instructions (BD OptEIA human IFN-γ ELISA Kit II from Becton Dickinson, #550612).

As depicted in Figure 26, the reference IgGs show no to weak induction of IFN-γ secretion, whereas the "2+1 IgG scFab" construct is able to activate human T cells to secrete IFN-γ.

### Example 6

### Re-directed T cell cytotoxicity mediated by cross-linked bispecific constructs targeting CD3 on T cells and MCSP or EGFR on tumor cells (LDH release assay)

In a first series of experiments, bispecific constructs targeting CD3 and MCSP were analyzed for their potential to induce T cell-mediated apoptosis in tumor target cells upon crosslinkage of the construct via binding of the antigen binding moieties to their respective target antigens on cells (Figures 27-38).

In one experiment purified "2+1 IgG scFab" (SEQ ID NOs 5, 21, 23) and "2+1 IgG Crossfab" (SEQ ID NOs 3, 5, 29, 33) constructs targeting human CD3 and human MCSP, and the corresponding "(scFv)₂" molecule, were compared. Briefly, huMCSP-expressing MDA-MB-435 human melanoma target cells were harvested with Cell Dissociation Buffer, washed and resuspendend in AIM-V medium (Invitrogen # 12055-091). 30 000 cells per well were plated in a round-bottom 96-well plate and the respective dilution of the construct was added at the indicated concentration. All constructs and corresponding control IgGs were adjusted to the same molarity. Human pan T effector cells were added to obtain a final E:T ratio of 5:1. As a positive control for the activation of human pan T cells, 1 µg/ml PHA-M (Sigma #L8902; mixture of isolectins isolated from Phaseolus vulgaris) was used. For normalization, maximal lysis of the target cells (= 100%) was determined by incubation of the target cells with a final concentration of 1% Triton X-100. Minimal lysis (= 0%) refers to target cells co-incubated with effector cells, but without any construct or antibody. After an overnight incubation of 20 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 27, both "2+1" constructs induce apoptosis in target cells comparable to the "(scFv)₂" molecule.

Further, purified "2+1 IgG Crossfab" (SEQ ID NOs 3, 5, 29, 33) and "2+1 IgG scFab" constructs differing in their Fc domain, as well as the "(scFv)₂" molecule, were compared. The different mutations in the Fc domain (L234A+L235A (LALA), P329G and/or N297D, as indicated) reduce or abolish the (NK) effector cell function induced by constructs containing a wild-type (wt) Fc domain. Experimental procedures were as described above.

Figure 28 shows that all constructs induce apoptosis in target cells comparable to the "(scFv)₂" molecule.

Figure 29 shows the result of a comparison of the purified "2+1 IgG scFab" (SEQ ID NOs 5, 17, 19) and the "(scFv)₂" molecule for their potential to induce T cell-mediated apoptosis in tumor target cells. Experimental procedures were as decribed above, using huMCSP-expressing Colo-38 human melanoma target cells at an E:T ratio of 5:1, and an overnight incubation of 18.5 h. As depicted in the figure, the "2+1 IgG scFab" construct shows comparable cytotoxic activity to the "(scFv)₂" molecule.

Similarly, Figure 30 shows the result of a comparison of the purified "2+1 IgG scFab" construct (SEQ ID NOs 5, 17, 19)and the "(scFv)₂" molecule, using huMCSP-expressing Colo-38 human melanoma target cells at an E:T ratio of 5:1 and an incubation time of 18 h. As depicted in the figure, the "2+1 IgG scFab" construct shows comparable cytotoxic activity to the (scFv)₂ molecule.

Figure 31 shows the result of a comparison of the purified "2+1 IgG scFab" construct (SEQ ID NOs 5, 17, 19) and the "(scFv)₂" molecule, using huMCSP-expressing MDA-MB-435 human melanoma target cells at an E:T ratio of 5:1 and an overnight incubation of 23.5 h. As depicted in the figure, the construct induces apoptosis in target cells comparably to the "(scFv)₂" molecule. The "2+1 IgG scFab" construct shows reduced efficacy at the highest concentrations.

Furthermore, different bispecific constructs that are monovalent for both targets, human CD3 and human MCSP, as well as the corresponding "(scFv)₂" molecule were analyzed for their potential to induce T cell-mediated apoptosis. Figure 32 shows the results for the "1+1 IgG scFab, one-armed" (SEQ ID NOs 1, 3, 5) and "1+1 IgG scFab, one-armed inverted" (SEQ ID NOs 7, 9, 11) constructs, using huMCSP-expressing Colo-38 human melanoma target cells at an E:T ratio of 5:1, and an incubation time of 19 h. As depicted in the figure, both "1+1" constructs are less active than the "(scFv)₂" molecule, with the "1+1 IgG scFab, one-armed" molecule being superior to the "1+1 IgG scFab, one-armed inverted" molecule in this assay.

Figure 33 shows the results for the "1+1 IgG scFab" construct (SEQ ID NOs 5, 21, 213), using huMCSP-expressing Colo-38 human melanoma target cells at an E:T ratio of 5:1, and an incubation time of 20 h. As depicted in the figure, the "1+1 IgG scFab" construct is less cytotoxic than the "(scFv)₂" molecule.

In a further experiment the purified "2+1 IgG Crossfab" (SEQ ID NOs 3, 5, 29, 33), the "1+1 IgG Crossfab" (SEQ ID NOs 5, 29, 31, 33) and the "(scFv)₂" molecule were analyzed for their potential to induce T cell-mediated apoptosis in tumor target cells upon crosslinkage of the construct via binding of both target antigens, CD3 and MCSP, on cells. huMCSP-expressing MDA-MB-435 human melanoma cells were used as target cells, the E:T ratio was 5:1, and the incubation time 20 h. The results are shown in Figure 34. The "2+1 IgG Crossfab" construct induces apoptosis in target cells comparably to the "(scFv)₂" molecule. The comparison of the mono- and bivalent "IgG Crossfab" formats clearly shows that the bivalent one is much more potent.

In yet another experiment, the purified "2+1 IgG Crossfab" (SEQ ID NOs 3, 5, 29, 33) construct was analyzed for its potential to induce T cell-mediated apoptosis in different (tumor) target cells. Briefly, MCSP-positive Colo-38 tumor target cells, mesenchymal stem cells (derived from bone marrow, Lonza #PT-2501 or adipose tissue, Invitrogen #R7788-115) or pericytes (from placenta; PromoCell #C-12980), as indicated, were harvested with Cell Dissociation Buffer, washed and resuspendend in AIM-V medium (Invitrogen #12055-091). 30 000 cells per well were plated in a round-bottom 96-well plate and the respective antibody dilution was added at the indicated concentrations. Human PBMC effector cells isolated from fresh blood of a healthy donor were added to obtain a final E:T ratio of 25:1. After an incubation of 4 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 35, significant T-cell mediated cytotoxicity could be observed only with Colo-38 cells. This result is in line with Colo-38 cells expressing significant levels of MCSP, whereas mesenchymal stem cells and pericytes express MCSP only very weakly.

The purified "2+1 IgG scFab" (SEQ ID NOs 5, 17, 19) construct and the "(scFv)₂" molecule were also compared to a glycoengineered anti-human MCSP IgG antibody, having a reduced proportion of fucosylated N-glycans in its Fc domain (MCSP GlycoMab). For this experiment huMCSP-expressing Colo-38 human melanoma target cells and human PBMC effector cells were used, either at a fixed E:T ratio of 25:1 (Figure 36A), or at different E:T ratios from 20:1 to 1:10 (Figure 36B). The different molecules were used at the concentrations indicated in Figure 36A, or at a fixed concentration of 1667 pM (Figure 36B). Read-out was done after 21 h incubation. As depicted in Figure 36A and B, both bispecific constructs show a higher potency than the MSCP GlycoMab.

In another experiment, purified "2+1 IgG Crossfab" targeting cynomolgus CD3 and human MCSP (SEQ ID NOs 3, 5, 35, 37) was analyzed. Briefly, human MCSP-expressing MV-3 tumor target cells were harvested with Cell Dissociation Buffer, washed and resuspendend in DMEM containing 2% FCS and 1% GlutaMax. 30 000 cells per well were plated in a round-bottom 96-well plate and the respective dilution of construct or reference IgG was added at the concentrations indicated. The bispecific construct and the different IgG controls were adjusted to the same molarity. Cynomolgus PBMC effector cells, isolated from blood of healthy cynomolgus, were added to obtain a final E:T ratio of 3:1. After incubation for 24 h or 43 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 37, the bispecific construct induces concentration-dependent LDH release from target cells. The effect is stronger after 43 h than after 24 h. The anti-cynoCD3 IgG (clone FN-18) is also able to induce LDH release of target cells without being crosslinked.

Figure 38 shows the result of a comparison of the purified "2+1 IgG Crossfab" (SEQ ID NOs 3, 5, 29, 33) and the "(scFv)₂" construct, using MCSP-expressing human melanoma cell line (MV-3) as target cells and human PBMCs as effector cells with an E:T ratio of 10:1 and an incubation time of 26 h. As depicted in the figure, the "2+1 IgG Crossfab" construct is more potent in terms of EC50 than the "(scFv)₂" molecule.

In a second series of experiments, bispecific constructs targeting CD3 and EGFR were analyzed for their potential to induce T cell-mediated apoptosis in tumor target cells upon crosslinkage of the construct via binding of the antigen binding moieties to their respective target antigens on cells (Figures 39-41).

In one experiment purified "2+1 IgG scFab" (SEQ ID NOs 45, 47, 53) and "1+1 IgG scFab" (SEQ ID NOs 47, 53, 213) constructs targeting CD3 and EGFR, and the corresponding "(scFv)₂" molecule, were compared. Briefly, human EGFR-expressing LS-174T tumor target cells were harvested with trypsin, washed and resuspendend in AIM-V medium (Invitrogen # 12055-091). 30 000 cells per well were plated in a round-bottom 96-well-plate and the respective antibody dilution was added at the indicated concentrations. All constructs and controls were adjusted to the same molarity. Human pan T effector cells were added to obtain a final E:T ratio of 5:1. As a positive control for the activation of human pan T cells, 1 µg/ml PHA-M (Sigma #L8902) was used. For normalization, maximal lysis of the target cells (= 100%) was determined by incubation of the target cells with a final concentration of 1% Triton X-100. Minimal lysis (= 0%) refers to target cells co-incubated with effector cells, but without any construct or antibody. After an overnight incubation of 18 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 39, the "2+1 IgG scFab" construct shows comparable cytotoxic activity to the "(scFv)₂" molecule, whereas the "1+1 IgG scFab" construct is less active.

In another experiment the purified "1+1 IgG scFab, one-armed" (SEQ ID NOs 43, 45, 47), "1+1 IgG scFab, one-armed inverted" (SEQ ID NOs 11, 49, 51), "1+1 IgG scFab" (SEQ ID NOs 47, 53, 213), and the "(scFv)₂" molecule were compared. Experimental conditions were as described above, except for the incubation time which was 21 h.

As depicted in Figure 40, the "1+1 IgG scFab" construct shows a slightly lower cytotoxic activity than the "(scFv)₂" molecule in this assay. Both "1+1 IgG scFab, one-armed (inverted)" constructs are clearly less active than the "(scFv)₂" molecule.

In yet a further experiment the purified "1+1 IgG scFab, one-armed" (SEQ ID NO 43, 45, 47) and "1+1 IgG scFab, one-armed inverted" (SEQ ID NOs 11, 49, 51) constructs and the "(scFv)₂" molecule were compared. The incubation time in this experiment was 16 h, and the result is depicted in Figure 41. Incubated with human pan T cells, both "1+1 IgG scFab, one-armed (inverted)" constructs are less active than the "(scFv)₂" molecule, but show concentration-dependent release of LDH from target cells (Figure 41A). Upon co-cultivation of the LS-174T tumor cells with naive T cells isolated from PBMCs, the constructs had only a basal activity - the most active among them being the "(scFv)₂" molecule (Figure 41B).

In a further experiment, purified "1+1 IgG scFab, one-armed inverted" (SEQ ID NOs 11, 51, 55), "1+1 IgG scFab" (57, 61, 213), and "2+1 IgG scFab" (57, 59, 61) targeting CD3 and Fibroblast Activation Protein (FAP), and the corresponding "(scFv)₂" molecule were analyzed for their potential to induce T cell-mediated apoptosis in human FAP-expressing fibroblasts GM05389 cells upon crosslinkage of the construct via binding of both targeting moieties to their respective target antigens on the cells. Briefly, human GM05389 target cells were harvested with trypsin on the day before, washed and resuspendend in AIM-V medium (Invitrogen #12055-091). 30 000 cells per well were plated in a round-bottom 96-well plate and incubated overnight at 37°C, 5% CO₂ to allow the cells to recover and adhere. The next day, the cells were centrifuged, the supernatant was discarded and fresh medium, as well as the respective dilution of the constructs or reference IgGs was added at the indicated concentrations. All constructs and controls were adjusted to the same molarity. Human pan T effector cells were added to obtain a final E:T ratio of 5:1. As a positive control for the activation of human pan T cells, 5 µg/ml PHA-M (Sigma #L8902) was used. For normalization, maximal lysis of the target cells (= 100%) was determined by incubation of the target cells with a final concentration of 1% Triton X-100. Minimal lysis (= 0%) refers to target cells co-incubated with effector cells, but without any construct or antibody. After an additional overnight incubation of 18 h at 37°C, 5% CO₂, LDH release of apoptotic/necrotic target cells into the supernatant was measured with the LDH detection kit (Roche Applied Science, #11 644 793 001), according to the manufacturer's instructions.

As depicted in Figure 42, the "2+1 IgG scFab" construct shows comparable cytotoxic activity to the "(scFv)₂" molecule in terms of EC50 values. The "1+1 IgG scFab, one-armed inverted" construct is less active than the other constructs tested in this assay.

In another set of experiments, the CD3/MCSP "2+1 IgG Crossfab, linked light chain" (see SEQ ID NOs 3, 5, 29, 179) was compared to the CD3/MCSP "2+1 IgG Crossfab" (see SEQ ID NOs 3, 5, 29, 33). Briefly, target cells (human Colo-38, human MV-3 or WM266-4 melanoma cells) were harvested with Cell Dissociation Buffer on the day of the assay (or with trypsin one day before the assay was started), washed and resuspended in the appropriate cell culture medium (RPMI1640, including 2% FCS and 1% Glutamax). 20 000 - 30 000 cells per well were plated in a flat-bottom 96-well plate and the respective antibody dilution was added as indicated (triplicates). PBMCs as effector cells were added to obtain a final effector-to-target cell (E:T) ratio of 10:1. All constructs and controls were adjusted to the same molarity, incubation time was 22 h. Detection of LDH release and normalization was done as described above.

Figure 49 to 52 show the result of four assays performed with MV-3 melanoma cells (Figure 49), Colo-38 cells (Figure 50 and 51) or WM266-4 cells (Figure 52). As shown in Figure 49, the construct with the linked light chain was less potent compared to the one without the linked light chain in the assay with MV-3 cells as target cells. As shown in Figure 50 and 51, the construct with the linked light chain was more potent compared to the one without the linked light chain in the assays with high MCSP expressing Colo-38 cells as target cells. Finally, as shown in Figure 52, there was no significant difference between the two constructs when high MCSP-expressing WM266-4 cells were used as target cells.

In another experiment, two CEA-targeting "2+1 IgG Crossfab, inverted" constructs were compared, wherein in the Crossfab fragment either the V regions (VL/VH, see SEQ ID NOs 33, 63, 65, 67) or the C regions (CL/CH1, see SEQ ID NOs 65, 67, 183, 197) were exchanged. The assay was performed as described above, using human PBMCs as effector cells and human CEA-expressing target cells. Target cells (MKN-45 or LS-174T tumor cells) were harvested with trypsin-EDTA (LuBiosciences #25300-096), washed and resuspendend in RPMI1640 (Invitrogen #42404042), including 1% Glutamax (LuBiosciences #35050087) and 2% FCS. 30 000 cells per well were plated in a round-bottom 96-well plate and the bispecific constructs were added at the indicated concentrations. All constructs and controls were adjusted to the same molarity. Human PBMC effector cells were added to obtain a final E:T ratio of 10:1, incubation time was 28 h. EC50 values were calculated using the GraphPad Prism 5 software.

As shown in Figure 61, the construct with the CL/CH1 exchange shows slightly better activity on both target cell lines than the construct with the VL/VH exchange. Calculated EC50 values were 115 and 243 pM on MKN-45 cells, and 673 and 955 pM on LS-174T cells, for the CL/CH1-exchange construct and the VL/VH-exchange construct, respectively.

Similarly, two MCSP-targeting "2+1 IgG Crossfab" constructs were compared, wherein in the Crossfab fragment either the V regions (VL/VH, see SEQ ID NOs 33, 189, 191, 193) or the C regions (CL/CH1, see SEQ ID NOs 183, 189, 193, 195) were exchanged. The assay was performed as described above, using human PBMCs as effector cells and human MCSP-expressing target cells. Target cells (WM266-4) were harvested with Cell Dissociation Buffer (LuBiosciences #13151014), washed and resuspendend in RPMI1640 (Invitrogen #42404042), including 1% Glutamax (LuBiosciences #35050087) and 2% FCS. 30 000 cells per well were plated in a round-bottom 96-well plate and the constructs were added at the indicated concentrations. All constructs and controls were adjusted to the same molarity. Human PBMC effector cells were added to obtain a final E:T ratio of 10:1, incubation time was 26 h. EC50 values were calculated using the GraphPad Prism 5 software.

As depicted in Figure 62, the two constructs show comparable activity, the construct with the CL/CH1 exchange having a slightly lower EC50 value (12.9 pM for the CL/CH1-exchange construct, compared to 16.8 pM for the VL/VH-exchange construct).

Figure 63 shows the result of a similar assay, performed with human MCSP-expressing MV-3 target cells. Again, both constructs show comparable activity, the construct with the CL/CH1 exchange having a slightly lower EC50 value (approximately 11.7 pM for the CL/CH1-exchange construct, compared to approximately 82.2 pM for the VL/VH-exchange construct). Exact EC50 values could not be calculated, since the killing curves did not reach a plateau at high concentrations of the compounds.

In a further experiment, the CD3/MCSP "2+1 IgG Crossfab" (see SEQ ID NOs 3, 5, 29, 33) and "1+1 IgG Crossfab" (see SEQ ID NOs 5, 29, 33, 181) constructs were compared to the CD3/MCSP "1+1 CrossMab" (see SEQ ID NOs 5, 23, 183, 185). The assay was performed as described above, using human PBMCs as effector cells and WM266-4 or MV-3 target cells (E:T ratio = 10:1) and an incubation time of 21 h.

As shown in Figure 64, the "2+1 IgG Crossfab" construct is the most potent molecule in this assay, followed by the "1+1 IgG Crossfab" and the "1+1 CrossMab". This ranking is even more pronounced with MV-3 cells, expressing medium levels of MCSP, compared to high MCSP expressing WM266-4 cells. The calculated EC50 values on MV-3 cells were 9.2, 40.9 and 88.4 pM, on WM266-4 cells 33.1, 28.4 and 53.9 pM, for the "2+1 IgG Crossfab", the "1+1 IgG Crossfab" and the "1+1 CrossMab", respectively.

In a further experiment, different concentrations of the "1+1 IgG Crossfab LC fusion" construct (SEQ ID NOs 183, 209, 211, 213) were tested, using MKN-45 or LS-174T tumor target cells and human PBMC effector cells at an E:T ratio of 10:1 and an incubation time of 28 hours. As shown in Figure 65, the "1+1 IgG Crossfab LC fusion" construct induced apoptosis in MKN-45 target cells with a calculated EC50 of 213 pM, whereas the calculated EC50 is 1.56 nM with LS-174T cells, showing the influence of the different tumor antigen expression levels on the potency of the bispecific constructs within a certain period of time.

In yet another experiment, the "1+1 IgG Crossfab LC fusion" construct (SEQ ID NOs 183, 209, 211, 213) was compared to a untargeted "2+1 IgG Crossfab" molecule. MC38-huCEA tumor cells and human PBMCs (E:T ratio = 10:1) and an incubation time of 24 hours were used. As shown in Figure 66, the "1+1 IgG Crossfab LC fusion" construct induced apoptosis of target cells in a concentration-dependent manner, with a calculated EC50 value of approximately 3.2 nM. In contrast, the untargeted "2+1 IgG Crossfab" showed antigen-independent T cell-mediated killing of target cells only at the highest concentration.

In a final experiment, the "2+1 IgG Crossfab (V9)" (SEQ ID NOs 3, 5, 29, 33), the "2+1 IgG Crossfab, inverted (V9)" (SEQ ID NOs 5, 23, 183, 187), the "2+1 IgG Crossfab (anti-CD3)" (SEQ ID NOs 5, 23, 215, 217), the "2+1 IgG Crossfab, inverted (anti-CD3)" (SEQ ID NOs 5, 23, 215, 219) were compared, using human MCSP-positive MV-3 or WM266-4 tumor cells and human PBMCs (E:T ratio = 10:1), and an incubation time of about 24 hours. As depicted in Figure 67, the T cell-mediated killing of the "2+1 IgG Crossfab, inverted" constructs seems to be slightly stronger or at least equal to the one induced by the "2+1 IgG Crossfabt" constructs for both CD3 binders. The calculated EC50 values were as follows:

| **EC50 [pM]** | 2+1 IgG Crossfab (V9) | 2+1 IgG Crossfab inverted (V9) | 2+1 IgG Crossfab (anti-CD3) | 2+1 IgG Crossfab, inverted (anti-CD3) |
|---|---|---|---|---|
| MV-3 | 10.0 | 4.1 | 11.0 | 3.0 |
| WM266-4 | 12.4 | 3.7 | 11.3 | 7.1 |

### Example 7

### CD107a/b assay

Purified "2+1 IgG scFab" construct (SEQ ID NOs 5, 17, 19) and the "(scFv)₂" molecule, both targeting human MCSP and human CD3, were tested by flow cytometry for their potential to up-regulate CD107a and intracellular perforin levels in the presence or absence of human MCSP-expressing tumor cells.

Briefly, on day one, 30 000 Colo-38 tumor target cells per well were plated in a round-bottom 96-well plate and incubated overnight at 37°C, 5% CO₂ to let them adhere. Primary human pan T cells were isolated on day 1 or day 2 from Buffy Coat, as described.

On day two, 0.15 million effector cells per well were added to obtain a final E:T ratio of 5:1. FITC-conjugated CD107a/b antibodies, as well as the different bispecific constructs and controls are added. The different bispecific molecules and antibodies were adjusted to same molarities to obtain a final concentration of 9.43 nM. Following a 1 h incubation step at 37°C, 5% CO₂, monensin was added to inhibit secretion, but also to neutralize the pH within endosomes and lysosomes. After an additional incubation time of 5 h, cells were stained at 4°C for 30 min for surface CD8 expression. Cells were washed with staining buffer (PBS / 0.1% BSA), fixed and permeabilized for 20 min using the BD Cytofix/Cytoperm Plus Kit with BD Golgi Stop (BD Biosciences #554715). Cells were washed twice using 1 x BD Perm/Wash buffer, and intracellular staining for perforin was performed at 4°C for 30 min. After a final washing step with 1 x BD Perm/Wash buffer, cells were resuspended in PBS / 0.1% BSA and analyzed on FACS CantoII (all antibodies were purchased from BD Biosciences or BioLegend).

Gates were set either on all CD107a/b positive, perforin-positive or double-positive cells, as indicated (Figure 43). The "2+1 IgG scFab" construct was able to activate T cells and up-regulate CD107a/b and intracellular perforin levels only in the presence of target cells (Figure 43A), whereas the "(scFv)₂" molecule shows (weak) induction of activation of T cells also in the absence of target cells (Figure 43B). The bivalent reference anti-CD3 IgG results in a lower level of activation compared to the "(scFv)₂" molecule or the other bispecific construct.

### Example 8

### Proliferation assay

The purified "2+1 IgG scFab" (SEQ ID NOs 5, 17, 19) and "(scFv)₂" molecules, both targeting human CD3 and human MCSP, were tested by flow cytometry for their potential to induce proliferation of CD8⁺ or CD4⁺ T cells in the presence and absence of human MCSP-expressing tumor cells.

Briefly, freshly isolated human pan T cells were adjusted to 1 million cells per ml in warm PBS and stained with 1 µM CFSE at room temperature for 10 minutes. The staining volume was doubled by addition of RPMI1640 medium, containing 10% FCS and 1% GlutaMax. After incubation at room temperature for further 20 min, the cells were washed three times with prewarmed medium to remove remaining CFSE. MCSP-positive Colo-38 cells were harvested with Cell Dissociation buffer, counted and checked for viability. Cells were adjusted to 0.2 x 10⁶ (viable) cells per ml in AIM-V medium, 100 µl of this cell suspension were pipetted per well into a round-bottom 96-well plate (as indicated). 50 µl of the (diluted) bispecific constructs were added to the cell-containing wells to obtain a final concentration of 1 nM. CFSE-stained human pan T effector cells were adjusted to 2 x 10⁶ (viable) cells per ml in AIM-V medium. 50 µl of this cell suspension was added per well of the assay plate (see above) to obtain a final E:T ratio of 5:1. To analyze whether the bispecific constructs are able to activate T cells only in the presence of target cells, expressing the tumor antigen huMCSP, wells were included that contained 1 nM of the respective bispecific molecules as well as PBMCs, but no target cells. After incubation for five days at 37°C, 5% CO₂, cells were centrifuged (5 min, 350 x g) and washed twice with 150 µl/well PBS, including 0.1% BSA. Surface staining for CD8 (mouse IgG1,κ; clone HIT8a; BD #555635), CD4 (mouse IgG1,κ; clone RPA-T4 ; BD #560649), or CD25 (mouse IgG1,κ; clone M-A251; BD #555434) was performed at 4°C for 30 min, according to the supplier's suggestions. Cells were washed twice with 150 µl/well PBS containing 0.1% BSA, resuspended in 200 µl/well PBS with 0.1% BSA, and analyzed using a FACS CantoII machine (Software FACS Diva). The relative proliferation level was determined by setting a gate around the non-proliferating cells and using the cell number of this gate relative to the overall measured cell number as the reference.

Figure 44 shows that all constructs induce proliferation of CD8⁺ T cells (A) or CD4⁺ T cells (B) only in the presence of target cells, comparably to the "(scFv)₂" molecule. In general, activated CD8⁺ T cells proliferate more than activated CD4⁺ T cells in this assay.

### Example 9

### Cytokine release assay

The purified "2+1 IgG scFab" construct (SEQ ID NOs 5, 17, 19) and the "(scFv)₂"molecule, both targeting human MCSP and human CD3, were analyzed for their ability to induce T cell-mediated *de novo* secretion of cytokines in the presence or absence of tumor target cells.

Briefly, human PBMCs were isolated from Buffy Coats and 0.3 million cells were plated per well into a round-bottom 96-well plate. Colo-38 tumor target cells, expressing human MCSP, were added to obtain a final E:T-ratio of 10:1. Bispecific constructs and IgG controls were added at 1 nM final concentration and the cells were incubated for 24 h at 37°C, 5% CO₂. The next day, the cells were centrifuged for 5 min at 350 x g and the supernatant was transferred into a new deep-well 96-well-plate for the subsequent analysis. The CBA analysis was performed according to manufacturer's instructions for FACS CantoII, using the Human Th1/Th2 Cytokine Kit II (BD #551809).

Figure 45 shows levels of the different cytokine measured in the supernatant. In the presence of target cells the main cytokine secreted upon T cell activation is IFN-γ. The "(scFv)₂" molecule induces a slightly higher level of IFN-γ than the "2+1 IgG scFab" construct. The same tendency might be found for human TNF, but the overall levels of this cytokine were much lower compared to IFN-γ. There was no significant secretion of Th2 cytokines (IL-10 and IL-4) upon activation of T cells in the presence (or absence) of target cells. In the absence of Colo-38 target cells, only very weak induction of TNF secretion was observed, which was highest in samples treated with the "(scFv)₂" molecule.

In a second experiment, the following purified bispecific constructs targeting human MCSP and human CD3 were analyzed: the "2+1 IgG Crossfab" construct (SEQ ID NOs 3, 5, 29, 33), the "(scFv)₂" molecule, as well as different "2+1 IgG scFab" molecules comprising either a wild-type or a mutated (LALA, P329G and/or N297D, as indicated) Fc domain. Briefly, 280 µl whole blood from a healthy donor were plated per well of a deep-well 96-well plate. 30 000 Colo-38 tumor target cells, expressing human MCSP, as well as the different bispecific constructs and IgG controls were added at 1 nM final concentration. The cells were incubated for 24 h at 37°C, 5% CO₂ and then centrifuged for 5 min at 350 x g. The supernatant was transferred into a new deep-well 96-well-plate for the subsequent analysis. The CBA analysis was performed according to manufacturer's instructions for FACS CantoII, using the combination of the following CBA Flex Sets: human granzyme B (BD #560304), human IFN-γ Flex Set (BD #558269), human TNF Flex Set (BD #558273), human IL-10 Flex Set (BD #558274), human IL-6 Flex Set (BD #558276), human IL-4 Flex Set (BD #558272), human IL-2 Flex Set (BD #558270).

Figure 46 shows the levels of the different cytokine measured in the supernatant. The main cytokine secreted in the presence of Colo-38 tumor cells was IL-6, followed by IFN-γ. In addition, also the levels of granzyme B strongly increased upon activation of T cells in the presence of target cells. In general, the "(scFv)₂" molecule induced higher levels of cytokine secretion in the presence of target cells (Figure 46, A and B). There was no significant secretion of Th2 cytokines (IL-10 and IL-4) upon activation of T cells in the presence (or absence) of target cells.

In this assay, there was a weak secretion of IFN-γ, induced by different "2+1 IgG scFab" constructs, even in the absence of target cells (Figure 46, C and D). Under these conditions, no significant differences could be observed between "2+1 IgG scFab" constructs with a wild-type or a mutated Fc domain.

## Claims

1. A T cell activating bispecific antigen binding molecule comprising a first and a second antigen binding moiety, one of which is a Fab molecule capable of specific binding to an activating T cell antigen and the other one of which is a Fab molecule capable of specific binding to a target cell antigen, and an Fc domain composed of a first and a second subunit capable of stable association;
wherein the first antigen binding moiety is a crossover Fab molecule wherein either the variable or the constant regions of the Fab light chain and the Fab heavy chain are exchanged; wherein the first and the second antigen binding moiety are fused to each other, optionally via a peptide linker; and
wherein the T cell activating bispecific antigen binding molecule comprises not more than one antigen binding moiety capable of specific binding to an activating T cell antigen.

2. The T cell activating bispecific antigen binding molecule of claim 1, wherein the second antigen binding moiety is fused at the C-terminus of the Fab heavy chain to the N-terminus of the Fab heavy chain of the first antigen binding moiety, wherein optionally
i) the Fab light chain of the first antigen binding moiety and the Fab light chain of the second antigen binding moiety are fused to each other, optionally via a peptide linker; and/or
ii) the first antigen binding moiety is fused at the C-terminus of the Fab heavy chain to the N-terminus of the first or second subunit of the Fc domain.

3. The T cell activating bispecific antigen binding molecule of claim 1, wherein the first antigen binding moiety is fused at the C-terminus of the Fab heavy chain to the N-terminus of the Fab heavy chain of the second antigen binding moiety, wherein optionally
i) the Fab light chain of the first antigen binding moiety and the Fab light chain of the second antigen binding moiety are fused to each other, optionally via a peptide linker; and/or
ii) wherein the second antigen binding moiety is fused at the C-terminus of the Fab heavy chain to the N-terminus of the first or the second subunit of the Fc domain.

4. The T cell activating bispecific antigen binding molecule of claim 1, wherein the second antigen binding moiety is fused at the C-terminus of the Fab light chain to the N-terminus of the Fab light chain of the first antigen binding moiety, and wherein optionally the second antigen binding moiety is fused at the C-terminus of the Fab heavy chain to the N-terminus of the first or the second subunit of the Fc domain.

5. The T cell activating bispecific antigen binding molecule of any one of claims 1 to 4, comprising a third antigen binding moiety which is a Fab molecule capable of specific binding to a target cell antigen, wherein optionally the third antigen binding moiety is fused at the C-terminus of the Fab heavy chain to the N-terminus of the first or second subunit of the Fc domain, and wherein optionally
i) the second and the third antigen binding moiety are each fused at the C-terminus of the Fab heavy chain to the N-terminus of one of the subunits of the Fc domain, and the first antigen binding moiety is fused at the C-terminus of the Fab heavy chain to the N-terminus of the Fab heavy chain of the second antigen binding moiety; or
ii) the first and the third antigen binding moiety are each fused at the C-terminus of the Fab heavy chain to the N-terminus of one of the subunits of the Fc domain, and the second antigen binding moiety is fused at the C-terminus of the Fab heavy chain to the N-terminus of the Fab heavy chain of the first antigen binding moiety.

6. The T cell activating bispecific antigen binding molecule of any one of the preceding claims, wherein
i) the T cell activating bispecific antigen binding molecule is as defined in claim 5(i) and wherein the second and the third antigen binding moiety and the Fc domain are part of an immunoglobulin molecule, particularly an IgG class immunoglobulin;
ii) the Fc domain is an IgG, specifically an IgG₁ or IgG₄, Fc domain;
iii) the Fc domain is a human Fc domain; and/or
iv) the Fc domain comprises a modification promoting the association of the first and the second subunit of the Fc domain, wherein optionally in the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

7. The T cell activating bispecific antigen binding molecule of any one of the preceding claims, wherein
i) the Fc domain exhibits reduced binding affinity to an Fc receptor and/or reduced effector function, as compared to a native IgG1 Fc domain;
ii) the Fc domain comprises one or more amino acid substitution that reduces binding to an Fc receptor and/or effector function;
iii) the Fc domain comprises one or more amino acid substitution that reduces binding to an Fc receptor and/or effector function, wherein said one or more amino acid substitution is at one or more position selected from the group of L234, L235, and P329 (EU numbering); and/or
(iv) each subunit of the Fc domain comprises three amino acid substitutions that reduce binding to an activating Fc receptor and/or effector function wherein said amino acid substitutions are L234A, L235A and P329G (EU numbering).

8. The T cell activating bispecific antigen binding molecule of claim 7, wherein
i) the Fc receptor is an Fcγ receptor; or
ii) the effector function is antibody-dependent cell-mediated cytotoxicity (ADCC).

9. The T cell activating bispecific antigen binding molecule of any one of the preceding claims, wherein
i) the activating T cell antigen is CD3; and/or
ii) the target cell antigen is selected from the group consisting of: Melanoma-associated Chondroitin Sulfate Proteoglycan (MCSP), Epidermal Growth Factor Receptor (EGFR), CD19, CD20, CD33, Carcinoembryonic Antigen (CEA) and Fibroblast Activation Protein (FAP).

10. An isolated polynucleotide encoding the T cell activating bispecific antigen binding molecule of any one of claims 1 to 9.

11. A method of producing the T cell activating bispecific antigen binding molecule of any one of claims 1 to 9, comprising the steps of a) culturing a host cell comprising the isolated polynucleotide of claim 10, or a vector comprising the isolated polynucleotide of claim 10, under conditions suitable for the expression of the T cell activating bispecific antigen binding molecule and b) recovering the T cell activating bispecific antigen binding molecule.

12. A pharmaceutical composition comprising the T cell activating bispecific antigen binding molecule of any one of claims 1 to 9 and a pharmaceutically acceptable carrier.

13. The T cell activating bispecific antigen binding molecule of any one of claims 1 to 9 or the pharmaceutical composition of claim 12
i) for use as a medicament;
ii) for use in the treatment of a disease in an individual in need thereof;
iii) for use in the treatment of a disease in an individual in need thereof wherein the disease is cancer; or
iv) for use in a method of inducing lysis of a target cell in an individual , comprising contacting a target cell with the T cell activating bispecific antigen binding molecule in the presence of a T cell, wherein the target cell is a tumor cell.

## Patentansprüche

1. T-Zellen-aktivierendes bispezifisches antigenbindendes Molekül, umfassend eine erste und eine zweite antigenbindende Gruppierung, von denen eine ein Fab-Molekül ist, das dazu in der Lage ist, spezifisch an ein aktivierendes T-Zellen-Antigen zu binden, und die andere ein Fab-Molekül ist, das dazu in der Lage ist, spezifisch an ein Target-Zellen-Antigen zu binden, und eine Fc-Domäne, die aus einer ersten und einer zweiten Untereinheit besteht, die zur stabilen Assoziation in der Lage ist;
wobei die erste antigenbindende Gruppierung ein Fab-Kreuzmolekül ist, wobei entweder die variablen oder die konstanten Regionen der Fab-Leichtkette und der Fab-Schwerkette ausgetauscht sind; wobei die erste und die zweite antigenbindende Gruppierung aneinander fusioniert sind, gegebenenfalls über einen Peptidlinker; und
wobei das T-Zellen-aktivierende bispezifische antigenbindende Molekül nicht mehr als eine antigenbindende Gruppierung umfasst, die zur spezifischen Bindung an ein aktivierendes T-Zellen-Antigen in der Lage ist.

2. T-Zellen-aktivierendes bispezifisches antigenbindendes Molekül nach Anspruch 1, wobei die zweite antigenbindende Gruppierung am C-Terminus der Fab-Schwerkette an den N-Terminus der Fab-Schwerkette der ersten antigenbindenden Gruppierung fusioniert ist, wobei gegebenenfalls:
i) die Fab-Leichtkette der ersten antigenbindenden Gruppierung und die Fab-Leichtkette der zweiten antigenbindenden Gruppierung aneinander fusioniert sind, gegebenenfalls über einen Peptidlinker; und/oder
ii) die erste antigenbindende Gruppierung am C-Terminus der Fab-Schwerkette an den N-Terminus der ersten oder der zweiten Untereinheit der Fc-Domäne fusioniert ist.

3. T-Zellen-aktivierendes bispezifisches antigenbindendes Molekül nach Anspruch 1, wobei die erste antigenbindende Gruppierung am C-Terminus der Fab-Schwerkette an den N-Terminus der Fab-Schwerkette der zweiten antigenbindenden Gruppierung fusioniert ist, wobei gegebenenfalls
i) die Fab-Leichtkette der ersten antigenbindenden Gruppierung und die Fab-Leichtkette der zweiten antigenbindenden Gruppierung aneinander fusioniert sind, gegebenenfalls über einen Peptidlinker; und/oder
ii) wobei die zweite antigenbindende Gruppierung am C-Terminus der Fab-Schwerkette an den N-Terminus der ersten oder der zweiten Untereinheit der Fc-Domäne fusioniert ist.

4. T-Zellen-aktivierendes bispezifisches antigenbindendes Molekül nach Anspruch 1, wobei die zweite antigenbindende Gruppierung am C-Terminus der Fab-Leichtkette an den N-Terminus der Fab-Leichtkette der ersten antigenbindenden Gruppierung fusioniert ist und wobei die zweite antigenbindende Gruppierung gegebenenfalls am C-Terminus der Fab-Schwerkette an den N-Terminus der ersten oder der zweiten Untereinheit der Fc-Domäne fusioniert ist.

5. T-Zellen-aktivierendes bispezifisches antigenbindendes Molekül nach einem der Ansprüche 1 bis 4, umfassend eine dritte antigenbindende Gruppierung, die ein Fab-Molekül ist, das zur spezifischen Bindung an ein Target-Zellen-Antigen in der Lage ist, wobei die dritte antigenbindende Gruppierung gegebenenfalls am C-Terminus der Fab-Schwerkette an den N-Terminus der ersten oder zweiten Untereinheit der Fc-Domäne gebunden ist und wobei gegebenenfalls:
i) die zweite und die dritte antigenbindende Gruppierung jeweils am C-Terminus der Fab-Schwerkette an den N-Terminus einer der Untereinheiten der Fc-Domäne fusioniert sind, und die erste antigenbindende Gruppierung am C-Terminus der Fab-Schwerkette an den N-Terminus der Fab-Schwerkette der zweiten antigenbindenden Gruppierung fusioniert ist; oder
ii) die erste und die dritte antigenbindende Gruppierung jeweils am C-Terminus der Fab-Schwerkette an den N-Terminus einer der Untereinheiten der Fc-Domäne fusioniert sind und die zweite antigenbindende Gruppierung am C-Terminus der Fab-Schwerkette an den N-Terminus der Fab-Schwerkette der ersten antigenbindenden Gruppierung fusioniert ist.

6. T-Zellen-aktivierendes bispezifisches antigenbindendes Molekül nach einem der vorangegangenen Ansprüche, wobei
i) das T-Zellen-aktivierende bispezifische antigenbindende Molekül wie in Anspruch 5(i) definiert ist und wobei die zweite und die dritte antigenbindende Gruppierung und die Fc-Domäne Teil eines Immunglobulinmoleküls sind, insbesondere eines Immunglobulins der IgG-Klasse;
ii) die Fc-Domäne eine IgG-, im Speziellen eine IgG₁- oder IgG₄-Fc-Domäne ist;
vi) die Fc-Domäne eine menschliche Fc-Domäne ist; und/oder
iv) die Fc-Domäne eine Modifikation umfasst, die die Assoziation der ersten und zweiten Fc-Domänen-Untereinheit fördert, wobei in der CH3-Domäne der ersten Untereinheit der Fc-Domäne gegebenenfalls ein Aminosäurerest durch einen Aminosäurerest ersetzt ist, der ein größeres Seitenkettenvolumen aufweist, wodurch ein Vorsprung innerhalb der CH3-Domäne der ersten Untereinheit erzeugt wird, der in einem Hohlraum innerhalb der CH3-Domäne der zweiten Untereinheit positionierbar ist und in der CH3-Domäne der zweiten Untereinheit der Fc-Domäne ein Aminosäurerest durch einen Aminosäurerest ersetzt ist, der ein kleineres Seitenkettenvolumen aufweist, wodurch ein Hohlraum innerhalb der CH3-Domäne der zweiten Untereinheit erzeugt wird, innerhalb dessen der Vorsprung innerhalb der CH3-Domäne der ersten Untereinheit positionierbar ist.

7. T-Zellen-aktivierendes bispezifisches antigenbindendes Molekül nach einem der vorangegangenen Ansprüche, wobei
i) die Fc-Domäne verglichen mit einer nativen IgG1-Fc-Domäne eine reduzierte Bindungsaffinität für den Fc-Rezeptor und/oder eine reduzierte Effektor-Funktion aufweist;
ii) die Fc-Domäne eine oder mehrere Aminosäuresubstitutionen umfasst, die die Bindung an einen Fc-Rezeptor und/oder die Effektor-Funktion reduziert;
iii) die Fc-Domäne eine oder mehrere Aminosäuresubstitutionen umfasst, die die Bindung an einen Fc-Rezeptor und/oder die Effektor-Funktion reduziert, wobei die eine oder mehrere Aminosäuresubstitutionen an einer oder mehreren Positionen vorliegt, die aus der Gruppe aus L234, L235 und P329 (EU-Nummerierung) ausgewählt sind; und/oder
(iv) jede Untereinheit der Fc-Domäne drei Aminosäuresubstitutionen umfasst, die die Bindung an einen aktivierenden Fc-Rezeptor und/oder die Effektor-Funktionen reduzieren, wobei die Aminosäuresubstitutionen L234A, L235A und P329G (EU-Nummerierung) sind.

8. T-Zellen-aktivierendes bispezifisches antigenbindendes Molekül nach Anspruch 7, wobei
i) der Fc-Rezeptor ein Fcy-Rezeptor ist; oder
ii) die Effektor-Funktion antikörperabhängige zellvermittelte Zytotoxizität (ADCC) ist.

9. T-Zellen-aktivierendes bispezifisches antigenbindendes Molekül nach einem der vorangegangenen Ansprüche, wobei
i) das aktivierende T-Zellen-Antigen CD3 ist; und/oder
ii) das Target-Zellen-Antigen aus der Gruppe bestehend aus den Folgenden ausgewählt ist: Melanomassoziiertem Chondroitinsulfat-Proteoglycan (MCSP), Epidermiswachstumsfaktorrezeptor (EGFR), CD19, CD20, CD33, karzinoembryonischem Antigen (CEA) und Fibroblasten-Aktivierungsprotein (FAP).

10. Isoliertes Polynucleotid, das für ein T-Zellen-aktivierendes bispezifisches antigenbindendes Molekül nach einem der Ansprüche 1 bis 9 kodiert.

11. Verfahren zur Herstellung eines T-Zellen-aktivierenden bispezifischen antigenbindenden Moleküls nach einem der Ansprüche 1 bis 9, umfassend die folgenden Schritte: a) Kultivieren einer Wirtszelle, umfassend ein isoliertes Polynucleotid nach Anspruch 10 oder eines Vektors, umfassend ein isoliertes Polynucleotid nach Anspruch 10 unter Bedingungen, die für die Expression des T-Zellen-aktivierenden bispezifischen antigenbindenden Moleküls geeignet sind, und b) Gewinnen des T-Zellen-aktivierenden bispezifischen antigenbindenden Moleküls.

12. Pharmazeutische Zusammensetzung, umfassend ein T-Zellen-aktivierendes bispezifisches antigenbindendes Molekül nach einem der Ansprüche 1 bis 9 und einen pharmazeutisch annehmbaren Träger.

13. T-Zellen-aktivierendes bispezifisches antigenbindendes Molekül nach einem der Ansprüche 1 bis 9 oder pharmazeutische Zusammensetzung nach Anspruch 12
i) zur Verwendung als Medikament;
ii) zur Verwendung bei der Behandlung einer Krankheit in einem Individuum, das diese benötigt;
iii) zur Verwendung bei der Behandlung einer Krankheit in einem Individuum, das diese benötigt, wobei die Krankheit Krebs ist; oder
iv) zur Verwendung in einem Verfahren zum Auslösen der Lyse einer Target-Zelle in einem Individuum, umfassend das Kontaktieren einer Target-Zelle mit dem T-Zellen-aktivierenden bispezifischen antigenbindenden Moleküls in der Gegenwart einer T-Zelle, wobei die Target-Zelle eine Tumorzelle ist.

## Revendications

1. Molécule bispécifique de liaison à l'antigène activant les lymphocytes T comprenant une première et une deuxième fraction de liaison à l'antigène, dont une est une molécule Fab capable de se lier spécifiquement à un antigène activant les lymphocytes T et l'autre est une molécule Fab capable de se lier spécifiquement à un antigène de cellule cible, et un domaine Fc se composant d'un premier et d'un second sous-motif capable de réaliser une association stable ;
dans laquelle la première fraction de liaison à l'antigène est une molécule Fab croisée dans laquelle les régions variables ou les régions constantes de la chaîne légère Fab et de la chaîne lourde Fab sont échangées ; dans laquelle la première et la deuxième fraction de liaison à l'antigène sont fusionnées l'une à l'autre, éventuellement par l'intermédiaire d'un lieur peptidique ; et
la molécule bispécifique de liaison à l'antigène activant les lymphocytes T ne comprenant qu'une seule fraction de liaison à l'antigène capable de se lier spécifiquement à un antigène activant les lymphocytes T.

2. Molécule bispécifique de liaison à l'antigène activant les lymphocytes T selon la revendication 1, dans laquelle la deuxième fraction de liaison à l'antigène est fusionnée, au niveau de l'extrémité C-terminale de la chaîne lourde Fab, à l'extrémité N-terminale de la chaîne lourde Fab de la première fraction de liaison à l'antigène, dans laquelle, éventuellement,
i) la chaîne légère Fab de la première fraction de liaison à l'antigène et la chaîne légère Fab de la deuxième fraction de liaison à l'antigène sont fusionnées l'une à l'autre, éventuellement par l'intermédiaire d'un lieur peptidique ; et/ou
ii) la première fraction de liaison à l'antigène est fusionnée, au niveau de l'extrémité C-terminale de la chaîne lourde Fab, à l'extrémité N-terminale du premier ou du second sous-motif du domaine Fc.

3. Molécule bispécifique de liaison à l'antigène activant les lymphocytes T selon la revendication 1, dans laquelle la première fraction de liaison à l'antigène est fusionnée, au niveau de l'extrémité C-terminale de la chaîne lourde Fab, à l'extrémité N-terminale de la chaîne lourde Fab de la deuxième fraction de liaison à l'antigène, dans laquelle, éventuellement,
i) la chaîne légère Fab de la première fraction de liaison à l'antigène et la chaîne légère Fab de la deuxième fraction de liaison à l'antigène sont fusionnées l'une à l'autre, éventuellement par l'intermédiaire d'un lieur peptidique ; et/ou
ii) la deuxième fraction de liaison à l'antigène est fusionnée, au niveau de l'extrémité C-terminale de la chaîne lourde Fab, à l'extrémité N-terminale du premier ou du second sous-motif du domaine Fc.

4. Molécule bispécifique de liaison à l'antigène activant les lymphocytes T selon la revendication 1, dans laquelle la deuxième fraction de liaison à l'antigène est fusionnée, au niveau de l'extrémité C-terminale de la chaîne légère Fab, à l'extrémité N-terminale de la chaîne légère Fab de la première fraction de liaison à l'antigène, et dans laquelle, éventuellement, la deuxième fraction de liaison à l'antigène est fusionnée, au niveau de l'extrémité C-terminale de la chaîne lourde Fab, à l'extrémité N-terminale du premier ou du second sous-motif du domaine Fc.

5. Molécule bispécifique de liaison à l'antigène activant les lymphocytes T selon l'une quelconque des revendications 1 à 4, comprenant une troisième fraction de liaison à l'antigène qui est une molécule Fab capable de se lier spécifiquement à un antigène de cellule cible, dans laquelle, éventuellement, la troisième fraction de liaison à l'antigène est fusionnée, au niveau de l'extrémité C-terminale de la chaîne lourde Fab, à l'extrémité N-terminale du premier ou du second sous-motif du domaine Fc, et dans laquelle, éventuellement,
i) chacune des deuxième et troisième fractions de liaison à l'antigène est fusionnée, au niveau de l'extrémité C-terminale de la chaîne lourde Fab, à l'extrémité N-terminale d'un des sous-motifs du domaine Fc, et la première fraction de liaison à l'antigène est fusionnée, au niveau de l'extrémité C-terminale de la chaîne lourde Fab, à l'extrémité N-terminale de la chaîne lourde Fab de la deuxième fraction de liaison à l'antigène ; ou
ii) chacune des première et troisième fractions de liaison à l'antigène est fusionnée, au niveau de l'extrémité C-terminale de la chaîne lourde Fab, à l'extrémité N-terminale d'un des sous-motifs du domaine Fc, et la deuxième fraction de liaison à l'antigène est fusionnée, au niveau de l'extrémité C-terminale de la chaîne lourde Fab, à l'extrémité N-terminale de la chaîne lourde Fab de la première fraction de liaison à l'antigène.

6. Molécule bispécifique de liaison à l'antigène activant les lymphocytes T selon l'une quelconque des revendications précédentes, dans laquelle
i) la molécule bispécifique de liaison à l'antigène activant les lymphocytes T est telle que définie dans la revendication 5(i) et dans laquelle la deuxième et la troisième fraction de liaison à l'antigène et le domaine Fc font partie d'une molécule d'immunoglobuline, en particulier une immunoglobuline de la classe des IgG ;
ii) le domaine Fc est un domaine Fc d'IgG, plus particulièrement d'une IgG₁ ou d'une IgG₄ ;
iii) le domaine Fc est un domaine Fc humain ; et/ou
iv) le domaine Fc comprend une modification favorisant l'association du premier et du second sous-motif du domaine Fc, un résidu d'acide aminé étant éventuellement remplacé, dans le domaine CH3 du premier sous-motif du domaine Fc, par un résidu d'acide aminé présentant un volume de chaîne latérale plus grand, générant ainsi une protubérance au sein du domaine CH3 du premier sous-motif qui peut être positionnée dans une cavité au sein du domaine CH3 du second sous-motif, et un résidu d'acide aminé étant éventuellement remplacé, dans le domaine CH3 du second sous-motif du domaine Fc, par un résidu d'acide aminé présentant un volume de chaîne latérale plus petit, générant ainsi une cavité au sein du domaine CH3 du second sous-motif à l'intérieur de laquelle peut être positionnée la protubérance au sein du domaine CH3 du premier sous-motif.

7. Molécule bispécifique de liaison à l'antigène activant les lymphocytes T selon l'une quelconque des revendications précédentes, dans laquelle
i) le domaine Fc présente une affinité de liaison réduite vis-à-vis d'un récepteur Fc et/ou une fonction effectrice réduite, par comparaison avec un domaine Fc d'IgG1 native ;
ii) le domaine Fc comprend une ou plusieurs substitutions d'acides aminés qui réduisent la liaison à un récepteur Fc et/ou la fonction effectrice ;
iii) le domaine Fc comprend une ou plusieurs substitutions d'acides aminés qui réduisent la liaison à un récepteur Fc et/ou la fonction effectrice, ladite ou lesdites substitutions d'acides aminés étant présentes sur une ou plusieurs positions choisies dans le groupe constitué par L234, L235, et P329 (numérotation EU) ; et/ou
iv) chaque sous-motif du domaine Fc comprend trois substitutions d'acides aminés qui réduisent la liaison à un récepteur Fc d'activation et/ou la fonction effectrice, lesdites substitutions d'acides aminés étant présentes sur les positions L234A, L235A, et P329G (numérotation EU).

8. Molécule bispécifique de liaison à l'antigène activant les lymphocytes T selon la revendication 7, dans laquelle
i) le récepteur Fc est un récepteur Fcγ; ou
ii) la fonction effectrice est la cytotoxicité cellulaire dépendant des anticorps (ADCC).

9. Molécule bispécifique de liaison à l'antigène activant les lymphocytes T selon l'une quelconque des revendications précédentes, dans laquelle
i) l'antigène activant les lymphocytes T est le CD3 ; et/ou
ii) l'antigène de cellule cible est choisi dans le groupe constitué par : protéoglycane à chondroïtine sulfate associé à un mélanome (MCSP), récepteur de facteur de croissance de l'épiderme (EGFR), CD19, CD20, CD33, antigène carcinoembryonnaire (CEA) et protéine d'activation des fibroblastes (FAP).

10. Polynucléotide isolé codant pour la molécule bispécifique de liaison à l'antigène activant les lymphocytes T selon l'une quelconque des revendications 1 à 9.

11. Procédé de production de la molécule bispécifique de liaison à l'antigène activant les lymphocytes T selon l'une quelconque des revendications 1 à 9 comprenant les étapes de a) mise en culture d'une cellule hôte comprenant le polynucléotide isolé selon la revendication 10, ou un vecteur comprenant le polynucléotide isolé selon la revendication 10, dans des conditions appropriées pour l'expression de la molécule bispécifique de liaison à l'antigène activant les lymphocytes T et b) récupération de la molécule bispécifique de liaison à l'antigène activant les lymphocytes T.

12. Composition pharmaceutique comprenant la molécule bispécifique de liaison à l'antigène activant les lymphocytes T selon l'une quelconque de revendications 1 à 9 et un véhicule pharmaceutiquement acceptable.

13. Molécule bispécifique de liaison à l'antigène activant les lymphocytes T selon l'une quelconque des revendications 1 à 9 ou composition pharmaceutique selon la revendication 12
i) destinée à être utilisée comme médicament ;
ii) destinée à être utilisée dans le traitement d'une maladie chez un individu qui en a besoin ;
iii) destinée à être utilisée dans le traitement d'une maladie chez un individu qui en a besoin, ladite maladie étant un cancer ; ou
iv) destinée à être utilisée dans une méthode d'induction d'une lyse d'une cellule cible chez un individu, comprenant la mise en contact d'une cellule cible avec la molécule bispécifique de liaison à l'antigène activant les lymphocytes T en présence d'un lymphocyte T, la cellule cible étant une cellule tumorale.
